(19) Europäisches Patentamt
European Patent Office
Office européen des brevets

(11) EP 4 419 207 B1

(12) EUROPEAN PATENT SPECIFICATION

(45) Date of publication and mention of the grant of the patent:
**23.07.2025 Bulletin 2025/30**

(21) Application number: **22797458.1**

(22) Date of filing: **21.10.2022**

(51) International Patent Classification (IPC):
***C07D 401/12*** *(2006.01)* ***A61K 31/395*** *(2006.01)*
***A61P 35/00*** *(2006.01)*

(52) Cooperative Patent Classification (CPC):
**C07D 401/12; A61P 35/00**

(86) International application number:
**PCT/GB2022/052688**

(87) International publication number:
**WO 2023/067354 (27.04.2023 Gazette 2023/17)**

# (54) HETEROCYCLIC COMPOUNDS FOR USE IN THE TREATMENT OF CANCER

HETEROCYCLISCHE VERBINDUNGEN ZUR VERWENDUNG BEI DER BEHANDLUNG VON KREBS

COMPOSÉS HÉTÉROCYCLIQUES DESTINÉS À ÊTRE UTILISÉS DANS LE TRAITEMENT DU CANCER

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **21.10.2021 GB 202115150**

(43) Date of publication of application:
**28.08.2024 Bulletin 2024/35**

(73) Proprietor: **Artios Pharma Limited**
**Cambridge CB22 3AT (GB)**

(72) Inventors:
- **DAVIS, Owen**
  **Cambridge CB22 3AT (GB)**
- **HEALD, Robert**
  **Cambridge CB22 3AT (GB)**
- **STOCKLEY, Martin**
  **Cambridge CB22 3AT (GB)**
- **FINCH, Harry**
  **Cambridge CB22 3AT (GB)**
- **MANN, Sam**
  **Cambridge CB22 3AT (GB)**

(74) Representative: **Sagittarius IP**
**Marlow International**
**Parkway**
**Marlow SL7 1YL (GB)**

(56) References cited:
**WO-A1-2021/028643**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 419 207 B1

## Description

### FIELD OF THE INVENTION

The invention relates to heterocyclic derivatives and their use in the treatment and prophylaxis of cancer, and to compositions containing said derivatives and processes for their preparation.

### BACKGROUND OF THE INVENTION

Robust repair of DNA double-strand breaks (DSBs) is essential for the maintenance of genome stability and cell viability. DSBs can be repaired by one of three main pathways: homologous recombination (HR), non-homologous end-joining (NHEJ) and alternative NHEJ (alt-NHEJ). Microhomology-mediated end-joining (MMEJ) is the most well characterised alt-NHEJ mechanism. HR-mediated repair is a high-fidelity mechanism essential for accurate error-free repair, preventing cancer-predisposing genomic stability. Conversely, NHEJ and MMEJ are error-prone pathways that can leave mutational scars at the site of repair. MMEJ can function parallel to both HR and NHEJ pathways (Truong et al. PNAS 2013, 110 (19), 7720-7725).

The survival of cancer cells, unlike normal cells, is often dependent on the mis-regulation of DNA damage response (DDR) pathways. For example, an increased dependency on one pathway (often mutagenic) to cope with either the inactivation of another one, or the enhanced replication stress resulting from increased proliferation. An aberrant DDR can also sensitise cancer cells to specific types of DNA damage, thus, defective DDR can be exploited to develop targeted cancer therapies. Crucially, cancer cells with impairment or inactivation of HR and NHEJ become hyper-dependent on MMEJ-mediated DNA repair. Genetic, cell biological and biochemical data have identified Polθ (UniProtKB - 075417 (DPOLQ_HUMAN) as the key protein in MMEJ (Kent et al. Nature Structural & Molecular Biology (2015), 22(3), 230-237, Mateos-Gomez et al. Nature (2015), 518(7538), 254-257). Polθ is multifunctional enzyme, which comprises an N-terminal helicase domain (SF2 HEL308-type) and a C-terminal low-fidelity DNA polymerase domain (A-type) (Wood & Doublié DNA Repair (2016), 44, 22-32). Both domains have been shown to have concerted mechanistic functions in MMEJ. The helicase domain mediates the removal of RPA protein from ssDNA ends and stimulates annealing. The polymerase domain extends the ssDNA ends and fills the remaining gaps.

Therapeutic inactivation of Polθ would thus disable the ability of cells to perform MMEJ and provide a novel targeted strategy in an array of defined tumour contexts. Firstly, Polθ has been shown to be essential for the survival of HR-defective (HRD) cells (e.g. synthetic lethal with FA/BRCA-deficiency) and is up-regulated in HRD tumour cell lines (Ceccaldi et al. Nature (2015), 518(7538), 258-262). *In vivo* studies also show that Polθ is significantly over-expressed in subsets of HRD ovarian, uterine and breast cancers with associated poor prognosis (Higgins et al. Oncotarget (2010), 1, 175-184, Lemée et al. PNAS (2010), 107(30), 13390-13395, Ceccaldi *et al.* (2015), *supra*). Importantly, Polθ is largely repressed in normal tissues but has been shown to be upregulated in matched cancer samples thus correlating elevated expression with disease (Kawamura et al. International Journal of Cancer (2004), 109(1), 9-16). Secondly, its suppression or inhibition confers radio-sensitivity in tumour cells. Finally, Polθ inhibition could conceivably prevent the MMEJ-dependent functional reversion of BRCA2 mutations that underlies the emergence of cisplatin and PARPi resistance in tumours.

There is therefore a need to provide effective Polθ inhibitors for the treatment of cancer.

### SUMMARY OF THE INVENTION

According to a first aspect of the invention, there is provided a compound of formula (I):

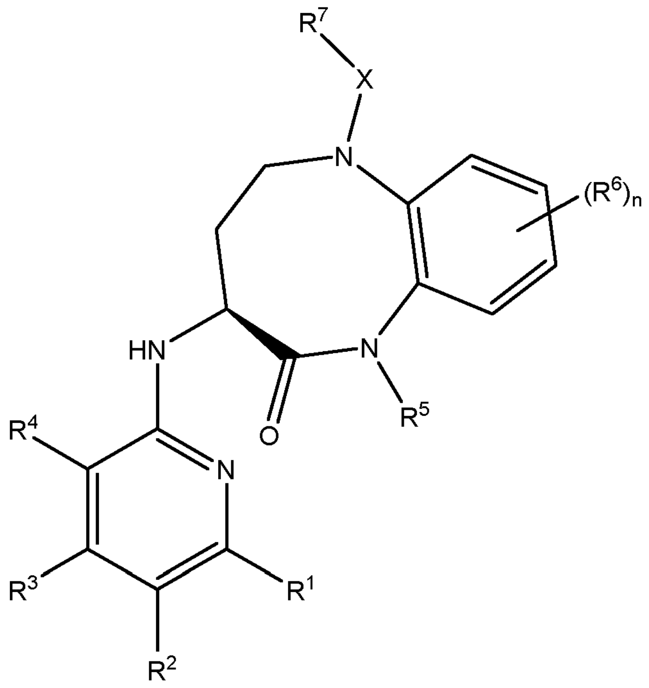

(I)

or a tautomeric or a stereochemically isomeric form, a pharmaceutically acceptable salt or a solvate thereof, wherein:

$R^1$, $R^2$, $R^3$ and $R^4$ independently represent hydrogen, $C_{1-6}$ alkyl, $C_{2-6}$ alkenyl, $C_{1-6}$ alkoxy, halogen, halo$C_{1-6}$ alkyl, halo$C_{1-6}$ alkoxy, $C_{3-8}$ cycloalkyl, cyano or $-NR^xR^y$;
$R^5$ represents hydrogen or $C_{1-6}$ alkyl;
n represents an integer selected from 0, 1, 2 or 3;
$R^6$ represents halogen or $C_{1-6}$ alkyl;
X represents a $C_1$-$C_4$ alkylene group optionally substituted by an O, OH or $CONR^8$ group;
$R^8$ represents hydrogen or $C_{1-6}$ alkyl;
$R^7$ represents a -$Het^A$-CO-CH=$CHR^9$ or -$Het^B$-Y-$Het^C$-CO-CH=$CHR^9$ group;
Y represents a bond or -O-;
$R^9$ represents hydrogen, $-CH_2-NR^xR^y$ or -$Het^D$;
$Het^A$, $Het^B$, $Het^C$ and $Het^D$ each represent a heterocyclyl or a heteroaryl group optionally substituted by one or more (e.g. 1, 2 or 3) substituents such as $C_{1-6}$ alkyl, $C_{2-6}$ alkenyl, $C_{2-6}$ alkynyl, $C_{1-6}$ alkoxy, -COO-$C_{2-6}$ alkynyl, -CO-$C_{1-6}$ alkyl, hydroxy and oxo; and
$R^x$ and $R^y$ independently represent hydrogen, $C_{1-6}$ alkyl, $C_{2-6}$ alkenyl, $C_{2-6}$ alkynyl, $C_{1-6}$ alkoxy, or $R^x$ and $R^y$ together with the nitrogen atom to which they are attached join to form a nitrogen containing heterocyclic ring which may be optionally substituted by one or more $C_{1-6}$ alkyl groups.

## DETAILED DESCRIPTION OF THE INVENTION

### *Definitions*

The term 'halo' or 'halogen' as used herein refers to fluorine, chlorine, bromine or iodine.

The term 'cyano' as used herein refers to a group where a carbon atom is triple bonded to a nitrogen atom.

The term '$C_{1-6}$ alkyl' as used herein as a group or part of a group refers to a linear or branched saturated hydrocarbon group containing from 1 to 6 carbon atoms. Examples of such groups include methyl, ethyl, n-propyl, iso-propyl, butyl, iso-butyl, *tert-butyl,* pentyl, hexyl and the like.

The term '$C_{2-6}$ alkenyl' as used herein as a group or part of a group refers to a linear or branched unsaturated hydrocarbon group containing from 2 to 6 carbon atoms and at least one double bond. Examples of such groups include ethenyl, propenyl, butenyl, pentenyl, hexenyl and the like.

The term '$C_{1-6}$ alkoxy' as used herein as a group or part of a group refers to a $C_{1-6}$ alkyl group which contains an oxygen atom wherein $C_{1-6}$ alkyl is as defined herein. Examples of such groups include methoxy, ethoxy or propoxy.

The term "$C_1$-$C_4$ alkylene" as used herein as a group or part of a group refers to a $-(CH_2)_{1-4}$ group. Examples of such groups include methylene, ethylene, propylene and butylene.

The term 'halo$C_{1-6}$ alkyl' as used herein as a group or part of a group refers to a $C_{1-6}$ alkyl group as defined herein wherein one or more than one hydrogen atom is replaced with a halogen. The term 'halo$C_{1-6}$ alkyl' therefore includes monohalo$C_{1-6}$ alkyl and also polyhalo$C_{1-6}$ alkyl. There may be one, two, three or more hydrogen atoms replaced with a halogen, so the halo$C_{1-6}$ alkyl may have one, two, three or more halogens. Examples of such groups include fluoroethyl, fluoromethyl, trifluoromethyl or trifluoroethyl and the like.

The term "$C_{3-8}$ cycloalkyl" as used herein refers to a saturated monocyclic hydrocarbon ring of 3 to 8 carbon atoms. Examples of such groups include cyclopropyl, cyclobutyl, cyclopentyl, cyclohexyl, cycloheptyl, cyclooctyl and the like.

The term 'oxo' as used herein refers to the group =O.

The term 'amino' as used herein refers to the group -NR'R", wherein R' and R" independently represent a hydrogen or $C_{1-6}$ alkyl group.

The term 'heterocyclyl' as used herein refers to a monocyclic or bicyclic non-aromatic, partially saturated or fully saturated ring system containing for example 3 to 12 ring members. Each ring may contain up to five heteroatoms typically selected from nitrogen, sulfur and oxygen.

Particular examples of 'heterocyclyl' include morpholine, piperidine (*e.g.* piperidin-1-yl, piperidin-2-yl, piperidin-3-yl and piperidin-4-yl), piperidinone, pyrrolidine (*e.g.* pyrrolidin-1-yl, pyrrolidin-2-yl and pyrrolidin-3-yl), pyrrolidone, azetidine, pyran (2H-pyran or 4H-pyran), dihydrothiophene, dihydropyran, dihydrofuran, dihydrothiazole, tetrahydrofuran, tetrahydrothiophene, dioxane, tetrahydropyran (*e.g.* tetrahydropyran-4-yl), imidazoline, imidazolidinone, oxazoline, thiazoline, pyrazolin-2-yl, pyrazolidine, piperazinone and piperazine.

It will be appreciated that the term 'heterocyclyl" includes reference to spiro and bridged heterocyclic derivatives. Examples of such spiro and bridged heterocyclic derivatives include: hexahydropyrrolo[2,3-c]pyrrolidinyl, diazaspiro[3.3]heptanyl, diazaspiro[3.4]octanyl, diazaspiro[4.4]nonyl, oxa-azaspiro[3.4]octanyl, oxa-azaspiro[4.4]nonyl, tetrahydrofuro[3,4-c]pyrrolidinyl, oxa-azaspiro[3.3]heptyl, diazaspiro[3.5]nonanyl, diazaspiro[4.4]nonanyl, Idiazaspiro[4.5]decanyl, diazaspiro[3.4]octanyl, diazaspiro[3.5]nonanyl, octahydro-naphthyridinyl, tetrahydropyrazino-oxazinyl, oxadiazaspiro[3.4]octanyl, oxadiazaspiro[3.5]nonanyl, oxadiazaspiro[4.5]decanyl, oxadiazospiro[5.5]undecanyl, triazaspiro[3.5]nonanyl, diazabicyclo[2.2.1]heptan-2-yl and oxabicyclo[2.2.1]heptanyl.

The term 'heteroaryl' as used herein denotes a heterocyclyl group having aromatic character. The term "heteroaryl" embraces polycyclic (e.g. bicyclic) ring systems wherein one or more rings are non-aromatic, provided that at least one ring is aromatic. In such polycyclic systems, the group may be attached by the aromatic ring, or by a non-aromatic ring.

Examples of heteroaryl groups are monocyclic and bicyclic groups containing from five to twelve ring members, and more usually from five to ten ring members.

Examples of five membered heteroaryl groups include but are not limited to pyrrole, furan, thiophene, imidazole, furazan, oxazole, oxadiazole, oxatriazole, isoxazole, thiazole, thiadiazole, isothiazole, pyrazole, triazole and tetrazole groups.

Examples of six membered heteroaryl groups include but are not limited to pyridine, pyrazine, pyridazine, pyrimidine and triazine.

The term 'optionally substituted' as used herein refers to a group which may be substituted or unsubstituted by a substituent as herein defined.

***Embodiments***

In one embodiment, $R^1$ represents:

hydrogen;
$C_{1-6}$ alkyl (such as methyl or ethyl);
$C_{2-6}$ alkenyl (such as ethenyl);$C_{1-6}$ alkoxy (such as methoxy);
halogen (such as chlorine); or
$-NR^xR^y$ (such as $-N(Me)_2$ or -N(Me)(Et)).

In a further embodiment, $R^1$ represents $C_{1-6}$ alkyl (such as methyl).

In a yet further embodiment, $R^1$ represents methyl.

In one embodiment, $R^2$ represents:

hydrogen;
halogen (such as chlorine); or

$C_{1-6}$ alkyl (such as methyl).

In a further embodiment, $R^2$ represents hydrogen.

In one embodiment, $R^3$ represents:

$C_{1-6}$ alkyl (such as methyl, ethyl or isopropyl);
$C_{2-6}$ alkenyl (such as $-C(=CH_2)(Me)$);
halogen (such as bromine);
halo$C_{1-6}$ alkyl (such as trifluoromethyl or $-C(H)(Me)-CF_3$); or
halo$C_{1-6}$ alkoxy (such as difluoromethoxy).

In a further embodiment, $R^3$ represents halo$C_{1-6}$ alkyl (such as trifluoromethyl).

In a yet further embodiment, $R^3$ represents trifluoromethyl.

In one embodiment, $R^4$ represents:

hydrogen; or
cyano.

In a further embodiment, $R^4$ represents cyano.

In one embodiment, $R^5$ represents hydrogen, methyl or ethyl.

In a further embodiment, $R^5$ represents hydrogen or methyl.

In a yet further embodiment, $R^5$ represents methyl.

In one embodiment, the compound of formula (I) is a compound of formula $(I)^a$:

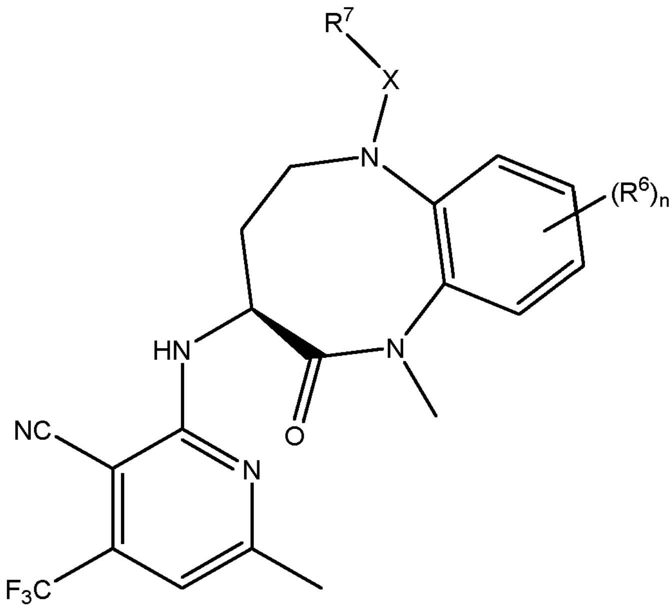

$(I)^a$

or a tautomeric or a stereochemically isomeric form, a pharmaceutically acceptable salt or a solvate thereof, wherein $R^6$, n, X and $R^7$ are as defined herein.

In one embodiment, n represents an integer selected from 0, 1 or 2. In a further embodiment, n represents an integer which is 0. In an alternative embodiment, n represents an integer which is 1. In an alternative embodiment, n represents an integer which is 2.

In one embodiment, $R^6$ represents halogen (such as fluorine or chlorine) or methyl. In a further embodiment, $R^6$ represents fluorine. In an alternative embodiment, $R^6$ represents chlorine. In a yet further alternative embodiment, $R^6$ represents methyl.

In one embodiment, n represents an integer which is 1 and $R^6$ represents fluorine. In an alternative embodiment, n represents an integer which is 1 and $R^6$ represents chlorine. In a yet further alternative embodiment, n represents an integer which is 1 and $R^6$ represents methyl.

In one embodiment, n represents an integer which is 2 and both $R^6$ groups represent fluorine.

In an alternative embodiment, n represents an integer which is 2 and one of $R^6$ represents fluorine and the other represents chlorine.

In one embodiment, X represents a $C_1$-$C_4$ alkylene group (such as $-CH_2-$, $-(CH_2)_2-$, $-(CH_2)_3-$ or $-(CH_2)_4-$) optionally substituted by an O (such as $-(CH_2)_2-O-$, $-(CH_2)_2-O-CH_2-$ or $-(CH_2)_3-O-$), OH (such as $-CH_2-CH(OH)-CH_2-$) or $CONR^8$ (such as $-(CH_2)_2-N(Me)-CO-$) group.

In a yet further embodiment, X represents $-CH_2-$, $-(CH_2)_2-$, $-(CH_2)_2-O-$, $-(CH_2)_2-O-CH_2-$,$-(CH_2)_3-$, $-(CH_2)_3-O-$, $-(CH_2)_2-N(Me)-CO-$ or $-CH_2-CH(OH)-CH_2-$.

In a further embodiment, X represents$-(CH_2)_2-$ or $-(CH_2)_3-O-$.

In one embodiment, $R^8$ represents hydrogen or methyl.

In a further embodiment, $R^8$ represents methyl.

In one embodiment, $R^7$ is a $-Het^A-CO-CH=CHR^9$ group.

In one embodiment, $Het^A$ represents a nitrogen containing heterocyclyl ring. In a further embodiment, $Het^A$ is linked to the $-CO-CH=CHR^6$ group via a nitrogen atom.

In a yet further embodiment, $Het^A$ represents 2-azaspiro[3.3]heptan-6-yl, azetidinyl, 2,6-diazaspiro[3.3]heptan-2-yl, 2,6-diazaspiro[3.5]nonan-6-yl, 2,7-diazaspiro[3.5]nonan-7-yl, 2,7-diazaspiro[4.4]nonan-2-yl, 5-oxa-2,7-diazaspiro[3.4]octan-7-yl, 5-oxa-2,8-diazaspiro[3.5]nonan-8-yl, 6-oxa-2,9-diazaspiro[4.5]decan-9-yl, piperazinyl, piperidinyl, pyrrolidinyl, 2,5,8-triazaspiro[3.5]nonan-8-yl, tetrahydro-1'*H*-spiro[azetidine-3,6'-pyrazino[2,1-*c*][1,4]oxazin]-8'(7'*H*)-yl, or octahydro-2*H*-pyrazino[1,2-a]pyrazin-2-yl, optionally substituted by one or more (e.g. 1, 2 or 3) substituents such as $C_{1-6}$ alkyl (e.g. methyl), oxo, $C_{2-6}$ alkynyl (e.g. propynyl), $-COO-C_{2-6}$ alkynyl (e.g. -COO-propynyl), $-CO-C_{1-6}$ alkyl (e.g.-CO-Me), or $C_{1-6}$ alkoxy (e.g. methoxy).

In a still yet further embodiment, $Het^A$ represents azetidinyl or triazaspiro[3.5]nonanyl, such as unsubstituted azetidinyl or unsubstituted triazaspiro[3.5]nonanyl.

In one embodiment, X represents $-(CH_2)_3-O-$ and $Het^A$ represents azetidinyl. In an alternative embodiment, X represents $-(CH_2)_2-$ and $Het^A$ represents triazaspiro[3.5]nonanyl.

In an alternative embodiment, $R^7$ is a $-Het^B-Y-Het^C-CO-CH=CHR^9$ group. In one embodiment, Y represents a bond. In an alternative embodiment, Y represents -O-.

In a further embodiment, $R^7$ is -azetidinyl-piperazinyl-$CO-CH=CHR^9$, -morpholinyl-azetidinyl-$CO-CH=CHR^9$, -piperazinyl-azetidinyl-$CO-CH=CHR^9$, -piperidinyl-azetidinyl-$CO-CH=CHR^9$,-piperidinyl-O-azetidinyl-$CO-CH=CHR^9$, -2,5-diazabicyclo[2.2.1]heptan-2-yl-azetidinyl-$CO-CH=CHR^9$, -pyrrolidinyl-O-azetidinyl-$CO-CH=CHR^9$, -triazolyl-azetidinyl-$CO-CH=CHR^9$, or-oxadiazolyl-azetidinyl-$CO-CH=CHR^9$, optionally substituted by one or more (e.g. 1, 2 or 3) substituents such as $C_{1-6}$ alkyl (e.g. methyl), hydroxy or oxo.

In a yet further embodiment, $R^7$ is -piperazinyl-azetidinyl-$CO-CH=CHR^9$.

In one embodiment, X represents $-(CH_2)_2-$ and $R^7$ is -piperazinyl-azetidinyl-$CO-CH=CHR^9$.

In one embodiment, $Het^B$ represents a monocyclic heterocyclyl or heteroaryl ring. In a further embodiment, $Het^B$ represents a monocyclic heterocyclyl ring selected from: azetidinyl, morpholinyl, pyrrolidinyl, piperazinyl, piperidinyl or 2,5-diazabicyclo[2.2.1]heptan-2-yl or a monocyclic heteroaryl ring selected from triazolyl or oxadiazolyl, any of which may be optionally substituted by one or more (e.g. 1, 2 or 3) substituents such as $C_{1-6}$ alkyl (e.g. methyl), hydroxy or oxo.

In a yet further embodiment, $Het^B$ represents piperazinyl, such as unsubstituted piperazinyl.

In one embodiment, $Het^C$ represents a nitrogen containing heterocyclyl ring. In a further embodiment, $Het^C$ is linked to the $-CO-CH=CHR^6$ group via a nitrogen atom.

In a yet further embodiment, $Het^C$ represents a monocyclic heterocyclyl. In a still yet further embodiment, $Het^C$ represents azetidinyl, piperazinyl, optionally substituted by one or more (e.g. 1, 2 or 3) substituents such as hydroxy.

In a still yet further embodiment, $Het^C$ represents azetidinyl, such as unsubstituted azetidinyl.

In one embodiment, $R^9$ represents hydrogen.

In an alternative embodiment, $R^9$ represents $-CH_2-NR^xR^y$ (such as $-CH_2-N(Me)_2$, $-CH_2-N(Me)(-(CH_2)_2-OMe)$ or $-CH_2-N(Me)(-CH_2-C{\equiv}CH)$.

In one embodiment, $R^\times$ and $R^y$ independently represent hydrogen, $C_{1-6}$ alkyl (such as methyl), $C_{2-6}$ alkynyl (such as $-CH_2-C{\equiv}CH$) or $C_{1-6}$ alkoxy (such as $-(CH_2)_2-OMe$). In a further embodiment, both of $R^\times$ and $R^y$ represent $C_{1-6}$ alkyl (such as methyl). In an alternative embodiment, one of $R^\times$ and $R^y$ represents $C_{1-6}$ alkyl (such as methyl) and the other represents $C_{2-6}$ alkynyl (such as $-CH_2-C{\equiv}CH$) or $C_{1-6}$ alkoxy (such as $-(CH_2)_2-OMe$).

In an alternative embodiment, $R^9$ represents $-Het^D$.

In one embodiment, $Het^D$ represents a monocyclic heterocyclyl. In a further embodiment, $Het^D$ represents pyrrolidinyl, such as C-linked pyrrolidinyl (e.g. pyrrolidin-2-yl) optionally substituted by one or more (e.g. 1, 2 or 3) substituents such as $C_{1-6}$ alkyl (e.g. methyl).

In one embodiment, the invention provides a compound of formula (I) which is the free base of a compound of Examples 1-72 or a pharmaceutically acceptable salt or solvate thereof.

A reference to a compound of the formula (I) and sub-groups thereof also includes ionic forms, salts, solvates, stereochemical isomers tautomers, N-oxides, thereof, for example, as discussed below; preferably, the salts or tautomers

or stereochemical isomers or N-oxides or solvates thereof; and more preferably, the salts or tautomers or N-oxides or solvates thereof, even more preferably the salts or tautomers or solvates thereof. Hereinafter, compounds and their ionic forms, salts, solvates, stereochemical isomers, tautomers, N-oxides, thereof as defined in any aspect of the invention (except intermediate compounds in chemical processes) are referred to as "compounds of the invention".

***Salts***

Certain compounds of the formula (I) can exist in the form of salts, for example acid addition salts or, in certain cases salts of organic and inorganic bases such as carboxylate, sulfonate and phosphate salts. All such salts are within the scope of this invention, and references to compounds of the formula (I) include the salt forms of the compounds.

The salts of the present invention can be synthesized from the parent compound that contains a basic or acidic moiety by conventional chemical methods such as methods described in *Pharmaceutical Salts: Properties, Selection, and Use,* P. Heinrich Stahl (Editor), Camille G. Wermuth (Editor), ISBN: 3-90639-026-8, Hardcover, 388 pages, August 2002. Generally, such salts can be prepared by reacting the free acid or base forms of these compounds with the appropriate base or acid in water or in an organic solvent, or in a mixture of the two; generally, non-aqueous media such as ether, ethyl acetate, ethanol, isopropanol, or acetonitrile are used.

Acid addition salts (*mono-* or *di*-salts) may be formed with a wide variety of acids, both inorganic and organic. Examples of acid addition salts include *mono-* or *di*-salts formed with an acid selected from the group consisting of acetic, 2,2-dichloroacetic, adipic, alginic, ascorbic (*e.g*. L-ascorbic), L-aspartic, benzenesulfonic, benzoic, 4-acetamidobenzoic, butanoic, (+) camphoric, camphor-sulfonic, (+)-(1S)-camphor-10-sulfonic, capric, caproic, caprylic, cinnamic, citric, cyclamic, dodecylsulfuric, ethane-1,2-disulfonic, ethanesulfonic, 2-hydroxyethanesulfonic, formic, fumaric, galactaric, gentisic, glucoheptonic, D-gluconic, glucuronic (*e.g.* D-glucuronic), glutamic *(e.g.* L-glutamic), $\alpha$-oxoglutaric, glycolic, hippuric, hydrohalic acids (*e.g.* hydrobromic, hydrochloric, hydriodic), isethionic, lactic (*e.g.* (+)-L-lactic, ($\pm$)-DL-lactic), lactobionic, maleic, malic, (-)-L-malic, malonic, ($\pm$)-DL-mandelic, methanesulfonic, naphthalene-2-sulfonic, naphthalene-1,5-disulfonic, 1-hydroxy-2-naphthoic, nicotinic, nitric, oleic, orotic, oxalic, palmitic, pamoic, phosphoric, propionic, pyruvic, L-pyroglutamic, salicylic, 4-amino-salicylic, sebacic, stearic, succinic, sulfuric, tannic, (+)-L-tartaric, thiocyanic, p-toluenesulfonic, undecylenic and valeric acids, as well as acylated amino acids and cation exchange resins.

One particular group of salts consists of salts formed from acetic, hydrochloric, hydriodic, phosphoric, nitric, sulfuric, citric, lactic, succinic, maleic, malic, isethionic, fumaric, benzenesulfonic, toluenesulfonic, methanesulfonic (mesylate), ethanesulfonic, naphthalenesulfonic, valeric, acetic, propanoic, butanoic, malonic, glucuronic and lactobionic acids. One particular salt is the hydrochloride salt.

Where the compounds of the formula (I) contain an amine function, these may form quaternary ammonium salts, for example by reaction with an alkylating agent according to methods well known to the skilled person. Such quaternary ammonium compounds are within the scope of formula (I).

The compounds of the invention may exist as *mono-* or *di*-salts depending upon the $pK_a$ of the acid from which the salt is formed.

It will be appreciated that for use in medicine the salts of the compounds of formula (I) should be pharmaceutically acceptable. Suitable pharmaceutically acceptable salts will be apparent to those skilled in the art. Pharmaceutically acceptable salts include those described by Berge, Bighley and Monkhouse, J. Pharm. Sci. 1977, 66, pp. 1-19. Such pharmaceutically acceptable salts include acid addition salts formed with inorganic acids e.g. hydrochloric, hydrobromic, sulfuric, nitric or phosphoric acid and organic acids e.g. succinic, maleic, acetic, fumaric, citric, tartaric, benzoic, p-toluenesulfonic, methanesulfonic or naphthalenesulfonic acid. Other salts e.g. oxalates or formates may be used, for example in the isolation of compounds of formula (I) and are included within the scope of this invention. However, salts that are not pharmaceutically acceptable may also be prepared as intermediate forms which may then be converted into pharmaceutically acceptable salts. Such non-pharmaceutically acceptable salts forms, which may be useful, for example, in the purification or separation of the compounds of the invention, also form part of the invention. Certain of the compounds of formula (I) may form acid addition salts with one or more equivalents of the acid. The present invention includes within its scope all possible stoichiometric and non-stoichiometric forms.

***Solvates***

Those skilled in the art of organic chemistry will appreciate that many organic compounds can form complexes with solvents in which they are reacted or from which they are precipitated or crystallized. These complexes are known as "solvates". For example, a complex with water is known as a "hydrate". Pharmaceutically acceptable solvates of the compound of the invention are within the scope of the invention. In one embodiment, the pharmaceutically acceptable solvates of the compounds of the invention include the hydrate thereof.

In one embodiment, said crystalline form of the compounds of formula (I) is a cocrystal or coformer. Such a cocrystal or coformer may be prepared using water-soluble molecules such as saccharin, caffeine, nicotinamide or

carboxylic acids. Coformers may be prepared as described in Emami S et al (2018) BioImpacts 8(4), 305-320.

### *N-Oxides*

Compounds of the formula (I) containing an amine function may also form N-oxides. A reference herein to a compound of the formula (I) that contains an amine function also includes the N-oxide.

Where a compound contains several amine functions, one or more than one nitrogen atom may be oxidised to form an N-oxide. Particular examples of N-oxides are the N-oxides of a tertiary amine or a nitrogen atom of a nitrogen-containing heterocycle.

N-Oxides can be formed by treatment of the corresponding amine with an oxidizing agent such as hydrogen peroxide or a *per*-acid (*e.g.* a peroxycarboxylic acid), see for example Advanced Organic Chemistry, by Jerry March, 4th Edition, Wiley Interscience. More particularly, N-oxides can be made by the procedure of L. W. Deady (Syn. Commun. 1977, 7, 509-514) in which the amine compound is reacted with *m*-chloroperoxybenzoic acid (mCPBA), for example, in an inert solvent such as dichloromethane.

Also included within the scope of the compound and various salts of the invention are polymorphs thereof.

### *Enantiomers*

Where chiral centres are present in compounds of formula (I), the present invention includes within its scope all possible enantiomers and diastereoisomers, including mixtures thereof. The different isomeric forms may be separated or resolved one from the other by conventional methods, or any given isomer may be obtained by conventional synthetic methods or by stereospecific or asymmetric syntheses. The invention also extends to any tautomeric forms or mixtures thereof.

### *Purity*

Since the compounds of formula (I) are intended for use in pharmaceutical compositions it will readily be understood that they are each preferably provided in substantially pure form, for example at least 60% pure, more suitably at least 75% pure and preferably at least 85%, especially at least 98% pure (% are given on a weight for weight basis). Impure preparations of the compounds may be used for preparing the more pure forms used in the pharmaceutical compositions.

### *Processes*

According to a further aspect of the present invention there is provided a process for the preparation of compounds of formula (I) and derivatives thereof. The following schemes are examples of synthetic schemes that may be used to synthesise the compounds of the invention. In the following schemes reactive groups can be protected with protecting groups and de-protected according to well established techniques.

According to a further aspect of the invention there is provided a process for preparing a compound of formula (I) as herein defined which comprises:

(a) reacting a compound of formula (II):

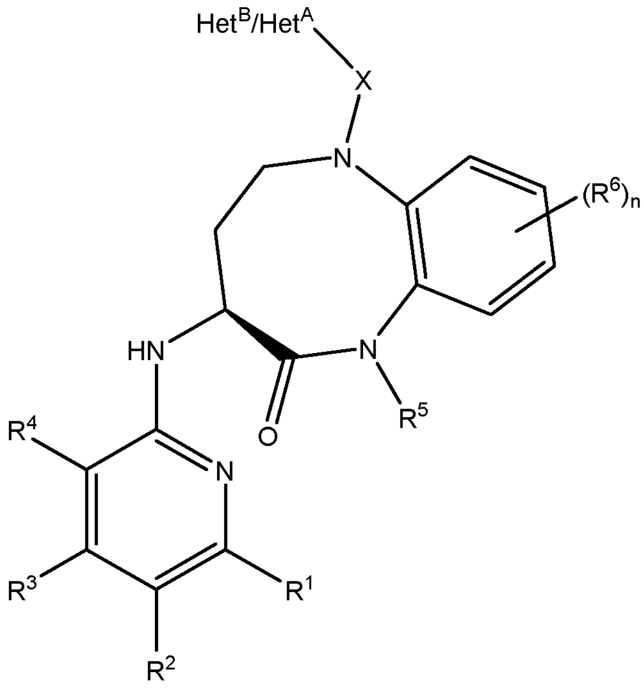

(II)

wherein $R^1$, $R^2$, $R^3$, $R^4$, $R^5$, $R^6$, n, X, $Het^A$ and $Het^B$ are as defined herein, with a compound of formula -CO-CH=CHR$^9$ or -Y-Het$^C$-CO-CH=CHR$^9$, wherein Y, $Het^C$ and $R^9$ are as defined herein;

(b) reacting a compound of formula (III):

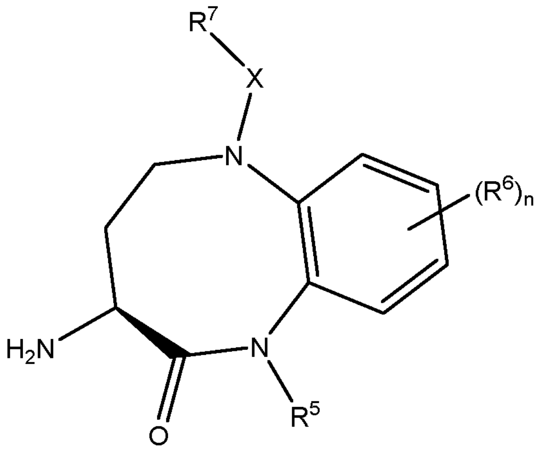

(III)

wherein $R^5$, $R^6$, n, X and $R^7$ are as defined herein, with a compound of formula (IV):

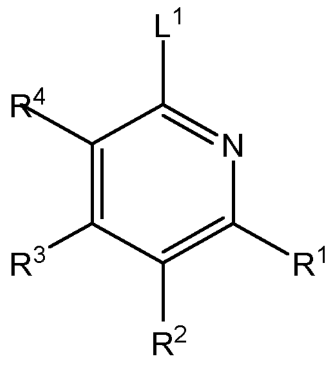

(IV)

wherein $R^1$, $R^2$, $R^3$ and $R^4$ are as defined herein and $L^1$ represents a suitable leaving group, such as a halogen atom (e.g. chlorine);

(c) deprotection of a protected derivative of a compound of formula (I);

(d) interconversion of a compound of formula (I) or protected derivative thereof to a further compound of formula (I) or protected derivative thereof; and

(e) optional formation of a pharmaceutically acceptable salt of a compound of formula (I).

Process (a) typically comprises reacting a compound of formula (II) with the compound of formula -CQ-CH=CHR$^9$ or -Y-Het$^C$-CO-CH=CHR$^9$ in a suitable solvent, such as DCM in the presence of suitable reagents, such as HATU and DIPEA.

Process (b) typically comprises reacting a compound of formula (III) with a compound of formula (IV) in the presence of suitable reagents, such as DIPEA in NMP, under suitable conditions, such as heating to a suitable temperature (such as 100 °C).

Compounds of formula (II) and (III) may be prepared in accordance with the procedures described herein. For example, compounds of formula (II) may be prepared in accordance with the experimental procedure described in Example 1 and compounds of formula (III) may be prepared in accordance with the experimental procedure described in Example 44.

Compounds of formula -CO-CH=CHR$^9$, -Y-Het$^C$-CO-CH=CHR$^9$ and (IV) are either known or may be prepared in accordance with known procedures.

A wide range of well known functional group interconversions for process (d) are known by a person skilled in the art for converting a precursor compound to a compound of formula (I) and are described in Advanced Organic Chemistry by Jerry March, 4th Edition, John Wiley & Sons, 1992. For example possible metal catalysed functionalisations such as using organo-tin reagents (the Stille reaction), Grignard reagents and reactions with nitrogen nucleophiles are described in 'Palladium Reagents and Catalysts' [Jiro Tsuji, Wiley, ISBN 0-470-85032-9] and Handbook of OrganoPalladium Chemistry for Organic Synthesis [Volume 1, Edited by Ei-ichi Negishi, Wiley, ISBN 0-471-31506-0].

If appropriate, the reactions described herein are followed or preceded by one or more reactions known to the skilled of the art and are performed in an appropriate order to achieve the requisite substitutions on each of the variables defined herein to afford other compounds of formula (I). Non-limiting examples of such reactions whose conditions can be found in the literature include:

protection of reactive functions,
deprotection of reactive functions,
halogenation,
dehalogenation,
dealkylation,
alkylation of amine, aniline, alcohol and phenol,
Mitsunobu reaction on hydroxyl groups,
cycloaddition reactions on appropriate groups,
reduction of nitro, esters, cyano, aldehydes,
transition metal-catalyzed coupling reactions,
acylation,

sulfonylation/introduction of sulfonyl groups,
saponification/hydrolysis of esters groups,
amidification or transesterification of ester groups,
esterification or amidification of carboxylic groups,
halogen exchange,
nucleophilic substitution with amine, thiol or alcohol,
reductive amination,
oxime formation on carbonyl and hydroxylamine groups,
S-oxidation,
N-oxidation,
salification.

It is recognised that the sequence of reactions involving aryl coupling and reduction may be varied. It is also recognised that a wide range of palladium based catalysts are suitable for conducting aryl coupling reactions.

It may also be recognised that isomer separation may occur at any suitable stage in the synthetic sequence. It should be stressed that such chiral separation forms a key aspect of the invention and that such separation may be conducted in accordance with the methodology described herein or may be conducted in accordance with known methodology. It is also recognised that it may be beneficial to temporarily form a protected derivative of an intermediate in the synthesis, for example, a Boc-protected amine, or SEM-protected amide, in order to facilitate chromatographic separation, chiral resolution or to give improved solubility or yields in particular steps.

In many of the reactions described above, it may be necessary to protect one or more groups to prevent reaction from taking place at an undesirable location on the molecule. Examples of protecting groups, and methods of protecting and de-protecting functional groups, can be found in Protective Groups in Organic Synthesis (T. Green and P. Wuts; 4th Edition; John Wiley and Sons, 2007).

A hydroxy group may be protected, for example, as an ether (-OR) or an ester (-OC(=O)R), for example, as: a *tert-butyl* ether; a tetrahydropyranyl (THP) ether; a benzyl, benzhydryl (diphenylmethyl), or trityl (triphenylmethyl) ether; a trimethylsilyl or *tert*-butyldimethylsilyl ether; or an acetyl ester (-OC(=O)$CH_3$).

An amine group may be protected, for example, as an amide (-NRCO-R) or a carbamate (-NRCO-OR), for example, as: a methyl amide (-NHCO-$CH_3$); a benzyl carbamate (-NHCO-$OCH_2C_6$Hs, -NH-Cbz or NH-Z); as a *tert-butyl* carbamate (-NHCOOC$(CH_3)_3$, NH-Boc); 2-biphenyl-2-propyl carbamate (-NHCO-OC$(CH_3)_2C_6H_4C_6H_5$, NH-Boc), as a 9-fluorenylmethyl carbamate (-NH-Fmoc), as a 6-nitroveratryl carbamate (-NH-Nvoc), as a 2-trimethylsilylethyl carbamate (-NH-Teoc), as a 2,2,2-trichloroethyl carbamate (-NH-Troc), as an allyl carbamate (-NH-Alloc), or as a 2(-phenylsulfonyl)ethyl carbamate (-NH-Psec).

Other protecting groups for amines, such as cyclic amines and heterocyclic N-H groups, include toluenesulfonyl (tosyl) and methanesulfonyl (mesyl) groups, benzyl groups such as a para-methoxybenzyl (PMB) group and tetrahydropyranyl (THP) groups.

A carboxylic acid group may be protected as an ester for example, as: an $C_{1-7}$ alkyl ester (*e.g.* a methyl ester; a *tert*-butyl ester); a $C_{1-7}$ haloalkyl ester (*e.g.* a $C_{1-7}$ trihaloalkyl ester); a tri$C_{1-7}$ alkylsilyl-$C_{1-7}$ alkyl ester; or a $C_{5-20}$ aryl-$C_{1-7}$ alkyl ester (*e.g.* a benzyl ester; a nitrobenzyl ester; *para*-methoxybenzyl ester.

It will be understood by those skilled in the art that certain compounds of the invention can be converted into other compounds of the invention according to standard chemical methods.

Pharmaceutically acceptable salts may be prepared conventionally by reaction with the appropriate acid or acid derivative.

### *Therapeutic Utility*

The compounds of the invention, subgroups and examples thereof, are inhibitors of Polθ polymerase activity, and which may be useful in preventing or treating disease states or conditions described herein. In addition, the compounds of the invention, and subgroups thereof, will be useful in preventing or treating diseases or condition mediated by Polθ. References to the preventing or prophylaxis or treatment of a disease state or condition such as cancer include within their scope alleviating or reducing the incidence of cancer.

Thus, for example, it is envisaged that the compounds of the invention will be useful in alleviating or reducing the incidence of cancer.

The compounds of the present invention may be useful for the treatment of the adult population. The compounds of the present invention may be useful for the treatment of the pediatric population.

As a consequence of their inhibition of Polθ, the compounds will be useful in providing a means of disabling the ability of cells to perform MMEJ. It is therefore anticipated that the compounds may prove useful in treating or preventing proliferative disorders such as cancers. In addition, the compounds of the invention may be useful in the treatment of

diseases in which there is a disorder associated with cell accumulation.

Without being bound by theory it is expected that the Polθ inhibitors of the present invention will demonstrate certain properties for them to be of particular utility in the therapeutic treatment of certain cancers. For example, in one embodiment, the Polθ inhibitors of the present invention are suitably lethal in BRCA1 and BRCA2 deficient primary and secondary solid tumours, including breast, ovarian, prostate and pancreas.

In a further embodiment, the Polθ inhibitors of the present invention are suitably lethal in a variety of primary and secondary solid tumours which are HRD by mechanisms other than BRCA deficiency, such as those with promoter hypermethylation. In these tumours where no DSB repair pathway may be fully down regulated the Polθi may be given along with another DDR modulator such as a PARP inhibitor, a DNA-PK inhibitor, an ATR inhibitor, an ATM inhibitor, a wee1 inhibitor or a CHK1 inhibitor.

In a further embodiment, the Polθ inhibitors of the present invention are suitably lethal in primary and secondary breast, ovarian, prostate and pancreatic tumours retaining BRCA1 deficiency but which, following or not following exposure to PARPi medication, are resistant to PARPi treatment.

In a further embodiment, the Polθ inhibitors of the present invention suitably increase the ORR including CRR, will delay the onset of PARPi resistance, will increase the time to relapse and DFS, and will increase the OS of HRD (BRCA1/2 deficient and other HRD mechanisms) primary and secondary tumours (breast, ovarian, prostate and pancreas) when given with PARPi treatment programmes.

In a further embodiment, the Polθ inhibitors of the present invention suitably show synthetic sickness and/or synthetic lethality in a variety of tumours with loss of ATM activity ($ATM^{-/-}$) particularly in the context of WT p53. Tumour types will include around 10% of all solid tumours including gastric, lung, breast, and CRC, along with CLL. Co-medicating with another DDR modifier, such as a DNA-PK inhibitor, PARP inhibitor or ATR inhibitor, may further enhance such activity. Polθ inhibitors will resensitise CLL to classical chemotherapy and chemo-immunotherapy where drug resistance has emerged. Thus, according to a further embodiment, the pharmaceutical composition of the present invention additionally comprises a DNA-PK inhibitor, PARP inhibitor, or ATR inhibitor.

In a further embodiment, the Polθ inhibitors of the present invention suitably show synthetic sickness and/or synthetic lethality in a variety of tumours deficient in the DNA double strand break repair process of non-homologous end-joining (NHEJ-D). Tumour types will include approximately 2-10% of all solid tumours including prostate, pancreatic, cervical, breast, lung, bladder and oesophageal. Co-medicating with another DDR modifier, such as a PARP inhibitor, ATM inhibitor, wee1 inhibitor, CHK inhibitor, or ATR inhibitor, may further enhance such activity. Polθ inhibitors will further sensitise NHEJD cancer cells to DNA DSB inducing chemotherapies and to ionising radiation based therapies. Thus, according to a further embodiment, the pharmaceutical composition of the present invention additionally comprises a PARP inhibitor, ATM inhibitor, wee1 inhibitor, CHK inhibitor, or ATR inhibitor.

In a further embodiment, the Polθ inhibitors of the present invention suitably reduce the DNA replication stress response during the chemotherapy of HR proficient tumours such as ovarian, NSCL and breast tumours over expressing Polθ. This will increase the ORR to treatment and increase OS. Such effects are particularly likely with cytarabine (Ara-C) and hydroxyurea used in a wide variety of leukemias including CML, and the management of squamous cell carcinomas.

In a further embodiment, the Polθ inhibitors of the present invention suitably selectively sensitise solid tumours to radiotherapy, including EBRTand brachytherapy, with little or no sensitisation of normal tissues. In a fractionated curative-intent setting this will increase loco-regional control driving increased survival. This will be particularly evident in the management of NSCLC, SCCH&N, rectal cancer, prostate cancer and pancreatic cancer.

In a further embodiment, the Polθ inhibitors of the present invention suitably show synthetic sickness and/or synthetic lethality in PTEN deleted tumours such as CaP, with or without comedication with a PARPi. Furthermore, such tumours will exhibit exquisite sensitivity to radiotherapy both by dint of the PTEN deletion as well as the Polθ inhibitor induced radiosensitivity.

In a further embodiment, the Polθ inhibitors of the present invention suitably suppress TLS polymerase activity, sensitising primary and secondary solid tumours (e.g. breast, lung, ovarian, CRC) to drugs (e.g. cisplatin, mitomycin and cyclophosphamide) as well as reducing the acquisition of drug-induced mutations implicated in tumour resistance leading to prolongation of remission and increased TTR.

In a further embodiment, the Polθ inhibitors of the present invention suitably resensitise BCR-ABL-positive CML which is has developed imatinib resistance, as well as other solid tumours with elevated ligase IIIα levels, reduced ligase IV levels and increased dependence upon altEJ DSB repair.

In a further embodiment, the Polθ inhibitors of the present invention suitably show synthetic sickness and/or synthetic lethality in aromatase inhibitor resistant $ER^-$ primary and secondary breast cancers, again showing elevated ligase IIIα levels, reduced ligase IV levels and increased dependence upon altEJ DSB repair.

According to a further aspect of the invention there is a provided a compound of formula (I) as defined herein for use in the treatment of tumours characterised by a deficiency in homologous recombination (HRD).

It will be appreciated that references herein to "deficiency in homologous recombination (HRD)" refer to any genetic variation which results in a deficiency or loss of function of the resultant homologous recombination gene.

Examples of said genetic variation include mutations (e.g. point mutations), substitutions, deletions, single nucleotide polymorphisms (SNPs), haplotypes, chromosome abnormalities, Copy Number Variation (CNV), epigenetics, DNA inversions, reduction in expression and mis-localisation.

In one embodiment, said homologous recombination genes are selected from any of: ATM, ATR, BRCA1, BRCA2, BARD1, RAD51C, RAD50, CHEK1, CHEK2, FANCA, FANCB, FANCC, FANCD2, FANCE, FANCF, FANCG, FANCI, FANCL, FANCM, PALB2 (FANCN), FANCP (BTBD12), ERCC4 (FANCQ), PTEN, CDK12, MRE11, NBS1, NBN, CLASPIN, BLM, WRN, SMARCA2, SMARCA4, LIG1, RPA1, RPA2, BRIP1 and PTEN.

It will be appreciated that references herein to "non-homologous end-joining deficiency (NHEJD)" refer to any genetic variation which results in a deficiency or loss of function of the resultant homologous recombination gene. Examples of said genetic variation include mutations (e.g. point mutations), substitutions, deletions, single nucleotide polymorphisms (SNPs), haplotypes, chromosome abnormalities, Copy Number Variation (CNV), epigenetics, DNA inversions, reduction in expression and mis-localisation.

In one embodiment, said non-homologous end-joining genes are selected from any one or more of: LIG4, NHEJ1, POLL, POLM, PRKDC, XRCC4, XRCC5, XRCC6, and DCLRE1C.

According to a further aspect of the invention there is a provided a compound of formula (I) as defined herein for use in the treatment of tumours which overexpress Pol$\theta$.

According to a further aspect of the invention there is a provided a compound of formula (I) as defined herein for use in the treatment of tumours which have elevated ligase III$\alpha$ levels, reduced ligase IV levels and increased dependence upon altEJ DSB repair.

Examples of cancers (and their benign counterparts) which may be treated (or inhibited) include, but are not limited to tumours of epithelial origin (adenomas and carcinomas of various types including adenocarcinomas, squamous carcinomas, transitional cell carcinomas and other carcinomas) such as carcinomas of the bladder and urinary tract, breast, gastrointestinal tract (including the esophagus, stomach (gastric), small intestine, colon, rectum and anus), liver (hepatocellular carcinoma), gall bladder and biliary system, exocrine pancreas, kidney, lung (for example adenocarcinomas, small cell lung carcinomas, non-small cell lung carcinomas, bronchioalveolar carcinomas and mesotheliomas), head and neck (for example cancers of the tongue, buccal cavity, larynx, pharynx, nasopharynx, tonsil, salivary glands, nasal cavity and paranasal sinuses), ovary, fallopian tubes, peritoneum, vagina, vulva, penis, cervix, myometrium, endometrium, thyroid (for example thyroid follicular carcinoma), adrenal, prostate, skin and adnexae (for example melanoma, basal cell carcinoma, squamous cell carcinoma, keratoacanthoma, dysplastic naevus); haematological malignancies (i.e. leukemias, lymphomas) and premalignant haematological disorders and disorders of borderline malignancy including haematological malignancies and related conditions of lymphoid lineage (for example acute lymphocytic leukemia [ALL], chronic lymphocytic leukemia [CLL], B-cell lymphomas such as diffuse large B-cell lymphoma [DLBCL], follicular lymphoma, Burkitt's lymphoma, mantle cell lymphoma, MALT lymphoma, T-cell lymphomas and leukaemias, natural killer [NK] cell lymphomas, Hodgkin's lymphomas, hairy cell leukaemia, monoclonal gammopathy of uncertain significance, plasmacytoma, multiple myeloma, and post-transplant lymphoproliferative disorders), and haematological malignancies and related conditions of myeloid lineage (for example acute myelogenous leukemia [AML], chronic myelogenous leukemia [CML], chronic myelomonocytic leukemia [CMML], hypereosinophilic syndrome, myeloproliferative disorders such as polycythaemia vera, essential thrombocythaemia and primary myelofibrosis, myeloproliferative syndrome, myelodysplastic syndrome, and promyelocytic leukemia); tumours of mesenchymal origin, for example sarcomas of soft tissue, bone or cartilage such as osteosarcomas, fibrosarcomas, chondrosarcomas, rhabdomyosarcomas, leiomyosarcomas, liposarcomas, angiosarcomas, Kaposi's sarcoma, Ewing's sarcoma, synovial sarcomas, epithelioid sarcomas, gastrointestinal stromal tumours, benign and malignant histiocytomas, and dermatofibrosarcoma protuberans; tumours of the central or peripheral nervous system (for example astrocytomas, gliomas and glioblastomas, meningiomas, ependymomas, pineal tumours and schwannomas); endocrine tumours (for example pituitary tumours, adrenal tumours, islet cell tumours, parathyroid tumours, carcinoid tumours and medullary carcinoma of the thyroid); ocular and adnexal tumours (for example retinoblastoma); germ cell and trophoblastic tumours (for example teratomas, seminomas, dysgerminomas, hydatidiform moles and choriocarcinomas); and paediatric and embryonal tumours (for example medulloblastoma, neuroblastoma, Wilms tumour, and primitive neuroectodermal tumours); or syndromes, congenital or otherwise, which leave the patient susceptible to malignancy (for example Xeroderma Pigmentosum).

Many diseases are characterized by persistent and unregulated angiogenesis. Chronic proliferative diseases are often accompanied by profound angiogenesis, which can contribute to or maintain an inflammatory and/or proliferative state, or which leads to tissue destruction through the invasive proliferation of blood vessels. Tumour growth and metastasis have been found to be angiogenesis-dependent. Compounds of the invention may therefore be useful in preventing and disrupting initiation of tumour angiogenesis. In particular, the compounds of the invention may be useful in the treatment of metastasis and metastatic cancers.

Metastasis or metastatic disease is the spread of a disease from one organ or part to another non-adjacent organ or part. The cancers which can be treated by the compounds of the invention include primary tumours (i.e. cancer cells at

the originating site), local invasion (cancer cells which penetrate and infiltrate surrounding normal tissues in the local area), and metastatic (or secondary) tumours ie. tumours that have formed from malignant cells which have circulated through the bloodstream (haematogenous spread) or via lymphatics or across body cavities (trans-coelomic) to other sites and tissues in the body.

Particular cancers include hepatocellular carcinoma, melanoma, oesophageal, renal, colon, colorectal, lung e.g. mesothelioma or lung adenocarcinoma, breast, bladder, gastrointestinal, ovarian and prostate cancers.

A further aspect provides the use of a compound for the manufacture of a medicament for the treatment of a disease or condition as described herein, in particular cancer.

The compounds may also be useful in the treatment of tumour growth, pathogenesis, resistance to chemo- and radio-therapy by sensitising cells to chemotherapy and as an anti-metastatic agent.

The potency of the compounds of the invention as inhibitors of Polθ can be measured using the biological and biophysical assays set forth in the examples herein and the level of affinity exhibited by a given compound can be defined in terms of the $IC_{50}$ value. Particular compounds of the present invention are compounds having an $IC_{50}$ value of less than 1μM, more particularly less than 0.1 μM.

A role for the loss of Polθ enhancing the efficacy of CRISPR mediated gene editing has been described in WO 2017/062754. Thus, Polθ inhibitory compounds are likely to be useful in enhancing the efficiency of CRISPR based editing methodologies and/or CRISPR based editing therapeutics. Furthermore, compound mediated Polθ inhibition is likely to reduce the frequency of random integration events and thus provide a route to ameliorate any safety concerns of CRISPR mediated technology. Thus, according to a further aspect of the invention, there is provided the use of a compound of formula (I) as defined herein in a CRISPR based editing methodology and/or CRISPR based editing therapeutics, such as the enhancement of efficiency of CRISPR based editing methodology and/or CRISPR based editing therapeutics.

***Pharmaceutical Compositions***

While it is possible for the active compound to be administered alone, it is preferable to present it as a pharmaceutical composition (e.g. formulation). In one embodiment this is a sterile pharmaceutical composition.

Thus, the present invention further provides pharmaceutical compositions, as defined above, and methods of making a pharmaceutical composition comprising (e.g admixing) at least one compound of formula (I) (and sub-groups thereof as defined herein), together with one or more pharmaceutically acceptable excipients and optionally other therapeutic or prophylactic agents, as described herein.

The pharmaceutically acceptable excipient(s) can be selected from, for example, carriers (e.g. a solid, liquid or semi-solid carrier), adjuvants, diluents, fillers or bulking agents, granulating agents, coating agents, release-controlling agents, binding agents, disintegrants, lubricating agents, preservatives, antioxidants, buffering agents, suspending agents, thickening agents, flavouring agents, sweeteners, taste masking agents, stabilisers or any other excipients conventionally used in pharmaceutical compositions. Examples of excipients for various types of pharmaceutical compositions are set out in more detail below.

The term "pharmaceutically acceptable" as used herein pertains to compounds, materials, compositions, and/or dosage forms which are, within the scope of sound medical judgment, suitable for use in contact with the tissues of a subject (e.g. human) without excessive toxicity, irritation, allergic response, or other problem or complication, commensurate with a reasonable benefit/risk ratio. Each carrier, excipient, etc. must also be "acceptable" in the sense of being compatible with the other ingredients of the formulation.

Pharmaceutical compositions containing compounds of the formula (I) can be formulated in accordance with known techniques, see for example, Remington's Pharmaceutical Sciences, Mack Publishing Company, Easton, PA, USA.

The pharmaceutical compositions can be in any form suitable for oral, parenteral, topical, intranasal, intrabronchial, sublingual, ophthalmic, otic, rectal, intra-vaginal, or transdermal administration. Where the compositions are intended for parenteral administration, they can be formulated for intravenous, intramuscular, intraperitoneal, subcutaneous administration or for direct delivery into a target organ or tissue by injection, infusion or other means of delivery. The delivery can be by bolus injection, short term infusion or longer term infusion and can be via passive delivery or through the utilisation of a suitable infusion pump or syringe driver.

Pharmaceutical formulations adapted for parenteral administration include aqueous and non-aqueous sterile injection solutions which may contain anti-oxidants, buffers, bacteriostats, co-solvents, surface active agents, organic solvent mixtures, cyclodextrin complexation agents, emulsifying agents (for forming and stabilizing emulsion formulations), liposome components for forming liposomes, gellable polymers for forming polymeric gels, lyophilisation protectants and combinations of agents for, *inter alia,* stabilising the active ingredient in a soluble form and rendering the formulation isotonic with the blood of the intended recipient. Pharmaceutical formulations for parenteral administration may also take the form of aqueous and non-aqueous sterile suspensions which may include suspending agents and thickening agents (R. G. Strickly, Solubilizing Excipients in oral and injectable formulations, Pharmaceutical Research, Vol

21(2) 2004, p 201-230).

The formulations may be presented in unit-dose or multi-dose containers, for example sealed ampoules, vials and prefilled syringes, and may be stored in a freeze-dried (lyophilised) condition requiring only the addition of the sterile liquid carrier, for example water for injections, immediately prior to use. In one embodiment, the formulation is provided as an active pharmaceutical ingredient in a bottle for subsequent reconstitution using an appropriate diluent.

The pharmaceutical formulation can be prepared by lyophilising a compound of formula (I), or sub-groups thereof. Lyophilisation refers to the procedure of freeze-drying a composition. Freeze-drying and lyophilisation are therefore used herein as synonyms.

Extemporaneous injection solutions and suspensions may be prepared from sterile powders, granules and tablets.

Pharmaceutical compositions of the present invention for parenteral injection can also comprise pharmaceutically acceptable sterile aqueous or non-aqueous solutions, dispersions, suspensions or emulsions as well as sterile powders for reconstitution into sterile injectable solutions or dispersions just prior to use.

Examples of suitable aqueous and nonaqueous carriers, diluents, solvents or vehicles include water, ethanol, polyols (such as glycerol, propylene glycol, polyethylene glycol, and the like), carboxymethylcellulose and suitable mixtures thereof, vegetable oils (such as sunflower oil, safflower oil, corn oil or olive oil), and injectable organic esters such as ethyl oleate. Proper fluidity can be maintained, for example, by the use of thickening or coating materials such as lecithin, by the maintenance of the required particle size in the case of dispersions, and by the use of surfactants.

The compositions of the present invention may also contain adjuvants such as preservatives, wetting agents, emulsifying agents, and dispersing agents. Prevention of the action of microorganisms may be ensured by the inclusion of various antibacterial and antifungal agents, for example, paraben, chlorobutanol, phenol, sorbic acid, and the like. It may also be desirable to include agents to adjust tonicity such as sugars, sodium chloride, and the like. Prolonged absorption of the injectable pharmaceutical form may be brought about by the inclusion of agents which delay absorption such as aluminum monostearate and gelatin.

In one particular embodiment of the invention, the pharmaceutical composition is in a form suitable for i.v. administration, for example by injection or infusion. For intravenous administration, the solution can be dosed as is, or can be injected into an infusion bag (containing a pharmaceutically acceptable excipient, such as 0.9% saline or 5% dextrose), before administration.

In another particular embodiment, the pharmaceutical composition is in a form suitable for sub-cutaneous (s.c.) administration.

Pharmaceutical dosage forms suitable for oral administration include tablets (coated or uncoated), capsules (hard or soft shell), caplets, pills, lozenges, syrups, solutions, powders, granules, elixirs and suspensions, sublingual tablets, wafers or patches such as buccal patches.

Thus, tablet compositions can contain a unit dosage of active compound together with an inert diluent or carrier such as a sugar or sugar alcohol, eg; lactose, sucrose, sorbitol or mannitol; and/or a non-sugar derived diluent such as sodium carbonate, calcium phosphate, calcium carbonate, or a cellulose or derivative thereof such as microcrystalline cellulose (MCC), methyl cellulose, ethyl cellulose, hydroxypropyl methyl cellulose, and starches such as corn starch. Tablets may also contain such standard ingredients as binding and granulating agents such as polyvinylpyrrolidone, disintegrants (e.g. swellable crosslinked polymers such as crosslinked carboxymethylcellulose), lubricating agents (e.g. stearates), preservatives (e.g. parabens), antioxidants (e.g. BHT), buffering agents (for example phosphate or citrate buffers), and effervescent agents such as citrate/bicarbonate mixtures. Such excipients are well known and do not need to be discussed in detail here.

Tablets may be designed to release the drug either upon contact with stomach fluids (immediate release tablets) or to release in a controlled manner (controlled release tablets) over a prolonged period of time or with a specific region of the GI tract.

Capsule formulations may be of the hard gelatin or soft gelatin variety and can contain the active component in solid, semi-solid, or liquid form. Gelatin capsules can be formed from animal gelatin or synthetic or plant derived equivalents thereof.

The solid dosage forms (eg; tablets, capsules etc.) can be coated or un-coated. Coatings may act either as a protective film (e.g. a polymer, wax or varnish) or as a mechanism for controlling drug release or for aesthetic or identification purposes. The coating (e.g. a Eudragit ™ type polymer) can be designed to release the active component at a desired location within the gastro-intestinal tract. Thus, the coating can be selected so as to degrade under certain pH conditions within the gastrointestinal tract, thereby selectively release the compound in the stomach or in the ileum, duodenum, jejenum or colon.

Instead of, or in addition to, a coating, the drug can be presented in a solid matrix comprising a release controlling agent, for example a release delaying agent which may be adapted to release the compound in a controlled manner in the gastrointestinal tract. Alternatively the drug can be presented in a polymer coating e.g. a polymethacrylate polymer coating, which may be adapted to selectively release the compound under conditions of varying acidity or alkalinity in the

gastrointestinal tract. Alternatively, the matrix material or release retarding coating can take the form of an erodible polymer (e.g. a maleic anhydride polymer) which is substantially continuously eroded as the dosage form passes through the gastrointestinal tract. In another alternative, the coating can be designed to disintegrate under microbial action in the gut. As a further alternative, the active compound can be formulated in a delivery system that provides osmotic control of the release of the compound. Osmotic release and other delayed release or sustained release formulations (for example formulations based on ion exchange resins) may be prepared in accordance with methods well known to those skilled in the art.

The compound of formula (I) may be formulated with a carrier and administered in the form of nanoparticles, the increased surface area of the nanoparticles assisting their absorption. In addition, nanoparticles offer the possibility of direct penetration into the cell. Nanoparticle drug delivery systems are described in "Nanoparticle Technology for Drug Delivery", edited by Ram B Gupta and Uday B. Kompella, Informa Healthcare, ISBN 9781574448573, published 13th March 2006. Nanoparticles for drug delivery are also described in J. Control. Release, 2003, 91 (1-2), 167-172, and in Sinha et al., Mol. Cancer Ther. August 1, (2006) 5, 1909.

The pharmaceutical compositions typically comprise from approximately 1% (w/w) to approximately 95% (w/w) active ingredient and from 99% (w/w) to 5% (w/w) of a pharmaceutically acceptable excipient or combination of excipients. Particularly, the compositions comprise from approximately 20% (w/w) to approximately 90%,% (w/w) active ingredient and from 80% (w/w) to 10% of a pharmaceutically acceptable excipient or combination of excipients. The pharmaceutical compositions comprise from approximately 1% to approximately 95%, particularly from approximately 20% to approximately 90%, active ingredient. Pharmaceutical compositions according to the invention may be, for example, in unit dose form, such as in the form of ampoules, vials, suppositories, pre-filled syringes, dragées, tablets or capsules.

The pharmaceutically acceptable excipient(s) can be selected according to the desired physical form of the formulation and can, for example, be selected from diluents (e.g solid diluents such as fillers or bulking agents; and liquid diluents such as solvents and co-solvents), disintegrants, buffering agents, lubricants, flow aids, release controlling (e.g. release retarding or delaying polymers or waxes) agents, binders, granulating agents, pigments, plasticizers, antioxidants, preservatives, flavouring agents, taste masking agents, tonicity adjusting agents and coating agents.

The skilled person will have the expertise to select the appropriate amounts of ingredients for use in the formulations. For example tablets and capsules typically contain 0-20% disintegrants, 0-5% lubricants, 0-5% flow aids and/or 0-99% (w/w) fillers/ or bulking agents (depending on drug dose). They may also contain 0-10% (w/w) polymer binders, 0-5% (w/w) antioxidants, 0-5% (w/w) pigments. Slow release tablets would in addition contain 0-99% (w/w) release-controlling (e.g. delaying) polymers (depending on dose). The film coats of the tablet or capsule typically contain 0-10% (w/w) polymers, 0-3% (w/w) pigments, and/or 0-2% (w/w) plasticizers.

Parenteral formulations typically contain 0-20% (w/w) buffers, 0-50% (w/w) cosolvents, and/or 0-99% (w/w) Water for Injection (WFI) (depending on dose and if freeze dried). Formulations for intramuscular depots may also contain 0-99% (w/w) oils.

Pharmaceutical compositions for oral administration can be obtained by combining the active ingredient with solid carriers, if desired granulating a resulting mixture, and processing the mixture, if desired or necessary, after the addition of appropriate excipients, into tablets, dragee cores or capsules. It is also possible for them to be incorporated into a polymer or waxy matrix that allow the active ingredients to diffuse or be released in measured amounts.

The compounds of the invention can also be formulated as solid dispersions. Solid dispersions are homogeneous extremely fine disperse phases of two or more solids. Solid solutions (molecularly disperse systems), one type of solid dispersion, are well known for use in pharmaceutical technology (see (Chiou and Riegelman, J. Pharm. Sci., 60, 1281-1300 (1971)) and are useful in increasing dissolution rates and increasing the bioavailability of poorly water-soluble drugs.

This invention also provides solid dosage forms comprising the solid solution described above. Solid dosage forms include tablets, capsules, chewable tablets and dispersible or effervescent tablets. Known excipients can be blended with the solid solution to provide the desired dosage form. For example, a capsule can contain the solid solution blended with (a) a disintegrant and a lubricant, or (b) a disintegrant, a lubricant and a surfactant. In addition a capsule can contain a bulking agent, such as lactose or microcrystalline cellulose. A tablet can contain the solid solution blended with at least one disintegrant, a lubricant, a surfactant, a bulking agent and a glidant. A chewable tablet can contain the solid solution blended with a bulking agent, a lubricant, and if desired an additional sweetening agent (such as an artificial sweetener), and suitable flavours. Solid solutions may also be formed by spraying solutions of drug and a suitable polymer onto the surface of inert carriers such as sugar beads ('non-pareils'). These beads can subsequently be filled into capsules or compressed into tablets.

The pharmaceutical formulations may be presented to a patient in "patient packs" containing an entire course of treatment in a single package, usually a blister pack. Patient packs have an advantage over traditional prescriptions, where a pharmacist divides a patient's supply of a pharmaceutical from a bulk supply, in that the patient always has access to the package insert contained in the patient pack, normally missing in patient prescriptions. The inclusion of a package insert has been shown to improve patient compliance with the physician's instructions.

Compositions for topical use and nasal delivery include ointments, creams, sprays, patches, gels, liquid drops and inserts (for example intraocular inserts). Such compositions can be formulated in accordance with known methods.

Examples of formulations for rectal or intra-vaginal administration include pessaries and suppositories which may be, for example, formed from a shaped moldable or waxy material containing the active compound. Solutions of the active compound may also be used for rectal administration.

Compositions for administration by inhalation may take the form of inhalable powder compositions or liquid or powder sprays, and can be administrated in standard form using powder inhaler devices or aerosol dispensing devices. Such devices are well known. For administration by inhalation, the powdered formulations typically comprise the active compound together with an inert solid powdered diluent such as lactose.

The compounds of the formula (I) will generally be presented in unit dosage form and, as such, will typically contain sufficient compound to provide a desired level of biological activity. For example, a formulation may contain from 1 nanogram to 2 grams of active ingredient, e.g. from 1 nanogram to 2 milligrams of active ingredient. Within these ranges, particular sub-ranges of compound are 0.1 milligrams to 2 grams of active ingredient (more usually from 10 milligrams to 1 gram, e.g. 50 milligrams to 500 milligrams), or 1 microgram to 20 milligrams (for example 1 microgram to 10 milligrams, e.g. 0.1 milligrams to 2 milligrams of active ingredient).

For oral compositions, a unit dosage form may contain from 1 milligram to 2 grams, more typically 10 milligrams to 1 gram, for example 50 milligrams to 1 gram, e.g. 100 miligrams to 1 gram, of active compound.

The active compound will be administered to a patient in need thereof (for example a human or animal patient) in an amount sufficient to achieve the desired therapeutic effect.

***Methods of Treatment***

The compounds of the formula (I) and sub-groups as defined herein may be useful in the prophylaxis or treatment of a range of disease states or conditions mediated by Polθ. Thus, according to a further aspect of the invention there is provided a compound for use in a method of treating a disease state or condition mediated by Polθ (e.g. cancer) which comprises administering to a subject in need thereof a compound of formula (I) as described herein. Examples of such disease states and conditions are set out above, and in particular include cancer.

The compounds are generally administered to a subject in need of such administration, for example a human or animal patient, particularly a human.

The compounds will typically be administered in amounts that are therapeutically or prophylactically useful and which generally are non-toxic. However, in certain situations (for example in the case of life threatening diseases), the benefits of administering a compound of the formula (I) may outweigh the disadvantages of any toxic effects or side effects, in which case it may be considered desirable to administer compounds in amounts that are associated with a degree of toxicity.

The compounds may be administered over a prolonged term to maintain beneficial therapeutic effects or may be administered for a short period only. Alternatively they may be administered in a continuous manner or in a manner that provides intermittent dosing (e.g. a pulsatile manner).

A typical daily dose of the compound of formula (I) can be in the range from 100 picograms to 100 milligrams per kilogram of body weight, more typically 5 nanograms to 25 milligrams per kilogram of bodyweight, and more usually 10 nanograms to 15 milligrams per kilogram (e.g. 10 nanograms to 10 milligrams, and more typically 1 microgram per kilogram to 20 milligrams per kilogram, for example 1 microgram to 10 milligrams per kilogram) per kilogram of bodyweight although higher or lower doses may be administered where required.

The compound of the formula (I) can be administered on a daily basis or on a repeat basis every 2, or 3, or 4, or 5, or 6, or 7, or 10 or 14, or 21, or 28 days for example.

The compounds of the invention may be administered orally in a range of doses, for example 1 to 1500 mg, 2 to 800 mg, or 5 to 500 mg, e.g. 2 to 200 mg or 10 to 1000 mg, particular examples of doses including 10, 20, 50 and 80 mg. The compound may be administered once or more than once each day. The compound can be administered continuously (i.e. taken every day without a break for the duration of the treatment regimen). Alternatively, the compound can be administered intermittently (i.e. taken continuously for a given period such as a week, then discontinued for a period such as a week and then taken continuously for another period such as a week and so on throughout the duration of the treatment regimen). Examples of treatment regimens involving intermittent administration include regimens wherein administration is in cycles of one week on, one week off; or two weeks on, one week off; or three weeks on, one week off; or two weeks on, two weeks off; or four weeks on two weeks off; or one week on three weeks off - for one or more cycles, e.g. 2, 3, 4, 5, 6, 7, 8, 9 or 10 or more cycles.

In one particular dosing schedule, a patient will be given an infusion of a compound of the formula (I) for periods of one hour daily for up to ten days in particular up to five days for one week, and the treatment repeated at a desired interval such as two to four weeks, in particular every three weeks.

More particularly, a patient may be given an infusion of a compound of the formula (I) for periods of one hour daily

for 5 days and the treatment repeated every three weeks.

In another particular dosing schedule, a patient is given an infusion over 30 minutes to 1 hour followed by maintenance infusions of variable duration, for example 1 to 5 hours, e.g. 3 hours.

In a further particular dosing schedule, a patient is given a continuous infusion for a period of 12 hours to 5 days, an in particular a continuous infusion of 24 hours to 72 hours.

In another particular dosing schedule, a patient is given the compound orally once a week.

In another particular dosing schedule, a patient is given the compound orally once-daily for between 7 and 28 days such as 7, 14 or 28 days.

In another particular dosing schedule, a patient is given the compound orally once-daily for 1 day, 2 days, 3 days, 5 days or 1 week followed by the required amount of days off to complete a one or two week cycle.

In another particular dosing schedule, a patient is given the compound orally once-daily for 2 weeks followed by 2 weeks off.

In another particular dosing schedule, a patient is given the compound orally once-daily for 2 weeks followed by 1 week off.

In another particular dosing schedule, a patient is given the compound orally once-daily for 1 week followed by 1 week off.

Ultimately, however, the quantity of compound administered and the type of composition used will be commensurate with the nature of the disease or physiological condition being treated and will be at the discretion of the physician.

It will be appreciated that Polθ inhibitors can be used as a single agent or in combination with other anticancer agents. Combination experiments can be performed, for example, as described in Chou TC, Talalay P. Quantitative analysis of dose-effect relationships: the combined effects of multiple drugs or enzyme inhibitors. Adv Enzyme Regulat 1984;22: 27-55.

The compounds as defined herein can be administered as the sole therapeutic agent or they can be administered in combination therapy with one of more other compounds (or therapies) for treatment of a particular disease state, for example a neoplastic disease such as a cancer as hereinbefore defined. For the treatment of the above conditions, the compounds of the invention may be advantageously employed in combination with one or more other medicinal agents, more particularly, with other anti-cancer agents or adjuvants (supporting agents in the therapy) in cancer therapy. Examples of other therapeutic agents or treatments that may be administered together (whether concurrently or at different time intervals) with the compounds of the formula (I) include but are not limited to:

- Topoisomerase I inhibitors;
- Antimetabolites;
- Tubulin targeting agents;
- DNA binder and topoisomerase II inhibitors;
- Alkylating Agents;
- Monoclonal Antibodies;
- Anti-Hormones;
- Signal Transduction Inhibitors;
- Proteasome Inhibitors;
- DNA methyl transferase inhibitors;
- Cytokines and retinoids;
- Chromatin targeted therapies;
- Radiotherapy; and
- Other therapeutic or prophylactic agents.

Particular examples of anti-cancer agents or adjuvants (or salts thereof), include but are not limited to any of the agents selected from groups (i)-(xlvi), and optionally group (xlvii), below:

(i) Platinum compounds, for example cisplatin (optionally combined with amifostine), carboplatin or oxaliplatin;

(ii) Taxane compounds, for example paclitaxel, paclitaxel protein bound particles (Abraxane™), docetaxel, cabazitaxel or larotaxel;

(iii) Topoisomerase I inhibitors, for example camptothecin compounds, for example camptothecin, irinotecan(CPT11), SN-38, or topotecan;

(iv) Topoisomerase II inhibitors, for example anti-tumour epipodophyllotoxins or podophyllotoxin derivatives for example etoposide, or teniposide;

(v) Vinca alkaloids, for example vinblastine, vincristine, liposomal vincristine (Onco-TCS), vinorelbine, vindesine, vinflunine or vinvesir;

(vi) Nucleoside derivatives, for example 5-fluorouracil (5-FU, optionally in combination with leucovorin), gemcitabine, capecitabine, tegafur, UFT, S1, cladribine, cytarabine (Ara-C, cytosine arabinoside), fludarabine, clofarabine, or nelarabine;

(vii) Antimetabolites, for example clofarabine, aminopterin, or methotrexate, azacitidine, cytarabine, floxuridine, pentostatin, thioguanine, thiopurine, 6-mercaptopurine, or hydroxyurea (hydroxycarbamide);

(viii) Alkylating agents, such as nitrogen mustards or nitrosourea, for example cyclophosphamide, chlorambucil, carmustine (BCNU), bendamustine, thiotepa, melphalan, treosulfan, lomustine (CCNU), altretamine, busulfan, dacarbazine, estramustine, fotemustine, ifosfamide (optionally in combination with mesna), pipobroman, procarbazine, streptozocin, temozolomide, uracil, mechlorethamine, methylcyclohexylchloroethylnitrosurea, or nimustine (ACNU);

(ix) Anthracyclines, anthracenediones and related drugs, for example daunorubicin, doxorubicin (optionally in combination with dexrazoxane), liposomal formulations of doxorubicin (eg. Caelyx™, Myocet™, Doxil™), idarubicin, mitoxantrone, epirubicin, amsacrine, or valrubicin;

(x) Epothilones, for example ixabepilone, patupilone, BMS-310705, KOS-862 and ZK-EPO, epothilone A, epothilone B, desoxyepothilone B (also known as epothilone D or KOS-862), aza-epothilone B (also known as BMS-247550), aulimalide, isolaulimalide, or luetherobin;

(xi) DNA methyl transferase inhibitors, for example temozolomide, azacytidine or decitabine, or SGI-110;

(xii) Antifolates, for example methotrexate, pemetrexed disodium, or raltitrexed;

(xiii) Cytotoxic antibiotics, for example antinomycin D, bleomycin, mitomycin C, dactinomycin, carminomycin, daunomycin, levamisole, plicamycin, or mithramycin;

(xiv) Tubulin-binding agents, for example combrestatin, colchicines or nocodazole;

(xv) Signal Transduction inhibitors such as Kinase inhibitors (e.g. EGFR (epithelial growth factor receptor) inhibitors, VEGFR (vascular endothelial growth factor receptor) inhibitors, PDGFR (platelet-derived growth factor receptor) inhibitors, MTKI (multi target kinase inhibitors), Raf inhibitors, mTOR inhibitors for example imatinib mesylate, erlotinib, gefitinib, dasatinib, lapatinib, dovotinib, axitinib, nilotinib, vandetanib, vatalinib, pazopanib, sorafenib, sunitinib, temsirolimus, everolimus (RAD 001), vemurafenib (PLX4032/RG7204), dabrafenib, encorafenib or an IκB kinase inhibitor such as SAR-113945, bardoxolone, BMS-066, BMS-345541, IMD-0354, IMD-2560, or IMD-1041, or MEK inhibitors such as Selumetinib (AZD6244) and Trametinib (GSK121120212);

(xvi) Aurora kinase inhibitors for example AT9283, barasertib (AZD1152), TAK-901, MK0457 (VX680), cenisertib (R-763), danusertib (PHA-739358), alisertib (MLN-8237), or MP-470;

(xvii) CDK inhibitors for example AT7519, roscovitine, seliciclib, alvocidib (flavopiridol), dinaciclib (SCH-727965), 7-hydroxy-staurosporine (UCN-01), JNJ-7706621, BMS-387032 (a.k.a. SNS-032), PHA533533, PD332991, ZK-304709, or AZD-5438;

(xviii) PKA/B inhibitors and PKB (akt) pathway inhibitors for example AKT inhibitors such as KRX-0401 (perifosine/NSC 639966), ipatasertib (GDC-0068; RG-7440), afuresertib (GSK-2110183; 2110183), MK-2206, MK-8156, AT13148, AZD-5363, triciribine phosphate (VQD-002; triciribine phosphate monohydrate (API-2; TCN-P; TCN-PM; VD-0002), RX-0201, NL-71-101, SR-13668, PX-316, AT13148, AZ-5363, Semaphore, SF1126, or Enzastaurin HCl (LY317615) or MTOR inhibitors such as rapamycin analogues such as RAD 001 (everolimus), CCI 779 (temsirolemus), AP23573 and ridaforolimus, sirolimus (originally known as rapamycin), AP23841 and AP23573, calmodulin inhibitors e.g. CBP-501 (forkhead translocation inhibitors), enzastaurin HCl (LY317615) or PI3K Inhibitors such as dactolisib (BEZ235), buparlisib (BKM-120; NVP-BKM-120), BYL719, copanlisib (BAY-80-6946), ZSTK-474, CUDC-907, apitolisib (GDC-0980; RG-7422), pictilisib (pictrelisib, GDC-0941, RG-7321), GDC-0032, GDC-0068, GSK-2636771, idelalisib (formerly CAL-101, GS 1101, GS-1101), MLN1117 (INK1117), MLN0128 (INK128), IPI-145 (INK1197), LY-3023414, ipatasertib, afuresertib, MK-2206, MK-8156, LY-3023414, LY294002, SF1126 or PI-103, or sonolisib (PX-866);

(xix) Hsp90 inhibitors for example AT13387, herbimycin, geldanamycin (GA), 17-allylamino-17-desmethoxygeldanamycin (17-AAG) e.g. NSC-330507, Kos-953 and CNF-1010, 17-dimethylaminoethylamino-17-demethoxygeldanamycin hydrochloride (17-DMAG) e.g. NSC-707545 and Kos-1022, NVP-AUY922 (VER-52296), NVP-BEP800, CNF-2024 (BIIB-021 an oral purine), ganetespib (STA-9090), SNX-5422 (SC-102112) or IPI-504;

(xx) Monoclonal Antibodies (unconjugated or conjugated to radioisotopes, toxins or other agents), antibody derivatives and related agents, such as anti-CD, anti-VEGFR, anti-HER2, anti-CTLA4, anti-PD-1 or anti-EGFR antibodies, for example rituximab (CD20), ofatumumab (CD20), ibritumomab tiuxetan (CD20), GA101 (CD20), tositumomab (CD20), epratuzumab (CD22), lintuzumab (CD33), gemtuzumab ozogamicin (CD33), alemtuzumab (CD52), galiximab (CD80), trastuzumab (HER2 antibody), pertuzumab (HER2), trastuzumab-DM1 (HER2), ertumaxomab (HER2 and CD3), cetuximab (EGFR), panitumumab (EGFR), necitumumab (EGFR), nimotuzumab (EGFR), bevacizumab (VEGF), catumaxumab (EpCAM and CD3), abagovomab (CA125), farletuzumab (folate receptor), elotuzumab (CS1), denosumab (RANK ligand), figitumumab (IGF1R), CP751,871 (IGF1R), mapatumumab (TRAIL receptor), metMAB (met), mitumomab (GD3 ganglioside), naptumomab estafenatox (5T4), siltuximab (IL6), or immunomodulating agents such as CTLA-4 blocking antibodies and/or antibodies against PD-1 and PD-L1 and/or PD-L2 for example ipilimumab (CTLA4), MK-3475 (pembrolizumab, formerly lambrolizumab, anti-PD-1), nivolumab (anti-PD-1), BMS-936559 (anti- PD-L1), MPDL320A, AMP-514 or MEDI4736 (anti-PD-L1), or tremelimumab (formerly ticilimumab, CP-675,206, anti-CTLA-4);
(xxi) Estrogen receptor antagonists or selective estrogen receptor modulators (SERMs) or inhibitors of estrogen synthesis, for example tamoxifen, fulvestrant, toremifene, droloxifene, faslodex, or raloxifene;
(xxii) Aromatase inhibitors and related drugs, such as exemestane, anastrozole, letrazole, testolactone aminoglutethimide, mitotane or vorozole;
(xxiii) Antiandrogens (i.e. androgen receptor antagonists) and related agents for example bicalutamide, nilutamide, flutamide, cyproterone, or ketoconazole;
(xxiv) Hormones and analogues thereof such as medroxyprogesterone, diethylstilbestrol (a.k.a. diethylstilboestrol) or octreotide;
(xxv) Steroids for example dromostanolone propionate, megestrol acetate, nandrolone (decanoate, phenpropionate), fluoxymestrone or gossypol,
(xxvi) Steroidal cytochrome P450 17alpha-hydroxylase-17,20-lyase inhibitor (CYP17), e.g. abiraterone;
(xxvii) Gonadotropin releasing hormone agonists or antagonists (GnRAs) for example abarelix, goserelin acetate, histrelin acetate, leuprolide acetate, triptorelin, buserelin, or deslorelin;
(xxviii) Glucocorticoids, for example prednisone, prednisolone, dexamethasone;
(xxix) Differentiating agents, such as retinoids, rexinoids, vitamin D or retinoic acid and retinoic acid metabolism blocking agents (RAMBA) for example accutane, alitretinoin, bexarotene, or tretinoin;
(xxx) Farnesyltransferase inhibitors for example tipifarnib;
(xxxi) Chromatin targeted therapies such as histone deacetylase (HDAC) inhibitors for example panobinostat, resminostat, abexinostat, vorinostat, romidepsin, belinostat, entinostat, quisinostat, pracinostat, tefinostat, mocetinostat, givinostat, CUDC-907, CUDC-101, ACY-1215, MGCD-290, EVP-0334, RG-2833, 4SC-202, romidepsin, AR-42 (Ohio State University), CG-200745, valproic acid, CKD-581, sodium butyrate, suberoylanilide hydroxamide acid (SAHA), depsipeptide (FR 901228), dacinostat (NVP-LAQ824), R306465/ JNJ-16241199, JNJ-26481585, trichostatin A, chlamydocin, A-173, JNJ-MGCD-0103, PXD-101, or apicidin;
(xxxii) Proteasome Inhibitors for example bortezomib, carfilzomib, delanzomib (CEP-18770), ixazomib (MLN-9708), oprozomib (ONX-0912) or marizomib;
(xxxiii) Photodynamic drugs for example porfimer sodium or temoporfin;
(xxxiv) Marine organism-derived anticancer agents such as trabectidin;
(xxxv) Radiolabelled drugs for radioimmunotherapy for example with a beta particle-emitting isotope (e.g. , Iodine -131, Yittrium -90) or an alpha particle-emitting isotope (e.g., Bismuth-213 or Actinium-225) for example ibritumomab or Iodine tositumomab;
(xxxvi) Telomerase inhibitors for example telomestatin;
(xxxvii) Matrix metalloproteinase inhibitors for example batimastat, marimastat, prinostat or metastat;
(xxxviii) Recombinant interferons (such as interferon-$\gamma$ and interferon $\alpha$) and interleukins (e.g. interleukin 2), for example aldesleukin, denileukin diftitox, interferon alfa 2a, interferon alfa 2b, or peginterferon alfa 2b;
(xxxix) Selective immunoresponse modulators for example thalidomide, or lenalidomide;
(xl) Therapeutic Vaccines such as sipuleucel-T (Provenge) or OncoVex;
(xli) Cytokine-activating agents include Picibanil, Romurtide, Sizofiran, Virulizin, or Thymosin;
(xlii) Arsenic trioxide;
(xliii) Inhibitors of G-protein coupled receptors (GPCR) for example atrasentan;
(xliv) Enzymes such as L-asparaginase, pegaspargase, rasburicase, or pegademase;
(xlv) DNA repair inhibitors such as PARP inhibitors for example, olaparib, velaparib, iniparib, rucaparib (AG-014699 or PF-01367338), talazoparib or AG-014699;
(xlvi) DNA damage response inhibitors such as ATM inhibitors AZD0156 MS3541, ATR inhibitors AZD6738, M4344, M6620 wee1 inhibitor AZD1775;
(xlvii) Agonists of Death receptor (e.g. TNF-related apoptosis inducing ligand (TRAIL) receptor), such as mapatumumab (formerly HGS-ETR1), conatumumab (formerly AMG 655), PRO95780, lexatumumab, dulanermin, CS-1008, apomab or recombinant TRAIL ligands such as recombinant Human TRAIL/Apo2 Ligand;
(xlviii) Prophylactic agents (adjuncts); i.e. agents that reduce or alleviate some of the side effects associated with chemotherapy agents, for example

- anti-emetic agents,
- agents that prevent or decrease the duration of chemotherapy-associated neutropenia and prevent complications that arise from reduced levels of platelets, red blood cells or white blood cells, for example interleukin-11 (e.g. oprelvekin), erythropoietin (EPO) and analogues thereof (e.g. darbepoetin alfa), colony-stimulating factor

analogs such as granulocyte macrophage-colony stimulating factor (GM-CSF) (e.g. sargramostim), and granulocyte-colony stimulating factor (G-CSF) and analogues thereof (e.g. filgrastim, pegfilgrastim),
- agents that inhibit bone resorption such as denosumab or bisphosphonates e.g. zoledronate, zoledronic acid, pamidronate and ibandronate,
- agents that suppress inflammatory responses such as dexamethasone, prednisone, and prednisolone,
- agents used to reduce blood levels of growth hormone and IGF-I (and other hormones) in patients with acromegaly or other rare hormone-producing tumours, such as synthetic forms of the hormone somatostatin e.g. octreotide acetate,
- antidote to drugs that decrease levels of folic acid such as leucovorin, or folinic acid,
- agents for pain e.g. opiates such as morphine, diamorphine and fentanyl,
- non-steroidal anti-inflammatory drugs (NSAID) such as COX-2 inhibitors for example celecoxib, etoricoxib and lumiracoxib,
- agents for mucositis e.g. palifermin,
- agents for the treatment of side-effects including anorexia, cachexia, oedema or thromoembolic episodes, such as megestrol acetate.

In one embodiment the anticancer is selected from recombinant interferons (such as interferon-$\gamma$ and interferon $\alpha$) and interleukins (e.g. interleukin 2), for example aldesleukin, denileukin diftitox, interferon alfa 2a, interferon alfa 2b, or peginterferon alfa 2b; interferon-$\alpha$2 (500 $\mu$/ml) in particular interferon-$\beta$; and signal transduction inhibitors such as kinase inhibitors (e.g. EGFR (epithelial growth factor receptor) inhibitors, VEGFR (vascular endothelial growth factor receptor) inhibitors, PDGFR (platelet-derived growth factor receptor) inhibitors, MTKI (multi target kinase inhibitors), Raf inhibitors, mTOR inhibitors for example imatinib mesylate, erlotinib, gefitinib, dasatinib, lapatinib, dovotinib, axitinib, nilotinib, vandetanib, vatalinib, pazopanib, sorafenib, sunitinib, temsirolimus, everolimus (RAD 001), vemurafenib (PLX4032/RG7204), dabrafenib, encorafenib or an I$\kappa$B kinase inhibitor such as SAR-113945, bardoxolone, BMS-066, BMS-345541, IMD-0354, IMD-2560, or IMD-1041, or MEK inhibitors such as Selumetinib (AZD6244) and Trametinib (GSK121120212), in particular Raf inhibitors (e.g. vemurafenib) or MEK inhibitors (e.g. trametinib).

Each of the compounds present in the combinations of the invention may be given in individually varying dose schedules and via different routes. As such, the posology of each of the two or more agents may differ: each may be administered at the same time or at different times. A person skilled in the art would know through his or her common general knowledge the dosing regimes and combination therapies to use. For example, the compound of the invention may be using in combination with one or more other agents which are administered according to their existing combination regimen. Examples of standard combination regimens are provided below.

The taxane compound is advantageously administered in a dosage of 50 to 400 mg per square meter (mg/$m^2$) of body surface area, for example 75 to 250 mg/$m^2$, particularly for paclitaxel in a dosage of about 175 to 250 mg/$m^2$ and for docetaxel in about 75 to 150 mg/$m^2$ per course of treatment.

The camptothecin compound is advantageously administered in a dosage of 0.1 to 400 mg per square meter (mg/$m^2$) of body surface area, for example 1 to 300 mg/$m^2$, particularly for irinotecan in a dosage of about 100 to 350 mg/$m^2$ and for topotecan in about 1 to 2 mg/$m^2$ per course of treatment.

The anti-tumour podophyllotoxin derivative is advantageously administered in a dosage of 30 to 300 mg per square meter (mg/$m^2$) of body surface area, for example 50 to 250mg/$m^2$, particularly for etoposide in a dosage of about 35 to 100 mg/$m^2$ and for teniposide in about 50 to 250 mg/$m^2$ per course of treatment.

The anti-tumour vinca alkaloid is advantageously administered in a dosage of 2 to 30 mg per square meter (mg/$m^2$) of body surface area, particularly for vinblastine in a dosage of about 3 to 12 mg/$m^2$ , for vincristine in a dosage of about 1 to 2 mg/$m^2$ , and for vinorelbine in dosage of about 10 to 30 mg/$m^2$ per course of treatment.

The anti-tumour nucleoside derivative is advantageously administered in a dosage of 200 to 2500 mg per square meter (mg/$m^2$) of body surface area, for example 700 to 1500 mg/$m^2$, particularly for 5-FU in a dosage of 200 to 500mg/$m^2$, for gemcitabine in a dosage of about 800 to 1200 mg/$m^2$ and for capecitabine in about 1000 to 2500 mg/$m^2$ per course of treatment.

The alkylating agents such as nitrogen mustard or nitrosourea is advantageously administered in a dosage of 100 to 500 mg per square meter (mg/$m^2$) of body surface area, for example 120 to 200 mg/$m^2$, particularly for cyclophosphamide in a dosage of about 100 to 500 mg/$m^2$ , for chlorambucil in a dosage of about 0.1 to 0.2 mg/kg, for carmustine in a dosage of about 150 to 200 mg/$m^2$ , and for lomustine in a dosage of about 100 to 150 mg/$m^2$ per course of treatment.

The anti-tumour anthracycline derivative is advantageously administered in a dosage of 10 to 75 mg per square meter (mg/$m^2$) of body surface area, for example 15 to 60 mg/$m^2$, particularly for doxorubicin in a dosage of about 40 to 75 mg/$m^2$, for daunorubicin in a dosage of about 25 to 45mg/$m^2$ , and for idarubicin in a dosage of about 10 to 15 mg/$m^2$ per course of treatment.

The antiestrogen agent is advantageously administered in a dosage of about 1 to 100 mg daily depending on the

particular agent and the condition being treated. Tamoxifen is advantageously administered orally in a dosage of 5 to 50 mg, particularly 10 to 20 mg twice a day, continuing the therapy for sufficient time to achieve and maintain a therapeutic effect. Toremifene is advantageously administered orally in a dosage of about 60mg once a day, continuing the therapy for sufficient time to achieve and maintain a therapeutic effect. Anastrozole is advantageously administered orally in a dosage of about 1mg once a day. Droloxifene is advantageously administered orally in a dosage of about 20-100mg once a day. Raloxifene is advantageously administered orally in a dosage of about 60mg once a day. Exemestane is advantageously administered orally in a dosage of about 25mg once a day.

Antibodies are advantageously administered in a dosage of about 1 to 5 mg per square meter ($mg/m^2$) of body surface area, or as known in the art, if different. Trastuzumab is advantageously administered in a dosage of 1 to 5 mg per square meter ($mg/m^2$) of body surface area, particularly 2 to $4mg/m^2$ per course of treatment.

Where the compound of the formula (I) is administered in combination therapy with one, two, three, four or more other therapeutic agents (particularly one or two, more particularly one), the compounds can be administered simultaneously or sequentially. In the latter case, the two or more compounds will be administered within a period and in an amount and manner that is sufficient to ensure that an advantageous or synergistic effect is achieved. When administered sequentially, they can be administered at closely spaced intervals (for example over a period of 5-10 minutes) or at longer intervals (for example 1, 2, 3, 4 or more hours apart, or even longer periods apart where required), the precise dosage regimen being commensurate with the properties of the therapeutic agent(s). These dosages may be administered for example once, twice or more per course of treatment, which may be repeated for example every 7, 14, 21 or 28 days.

In one embodiment is provided a compound of formula (I) for the manufacture of a medicament for use in therapy wherein said compound is used in combination with one, two, three, or four other therapeutic agents. In another embodiment is provided a medicament for treating cancer which comprises a compound of formula (I) wherein said medicament is used in combination with one, two, three, or four other therapeutic agents. The invention further provides use of a compound of formula (I) for the manufacture of a medicament for enhancing or potentiating the response rate in a patient suffering from a cancer where the patient is being treated with one, two, three, or four other therapeutic agents.

It will be appreciated that the particular method and order of administration and the respective dosage amounts and regimes for each component of the combination will depend on the particular other medicinal agent and compound of the present invention being administered, their route of administration, the particular tumour being treated and the particular host being treated. The optimum method and order of administration and the dosage amounts and regime can be readily determined by those skilled in the art using conventional methods and in view of the information set out herein.

The weight ratio of the compound according to the present invention and the one or more other anticancer agent(s) when given as a combination may be determined by the person skilled in the art. Said ratio and the exact dosage and frequency of administration depends on the particular compound according to the invention and the other anticancer agent(s) used, the particular condition being treated, the severity of the condition being treated, the age, weight, gender, diet, time of administration and general physical condition of the particular patient, the mode of administration as well as other medication the individual may be taking, as is well known to those skilled in the art. Furthermore, it is evident that the effective daily amount may be lowered or increased depending on the response of the treated subject and/or depending on the evaluation of the physician prescribing the compounds of the instant invention. A particular weight ratio for the present compound of formula (I) and another anticancer agent may range from 1/10 to 10/1, more in particular from 1/5 to 5/1, even more in particular from 1/3 to 3/1.

The compounds of the invention may also be administered in conjunction with non-chemotherapeutic treatments such as radiotherapy, photodynamic therapy, gene therapy; surgery and controlled diets.

The compounds of the present invention also have therapeutic applications in sensitising tumour cells for radiotherapy and chemotherapy. Hence the compounds of the present invention can be used as "radiosensitizer" and/or "chemosensitizer" or can be given in combination with another "radiosensitizer" and/or "chemosensitizer". In one embodiment the compound of the invention is for use as chemosensitiser.

The term "radiosensitizer" is defined as a molecule administered to patients in therapeutically effective amounts to increase the sensitivity of the cells to ionizing radiation and/or to promote the treatment of diseases which are treatable with ionizing radiation.

The term "chemosensitizer" is defined as a molecule administered to patients in therapeutically effective amounts to increase the sensitivity of cells to chemotherapy and/or promote the treatment of diseases which are treatable with chemotherapeutics.

In one embodiment the compound of the invention is administered with a "radiosensitizer" and/or "chemosensitizer". In one embodiment the compound of the invention is administered with an "immune sensitizer".

The term "immune sensitizer" is defined as a molecule administered to patients in therapeutically effective amounts to increase the sensitivity of cells to a Polθ inhibitor.

Many cancer treatment protocols currently employ radiosensitizers in conjunction with radiation of x-rays. Examples of x-ray activated radiosensitizers include, but are not limited to, the following: metronidazole, misonidazole, desmethylmisonidazole, pimonidazole, etanidazole, nimorazole, mitomycin C, RSU 1069, SR 4233, EO9, RB 6145,

nicotinamide, 5-bromodeoxyuridine (BUdR), 5- iododeoxyuridine (IUdR), bromodeoxycytidine, fluorodeoxyuridine (FudR), hydroxyurea, cisplatin, and therapeutically effective analogs and derivatives of the same.

Photodynamic therapy (PDT) of cancers employs visible light as the radiation activator of the sensitizing agent. Examples of photodynamic radiosensitizers include the following, but are not limited to: hematoporphyrin derivatives, Photofrin, benzoporphyrin derivatives, tin etioporphyrin, pheoborbide-a, bacteriochlorophyll-a, naphthalocyanines, phthalocyanines, zinc phthalocyanine, and therapeutically effective analogs and derivatives of the same.

Radiosensitizers may be administered in conjunction with a therapeutically effective amount of one or more other compounds, including but not limited to: compounds of the invention; compounds which promote the incorporation of radiosensitizers to the target cells; compounds which control the flow of therapeutics, nutrients, and/or oxygen to the target cells; chemotherapeutic agents which act on the tumour with or without additional radiation; or other therapeutically effective compounds for treating cancer or other diseases.

Chemosensitizers may be administered in conjunction with a therapeutically effective amount of one or more other compounds, including but not limited to: compounds of the invention; compounds which promote the incorporation of chemosensitizers to the target cells; compounds which control the flow of therapeutics, nutrients, and/or oxygen to the target cells; chemotherapeutic agents which act on the tumour or other therapeutically effective compounds for treating cancer or other disease. Calcium antagonists, for example verapamil, are found useful in combination with antineoplastic agents to establish chemosensitivity in tumor cells resistant to accepted chemotherapeutic agents and to potentiate the efficacy of such compounds in drug-sensitive malignancies.

Examples of immune sensitizers include the following, but are not limited to: immunomodulating agents, for example monoclonal antibodies such as immune checkpoint antibodies [e.g. CTLA-4 blocking antibodies and/or antibodies against PD-1 and PD-L1 and/or PD-L2 for example ipilimumab (CTLA4), MK-3475 (pembrolizumab, formerly lambrolizumab, anti-PD-1), nivolumab (anti-PD-1), BMS-936559 (anti- PD-L1), MPDL320A, AMP-514 or MEDI4736 (anti-PD-L1), or tremelimumab (formerly ticilimumab, CP-675,206, anti-CTLA-4)]; or Signal Transduction inhibitors; or cytokines (such as recombinant interferons); or oncolytic viruses; or immune adjuvants (e.g. BCG).

Immune sensitizers may be administered in conjunction with a therapeutically effective amount of one or more other compounds, including but not limited to: compounds of the invention; compounds which promote the incorporation of immune sensitizers to the target cells; compounds which control the flow of therapeutics, nutrients, and/or oxygen to the target cells; therapeutic agents which act on the tumour or other therapeutically effective compounds for treating cancer or other disease.

For use in combination therapy with another chemotherapeutic agent, the compound of the formula (I) and one, two, three, four or more other therapeutic agents can be, for example, formulated together in a dosage form containing two, three, four or more therapeutic agents i.e. in a unitary pharmaceutical composition containing all agents. In an alternative embodiment, the individual therapeutic agents may be formulated separately and presented together in the form of a kit, optionally with instructions for their use.

In one embodiment is provided a combination of a compound of formula (I) with one or more (e.g. 1 or 2) other therapeutic agents (e.g. anticancer agents as described above). In a further embodiment is provided a combination of a Polθ inhibitor as described herein and a PI3K/AKT pathway inhibitor selected from: apitolisib, buparlisib, Copanlisib, pictilisib, ZSTK-474, CUDC-907, GSK-2636771, LY-3023414, ipatasertib, afuresertib, MK-2206, MK-8156, Idelalisib, BEZ235 (dactolisib), BYL719, GDC- 0980, GDC-0941, GDC-0032 and GDC-0068.

In another embodiment is provided a compound of formula (I) in combination with one or more (e.g. 1 or 2) other therapeutic agents (e.g. anticancer agents) for use in therapy, such as in the prophylaxis or treatment of cancer.

In one embodiment the pharmaceutical composition comprises a compound of formula (I) together with a pharmaceutically acceptable carrier and optionally one or more therapeutic agent(s).

In another embodiment the invention relates to the use of a combination according to the invention in the manufacture of a pharmaceutical composition for inhibiting the growth of tumour cells.

In a further embodiment the invention relates to a product containing a compound of formula (I) and one or more anticancer agent, as a combined preparation for simultaneous, separate or sequential use in the treatment of patients suffering from cancer.

## EXAMPLES

The invention will now be illustrated, but not limited, by reference to the specific embodiments described in the following examples.

### Abbreviations

| | |
|---|---|
| aq. | Aqueous |
| Boc | tert-Butyloxycarbonyl |
| Cbz | Benzyloxycarbonyl |
| DCE | 1,2-Dichloroethane |
| DCM | Dichloromethane |
| DIPEA | N,N-Diisopropylethylamine |
| DMAP | 4-Dimethylaminopyridine |
| DMF | Dimethylformamide |
| DMSO | Dimethylsulfoxide |
| EtOH | Ethanol |
| EtOAc | Ethyl acetate |
| FA | Formic acid |
| Fmoc | Fluorenylmethoxycarbonyl |
| h | hour(s) |
| HATU | 1-[Bis(dimethylamino)methylene]-1H-1,2,3-triazolo[4,5-b]pyridinium 3-oxide hexafluorophosphate |
| HPLC | High-performance liquid chromatography |
| MeCN | Acetonitrile |
| MeOH | Methanol |
| min | minutes |
| NMO | 4-Methylmorpholine 4-oxide |
| NMP | N-Methylpyrrolidinone |
| NMR | Nuclear magnetic resonance |
| Pd/C | Palladium on carbon |
| PE | Petroleum ether |
| rt | Room temperature or ambient temperature |
| sat. | Saturated solution |
| SFC | Supercritical Fluid Chromatography |
| TBAB | Tetrabutylammonium bromide |
| TBAF | Tetra-n-butylammonium fluoride |
| TEA | Triethylamine |
| TFA | Trifluoroacetic acid |
| THF | Tetrahydrofuran |
| TLC | Thin layer chromatography |
| TsOH | p-Toluenesulfonic acid monohydrate |
| XPhos-Pd-G2 | Chloro(2-dicyclohexylphosphino-2',4',6'-triisopropyl-1,1'-biphenyl)[2-(2'-amino-1,1'-biphenyl)]palladium(II) |

**Typical Preparative HPLC method**

For purification of samples by HPLC the following columns were typically used; SunFire C18, Xtimate C18, Phenomenex Gemini, Phenomenex Synergi C18, Phenomenex Luna, Waters Xbridge C18, Boston Prime C18 and Shimpack C18. Typical mobile phases used were water and MeCN, with either acidic or basic additives, such as formic acid (0.1% v/v) or ammonium hydroxide (0.05% v/v). A typical method started with 95% water:5% MeCN and decreasingly polar ratios of water and MeCN over a period of 5 to 12 min, with a typical flow rate of 25 mL/min. For mass-directed HPLC the typical mass spectrometer used was a Waters 3100 which detected masses between 100 and 700 g/mol.

**Intermediate 1: 2-Chloro-6-methyl-4-(trifluoromethyl)nicotinonitrile**

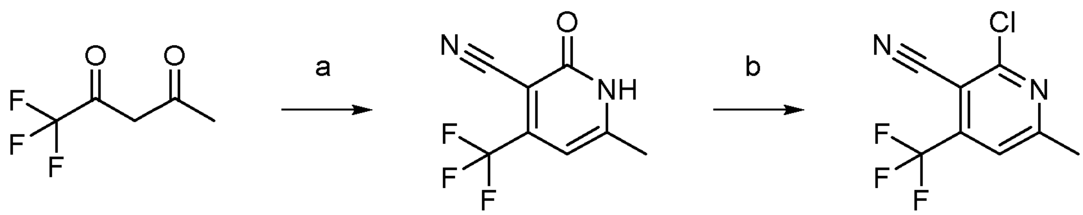

**Step a.** To a solution of 1,1,1-trifluoropentane-2,4-dione (25 g, 162 mmol) and 2-cyano-acetamide (15 g, 178 mmol) in EtOH (200 mL) was added diethylamine (5.93 g, 81.1 mmol). The mixture was stirred at 70 °C for 12 h. On completion, a solid precipitated from EtOH, which was collected by filtration and dried under vacuum to give 2-hydroxy-6-methyl-4-(trifluoromethyl)pyridine-3-carbonitrile (24.5 g, 121 mmol, crude) as a white solid.

m/z ES+ $[M+H]^+$ 203.0; $^1H$ NMR (400 MHz, DMSO-$d_6$) δ ppm 6.51 (s, 1H), 2.33 (s, 3H).

**Step b.** 2-Hydroxy-6-methyl-4-(trifluoromethyl)pyridine-3-carbonitrile (24.5 g, 121 mmol) was carefully added to $POCl_3$ (400 mL) at rt and the resultant solution was stirred at 110 °C for 12 h. Upon completion, $POCl_3$ was carefully removed by distillation to afford a residue, which was purified by column chromatography (PE to PE/EtOAc = 4/1) to give the title compound (24.5 g, 92% yield) as a pale yellow oil.

$^1H$ NMR (400 MHz, $CDCl_3$) δ ppm 7.50 (s, 1H), 2.73 (s, 3H).

**Intermediate 2a: tert-Butyl (2-amino-3-fluorophenyl)carbamate**

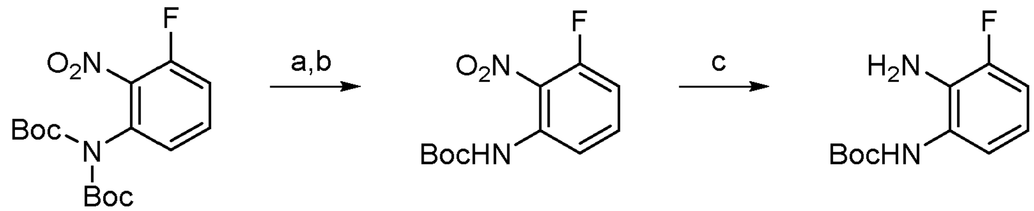


**Step a.** A solution of 3-fluoro-2-nitroaniline (200 g, 1.28 mol) in THF (2.0 L) was cooled to 0 °C. TEA (532 mL, 3.84 mol) and DMAP (78.0 g, 640 mmol) was added to the reaction mixture and stirred for 30 min. Di-*tert*-butyl dicarbonate (677 mL, 2.95 mol) was added drop wise to the reaction mixture and allowed to stir at rt for 12 h. Upon completion, the reaction mixture was partitioned between sat. aq. $NH_4Cl$ solution (2.0 L) and EtOAc (1.5 L). The aqueous layer was further extracted with EtOAc (2 x 500 mL) and dried over $Na_2SO_4$. The combined organic layer was evaporated to give crude *tert-butyl* (*tert*-butoxycarbonyl)(3-fluoro-2-nitrophenyl)carbamate as a brown oil (450 g, quantitative) which was used in the next step without purification.

**Step b.** TFA (193 mL, 2.53 mol) was added to a solution of *tert-butyl* (*tert*-butoxycarbonyl)(3-fluoro-2-nitrophenyl) carbamate (450 g, 1.26 mol) in DCM (4.50 L) at 0 °C. The reaction mixture was warmed to rt and stirred for 24 h. Upon completion, the reaction mixture was partitioned between sat. aq. $NH_4Cl$ solution (1.50 L) and DCM (1.50 L). The aqueous layer was further extracted with DCM (2 x 500 mL) and dried over $Na_2SO_4$. The combined organic layer was evaporated under vacuum and the crude product was triturated with hexane to give tert-butyl (3-fluoro-2-nitrophenyl)carbamate (250 g, 77% yield) as a brown solid.

$^1H$ NMR (400 MHz, DMSO-$d_6$) δ ppm 9.82 (s, 1H), 7.64 - 7.56 (m, 1H), 7.31 - 7.25 (m, 2H), 1.42(s, 9H).

**Step c.** To a solution of tert-butyl (3-fluoro-2-nitrophenyl) carbamate (250g, 976 mmol) in EtOH (1.5 L) and water (1.0 L), $NH_4Cl$ (522 g, 9.76 mol) was added and stirred for 15 min. Iron powder (436 g, 7.80 mol) was added to reaction mixture and stirred at 80 °C for 12 h. Upon completion, the reaction mixture was filtered through a bed of celite and washed with EtOAc (3 x 300 mL). The organic solvent was evaporated under vacuum and the residue partitioned between water (1.5 L) and EtOAc (1.2 L). The aqueous layer was further extracted by EtOAc (2 x 500 mL) and dried over $Na_2SO_4$. The combined organic layer was evaporated under vacuum and further purified by column chromatography (hexanes to hexanes/EtOAc = 4/1) to give the title compound (190 g, 86% yield) as a white solid.

m/z ES+ $[M+H]^+$ 227.33; $^1H$ NMR (400 MHz, DMSO-$d_6$) δ ppm 8.52 (s, 1H), 7.10 (d, *J* = 7.6 Hz, 1H), 6.81 (m,1H), 6.52 (m, 1H), 4.81 (s, 2H), 1.46 (s, 9H).

**Intermediate 2b: tert-Butyl (2-amino-4-fluorophenyl)carbamate**

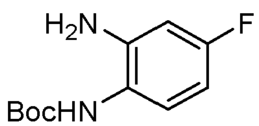

The title compound was prepared in a similar manner to **Intermediate 2a,** using 4-fluoro-2-nitroaniline in step a.

$^1H$ NMR (400 MHz, $CDCl_3$) δ ppm 7.16 - 7.11 (m, 1H), 6.54 - 6.44 (m, 2H), 6.03 (br s, 1H), 3.71 (br s, 2H), 1.45 (s,

9H).

**Intermediate 2c: tert-Butyl (2-amino-4-chlorophenyl)carbamate**

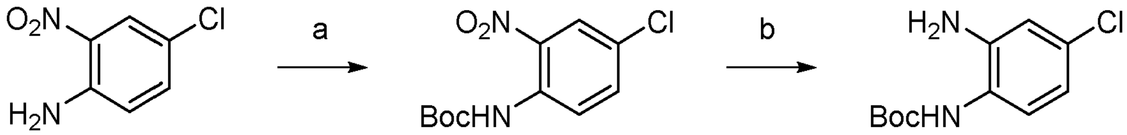


**Step a.** A solution of 4-chloro-2-nitro-aniline (10.0 g, 58.0 mmol) in DMF (100 mL) was treated with NaH (4.64 g, 116 mmol, 60% dispersion in mineral oil) at 0 °C and stirred for 30 min, before addition of di-tert-butyl dicarbonate (13.9 g, 63.7 mmol). The mixture was stirred at rt for 12 h. Upon completion, the reaction mixture was quenched with water (10 mL) at 0 °C, diluted with water (300 mL) and extracted with EtOAc (3 x 100 mL). The combined organic layers were washed with brine 150 mL (3 x 50 mL), dried over $Na_2SO_4$ and evaporated. The residue was triturated (PE/EtOAc = 20/1, 100mL) at rt for 30 min to give *tert*-butyl N-(4-chloro-2-nitro-phenyl)carbamate (12.0 g, 76% yield) as a yellow solid.

$^1H$ NMR (400 MHz, $CDCl_3$) δ ppm 9.61 (s, 1H), 8.59 (d, *J* = 9.2 Hz, 1H), 8.21 (d, *J* = 2.8 Hz, 1H), 7.59 - 7.56 (m, 1H), 1.56 (s, 9H).

**Step b.** A mixture of *tert-butyl* N-(4-chloro-2-nitro-phenyl)carbamate (9.0 g, 33.0 mmol), iron powder (5.53 g, 99.0 mmol), $NH_4Cl$ (17.6 g, 330 mmol) in EtOH (100 mL) and water (100 mL) was degassed and purged with $N_2$ for 3 times. The mixture was stirred at 60 °C for 2 h under $N_2$ atmosphere. Upon completion, the reaction mixture was filtered and evaporated. The crude product was triturated with water (50 mL) at rt for 30 min to give the title compound (7.00 g, 87% yield) as a yellow solid.

$^1H$ NMR (400 MHz, $CDCl_3$) δ ppm 7.20 (d, *J* = 8.0 Hz, 1H), 6.79 - 6.74 (m, 2H), 6.11 (br s, 1H), 3.83 (br s, 2H), 1.53 (s, 9H).

**Intermediate 3: (S)-2-((6-Allyl-7-fluoro-1-methyl-2-oxo-1,2,3,4,5,6-hexahydro-benzo[b][1,4]diazocin-3-yl)amino)-6-methyl-4-(trifluoromethyl)nicotinonitrile**

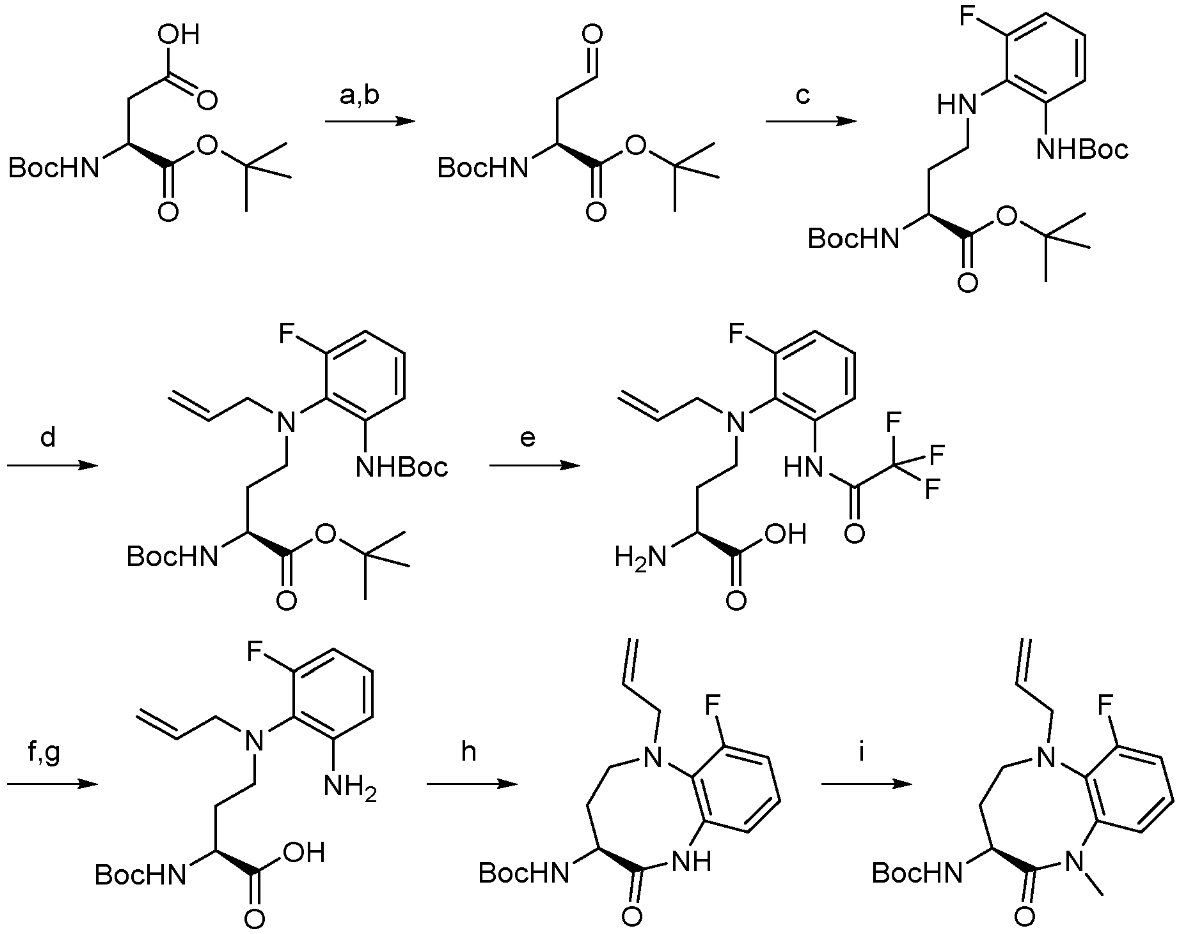


Step a. To a solution of (*S*)-4-(*tert*-butoxy)-3-((*tert*-butoxy carbonyl)amino)-4-oxobutanoic acid (CAS Number 34582-32-6; 400 g, 1.38 mol) in THF (4.0 L) at 0 °C, TEA (574 mL, 4.15 mol) was added dropwise and stirred for 10 min. Ethyl chloroformate (145 mL, 1.52 mol) was added to the reaction mixture by addition funnel at 0 °C and allowed to stir for 1 h. Upon completion, the solid was removed by filtration and the filtrate was added drop wise to a solution of $NaBH_4$ (131 g, 3.45 mol) in water (80 mL). The reaction mixture was allowed to warm from 0 °C to rt over 16 h. Upon completion, the reaction mixture was partitioned between water (3.0 L) and EtOAc (2.0 L). The aqueous layer was further extracted with EtOAc (2 x 500 mL) and dried over $Na_2SO_4$. The combined organic layer was evaporated under vacuum to obtain crude product which was purified by column chromatography (hexanes to hexanes/EtOAc = 3/1) to give *tert*-butyl (*tert*-butoxy carbonyl)-*L*-homoserinate as a pale yellow oil (250 g, 66% yield).

m/z ES+ $[M+H]^+$ 276.47; $^1H$ NMR (400 MHz, DMSO-$d_6$) δ ppm 7.05 (d, *J* = 4.8 Hz, 1H), 4.58 (t, *J* = 4.8 Hz, 1H), 3.93 - 3.88 (m, 1H), 3.45 - 3.34 (m, 2H), 1.79 - 1.60 (m, 2H), 1.41 (s, 9H), 1.38 (s, 9H).

**Step b.** To a solution *tert*-butyl (tert-butoxy carbonyl)-*L*-homoserinate (250 g, 908 mmol) in DCM (2.5 L) under $N_2$ atmosphere, Dess-Martin periodinane (385 g, 908 mmol) was added portion-wise at 0 °C. The reaction mixture was stirred at rt for 16 h. Upon completion, the reaction mixture was filtered through a pad of celite and washed with DCM (2 x 500 mL). The filtrate was collected and partitioned between water (3.0 L) and DCM (3.0 L). The aqueous was further extracted with DCM (2 x 500 mL) and dried over $Na_2SO_4$. The organic layer was evaporated under vacuum to give crude product which was further purified by column chromatography (hexanes to hexanes/EtOAc = 8/1) to give *tert*-butyl (*S*)-2-((*tert*-butoxycarbonyl)amino)-4-oxobutanoate as a yellow solid (200 g, 81% yield).

m/z ES+ $[M+H]^+$ 274.44.

**Step c.** To a solution of *tert-butyl* (*S*)-2-((*tert*-butoxy carbonyl)amino)-4-oxobutanoate (200 g, 732 mmol) and **Intermediate 2a** (166 g, 732 mmol) in DCM (2.0 L) was added 3 Å molecular sieves (20 g). The reaction mixture was cooled to 0 °C and acetic acid (20.9 mL, 366 mmol) was added to the reaction mixture and stirred at rt for 12 h. Sodium triacetoxyborohydride (233 g, 1.097 mol) was added to the reaction mixture and stirred for an additional 6 h. The reaction mixture was then partitioned between water (2.5 L) and DCM (1.5 L). The aqueous layer was further extracted with DCM (2 x 500 mL) and dried over $Na_2SO_4$. The combined organic layer was evaporated under vacuum to give crude tert-butyl (*S*)-2-((*tert*-butoxycarbonyl)amino)-4-((2-((*tert*-butoxycarbonyl)amino)-6-fluorophenyl)amino)butanoate as a yellow oil (260 g, 73%) which was used without purification in next step.

m/z ES+ $[M+H]^+$ 484.80.

**Step d.** A solution of *tert*-butyl (*S*)-2-((*tert*-butoxycarbonyl)amino)-4-((2-((tert-butoxycarbonyl)amino)-6-fluorophenyl)amino)butanoate (260 g, 538 mmol) in DMF (2.6 L) was cooled to 0 °C and $Na_2CO_3$ (228 g, 2.15 mol) was added. TBAF (1 M in THF; 54 mL, 54 mmol) was added drop-wise to the reaction mixture and stirred at 0 °C for 30 min. Allyl bromide (279 mL, 3.23 mol) was added drop-wise to the reaction mixture and stirred at 60 °C for 12 h. Upon completion, the reaction mixture was partitioned between water (2.0 L) and EtOAc (1.5 L). The aqueous layer was further extracted with EtOAc (2 x 500 mL) and dried over $Na_2SO_4$. The solvent was evaporated under vacuum and the crude was purified by column chromatography (hexanes to hexanes/EtOAc = 12/1) to give *tert*-butyl (*S*)-4-(allyl(2-((*tert*-butoxycarbonyl)amino)-6-fluorophenyl)amino)-2-((*tert*-butoxycarbonyl)amino)butanoate (125 g, 44% yield) as a pale yellow gummy solid.

$^1H$ NMR (400 MHz, $CDCl_3$) δ ppm 8.13-8.02 (br s, 1H), 7.90 (d, *J* = 8.0, 1H), 7.13 - 7.08 (m, 1H), 6.68 - 6.63 (m, 1H) 5.82 - 5.76 (m, 1H), 5.16 (d, *J* = 16.8Hz, 1H), 5.13 - 5.05 (m, 2H), 4.15 - 4.10 (m, 1H), 3.59 (br s, 2H), 3.07 (br s, 2H), 1.86 - 1.61 (m, 2H), 1.54 (s, 9H), 1.45 (s, 9H), 1.40 (s, 9H).

**Step e.** To a solution of *tert-butyl* (*S*)-4-(allyl(2-((*tert*-butoxycarbonyl)amino)-6-fluorophenyl)amino)-2-((*tert*-butoxycarbonyl)amino)butanoate (20.4 g, 39.0 mmol) in DCM (50 mL) was added TFA (120 mL). The mixture was stirred at rt for 20 h. Upon completion, the mixture was evaporated to give (*S*)-4-(allyl(2-fluoro-6-(2,2,2-trifluoroacetamido)phenyl)amino)-2-aminobutanoic acid as a TFA salt (18.5 g, crude) as a yellow gum.

m/z ES+ $[M+H]^+$ 363.9.

**Step f.** To a solution of (*S*)-4-(allyl(2-fluoro-6-(2,2,2-trifluoroacetamido)phenyl)amino)-2-aminobutanoic acid (TFA salt, 18.5 g, 38.8 mmol) in dioxane (120 mL) and water (120 mL) was added $NaHCO_3$ (9.77 g, 116 mmol) and di-*tert*-butyl dicarbonate (8.88 g, 40.7 mmol). The mixture was stirred at rt for 4 h. Upon completion, the reaction mixture was diluted with water (30 mL) and extracted with EtOAc (3 x 200 mL). The combined organic layers were washed with brine (300 mL), dried over $Na_2SO_4$ and evaporated to give (*S*)-4-(allyl(2-fluoro-6-(2,2,2-trifluoroacetamido)phenyl)amino)-2-((*tert*-butoxycarbonyl)amino)butanoic acid (16 g, crude) as a brown oil.

m/z ES+ $[M-tBu]^+$ 407.9.

**Step g.** To a solution of (*S*)-4-(allyl(2-fluoro-6-(2,2,2-trifluoroacetamido)phenyl)amino)-2-((*tert*-butoxycarbonyl)amino)butanoic acid (16.0 g, 34.5 mmol) in MeOH (160 mL) and water (80 mL) was added $K_2CO_3$ (14.3 g, 103 mmol). The mixture was stirred at rt for 12 h. Upon completion, the reaction mixture was diluted with water (100 mL) and extracted with EtOAc (3 x 200 mL). The organic layers were washed with brine (400 mL), dried over $Na_2SO_4$ and evaporated to give (S)-4-(allyl(2-amino-6-fluorophenyl)amino)-2-((*tert*-butoxycarbonyl)amino)butanoic acid (13.0 g, crude) as a yellow oil.

m/z ES+ $[M+H]^+$ 368.2.

**Step h.** To a solution of (*S*)-4-(allyl(2-amino-6-fluorophenyl)amino)-2-((*tert*-butoxycarbonyl)amino)butanoic acid (13.0 g, 35.38 mmol) in DCM (1000 mL) was added HATU (13.4 g, 35.3 mmol) and DIPEA (9.15 g, 70.7 mmol). The mixture was stirred at rt for 1 h. Upon completion, the reaction mixture was evaporated to give a residue. The residue was diluted with water (120 mL), extracted with DCM (3 x 150 mL). The combined organic layers were washed with brine (200 mL), dried over $Na_2SO_4$ and evaporated. The residue was purified by column chromatography (PE/EtOAc = 3/1) to give (*S*)-*tert*-butyl (6-allyl-7-fluoro-2-oxo-1,2,3,4,5,6-hexahydrobenzo[*b*][1,4]diazocin-3-yl)carbamate (10.8 g, 83% yield) as a white solid.

m/z ES+ $[M-tBu]^+$ 294.1; $^1H$ NMR (400 MHz, $CDCl_3$) δ ppm 7.40 (br s, 1H), 7.18 - 7.12 (m, 1H), 7.06 - 6.99 (m, 1H), 6.95 (d, *J* = 8.0 Hz, 1H), 5.77 - 5.64 (m, 1H), 5.52 (d, *J* = 6.4 Hz, 1H), 5.17 (dd, *J* = 1.6, 17.2 Hz, 1H), 5.01 (dd, *J* = 1.2, 10.0 Hz, 1H), 4.06 (t, *J* = 8.4 Hz, 1H), 3.76 - 3.65 (m, 1H), 3.64 - 3.55 (m, 1H), 3.33 - 3.21 (m, 1H), 3.11 - 2.99 (m, 1H), 2.11 - 2.00 (m, 1H), 1.78 (dd, *J* = 2.4, 13.6 Hz, 1H), 1.40 (s, 9H).

**Step i.** To a solution of (*S*)-tert-butyl (6-allyl-7-fluoro-2-oxo-1,2,3,4,5,6-hexahydrobenzo[*b*][1,4]diazocin-3-yl)carbamate (10.8 g, 30.9 mmol) and methyl iodide (17.5 g, 123 mmol) in DMF (100 mL) was added $K_2CO_3$ (12.8 g, 92.7 mmol). The mixture was stirred at rt for 12 h. Upon completion, the reaction mixture was diluted with water (300 mL) and extracted with EtOAc (4 x 200 mL). The combined organic layers were washed with water (2 x 150 mL) and brine (2 x 150 mL), dried over $Na_2SO_4$ and evaporated to give the title compound (10.7 g, crude) as a yellow oil.

m/z ES+ $[M-tBu]^+$ 307.8.

**Intermediate 4: (S)-2-((6-Allyl-7-fluoro-1-methyl-2-oxo-1,2,3,4,5,6-hexahydro-benzo[b][1,4]diazocin-3-yl)amino)-6-methyl-4-(trifluoromethyl)nicotinonitrile**

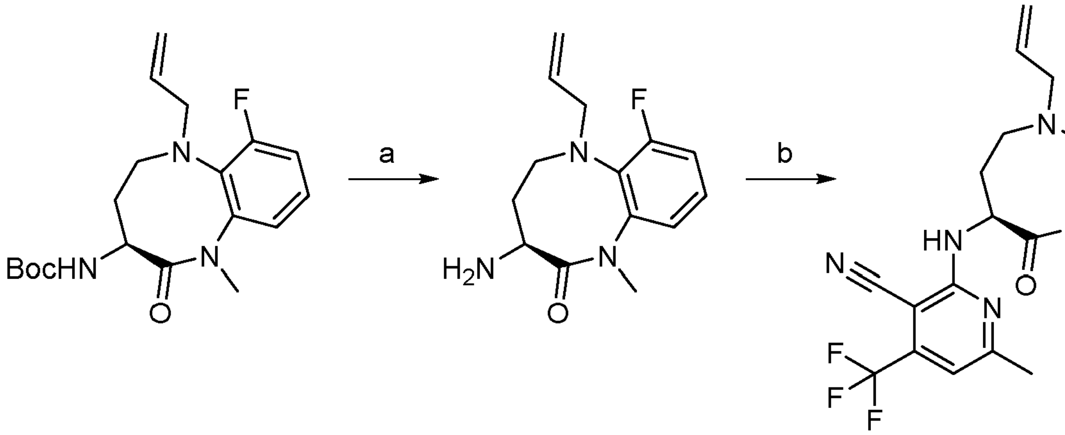


**Step a.** A solution of **Intermediate 3** (10.7 g, 29.4 mmol) in DCM (90 mL) was treated with TFA (27.7 g, 243 mmol) and stirred at rt for 3 h. Upon completion, the solution was adjusted to pH 8 with sat. aq. $NaHCO_3$ and extracted with EtOAc (3 x 150 mL). The combined organic layers were washed with brine (300 mL), dried over $Na_2SO_4$ and evaporated to give (S)-6-allyl-3-amino-7-fluoro-1-methyl-3,4,5,6-tetrahydrobenzo[*b*][1,4]diazocin-2(1H)-one (7.5 g, crude) as a yellow oil.

m/z ES+ $[M+H]^+$ 264.2.

**Step b.** To a solution of (*S*)-6-allyl-3-amino-7-fluoro-1-methyl-3,4,5,6-tetrahydrobenzo[*b*][1,4]diazocin-2(1H)-one (7.5 g, 28.4mmol) and **Intermediate 1** (6.60 g, 29.9 mmol) in NMP (40 mL) was added DIPEA (7.36 g, 56.9 mmol). The mixture was stirred at 100 °C for 1 h. Upon completion, the reaction mixture was diluted with water (150 mL) and extracted with EtOAc (3 x 150 mL). The combined organic layers were washed with water (2 x 100 mL) and brine (2 x 100 mL), dried over $Na_2SO_4$ and evaporated. The residue was purified by column chromatography (PE/EtOAc = 5/1) to give the title compound (10.0 g, 75% yield) as an off-white solid.

m/z ES+ $[M+H]^+$ 447.9; $^1$H NMR (400 MHz, $CDCl_3$) δ ppm 7.27 - 7.22 (m, 1H), 7.15 - 7.08 (m, 2H), 6.68 (s, 1H), 6.18 (d, *J = 7.2* Hz, 1H), 5.78 - 5.65 (m, 1H), 5.19 (dd, *J* = 1.6, 17.2 Hz, 1H), 5.06 (dd, *J* = 1.2, 10.0 Hz, 1H), 4.53 - 4.48 (m, 1H), 3.72 - 3.60 (m, 2H), 3.37 - 3.30 (m, 1H), 3.30 (s, 3H), 3.14 - 3.06 (m, 1H), 2.37 (s, 3H), 2.30 - 2.18 (m, 1H), 1.82 - 1.77 (m, 1H).

**Intermediate 5: (S)-2-((7-Fluoro-1-methyl-2-oxo-6-(2-oxoethyl)-1,2,3,4,5,6-hexahydrobenzo[b][1,4]diazocin-3-yl)amino)-6-methyl-4-(trifluoromethyl)nicotinonitrile**

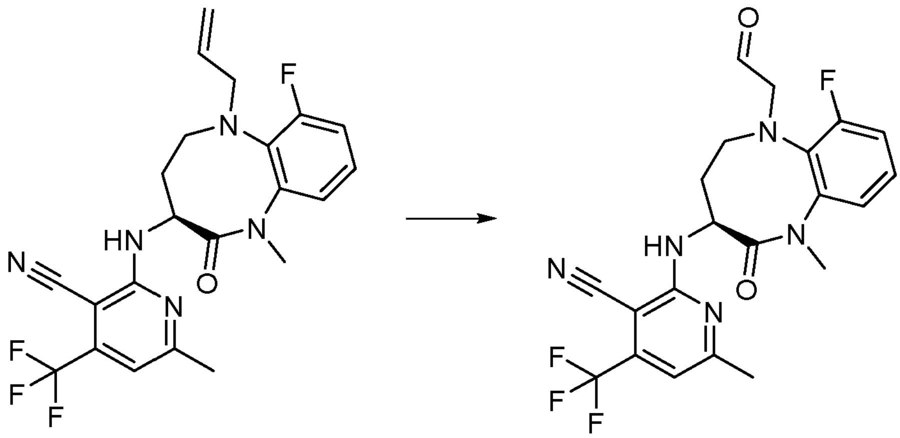

To a solution of **Intermediate** 4 (5 g, 11.1 mmol) in THF (50 mL) and water (20 mL) was added $NaIO_4$ (7.17 g, 33.5 mmol) and $OsO_4$ (284 mg, 1.12 mmol) at 0 °C. The mixture was stirred at 0 °C for 2 h. Upon completion, sat. aq. $Na_2S_2O_3$ (15 mL) was added to the mixture and was stirred at rt for 30 min. The mixture was extracted with EtOAc (3 x 50 mL). The combined organic layers were washed with sat. aq. $Na_2S_2O_3$ (2 x 50 mL), sat. aq. $NaHCO_3$ (2 x 50 mL) and brine (2 x 80 mL), dried over $Na_2SO_4$ and evaporated. The residue was purified by column chromatography (PE/EtOAc = 3/1) to give the title compound (3.10 g, 54% yield) as a brown solid.

m/z ES+ $[M+H]^+$ 450.2.

**Intermediate 6a: 5-Benzyl 2-(tert-butyl) 2,5,8-triazaspiro[3.5]nonane-2,5-dicarboxylate**

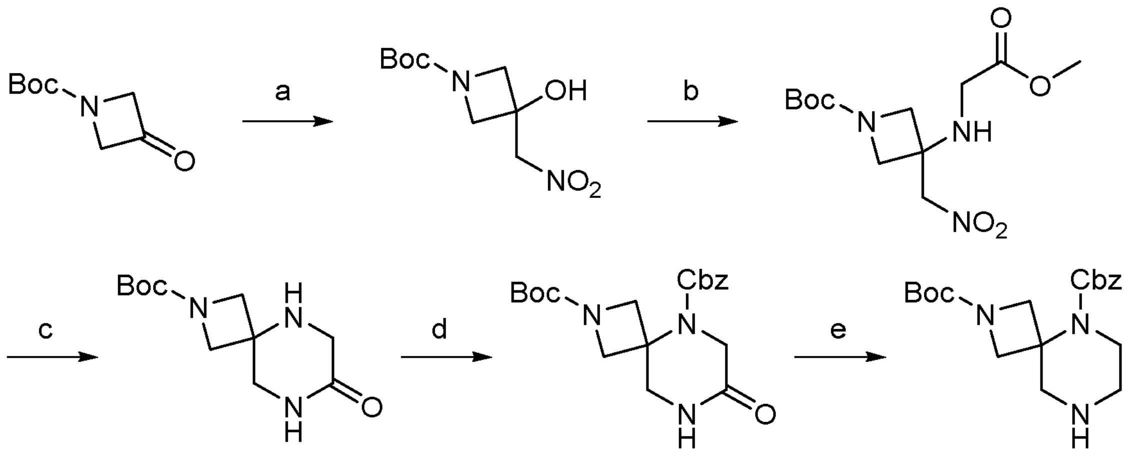


**Step** a. To a solution of *tert-butyl* 3-oxoazetidine-1-carboxylate (5.00 g, 29.2 mmol) in nitromethane (30 mL) was added TEA (591 mg, 5.84 mmol). The mixture was stirred at rt for 1 h. Upon completion, the reaction mixture was evaporated to give *tert-butyl* 3-hydroxy-3-(nitromethyl)azetidine-1-carboxylate (6.5 g, 95% yield) as a yellow solid.

$^1$H NMR (400 MHz, $CDCl_3$) δ ppm 4.70 (s, 2H), 4.00 (s, 4H), 1.44 (s, 9H).

**Step b.** To a solution of *tert*-butyl 3-hydroxy-3-(nitromethyl)azetidine-1-carboxylate (30.0 g, 129 mmol) and TEA (26.1 g, 258 mmol) in DCM (560 mL) was added a solution of methanesulphonyl chloride (14.8 g, 129 mmol) in DCM (140 mL) dropwise at -78 °C. The mixture was stirred at -78 °C for 1.5 h. In a separate flask; a suspension of methyl 2-aminoacetate HCl salt (32.4 g, 258 mmol) in DCM (1.0 L) was treated with TEA (26.1 g, 258 mmol, 36.0 mL) and stirred for 10 min. The resulting solution was then slowly added into the previous mixture at -78 °C. The mixture was warmed to rt and stirred for 16 h. Upon completion, the reaction was quenched with water (1.5 L) and extracted with DCM (3 x 1 L). The combined organic layers were dried over $Na_2SO_4$ and evaporated. The residue was purified by column chromatography (PE/EtOAc = 3/1) to give *tert-butyl* 3-[(2-methoxy-2-oxoethyl)amino]-3-(nitromethyl)azetidine-1-carboxylate (15.0 g, 38% yield) as a yellow solid.

$^1$H NMR (400 MHz, $CDCl_3$) δ ppm 4.00 (s, 4H), 3.96 - 3.84 (m, 2H), 3.76 (s, 3H), 3.48 (s, 2 H), 1.46 (s, 9H).

**Step c.** To a solution of *tert-butyl* 3-[(2-methoxy-2-oxo-ethyl)amino]-3-(nitromethyl)azetidine-1-carboxylate (15.0 g, 49.5 mmol) in MeOH (300 mL) was added Raney-Ni (10 g, 117 mmol) and the mixture was stirred at rt for 12 h under $H_2$ atmosphere (40 psi). Upon completion, the reaction mixture was filtered and evaporated to give *tert-butyl* 7-oxo-2,5,8-triazaspiro[3.5]nonane-2-carboxylate (12 g, crude) as a yellow solid, which was used in the next step directly.

**Step d.** To a solution of *tert-butyl* 7-oxo-2,5,8-triazaspiro[3.5]nonane-2-carboxylate (4.0 g, 16.6 mmol) in THF (60 mL) and water (20 mL) was added benzyl chloroformate (4.24 g, 24.9 mmol) and $Na_2CO_3$ (8.79 g, 82.9 mmol). The mixture was stirred at rt for 12 h. Upon completion, the reaction mixture was quenched with sat. aq. $NH_4Cl$ solution (20 mL), and then diluted with water (30 mL), extracted with EtOAc (3 x 50 mL). The combined organic layers were washed with brine (3 x 20 mL), dried over $Na_2SO_4$ and evaporated. The residue was purified by column chromatography (PE/EtOAc = 1/3) to give 5-benzyl 2-(*tert*-butyl) 7-oxo-2,5,8-triazaspiro[3.5]nonane-2,5-dicarboxylate (4.2 g, 67% yield) as a yellow solid.

m/z ES+ $[M+Na]^+$ 398.2; $^1$H NMR (400 MHz, $CDCl_3$) δ ppm 7.42 - 7.31 (m, 5H), 6.78 (s, 1H), 5.15 (s, 2H), 4.29 (d, *J* = 8.8 Hz, 2H), 4.18 - 4.09 (m, 2H), 3.80 (d, *J* =9.2 Hz, 2H), 3.63 (s, 2H), 1.44 (s, 9H).

**Step e.** To a solution of 5-benzyl *2-(tert-butyl)* 7-oxo-2,5,8-triazaspiro[3.5]nonane-2,5-dicarboxylate (2.00 g, 5.33 mmol) in THF (30 mL) was added borane dimethyl sulfide complex (10 M, 2.66 mL, 26.6 mmol) and the mixture was stirred at 0 °C for 5 h. Upon completion, the reaction mixture was quenched with MeOH (10 mL) at 0 °C and diluted with water (30 mL). The mixture was extracted with DCM (3 x 50 mL). The combined organic layers were washed with brine (3 x 20 mL), dried over $Na_2SO_4$ and evaporated. The residue was purified by reverse phase flash chromatography (water (0.1% FA)/MeCN) to give the title compound as a FA salt (630 mg, 32% yield).

m/z ES+ $[M+H]^+$ 362.4; $^1$H NMR (400 MHz, $CDCl_3$) δ ppm 8.23 - 8.10 (m, 1H), 7.45 - 7.31 (m, 5H), 5.16 (d, *J* = 6.0 Hz, 2H), 4.20 - 4.03 (m, 2H), 3.90 (d, *J* = 9.2 Hz, 1H), 3.72 (dd, *J* = 9.2, 5.6 Hz, 2H), 3.55 (s, 1H), 3.47 - 3.37 (m, 2H), 3.31 - 3.17 (m, 2H),1.50 - 1.38 (m, 9H).

**Intermediate 6b: *tert*-Butyl 5-methyl-2,5,8-triazaspiro[3.5]nonane-2-carboxylate**

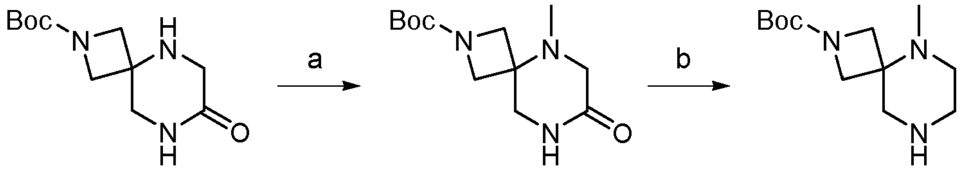


**Step a.** To a solution of *tert*-butyl 7-oxo-2,5,8-triazaspiro[3.5]nonane-2-carboxylate **(Intermediate 6a,** steps a-c; 1.0 g, 4.14 mmol) and aq. formaldehyde (1.85 g, 22.7 mmol, 37% w/w) in MeOH (30 mL) was added acetic acid (497 mg, 8.29 mmol) and $NaBH_3CN$ (442 mg, 7.05 mmol). The mixture was stirred at rt for 12 h. Upon completion, the reaction mixture was evaporated. The residue was diluted with sat. aq. $NaHCO_3$ (20 mL) and extracted with EtOAc (3 x 30 mL). The combined organic layers were washed with brine (20 mL), dried over $Na_2SO_4$ and evaporated to give the title compound (1.0 g, 94% yield) as a brown oil.

$^1$H NMR (400 MHz, $CDCl_3$) δ ppm 6.58 (s, 1H), 4.33 (s, 2H), 4.01 (d, *J* = 9.2 Hz, 2H), 3.64 (d, *J* = 9.2 Hz, 2H), 3.55 (d, *J* = 1.2 Hz, 2H), 2.39 (s, 3H), 1.44 (s, 9H).

Step b. To a solution of *tert-butyl* 5-methyl-7-oxo-2,5,8-triazaspiro[3.5]nonane-2-carboxylate (1.0 g, 3.92 mmol) in THF (15 mL) was added borane dimethyl sulfide complex (10 M, 1.96 mL, 19.6 mmol) portions at 0 °C. The mixture was stirred a rt for 12 h. Upon completion, the reaction mixture was quenched with MeOH (5 mL) at rt, and then evaporated. The residue was diluted with water (20 mL), treated with aq. 1 M HCl (5 mL) and washed with EtOAc (20 mL). The aqueous phase was adjusted pH to 8 with sat. aq. $NaHCO_3$ (20 mL) and extracted with EtOAc (3 x 20 mL). The organic layers were washed with brine (10 mL), dried over $Na_2SO_4$ and evaporated to give the title compound (160 mg, 16% yield) as a colorless oil. $^1$H NMR (400 MHz, $CDCl_3$) δ ppm 4.06 (d, *J* = 8.8 Hz, 2H), 3.60 - 3.56 (m, 2H), 3.37 - 3.10 (m, 2H), 2.81 - 2.75 (m, 3H), 2.44 (s, 3H), 2.39 - 2.34 (m, 1H), 1.46 (s, 9H).

**Intermediate 6c: *tert*-Butyl 3-hydroxy-3-(4-piperidyl)azetidine-1-carboxylate**

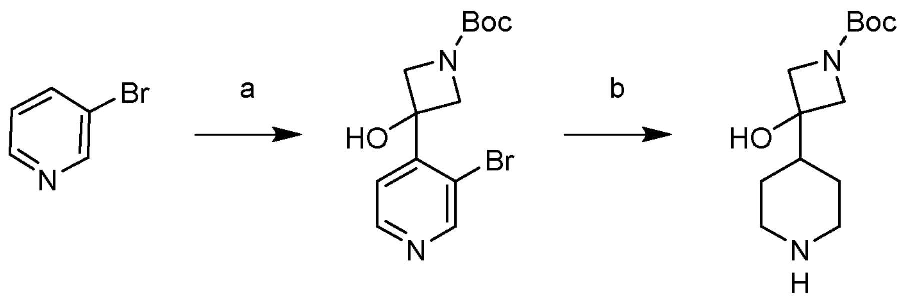


**Step a.** n-BuLi (2.5 M in hexane, 13.9 mL) was added dropwise to a solution of 2,2,6,6-tetramethylpiperidine (4.92 g, 34.8 mmol) in THF (50 mL) at -40 °C. The reaction mixture was warmed to -5 °C and stirred for 15 min, then cooled to -85 °C, and 3-bromopyridine (5.0 g, 31.6 mmol) in THF (10 mL) was added dropwise. The resulting mixture was stirred at - 78 °C for 25 min. Then a solution of *tert-butyl* 3-oxoazetidine-1-carboxylate (11.9 g, 69.6 mmol) in THF (10 mL) was added in one portion at -78 °C. The solution was stirred for 15 min at -78 °C. Upon completion, sat. aq. $NaHCO_3$ (10 mL) was added and the mixture was stirred at -78 °C for 10 min. The mixture was warmed to rt, diluted with water (100 mL) and extracted with EtOAc (2 x 100 mL). The combined organic layer was washed with brine (80 mL), dried over $Na_2SO_4$ and evaporated. The residue was purified by column chromatography (PE/EtOAc = 1/1) to give the title compound (9.0 g, 86% yield) as an oil.

m/z ES+ $[M+H]^+$ 329.0.

**Step b.** To a solution of *tert*-butyl 3-(3-bromo-4-pyridyl)-3-hydroxy-azetidine-1-carboxylate (5.0 g, 15.2 mmol) and TsOH (2.89 g, 15.2 mmol) in EtOH (10 mL) was added $PtO_2$ (862 mg, 3.80 mmol). The mixture was purged 3 times with $H_2$ and stirred for 12 h at rt under $H_2$ atmosphere. Upon completion, the mixture was washed with 5% aq. NaOH (20 mL) and diluted with water (50 mL). The aqueous mixture was extracted with EtOAc (4 x 50 mL). The combined organic layer and washed with brine (50 mL), dried over $Na_2SO_4$ and evaporated to give the title compound (480 mg, 12% yield) as a white solid.

$^1H$ NMR (400 MHz, $CDCl_3$) δ ppm 3.81 (d, *J* = 9.2 Hz, 2H), 3.67 (d, *J* = 9.2 Hz, 3H), 3.13 (s, 2H), 2.55 (s, 2H), 1.59 (d, *J* = 10.0 Hz, 3H), 1.46 (s, 2H), 1.37 (s, 10H).

**Intermediate 6d: *tert*-Butyl 3-(piperidin-4-yloxy)azetidine-1-carboxylate**

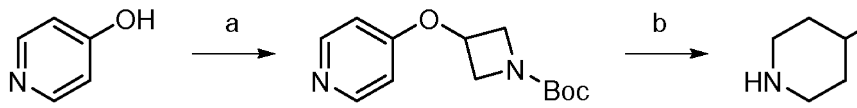


**Step a.** To a mixture of pyridin-4-ol (2.0 g, 21.0 mmol), *tert*-butyl 3-hydroxyazetidine-1-carboxylate (4.55 g, 26.3 mmol) and $PPh_3$ (6.90 g, 26.3 mmol) in THF (70 mL) was added DIAD (5.32 g, 26.3 mmol) dropwise at rt. The mixture was stirred at rt for 16 h under $N_2$ atmosphere. Upon completion, the reaction was evaporated. The residue was treated with 1 M aq. HCl (30 mL). The acidic mixture was washed with DCM (2 x 30 mL). The combined organic phase was extracted with 1 M aq. HCl (10 mL) and water (20 mL). The aqueous phases were combined, basified to pH ~12 with 1 M aq. NaOH, and extracted with DCM (4 x 50 mL). The combined organic layers were washed with brine (2 x 80 mL), dried over $Na_2SO_4$ and evaporated to give *tert-butyl* 3-(pyridin-4-yloxy)azetidine-1-carboxylate (1 g, crude) as a colorless oil.
m/z ES+ $[M+H]^+$ 251.2.
**Step b.** A mixture of *tert*-butyl 3-(pyridin-4-yloxy)azetidine-1-carboxylate (500 mg, 1.68 mmol), $PtO_2$ (125 mg, 0.55 mmol), TsOH (319 mg, 1.68 mmol) in EtOH (10 mL) was degassed and purged 3 times with $H_2$, and then stirred at rt for 20 h under $H_2$ atmosphere (15 psi). Upon completion, the mixture was poured into ice cold 1 M aq. NaOH (25 mL) and filtered through a Celite pad. The filtrate was evaporated to remove EtOH, and the remaining aqueous solution was extracted with DCM (3 x 25 mL). The combined organic layers were washed with brine (50 mL), dried over $Na_2SO_4$ and evaporated to give the title compound (300 mg, crude) as a colorless oil.
$^1H$ NMR (400 MHz, $CDCl_3$) δ ppm 4.35 - 4.28 (m, 1H), 4.08 (dd, *J* = 6.8, 9.2 Hz, 2H), 3.85 - 3.81 (m, 2H), 3.49 - 3.41 (m, 1H), 3.20 - 3.09 (m, 2H), 2.77 - 2.71 (m, 2H), 1.99 - 1.88 (m, 2H), 1.63 - 1.51 (m, 2H), 1.44 (s, 9H).

**Intermediate 6e: Benzyl (*S*)-3-(3-methylpiperazin-1-yl)azetidine-1-carboxylate**

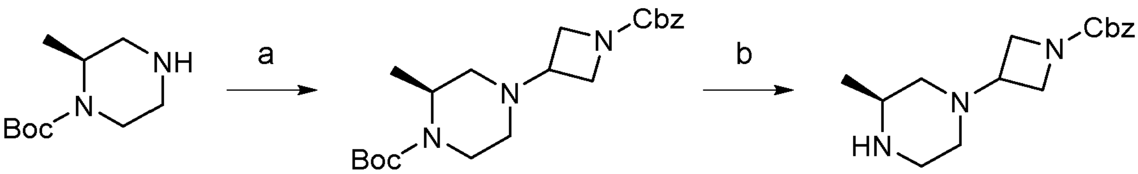


**Step a.** A mixture of *tert-butyl* (2S)-2-methylpiperazine-1-carboxylate (1.0 g, 4.99 mmol), benzyl 3-oxoazetidine-1-carboxylate (2.05 g, 9.99 mmol), $NaBH_3CN$ (627 mg, 9.99 mmol), 4 Å molecular sieves (2.0 g) and acetic acid (599 mg, 9.99 mmol) in DCE (10 mL) was degassed and purged 3 times with $N_2$. The reaction mixture was stirred at 60 °C for 24 h under $N_2$ atmosphere. Upon completion, the mixture was diluted with water (50 mL) and extracted with DCM (50 mL x 2). The combined organic layer was washed with brine (50 mL), dried over $Na_2SO_4$ and evaporated. The residue was purified by reverse phase flash chromatography (water (0.1% $NH_4OH$)/MeCN) to give *tert*-butyl (*S*)-4-(1-((benzyloxy)carbonyl) azetidin-3-yl)-2-methylpiperazine-1-carboxylate (300 mg, 15% yield) as an oil.
m/z ES+ $[M+H]^+$ 390.2.
**Step b**. A solution of tert-butyl (2*S*)-4-(1-benzyloxycarbonylazetidin-3-yl)-2-methylpiperazine-1-carboxylate (0.3 g, 0.77 mmol) in DCM (3 mL) was treated with TFA (878 mg, 7.70 mmol) and stirred at rt for 12 h. Upon completion, the mixture was evaporated to give a residue. The residue was diluted with water (30 mL), adjusted to pH 8~9 with sat. aq. $NaHCO_3$ (10 mL) and extracted with EtOAc (2 x 30 mL). The combined the organic layer was washed with brine (30 mL), dried over $Na_2CO_3$ and evaporated to give the title compound (0.2 g, crude) as an oil.
m/z ES+ $[M+H]^+$ 290.2.

**Intermediate 6f: Benzyl (*R*)-3-(3-methylpiperazin-1-yl)azetidine-1-carboxylate**

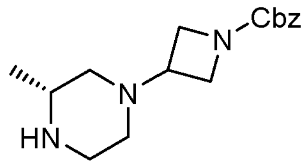

The title compound was prepared in a similar manner to **Intermediate 6e**, using tert-butyl (*R*)-2-methylpiperazine-1-carboxylate in step a.

$^{1}H$ NMR (400 MHz, $CDCl_3$) δ ppm 7.38 - 7.29 (m, 5H), 5.09 (s, 2H), 4.04 - 3.97 (m, 2H), 3.93 - 3.85 (m, 2H), 3.18 - 3.04 (m, 2H), 2.99 - 2.89 (m, 2H), 2.70 (d, *J* = 10.4 Hz, 2H), 2.09 - 1.93 (m, 1H), 1.69 (t, *J* = 10.4 Hz, 1H), 1.12 (d, *J* = 6.4 Hz, 3H).

**Intermediate 6g: Benzyl (R)-3-(2-methylpiperazin-1-yl)azetidine-1-carboxylate**

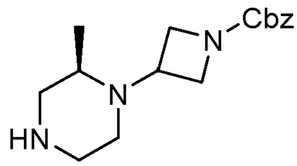

The title compound was prepared in a similar manner to **Intermediate 6e**, using (*R*)-*tert*-butyl 3-methylpiperazine-1-carboxylate in step a.

m/z ES+ $[M+H]^+$ 290.1.

**Intermediate 6h: Benzyl (S)-3-(2-methylpiperazin-1-yl)azetidine-1-carboxylate**

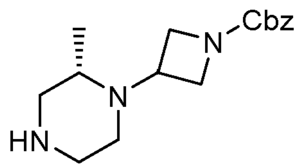

The title compound was prepared in a similar manner to **Intermediate 6e**, using (*S*)-*tert*-butyl 3-methylpiperazine-1-carboxylate in step a.

m/z ES+ $[M+H]^+$ 290.2.

**Intermediate 6i: Benzyl 3-((1*R*,4*R*)-2,5-diazabicyclo[2.2.1]heptan-2-yl)azetidine-1-carboxylate**

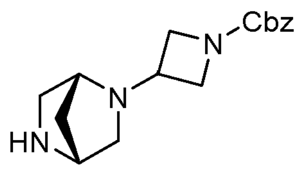

The title compound was prepared in a similar manner to Intermediate 6e, using (1*R*,4*R*)-*tert-butyl* 2,5-diazabicyclo[2.2.1]heptane-2-carboxylate in step a.

m/z ES+ $[M+H]^+$ 288.2.

**Intermediate 6j: Benzyl 3-((1*S*,4*S*)-2,5-diazabicyclo[2.2.1]heptan-2-yl)azetidine-1-carboxylate**

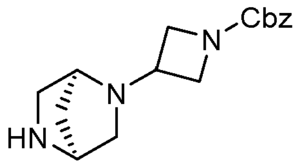

The title compound was prepared in a similar manner to **Intermediate 6e,** using (1*S,4S*)-*tert*-butyl 2,5-diazabicyclo[2.2.1]heptane-2-carboxylate in step a.

m/z ES+ $[M+H]^+$ 288.0.

**Intermediate 6k: 5-((9*H*-Fluoren-9-yl)methyl) 2-(*tert*-butyl) 2,5,8-triazaspiro[3.5]nonane-2,5-dicarboxylate**

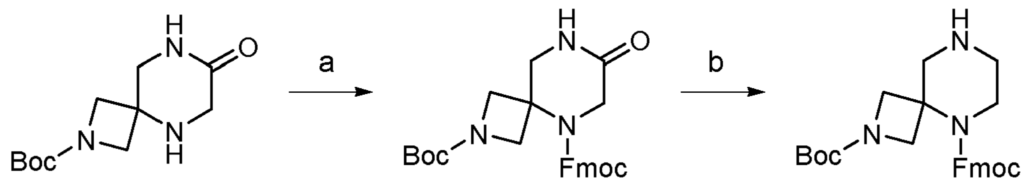


**Step a.** To a solution of *tert-butyl* 7-oxo-2,5,8-triazaspiro[3.5]nonane-2-carboxylate (**Intermediate 6a,** steps a-c; 6.00 g, 24.9 mmol) in dioxane (60 mL) and water (20 mL) was added fluorenylmethyloxycarbonyl chloride (9.65 g, 37.3 mmol) and $Na_2CO_3$ (6.27 g, 74.6 mmol). The mixture was stirred at rt for 12 h. Upon completion, the reaction mixture was quenched with sat. aq. $NH_4Cl$ solution (100 mL) at rt, then diluted with water (200 mL), and extracted with EtOAc (3 x 250 mL). The combined organic layers were washed with brine (3 x 100 mL), dried over $Na_2SO_4$ and evaporated. The residue was purified by column chromatography (PE/EtOAc = 1/3) to give 5-((9H-fluoren-9-yl)methyl) 2-(tert-butyl) 7-oxo-2,5,8-triazaspiro[3.5]nonane-2,5-dicarboxylate (6.00 g, 52% yield) as a white solid.

m/z ES+ [M+H]+ 464.4

**Step b.** To a solution of 5-((9*H*-fluoren-9-yl)methyl) 2-(*tert*-butyl) 7-oxo-2,5,8-triazaspiro[3.5]nonane-2,5-dicarboxylate (2.90 g, 6.26 mmol) in THF (30 mL) was slowly added borane dimethyl sulfide complex (10 M, 3.13 mL) and the mixture was stirred at 0 °C for 2 h. Upon completion, the reaction mixture was carefully quenched with MeOH (20 mL) at 0 °C, then diluted with water (50 mL) and extracted with EtOAc (3 x 100 mL). The combined organic layers were washed with brine (3 x 50 mL), dried over $Na_2SO_4$ and evaporated. The residue was purified by reverse phase flash chromatography (water (0.1% FA)/MeCN) to give the title compound (640 mg, 22% yield) as a yellow solid.

m/z ES+ [M+H]+ 450.2

**Intermediate 6l: tert-butyl (*R*)-hexahydro-1'*H*-spiro[azetidine-3,6'-pyrazino[2,1-*c*][1,4]oxazine]-1-carboxylate**

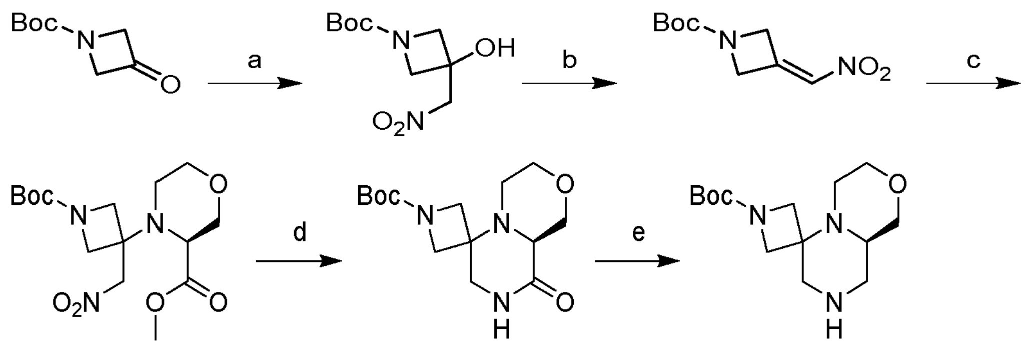


**Step a.** To a solution of *tert*-butyl 3-oxoazetidine-1-carboxylate (100 g, 584 mmol) in $MeNO_2$ (678 g, 11.1 mol) was added TEA (11.8 g, 117 mmol). The mixture was stirred at 25 °C for 1 h. Upon completion, the mixture was evaporated to give *tert*-butyl 3-hydroxy-3-(nitromethyl)azetidine-1-carboxylate (135 g, crude) as a yellow solid.

$^1H$ NMR (400 MHz, DMSO-$d_6$) $\delta$ ppm 6.45 (s, 1H), 4.86 (s, 2H), 4.04 (d, $J$ = 8.8 Hz, 2H), 3.74 (d, $J$ = 9.2 Hz, 2H), 1.38 (s, 9H).

**Step b.** A To a solution of *tert*-butyl 3-hydroxy-3-(nitromethyl)azetidine-1-carboxylate (2 g, 8.61 mmol) in DCM (20 mL) was added DAST (2.08 g, 12.9 mmol) dropwise at 0 °C. The reaction mixture was stirred at 0 °C for 30 min. Upon completion, the mixture was quenched with water (20 mL), and the layers were separated. The organic layer was washed with brine (50 mL), dried over $Na_2SO_4$ and evaporated. The crude product was purified by column chromatography (PE/EtOAc = 8/1) to give *tert-butyl* 3-(nitromethylene)azetidine-1-carboxylate (1.55 g, 84% yield) as a yellow solid.
$^1H$ NMR (400 MHz, $CDCl_3$) δ ppm 7.08 - 7.02 (m, 1H), 5.06 - 5.00 (m, 2H), 4.75 - 4.67 (m, 2H), 1.48 (s, 9H).
**Step c.** To a solution of *tert*-butyl 3-(nitromethylene)azetidine-1-carboxylate (2.01 g, 9.4 mmol) in DCM (20 mL) was added methyl (*S*)-morpholine-3-carboxylate (CAS: 741288-31-3, 1.5 g, 10.3 mmol) and the mixture was stirred at 25 °C for 12 h. Upon completion, the mixture was evaporated and purified by silica gel chromatography (PE/EtOAc =2/1) to give methyl (*S*)-4-(1-(*tert*-butoxycarbonyl)-3-(nitromethyl)azetidin-3-yl)morpholine-3-carboxylate (2.5 g, 74% yield) as a white solid.
m/z ES+ $[M+H]^+$ 360.1
**Step d.** A mixture of methyl (*S*)-4-(1-(*tert*-butoxycarbonyl)-3-(nitromethyl)azetidin-3-yl)morpholine-3-carboxylate (2.50 g, 7.0 mmol) and Raney-Ni (596 mg, 7.0 mmol) in MeOH (30 mL) was stirred at 25 °C for 12 h under an $H_2$ atmosphere. Upon completion, the mixture was evaporated to give *tert-butyl* (*S*)-9'-oxohexahydro-1'*H*-spiro[azetidine-3,6'-pyrazino[2,1-c][1,4]oxazine]-1-carboxylate (1.2 g, 58% yield) as a yellow solid.
m/z ES+ $[M+H]^+$ 298.2.
**Step e.** To a solution of *tert*-butyl (*S*)-9'-oxohexahydro-1'*H*-spiro[azetidine-3,6'-pyrazino[2,1-*c*][1,4]oxazine]-1-carboxylate (1.2 g, 4.0 mmol) in THF (10 mL) was added borane dimethyl sulfide complex (10 M in THF, 1.21 mL, 12.1 mmol) at 0 °C, the mixture was stirred at 0 °C for 1 h. Upon completion, the mixture was quenched with MeOH (6 mL) and evaporated to give the title compound (1 g, 87% yield) as a yellow solid.
m/z ES+ $[M+H]^+$ 284.0.

**Intermediate 6m: *tert*-butyl (*S*)-hexahydro-1'*H*-spiro[azetidine-3,6'-pyrazino[2,1-*c*][1,4]oxazine]-1-carboxylate**

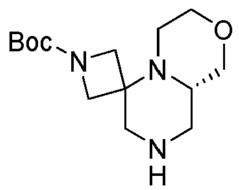

The title compound was prepared in a similar manner to **Intermediate 6l,** using methyl (*R*)-morpholine-3-carboxylate (CAS: 1187933-47-6) in step c.
m/z ES+ $[M+H]^+$ 284.3.

**Intermediate 6n: 5-benzyl 2-(*tert*-butyl) (*R*)-6-methyl-2,5,8-triazaspiro[3.5]nonane-2,5-dicarboxylate**

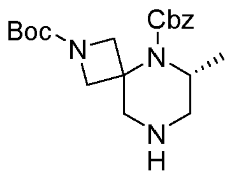

The title compound was prepared in a similar manner to **Intermediate 6a** using methyl *D*-alaninate (CAS: 14316-06-4) in step b.
m/z ES+ $[M+H]^+$ 376.2.

**Intermediate 6o: *tert*-butyl (*R*)-6-methyl-2,5,8-triazaspiro[3.5]nonane-2-carboxylate**

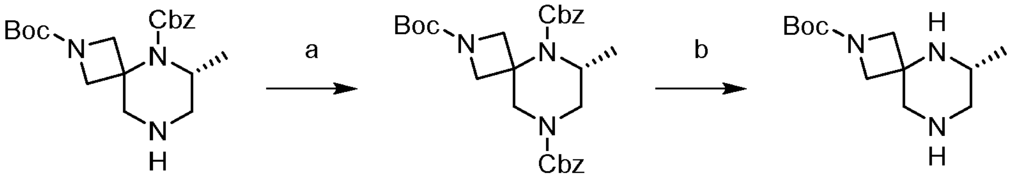


**Step** a. To a solution of **Intermediate 6n** (0.8 g, 2.13 mmol) in THF (9 mL) and water (3 mL) was added $Na_2CO_3$ (226 mg, 2.13 mmol) and CbzCl (545 mg, 3.20 mmol). The mixture was stirred at 25 °C for 12 h. Upon completion, the reaction mixture was quenched with sat. aq. $NH_4Cl$ (20 mL), further diluted with water (30 mL) and extracted with EtOAc (3 x 50 mL). The combined organic layers were washed with brine (3 x 20 mL), dried over $Na_2SO_4$ and evaporated. The residue was purified by column chromatography (PE/EtOAc = 5/1) to give 5,8-dibenzyl *2-(tert-butyl)* (*R*)-6-methyl-2,5,8-triazaspiro[3.5]nonane-2,5,8-tricarboxylate (1 g, 92% yield) as a colourless solid.

m/z ES+ $[M+H]^+$ 510.4; $^1H$ NMR (400 MHz, $CDCl_3$) δ ppm 7.37 - 7.27 (m, 10H), 5.25 - 5.08 (m, 4H), 4.51 - 4.98 (m, 1H), 4.37 - 4.23 (m, 2H), 4.00 - 3.96 (m, 1H), 3.77 - 3.48 (m, 4H), 3.26 - 3.22 (d, *J* = *13.2* Hz, 1H),1.44 (s, 9H), 1.17 (d, *J* = 6.0 Hz, 3H).

**Step b.** To a solution of 5,8-dibenzyl 2-(*tert*-butyl) (*R*)-6-methyl-2,5,8-triazaspiro[3.5]nonane-2,5,8-tricarboxylate (1.0 g, 1.96 mmol) in THF (10 mL) was added 10% Pd/C (208 mg) and the mixture was stirred at 25 °C for 12 h under a $H_2$ atmosphere (15 psi). Upon completion, the reaction mixture was filtered and evaporated. The residue was purified by reverse phase flash chromatography (water (0.1% FA)/MeCN) to give the title compound (450 mg, 95% yield) as a colourless oil.

$^1H$ NMR (400 MHz, $CDCl_3$) δ ppm 3.94 (d, *J* = 8.8 Hz, 1H), 3.76 - 3.68 (m, 2H), 3.56 (d, *J* = 9.2 Hz, 1H), 3.04 (d, *J* = 11.8 Hz, 1H), 2.89 - 2.84 (m, 1H), 2.82 - 2.78 (m, 1H), 2.73 - 2.66 (m, 1H), 2.34 - 2.28 (m, 1H), 1.44 (s, 9H), 1.02 (d, *J* = 6.0 Hz, 3H).

**Intermediate 7a: *trans*-1-(*tert*-Butoxycarbonyl)-4-methoxypyrrolidine-3-carboxylic acid**

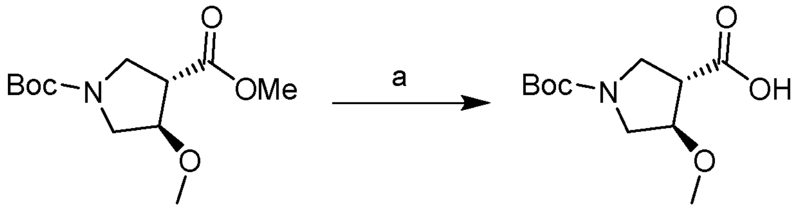


To a solution of *trans*-1-*tert*-butyl 3-methyl-4-methoxypyrrolidine-1,3-dicarboxylate (CAS Number 1428775-89-6; 200 mg, 0.77 mmol) in THF (2 mL) was added LiOH monohydrate (65 mg, 1.54 mmol) and water (2 mL). The mixture was stirred at rt for 2 h. Upon completion, the reaction mixture was adjusted pH ~ 6 by addition of acetic acid (2 mL), diluted with water (5 mL) and extracted with EtOAc (5 mL x 3). The combined organic layers were dried over $Na_2SO_4$, filtered and evaporated to give the title compound (180 mg, crude) as colourless oil, which was used into the next step without further purification.

$^1H$ NMR (400 MHz, $CDCl_3$) δ ppm 4.17 - 4.14 (m, 1H), 3.70 - 3.57 (m, 3H), 3.41 - 3.38 (m, 1H), 3.37 (s, 3H), 3.12 - 3.08 (m, 1H), 1.45 (s, 9H).

**Intermediate 7b**: ***tert*-butyl 3-(((*rel-trans*)-4-hydroxypyrrolidin-3-yl)oxy)azetidine-1-carboxylate**

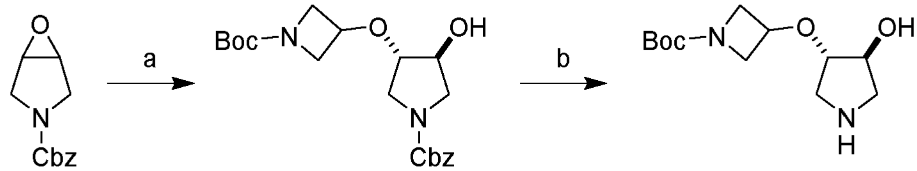


**Step a.** To a solution of benzyl 6-oxa-3-azabicyclo[3.1.0]hexane-3-carboxylate (CAS: 31865-25-5; 10.0 g, 45.6 mmol) in DMSO (40 mL) and water (40 mL) was added $Cs_2CO_3$ (22.3 g, 68.4 mmol) and *tert*-butyl 3-hydroxyazetidine-1-carboxylate (CAS: 141699-55-0; 9.5 g, 54.7 mmol). The reaction mixture was then stirred at 110 °C under microwave irradiation for 45 min. Upon completion, the reaction mixture was poured into cold water (50 mL) and extracted with EtOAc (3 x 100 mL). The combined organic layers were washed with cold water, brine, dried over $Na_2SO_4$ and evaporated. Purification by column chromatography gave benzyl (*rel-trans*)-3-((1-(*tert*-butoxycarbonyl)azetidin-3-yl)oxy)-4-hydroxypyrrolidine-1-carboxylate as an off-white solid (3.7 g, 21% yield).

m/z ES+ [M-$CO_2$*t*Bu]+ 293.6.

**Step b.** To a solution of benzyl (rel-trans)-3-((1-(tert-butoxycarbonyl)azetidin-3-yl)oxy)-4-hydroxypyrrolidine-1-carboxylate (3.7 g, 9.4 mmol) in MeOH (100 mL) was added 10% Pd/C (3.6 g) and the mixture was stirred at rt for 2 h under a $H_2$ atmosphere (15 psi). Upon completion, the reaction mixture was filtered and evaporated to give the title compound as an off-white solid (1.5 g, 62% yield).

m/z ES+ [M+H]+ 259.6.

**Intermediate 7c: *tert*-butyl 3-hydrazineylazetidine-1-carboxylate**

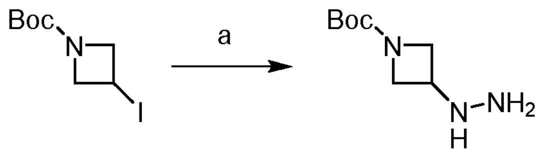


**Step a.** To a mixture of *tert-butyl* 3-iodoazetidine-1-carboxylate (5 g, 17.7 mmol) in EtOH (10 mL) was added hydrazine monohydrate (8.84 g, 177 mmol). The mixture was stirred at 85 °C for 48 h. Upon completion, the solution was cooled to rt, diluted with water (50 mL), extracted with DCM (3 x 50 mL) and evaporated to give the title compound (2 g, crude) as a colourless oil.

$^1$H NMR (400 MHz, $CDCl_3$) δ ppm 4.07 - 3.99 (m, 2H), 3.75 - 3.65 (m, 3H), 1.43 (s, 9H).

**Intermediate 7d: tert-butyl (*E*)-3-(*N*'-hydroxycarbamimidoyl)azetidine-1-carboxylate**

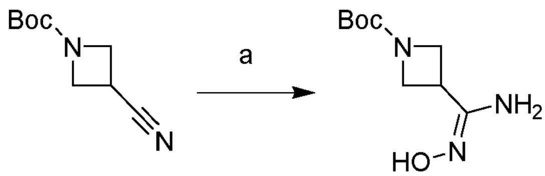


**Step a**: A mixture of *tert-butyl* 3-cyanoazetidine-1-carboxylate (CAS: 142253-54-1; 5.0 g, 27.44 mmol), hydroxylamine hydrochloride (2.67 g, 38.4 mmol) and $NaHCO_3$ (4.61 g, 54.9 mmol) in EtOH (50 mL) was stirred at 80 °C for 3 h under a $N_2$ atmosphere. Upon completion, the mixture was evaporated, diluted with water (30 mL) and extracted with EtOAc (2 x 30 mL). The combined layers were washed with brine (30 mL), dried over $Na_2SO_4$ and evaporated to give the title compound (2.5 g, 42 % yield) as a white solid.

$^1$H NMR (400 MHz, $CDCl_3$) δ ppm 9.20 (s, 1H), 5.63 (br s, 2H), 4.05 - 3.85 (m, 4H), 3.29 - 3.17 (m, 1H), 1.42 (s, 9H).

**Intermediate 8a: *tert*-Butyl 3-(3-oxopropoxy)azetidine-1-carboxylate**

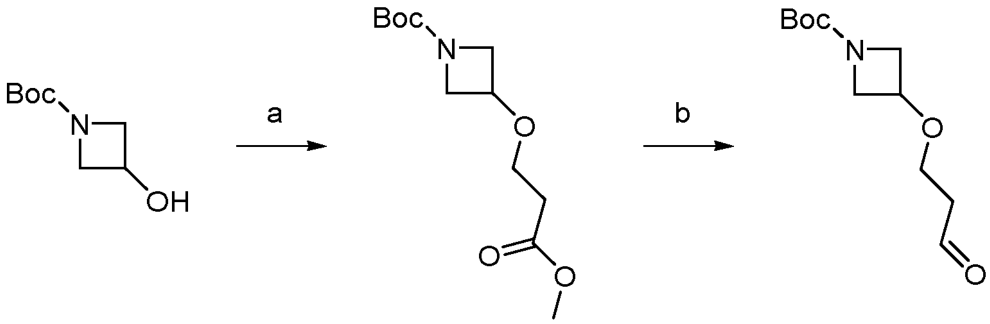


**Step a.** To a solution of *tert*-butyl 3-hydroxyazetidine-1-carboxylate (120 g, 693 mmol) in THF (1.2 L) was added NaH (4.16 g, 104 mmol, 60% dispersion in mineral oil) and stirred at 0 °C for 30 min. Methyl acrylate (298 g, 346 mmol) was added slowly and the mixture was stirred at 0 °C for 12 h. The reaction mixture was quenched with sat. aq. $NH_4Cl$ solution (300 mL) at rt, and then diluted with water (300 mL) and extracted with EtOAc (3 x 500 mL). The combined organic layers were washed with brine (3 x 100 mL), dried over $Na_2SO_4$ and evaporated. The residue was purified by column chromatography (PE/EtOAc = 5/1) to give *tert-butyl* 3-(3-methoxy-3-oxo-propoxy)azetidine-1-carboxylate (85.0 g, 47% yield) as a colorless oil.

$^1$H NMR (400 MHz, $CDCl_3$) δ ppm 4.29 - 4.21 (m, 1H), 4.07 (dd, *J* = 6.8, 9.6 Hz, 2H), 3.86 - 3.81 (m, 2H), 3.72 (s, 3H), 3.70 - 3.63 (m, 2H), 2.63 - 2.58 (m, 2H), 1.44 (s, 9H).

**Step b.** To a solution of *tert-butyl* 3-(3-methoxy-3-oxo-propoxy)azetidine-1-carboxylate (45.0 g, 174 mmol) in THF (100 mL) was added DIBAL-H (1 M in toluene, 520 mL, 520 mmol) and the mixture was stirred at -78 °C for 2 h. Upon completion, the reaction mixture was quenched with MeOH (20 mL) at -78 °C and then stirred at rt for 30 min. The combined organic layers were filtered and evaporated to give the title compound (35.0 g, 88% yield) as a colorless oil.

$^1$H NMR (400 MHz, $CDCl_3$) δ ppm 9.82 (t, *J* = 1.6 Hz, 1H), 4.29 - 4.20 (m, 1H), 4.11 - 4.07 (m, 2H), 3.85-3.82 (m, 2H), 3.75 - 3.68 (m, 2H), 2.77 - 2.62 (m, 2H), 1.45 (s, 9H).

**Intermediate 8b: *tert*-Butyl 6-(2-oxoethoxy)-2-azaspiro[3.3]heptane-2-carboxylate**

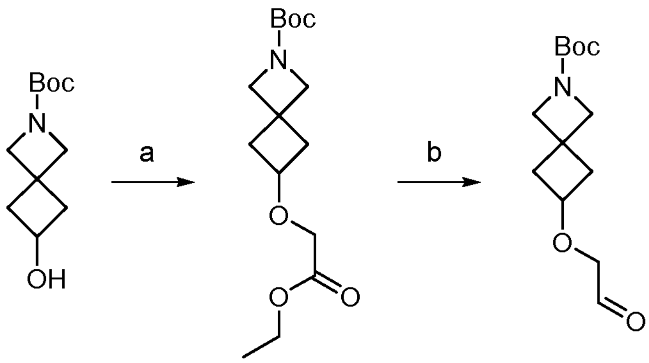


**Step a.** To a solution of *tert*-butyl 6-hydroxy-2-azaspiro[3.3]heptane-2-carboxylate (5.70 g, 26.7 mmol in DCM (60 mL) was added $Rh(OAc)_2$ (295 mg, 1.34 mmol) followed by addition a solution of ethyl 2-diazoacetate (4.57 g, 40.1 mmol) in DCM (180 mL) drop-wise over 2 h at 10 °C. The mixture was stirred at 10 °C for 1.5 h. Upon completion, the reaction mixture was filtered and evaporated. The residue was purified by column chromatography (PE/EtOAc = 1/1) to give *tert-butyl* 6-(2-ethoxy-2-oxo-ethoxy)-2-azaspiro[3.3]heptane- 2-carboxylate (4.60 g, 58% yield) as a pale yellow oil.

m/z ES+ $[M\text{-}tBu]^+$ 244.1; $^1$H NMR (400 MHz, DMSO-$d_6$) δ ppm 4.12 - 4.07 (m, 2H), 3.96 (s, 2H), 3.94 - 3.85 (m, 1H), 3.77 (d, *J* = 14.4 Hz, 4H), 2.45 - 2.37 (m, 2H), 2.06 - 1.99 (m, 2H), 1.35 (s, 9H), 1.19 - 1.17 (m, 3H).

**Step b.** To a solution of *tert-butyl* 6-(2-ethoxy-2-oxo-ethoxy)-2-azaspiro[3.3]heptane-2-carboxylate (4.60 g, 15.4 mmol) in THF (50 mL) was added DIBAL-H (1 M in toluene, 46.1 mL). The mixture was stirred at -60 °C for 2 h. Upon completion, the reaction mixture was quenched with MeOH (5 mL) at -60 °C, filtered and evaporated to give the title compound (4.00 g, crude) as pale yellow oil.

$^1$H NMR (400 MHz, DMSO-$d_6$) δ ppm 9.55 (s, 1H), 4.06 - 4.01 (m, 1H), 3.81 - 3.73 (m, 4H), 3.28 - 3.00 (m, 2H), 2.46 - 2.35 (m, 2H), 2.08 - 1.93 (m, 2H), 1.36 (s, 9H).

**Intermediate 8c: tert-Butyl 5-methyl-7-oxo-8-(2-oxoethyl)-2,5,8-triazaspiro[3.5]nonane-2-carboxylate**

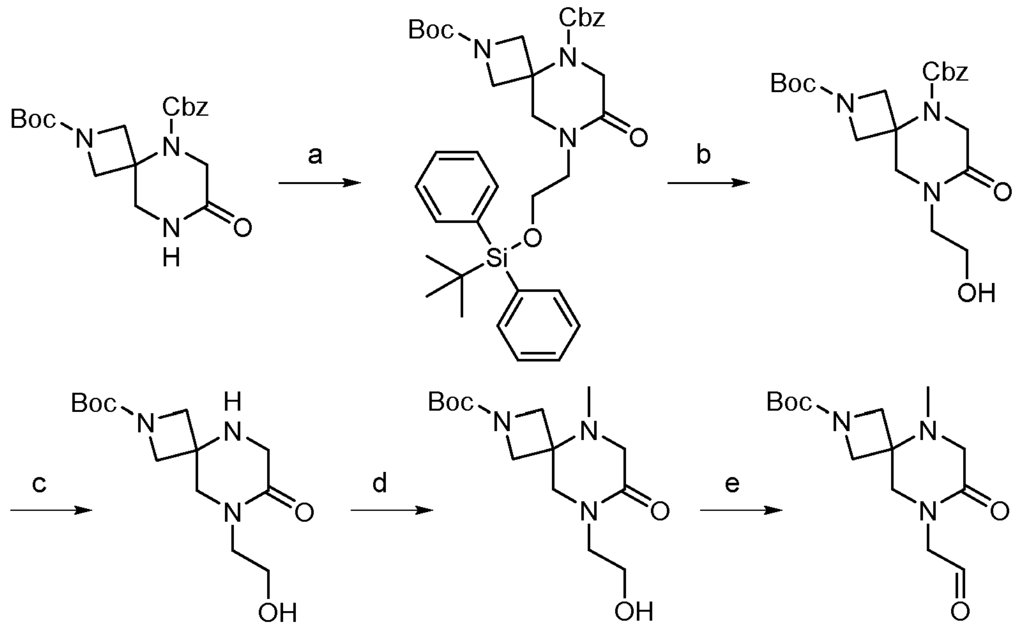


**Step a.** To a solution of 5-benzyl *2-tert-butyl* 7-oxo-2,5,8-triazaspiro[3.5]nonane-2,5-dicarboxylate **(Intermediate 6a,** steps a-d, 10 g, 26.6 mmol) in DMF (100 mL) was added $Cs_2CO_3$ (26.0 g, 79.9 mmol) and *tert*-butyl(2-iodoethoxy) diphenylsilane (16.4 g, 40.0 mmol). The mixture was stirred at rt for 16 h. Upon completion, the reaction mixture was diluted with water (100 mL) and extracted with EtOAc (3 x 80 mL). The combined organic layers were dried over $Na_2SO_4$ and evaporated. The residue was purified by column chromatography (PE/EtOAc = 1/1) to give 5-benzyl *2-tert-butyl* 8-(2-((*tert*-butyldiphenylsilyl)oxy)ethyl)-7-oxo-2,5,8-triazaspiro [3.5]nonane-2,5-dicarboxylate (12.4 g, 71% yield) as a pale yellow oil.

m/z ES+ $[M+H]^+$ 658.2; $^1H$ NMR (400 MHz, $CDCl_3$) $\delta$ ppm 7.64 (dd, *J* = 1.6, 7.6 Hz, 4H), 7.45 - 7.33 (m, 11H), 5.13 (s, 2H), 4.31 (d, *J* = 8.8 Hz, 2H), 4.14 (s, 2H), 3.84 (t, *J* = 5.2 Hz, 2H), 3.80 - 3.73 (m, 4H), 3.55 - 3.47 (m, 2H), 1.44 (s, 9H), 1.06 (s, 9H).

**Step b.** To a solution of 5-benzyl 2-*tert*-butyl 8-(2-((*tert*-butyldiphenylsilyl)oxy)ethyl)-7-oxo-2,5,8-triazaspiro [3.5] nonane-2,5-dicarboxylate (12.4 g, 18.9 mmol) in THF (90 mL) was added TBAF (1 M in THF, 38 mL). The mixture was stirred at rt for 1 h. Upon completion, the reaction mixture was diluted with water (50 mL) and extracted with EtOAc (3 x 30 mL). The combined organic layers were dried over $Na_2SO_4$ and evaporated. The residue was purified by column chromatography (PE/EtOAc = 1/1) to give 5-benzyl *2-tert-butyl* 8-(2-hydroxyethyl)-7-oxo-2,5,8-triazaspiro[3.5]nonane-2,5- dicarboxylate (7.10 g, 89% yield) as a pale yellow oil.

**Step c.** A mixture of 5-benzyl *2-tert-butyl* 8-(2-hydroxyethyl)-7-oxo-2,5,8-triazaspiro[3.5]nonane-2,5- dicarboxylate (7.10 g, 16.9 mmol), 10% Pd/C (2.00 g, 1.69 mmol) in 2-propanol (70 mL) was degassed and purged 3 times with $H_2$. The mixture was stirred at rt for 2 h under $H_2$ atmosphere (15 psi). Upon completion, the reaction mixture was filtered and evaporated to give *tert-butyl* 8-(2-hydroxyethyl)-7-oxo-2,5,8-triazaspiro[3.5]nonane-2-carboxylate (5.00 g, crude) as a yellow oil.

$^1H$ NMR (400 MHz, $CDCl_3$) $\delta$ ppm 3.87 - 3.79 (m, 6H), 3.60 (d, *J* = 6.0 Hz, 4H), 3.55 (t, *J* = 5.2 Hz, 2H), 1.45 (s, 9H).

**Step d** To a solution of *tert*-butyl 8-(2-hydroxyethyl)-7-oxo-2,5,8-triazaspiro[3.5]nonane-2-carboxylate (5 g, 17.5 mmol ) and aq. formaldehyde (7.8 g, 96.4 mmol, 37% w/w) in MeOH (50 mL) was added $NaBH_3CN$ (1.87 g, 29.8 mmol) and acetic acid (2.10 g, 35.1 mmol). The mixture was stirred at rt for 3 h. Upon completion, the reaction mixture was evaporated. The residue was purified by reverse phase flash chromatography (water (0.1% FA)/MeCN) to give *tert*-butyl 8-(2-hydroxyethyl)-5-methyl-7-oxo- 2,5,8-triazaspiro[3.5]nonane-2-carboxylate (3.68 g, 70% yield) as a yellow oil.

m/z ES+ $[M+H]^+$ 300.2.

**Step e.** To a solution of oxalyl chloride (2.14 g, 16.8 mmol) in DCM (5 mL) was added DMSO (2.63 g, 33.7 mmol) in DCM (5 mL) at -78 °C. The mixture was stirred at -78 °C for 15 min before addition of a solution of *tert-butyl* 8-(2-hydroxyethyl)-5-methyl-7-oxo-2,5,8-triazaspiro[3.5]nonane-2-carboxylate (1.68 g, 5.61 mmol) in DCM (5 mL). The reaction was stirred at -78 °C for 3 h. TEA (5.11 g, 50.5 mmol) was added to the mixture and stirred at - 78 °C for 30 min. Upon completion, the reaction mixture was diluted with water (30 mL) and extracted with a 10:1 mixture of DCM:2-propanol (3 x 20 mL). The combined organic layers were dried over $Na_2SO_4$ and evaporated to give the title compound (2.00 g, crude) as a pale yellow oil.

m/z ES+ $[M+H]^+$ 297.9; $^1H$ NMR (400 MHz, $CDCl_3$) δ ppm 9.62 - 9.58 (m, 1H), 4.17 - 4.07 (m, 1H), 3.91 - 3.66 (m, 1H), 3.65 - 3.57 (m, 1H), 3.52 - 3.47 (m, 1H), 3.44 (s, 4H), 3.41 - 3.38 (m, 1H), 3.20 - 3.05 (m, 1H), 2.61 (s, 3H), 1.46 - 1.35 (m, 9H).

**Intermediate 8d: *tert*-Butyl 6-oxo-7-(2-oxoethyl)-2,7-diazaspiro[4.4]nonane-2-carboxylate**

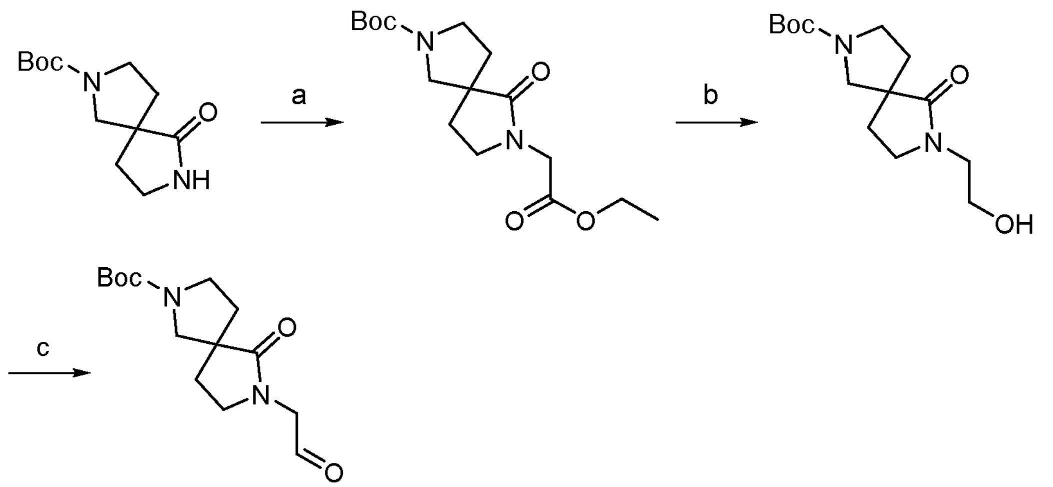


**Step a**. To a solution of *tert*-butyl 1-oxo-2,7-diazaspiro[4.4]nonane-7-carboxylate (8.00 g, 33.3 mmol) in THF (160 mL) was added NaH (4.00 g, 100 mmol, 60% dispersion in mineral oil) and stirred at 0 °C for 30 min. Ethyl 2-bromoacetate (11.1 g, 66.6 mmol) was added to the mixture and stirred at rt for 2 h. Upon completion, the reaction mixture was quenched with water (15 mL), and then diluted with water (50 mL) and extracted with EtOAc (3 x 40 mL). The combined organic layers were dried over $Na_2SO_4$ and evaporated. The residue was purified by column chromatography (PE/EtOAc= 1/1) to give tert-*butyl* 7-(2-hydroxyethyl)-6-oxo-2,7-diazaspiro[4.4]nonane-2-carboxylate (3.10 g, 27% yield) as a pale yellow oil.

m/z ES+ $[M+Na]^+$349.1.

**Step b.** To a solution of *tert*-butyl 7-(2-hydroxyethyl)-6-oxo-2,7-diazaspiro[4.4]nonane-2-carboxylate (3.70 g, 11.3 mmol) in THF (80 mL) was added $LiBH_4$ (741 mg, 34.0 mmol). The mixture was stirred at rt for 2 h. Upon completion, the reaction mixture was quenched with sat. aq. $NH_4Cl$ (20 mL), diluted with water (50 mL) and extracted with EtOAc (3 x 30 mL). The combined organic layers were dried over $Na_2SO_4$ and evaporated to give *tert-butyl* 7-(2-hydroxyethyl)-6-oxo-2,7-diazaspiro[4.4]nonane-2-carboxylate (3 g, crude) as an oil.

m/z ES+ $[M+Na]^+$307.1.

**Step c.** To a solution of oxalyl chloride (2.68 g, 21.1 mmol) in DCM (24 mL) was added DMSO (3.30 g, 42.2 mmol) in DCM (24 mL) at -78 °C. The mixture was stirred at -78 °C for 15 min before addition of a solution of *tert-butyl* 7-(2-hydroxyethyl)-6-oxo-2,7-diazaspiro[4.4]nonane-2-carboxylate (3.00 g, 10.5 mmol) in DCM (24 mL). The reaction was stirred at -78 °C for 30 min. TEA (8.54 g, 84.4 mmol) was added and stirred at -78 °C for 30 min. Upon completion, the reaction mixture was diluted with water (40 mL) and extracted with DCM (3 x 30 mL). The combined organic layers were washed with water (4 x 30 mL), dried over $Na_2SO_4$ and evaporated to give the title compound (3.00 g, crude) as a pale yellow oil.

m/z ES+ $[M-tBu]^+$ 227.1, $[M-CO_2tBu]^+$ 183.2

**Intermediate 8e: *trans-tert*-Butyl 3-methoxy-4-((2-oxoethoxy)methyl) pyrrolidine-1-carboxylate**

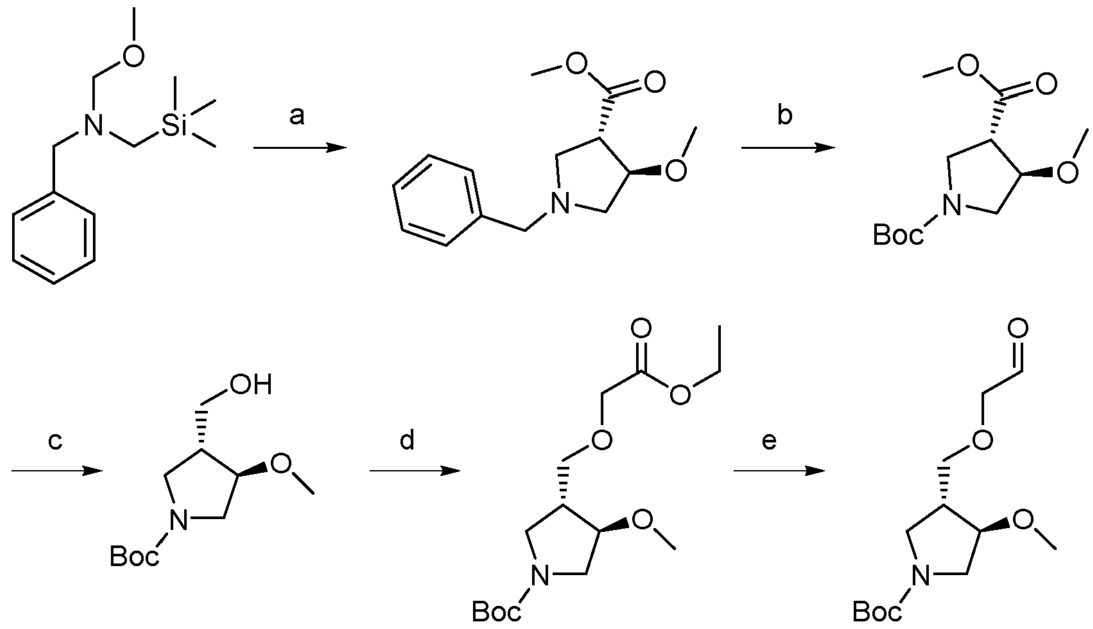


**Step a.** To a solution of methyl (*E*)-3-methoxy-2-methylacrylate (20.0 g, 172 mmol) in 2-methyltetrahydrofuran (240 mL) and TFA (3.93 g, 34.5 mmol) at 0 °C was added *N*-benzyl-1-methoxy-N-((trimethylsilyl)methyl)methanamine (81.8 g, 344 mmol) dropwise. The reaction was allowed to warm to rt and stirred for 2 h. Upon completion, the reaction mixture was diluted with sat. aq. $NaHCO_3$ (500 mL) and extracted with EtOAc (3 x 300 mL). The combined organic layers were dried over $Na_2SO_4$ and evaporated. The residue was purified by column chromatography (PE/EtOAc = 9/1) and reverse phase flash chromatography (water (0.1% $NH_4OH$)/MeCN) to give trans-methyl 1-benzyl-4-methoxypyrrolidine-3-carboxylate (29.0 g, 68% yield) as a brown liquid.

m/z ES+ $[M+H]^+$250.2.

**Step b.** A mixture of *trans*-methyl 1-benzyl-4-methoxypyrrolidine-3-carboxylate (10.0 g, 40.1 mmol) and 20% $Pd(OH)_2/C$ (2.82 g) in EtOH (100 mL) was degassed and purged 3 times with $H_2$. The mixture was stirred at rt for 3 h under $H_2$ atmosphere (15 psi). Upon completion, the reaction mixture was filtered and evaporated to give a residue. The residue was redissolved in DCM (60 mL) and treated with DIPEA (9.74 g, 75.4 mmol) and di-tert-butyl dicarbonate (12.3 g, 56.5 mmol). The mixture was stirred at rt for 16 h. Upon completion, the reaction mixture was diluted with water (100 mL) and extracted with EtOAc (3 x 50 mL). The combined organic layers were dried over $Na_2SO_4$ and evaporated. The residue was purified by column chromatography (PE/EtOAc = 5/1) to give *trans*-1-*tert*-butyl 3-methyl 4-methoxypyrrolidine-1,3-dicarboxylate (9.1 g, 93% yield) as a colorless liquid.

$^1H$ NMR (400 MHz, $CDCl_3$) δ ppm 4.16 - 4.11 (m, 2H), 3.73 (s, 3H), 3.63 (d, *J* = 13.6 Hz, 3H), 3.36 (s, 3H), 3.12 - 3.04 (m, 1H), 1.45 (s, 9H).

**Step c.** To a solution of *trans*-1-*tert*-butyl 3-methyl 4-methoxypyrrolidine-1,3-dicarboxylate (5.00 g, 19.3 mmol) in THF (50 mL) was added $LiBH_4$ (1.26 g, 57.9 mmol). The mixture was stirred at rt for 3 h. Upon completion, the reaction mixture was quenched with sat. aq. $NH_4Cl$ (20 mL), diluted with water (20 mL) and extracted with EtOAc (3 x 30 mL). The combined organic layers were dried over $Na_2SO_4$ and evaporated. The residue was purified by column chromatography (PE/EtOAc = 3/1) to give *trans-tert*-butyl 3-(hydroxymethyl)-4-methoxypyrrolidine-1-carboxylate (3.5 g, 78% yield) as a colorless oil.

$^1H$ NMR (400 MHz, DMSO-$d_6$) δ ppm 4.76 (t, *J* = 5.2 Hz, 1H), 3.75 - 3.67 (m, 1H), 3.42 - 3.34 (m, 1H), 3.33 (s, 2H), 3.22 (s, 3H), 3.32 - 3.18 (m, 2H), 3.10 (dd, *J* = 3.6, 10.8 Hz, 1H), 2.29 - 2.20 (m, 1H), 1.39 (s, 9H).

**Step d.** To a solution of *trans-tert*-butyl 3-(hydroxymethyl)-4-methoxypyrrolidine-1-carboxylate (2.50 g, 10.8 mmol) in THF (25 mL) was added NaH (865 mg, 21.6 mmol, 60% dispersion in mineral oil) and stirred at rt for 30 min. Then ethyl 2-bromoacetate (2.71 g, 16.2 mmol) was added and stirred at rt for 16 h. Upon completion, the reaction mixture was quenched with sat. aq. $NaHCO_3$ (10 mL), diluted with water (30 mL) and extracted with EtOAc (3 x 20 mL). The combined organic layers were dried over $Na_2SO_4$ and evaporated. The residue was purified by column chromatography (PE/EtOAc = 7/1) to give *trans-tert*-butyl 3-methoxy-4-((2-methoxy-2-oxoethoxy)methyl)pyrrolidine-1- carboxylate (2.00 g, 58% yield) as a colorless oil.

$^1H$ NMR (400 MHz, $CDCl_3$) δ ppm 4.21 (q, *J* = 7.2 Hz, 2H), 4.12 - 4.04 (m, 2H), 3.92 - 3.79 (m, 1H), 3.53 (d, *J* = 9.2 Hz, 2H), 3.48 (d, *J* = 6.4 Hz, 1H), 3.34 (s, 3H), 3.28 - 3.19 (m, 1H), 2.59 - 2.47 (m, 1H), 1.45 (s, 9H), 1.29 (t, *J* = 7.2 Hz, 3H).

**Step e.** To a solution of *trans-tert*-butyl 3-methoxy-4-((2-methoxy-2-oxoethoxy)methyl)pyrrolidine-1-carboxylate (2.00 g, 6.30 mmol) in THF (60 mL) was added DIBAL-H (1 M in toluene, 18.9 mL). The mixture was stirred at -78 °C for 2 h.

Upon completion, the reaction mixture was quenched with MeOH (2 mL) at -60 °C. The mixture was filtered and evaporated to give the title compound (2 g, crude) as a pale yellow oil, which was used in the next step directly.

**Intermediate 8f: *tert*-Butyl 3-(2-oxoethoxy)azetidine-1-carboxylate**

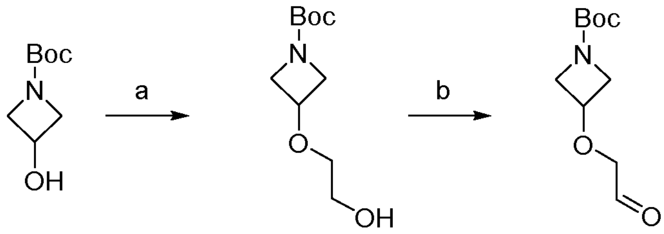


**Step a.** To a solution of *tert-butyl* 3-hydroxyazetidine-1-carboxylate (20.0 g, 115 mmol) in TEA (16.9 mL) was added TBAB (744 mg, 2.31 mmol) at rt, and then the mixture was stirred at rt for 5 h. Then 1,3-dioxolan-2-one (11.2 g, 127 mmol) was added. The mixture was refluxed at 100 °C for 48 h. Upon completion, the reaction was evaporated and purified by column chromatography (PE/EtOAc = 1/1) to give *tert-butyl* 3-(2-hydroxyethoxy) azetidine-1-carboxylate (11 g, 44% yield) as a yellow oil.

$^1$H NMR (400 MHz, $CDCl_3$) δ ppm 4.28 - 4.11 (m, 1H), 4.11 - 4.06 (m, 2H), 3.88 - 3.83 (m, 2H), 3.78 - 3.73 (m, 2H), 3.51 - 3.48 (m, 2H), 2.01 (t, *J* = 6.0 Hz, 1H), 1.44 (s, 9H).

**Step b.** To a solution of oxalyl dichloride (4.56 g, 35.9 mmol) in DCM (40 mL) was added dropwise DMSO (5.39 g, 71.8 mmol) at -78 °C. The mixture was stirred at -78 °C for 30 min. Then *tert-butyl* 3-(2-hydroxyethoxy) azetidine-1-carboxylate (3.00 g, 13.8 mmol) in DCM (60 mL) was added dropwise at -78 °C. The mixture was stirred at -78 °C for 30 min. Then TEA (6.99 g, 71.8 mmol) was added dropwise at -78 °C and the mixture was slowly warmed to rt. Upon completion, the reaction mixture was quenched with sat. aq. $NaHCO_3$ solution (50 mL) and extracted with DCM (3 x 50 mL). The combined organic layers were washed with brine (2 x 60 mL), dried over $Na_2SO_4$ and evaporated to give the title compound (3.08 g, crude) as a yellow oil.

$^1$H NMR (400 MHz, $CDCl_3$) δ ppm 9.70 (s, 1H), 4.29 (d, *J* = 6.4 Hz, 1H), 4.13 - 4.08 (m, 2H), 3.93 - 3.88 (m, 2H), 3.19-3.09 (m, 2H), 1.44 (s, 9H).

**Intermediate 8g: Benzyl 3-formyl-4-methyl-5-oxopiperazine-1-carboxylate**

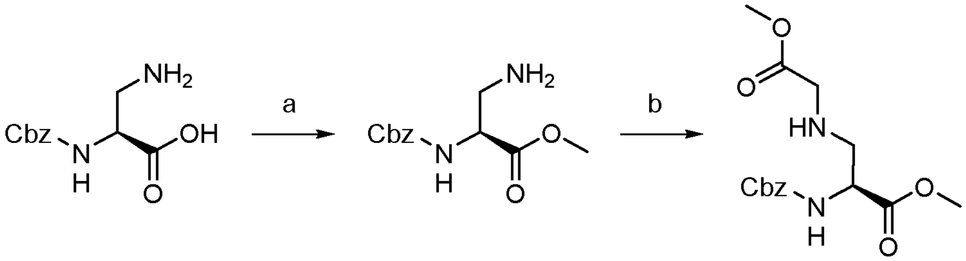

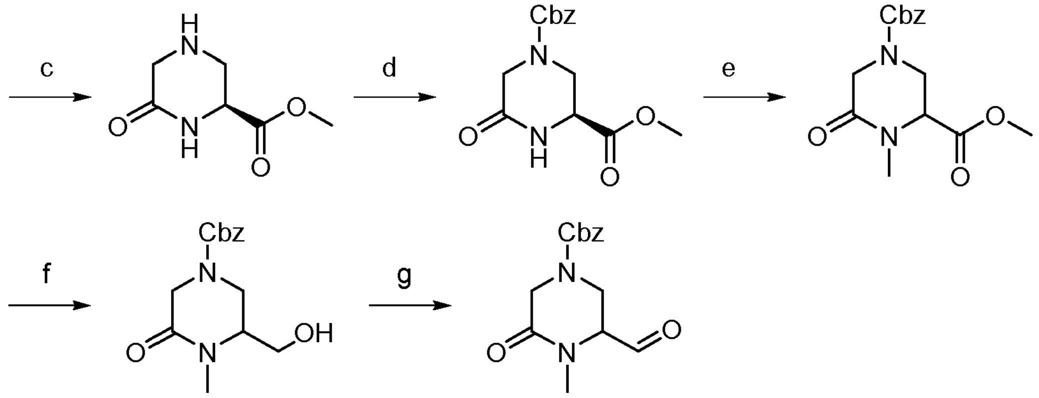


**Step a.** To a solution of (S)-3-amino-2-(((benzyloxy)carbonyl)amino)propanoic acid (50.0 g, 210 mmol) in MeOH (500 mL) was added $SOCl_2$ (30.0 g, 252 mmol). The reaction mixture was stirred at rt for 12 h. Upon completion, the reaction mixture was evaporated to give methyl (S)-3-amino-2-(((benzyloxy)carbonyl)amino)propanoate as a HCl salt (60.0 g, 99% yield) as a white solid.

m/z ES+ $[M+H]^+$ 253.1.

**Step b.** To a solution of methyl (S)-3-amino-2-(((benzyloxy)carbonyl)amino)propanoate (HCl salt, 60.0 g, 208 mmol) in THF (500 mL) was added DIPEA (80.6 g, 623 mmol). The reaction mixture was stirred at rt for 10 min. Then methyl 2-bromoacetate (63.6 g, 416 mmol) was added. The reaction mixture was stirred at rt for 2 h. Upon completion, the reaction mixture was diluted with water (500 mL) and extracted with EtOAc (200 mL). The organic layer was washed with brine (200 mL), filtered and evaporated. The residue was purified by column chromatography (PE/EtOAc = 1/1) to give methyl (*S*)-2-(((benzyloxy)carbonyl)amino)-3-((2-methoxy-2-oxoethyl)amino)propanoate (25.0 g, 37% yield) as a yellow oil.

m/z ES+ $[M+H]^+$ 325.1; $^1$H NMR (400 MHz, $CDCl_3$) δ ppm 7.42 - 7.28 (m, 5H), 5.81 (s, 1H), 5.13 (s, 2H), 4.47 - 4.36 (m, 1H), 3.83 - 3.65 (m, 6H), 3.39 (d, *J* = 3.2 Hz, 2H), 3.17 - 2.90 (m, 2H).

**Step c.** To a solution of methyl (S)-2-(((benzyloxy)carbonyl)amino)-3-((2-methoxy-2-oxoethyl)amino)propanoate (25.0 g, 77.1 mmol) in EtOH (150 mL) was added 10% Pd/C (3.0 g). The reaction mixture was stirred at 40 °C for 12 h under $H_2$ atmosphere (15 psi). Upon completion, the reaction mixture was filtered and evaporated to give methyl (S)-6-oxopiperazine-2-carboxylate (11.0 g, 90% yield) as a white solid.

m/z ES+ $[M+H]^+$ 158.2; $^1$H NMR (400 MHz, DMSO-$d_6$) δ ppm 7.82 (s, 1H), 4.06 - 3.97 (m, 1H), 3.74 - 3.59 (m, 3H), 3.17 (d, *J* = 1.2 Hz, 2H), 3.07 - 2.93 (m, 2H), 2.64 (s, 1H).

**Step d.** To a solution of methyl (*S*)-6-oxopiperazine-2-carboxylate (11.0 g, 69.6 mmol) and DIPEA (10.8 g, 83.5 mmol) in DCM (200 mL) was added benzyl chloroformate (14.2 g, 83.5 mmol). The reaction mixture was stirred at rt for 12 h. Upon completion, the reaction mixture was diluted with water (200 mL) and extracted with DCM (50 mL). The organic layer was washed with brine (200 mL), dried over $Na_2SO_4$ and evaporated. The residue was purified by column chromatography (EtOAc) to give 1-benzyl 3-methyl (*S*)-5-oxopiperazine-1,3-dicarboxylate (16.5 g, 81% yield) as a colorless oil.

m/z ES+ $[M+H]^+$ 293.2.

**Step e.** To a mixture of 1-benzyl 3-methyl (*S*)-5-oxopiperazine-1,3-dicarboxylate (15.0 g, 51.3 mmol) and $Cs_2CO_3$ (33.4 g, 103 mmol) in DMF (150 mL) was added methyl iodide (8.74 g, 61.6 mmol). The reaction mixture was stirred at rt for 1 h. Upon completion, the reaction mixture was diluted with water (400 mL) and extracted with EtOAc (400 mL). The organic layer was washed with brine (400 mL), dried over $Na_2SO_4$ and evaporated. The residue was purified by column chromatography (EtOAc) to give 1-benzyl 3-methyl 4-methyl-5-oxopiperazine-1,3-dicarboxylate (13.0 g, 83% yield) as a yellow oil.

m/z ES+ $[M+H]^+$ 307.2; $^1$H NMR (400 MHz, $CDCl_3$) δ ppm 7.41 - 7.29 (m, 5H), 5.24 - 5.04 (m, 2H), 4.62 - 4.41 (m, 2H), 4.06 - 3.81 (m, 2H), 3.80 - 3.38 (m, 4H), 2.99 (s, 3H).

**Step f.** To a suspension of $LiBH_4$ (4.05 g, 186 mmol) in THF (100 mL) was added a solution of 1-benzyl 3-methyl 4-methyl-5-oxopiperazine-1,3-dicarboxylate (19.0 g, 62.0 mmol) in THF (100 mL) at 0 °C. The reaction mixture was stirred at rt for 1 h, after which additional $LiBH_4$ (4.05 g, 186 mmol) was added. The reaction mixture was stirred at rt for an additional 1 h. Upon completion, the reaction mixture was slowly quenched with sat. aq. $NH_4Cl$ solution (200 mL) and extracted with EtOAc (200 mL). The organic layer was washed with brine (200 mL), dried over $Na_2SO_4$ and evaporated. The residue was purified by column chromatography (EtOAc) to give benzyl 3-(hydroxymethyl)-4-methyl-5-oxopiperazine-1-carboxylate (16.2 g, 94% yield) as a colorless oil.

m/z ES+ $[M+H]^+$ 279.0.

**Step g.** To a solution of $(COCl)_2$ (21.5 g, 169 mmol) in DCM (150 mL) was added DMSO (26.5 g, 338 mmol) at -78°C. The mixture was stirred at -78 °C for 10 min. Then a solution of benzyl 3-(hydroxymethyl)-4-methyl-5-oxopiperazine-1-carboxylate (15.7 g, 56.4 mmol) in DCM (50 mL) was added. The mixture was stirred at -78 °C for 30 min. TEA (57.1 g, 564 mmol) was added. The reaction mixture was stirred at -78 °C for 1 h. Upon completion, the reaction mixture was warmed to rt, diluted with water (200 mL) and extracted with EtOAc (200 mL). The organic extract was washed with brine (200 mL), dried over $Na_2SO_4$ and evaporated to give the title compound (13.0 g, 83% yield) as a yellow oil, which was used in the next step directly.

**Intermediate 8h: Benzyl (*S*)-2-formylmorpholine-4-carboxylate**

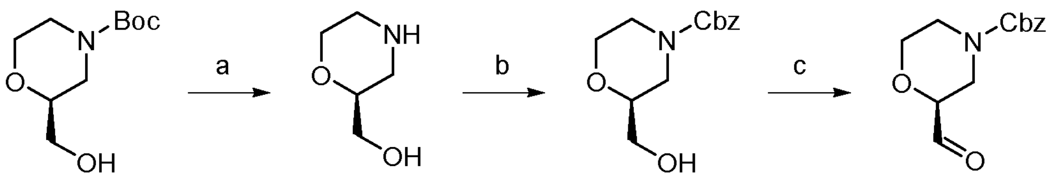


**Step a.** To a solution of tert-butyl (2*S*)-2-(hydroxymethyl)morpholine-4-carboxylate (5.00 g, 23.0 mmol) in HCl (4 M in EtOAc, 30 mL) was stirred at rt for 2 h. Upon completion, the reaction mixture was evaporated to give (*S*)-morpholin-2-ylmethanol as a HCl salt (3.50 g, 99% yield).

**Step b.** To a solution of (*S*)-morpholin-2-ylmethanol HCl salt (3.50 g, 22.8 mmol) in THF (45 mL) and water (15 mL) was added benzyl chloroformate (5.83 g, 34.2 mmol) and $NaHCO_3$ (5.74 g, 68.4 mmol). The mixture was stirred at rt for 3 h. Upon completion, the reaction mixture was diluted with water (100 mL) and extracted with EtOAc (3 x 100 mL). The combined organic layers were washed with brine (3 x 50 mL), dried over $Na_2SO_4$ and evaporated. The residue was purified by column chromatography (PE/EtOAc = 1/1) to give benzyl (*S*)-2-(hydroxymethyl)morpholine-4-carboxylate (4.90 g, 85% yield) as a colorless oil. $^1H$ NMR (400 MHz, $CDCl_3$) $\delta$ ppm 7.37 - 7.27 (m, 5H), 5.15 - 5.14 (m, 2H), 3.93 (s, 3H), 3.75 - 3.56 (m, 4H), 3.02 - 2.85 (m, 2H), 2.12 - 2.08 (m, 1H).

**Step c.** To a solution of benzyl (*S*)-2-(hydroxymethyl)morpholine-4-carboxylate (4.00 g, 15.9 mmol) in DCM (40 mL) was added Dess-Martin periodinane (13.5 g, 31.8 mmol) and the mixture was stirred at rt for 1 h. Upon completion, the reaction mixture was filtered and then quenched with sat. aq. $NaHCO_3$ (100 mL). The mixture was diluted with water (50 mL) and extracted with EtOAc (3 x 100 mL). The combined organic layers were washed with sat. aq. $Na_2S_2O_3$ (3 x 50 mL), dried over $Na_2SO_4$ and evaporated to give the title compound (3.90 g, crude) as a yellow oil.

$^1H$ NMR (400 MHz, $CDCl_3$) $\delta$ ppm 9.66 (s, 1H), 7.40 - 7.30 (m, 5H), 5.17 (s, 2H), 4.24 - 4.07 (m, 1H), 4.09 - 3.99 (m, 2H), 3.90 - 3.81 (m, 2H), 3.79 - 3.56 (m, 2H).

**Intermediate 8i: Benzyl (*R*)-2-formylmorpholine-4-carboxylatee**

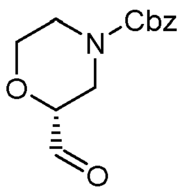

The title compound was prepared in a similar manner to **Intermediate 8h**, using *tert-butyl* (2*R*)-2-(hydroxymethyl)morpholine-4-carboxylate in step a.

$^1H$ NMR (400 MHz, $CDCl_3$) $\delta$ ppm 9.64 (s, 1H), 7.36 - 7.30 (m, 5H), 5.15 (s, 2H), 4.22 - 4.15 (m, 1H), 4.11 - 4.00 (m, 2H), 3.98 - 3.87 (m, 2H), 3.80 - 3.65 (m, 2H).

**Intermediate 9a: (*S,E*)-3-(1-Methylpyrrolidin-2-yl)acrylic acid**

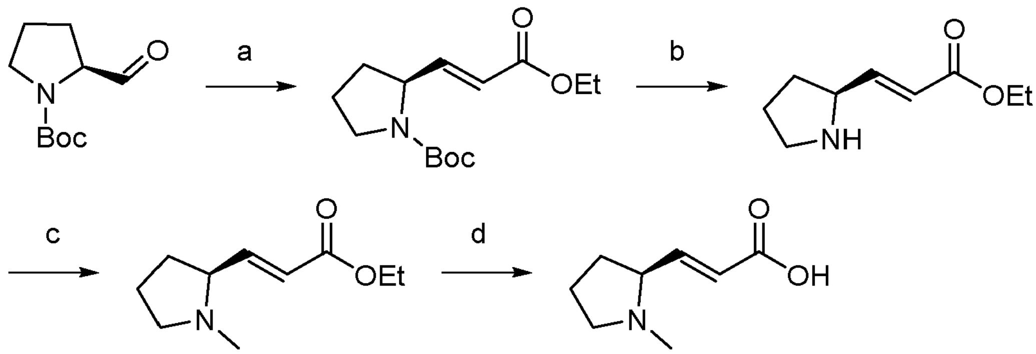


**Step a.** To a mixture of NaH (120 mg, 3.01 mmol, dispersion in mineral oil) in THF (5 mL) was added ethyl 2-diethoxyphosphorylacetate (675 mg, 3.01 mmol) at -78 °C. The mixture was stirred at -78 °C for 10 min. A solution of (*S*)-tert-butyl 2-formylpyrrolidine-1-carboxylate (0.5 g, 2.51 mmol) in THF (5 mL) was added. The reaction mixture was stirred at rt for 1 h. The reaction mixture was quenched with water (30 mL) and extracted with EtOAc (30 mL). The organic layer was washed with brine (30 mL), dried over $Na_2SO_4$ and evaporated. The residue was purified by column chromatography (PE/EtOAc = 5/1) to give *tert-butyl* (*S,E*)-2-(3-ethoxy-3-oxoprop-1-en-1-yl)pyrrolidine-1-carboxylate (0.64 g, 95% yield) as an oil.

$^1$H NMR (400 MHz, $CDCl_3$) δ ppm 6.93 - 6.72 (m, 1H), 5.82 (d, *J* = 15.6 Hz, 1H), 4.58 - 4.32 (m, 1H), 4.20 (d, *J* = 6.4 Hz, 2H), 3.44 (d, *J* = 5.2 Hz, 2H), 2.17 - 1.98 (m, 1H), 1.91 - 1.81 (m, 2H), 1.78 (d, *J* = 4.8 Hz, 1H), 1.50 - 1.39 (m, 9H), 1.34 - 1.25 (m, 3H).

**Step b.** To a solution of *tert-butyl* (*S,E*)-2-(3-ethoxy-3-oxoprop-1-en-1-yl)pyrrolidine-1-carboxylate (0.63 g, 2.34 mmol) in DCM (5 mL) was added TFA (1.54 g, 13.5 mmol). The reaction mixture was stirred at rt for 1 h. Upon completion, the reaction mixture was evaporated. The residue was diluted with sat. aq. $NaHCO_3$ (20 mL) and extracted with a 3:1 mixture of DCM:2-propanol (5 x 30 mL). The organic layer was dried over $Na_2SO_4$ and evaporated to give ethyl (*S,E*)-3-(pyrrolidin-2-yl)acrylate (0.39 g, 99% yield) as an oil.

m/z ES+ $[M+H]^+$ 170.2.

**Step c.** A solution of ethyl (*S,E*)-3-(pyrrolidin-2-yl)acrylate (0.35 g, 2.07 mmol) in DCM (7 mL) was treated with aq. formaldehyde (1.01 g, 12.4 mmol, 37% w/w) and $NaBH(OAc)_3$ (1.75 g, 8.27 mmol) at 0 °C. The reaction mixture was stirred at rt for 12 h. Upon completion, the reaction mixture was filtered and evaporated. The residue was diluted with water (30 mL) and extracted with a 3:1 mixture of DCM:2-propanol (5 x 30 mL). The organic layer was dried over $Na_2SO_4$ and evaporated to give ethyl (*S,E*)-3-(1-methylpyrrolidin-2-yl)acrylate (0.37 g, 98% yield) as a yellow oil.

$^1$H NMR (400 MHz, $CDCl_3$) δ ppm 6.94 (dd, *J* = 9.2, 15.6 Hz, 1H), 6.08 (d, *J* = 15.6 Hz, 1H), 4.27 - 4.19 (m, 2H), 3.60 - 3.40 (m, 2H), 2.90 - 2.78 (m, 1H), 2.60 (s, 3H), 2.30 - 2.11 (m, 2H), 2.05 - 1.96 (m, 2H), 1.31 (t, *J* = 7.2 Hz, 3H).

**Step d.** To a solution of ethyl (S,E)-3-(1-methylpyrrolidin-2-yl)acrylate (0.2 g, 1.09 mmol) in a mixed solvent of THF (2 mL) and water (2 mL) was added LiOH monohydrate (137 mg, 3.27 mmol). The reaction mixture was stirred at rt for 1 h. Upon completion, the reaction mixture was acidified to pH 5 with 1 M aq. HCl and evaporated. The residue was lyophilized to give the title compound (0.15 g, 89% yield, crude) as a yellow solid, which was used in the next step directly.

**Intermediate 9b: (*R,E*)-3-(1-Methylpyrrolidin-2-yl)acrylic acid**

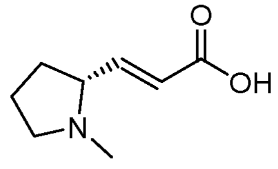

The title compound was prepared in a similar manner to **Intermediate 9a,** using (*R*)-*tert*-butyl 2-formylpyrrolidine-1-carboxylate in step a. The crude product was used in the next step directly.

**Intermediate 10: (*E*)-4-Bromobut-2-enoyl chloride**

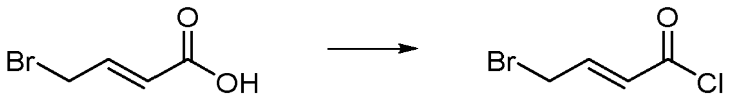

A mixture of (*E*)-4-bromobut-2-enoic acid (100 mg, 0.61 mmol) and DMF (4 mg, 0.06 mmol) in DCM (1 mL) was degassed and purged 3 times with $N_2$, and then a solution of oxalyl chloride (154 mg, 1.21 mmol) in DCM (0.2 mL) was added at 0 °C. The mixture was stirred at 0 °C for 1 h under a $N_2$ atmosphere. Upon completion, the reaction mixture was evaporated at rt to give the title compound (110 mg, crude) as a yellow oil, which was used in the next step directly.

**Intermediate 11: (*S*)-2-((7-chloro-6-(2,2-dimethoxyethyl)-1-methyl-2-oxo-1,2,3,4,5,6-hexahydrobenzo[*b*][1,4]diazocin-3-yl)amino)-6-methyl-4-(trifluoromethyl)nicotinonitrile**

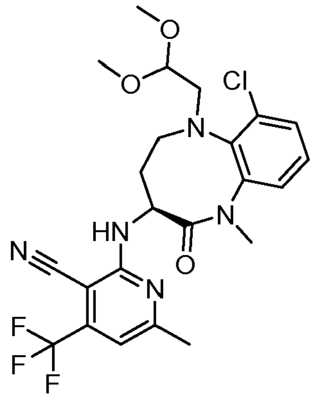

The title compound was prepared in a similar manner to **Example 40,** steps a-g, using 2,2-dimethoxyacetaldehyde (CAS: 51673-84-8) in step a, and 1-chloro-2-fluoro-3-nitrobenzene (CAS: 2106-49-2) in step b.

$^1H$ NMR (400 MHz, $CDCl_3$) δ ppm 7.44 (d, *J* = 7.6 Hz, 1H), 7.26 - 7.19 (m, 2H), 6.68 (s, 1H), 6.18 (d, *J* = 7.2 Hz, 1H), 4.43 (dd, *J* = 7.2, 10.8 Hz, 1H), 4.27 (t, *J* = 5.2 Hz, 1H), 3.44 - 3.38 (m, 1H), 3.37 - 3.33 (m, 9H), 3.26 - 3.17 (m, 1H), 3.16 - 3.10 (m, 1H), 3.08 - 3.01 (m, 1H), 2.38 - 2.35 (m, 4H), 1.76 - 1.70 (m, 1H).

m/z ES+ $[M+Na]^+$ 534.1.

**Intermediate 12: (S)-2-((7-chloro-1-methyl-2-oxo-6-(2-oxoethyl)-1,2,3,4,5,6-hexahydrobenzo[*b*][1,4]diazocin-3-yl)amino)-6-methyl-4-(trifluoromethyl)nicotinonitrile**

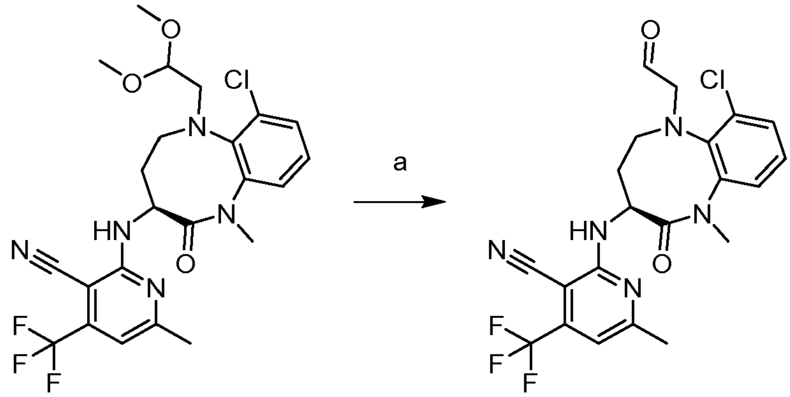


**Step a.** A solution of **Intermediate 11** (200 mg, 0.39 mmol) in formic acid (18.8 mg, 0.39 mmol) was stirred at 25 °C for 1 h. Upon completion, the reaction mixture was evaporated and the residue was purified by Prep-TLC (PE/EtOAc=1/1) to give the title compound (170 mg, 88% yield) as brown oil.

m/z ES+ $[M+H]^+$ 466.0.

**Intermediate 13: (*S*)-2-((1,7-dimethyl-2-oxo-6-(2-oxoethyl)-1,2,3,4,5,6-hexahydrobenzo[*b*][1,4]diazocin-3-yl) amino)-6-methyl-4-(trifluoromethyl)nicotinonitrile**

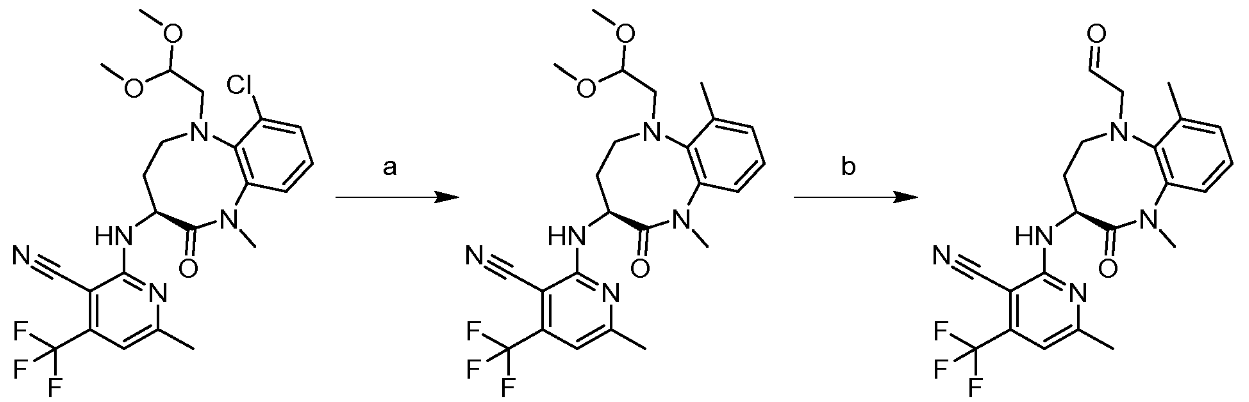


**Step a.** A mixture of **Intermediate 11** (750 mg, 1.5 mmol), methylboronic acid (1.75 g, 29.3 mmol), $Cs_2CO_3$ (1.43 g, 4.40 mmol) and Xphos-Pd-G2 (231 mg, 0.29 mmol) in toluene (8 mL) was stirred at 110 °C for 16 h under a $N_2$ atmosphere. Upon completion, the reaction mixture was filtered, the filtrate diluted with water (10 mL), extracted with DCM (3 x 5 mL), dried over $Na_2SO_4$, evaporated. The residue was purified by column chromatography (PE/EtOAc = 3/1) to give **(*S*)-2-((6-(2,2-dimethoxyethyl)-1,7-dimethyl-2-oxo-1,2,3,4,5,6-hexahydrobenzo[*b*][1,4]diazocin-3-yl)amino)-6-methyl-4-(trifluoromethyl)nicotinonitrile** (540 mg, 66% yield) as yellow oil.

m/z ES+ $[M+H]^+$ 492.2.

**Step b.** This step was conducted in a similar manner to **Intermediate 12**, step a.

m/z ES+ $[M+H]^+$ 446.3.

**Intermediate 14: (*S*)-2-((1-ethyl-7-fluoro-2-oxo-6-(2-oxoethyl)-1,2,3,4,5,6-hexahydrobenzo[*b*][1,4]diazocin-3-yl) amino)-6-methyl-4-(trifluoromethyl)nicotinonitrile**

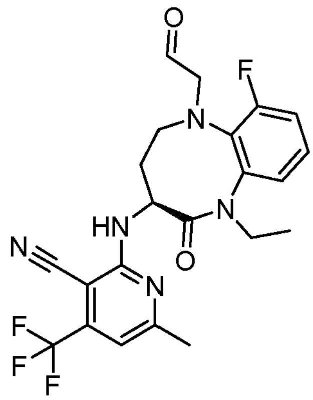

The title compound was prepared in a similar manner to **Intermediate 11** and **Intermediate 12.**

m/z ES+ $[M+H]^+$ 464.1

**Intermediate 15: (*S*)-2-(4-((3-cyano-6-methyl-4-(trifluoromethyl)pyridin-2-yl)amino)-10-fluoro-6-methyl-5-oxo-3,4,5,6-tetrahydrobenzo[b][1,4]diazocin-1(2*H*)-yl)acetic acid**

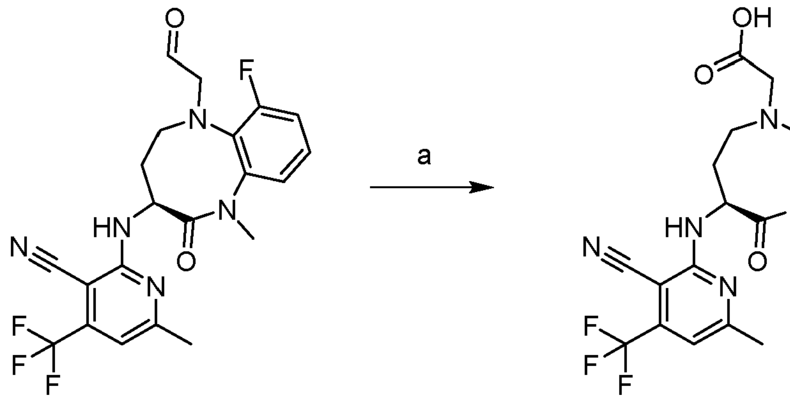


**Step a.** To a solution of **Intermediate 5** (200 mg, 0.45 mmol), $NaH_2PO_4$ (160 mg, 1.34 mmol) and 2-methylbut-2-ene (437 mg, 6.0 mmol) in *t*-BuOH (2 mL) and $H_2O$ (2 mL) was added sodium chlorite (121 mg, 1.34 mmol). The mixture was stirred at 25 °C for 12 h. Upon completion, the reaction mixture was acidified to pH = 5 using aq. 0.5 M HCl, extracted with EtOAc (3 x 10 mL), washed with brine (30 mL), dried over $Na_2SO_4$, and evaporated. The residue was purified by Prep-TLC (PE/EtOAc=1/3) to give the title compound (100 mg, 43% yield) as off-white solid.

$^1H$ NMR (400 MHz, $CDCl_3$) δ ppm 7.35 - 7.28 (m, 1H), 7.21 - 7.18 (m, 1H), 7.17 - 7.12 (m, 1H), 6.71 (s, 1H), 6.37 (d, *J* = 7.2 Hz, 1H), 4.59 - 4.54 (m, 1H), 3.87 (s, 2H), 3.52 - 3.46 (m, 1H), 3.36 (s, 3H), 3.29 - 3.22 (m, 1H), 2.38 (s, 3H), 2.36 - 2.28 (m, 1H), 1.99 - 1.91 (m, 1H). m/z ES+ $[M+H]^+$ 466.1.

**Example 1**

**(*S,E*)-2-((6-(2-(2-(4-(Dimethylamino)but-2-enoyl)-5-oxa-2,8-diazaspiro[3.5]nonan-8-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile (E1)**

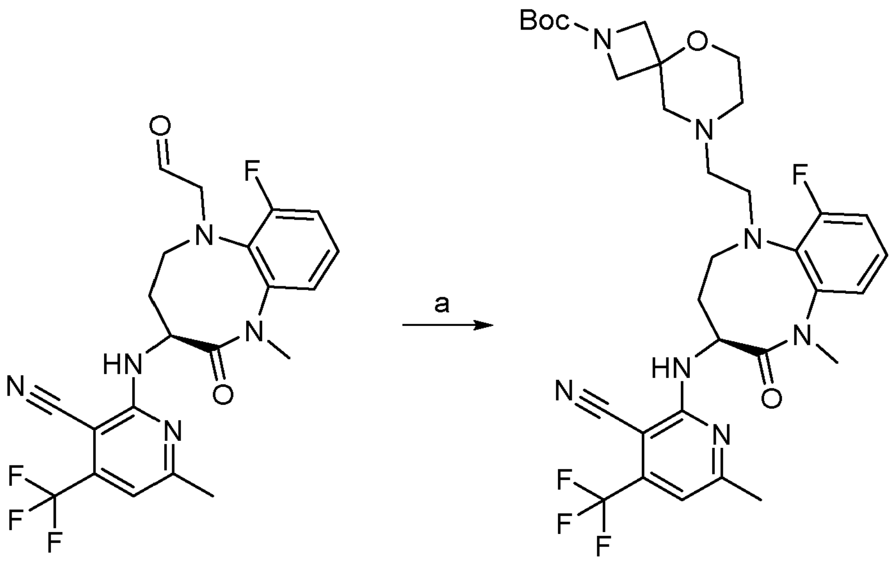

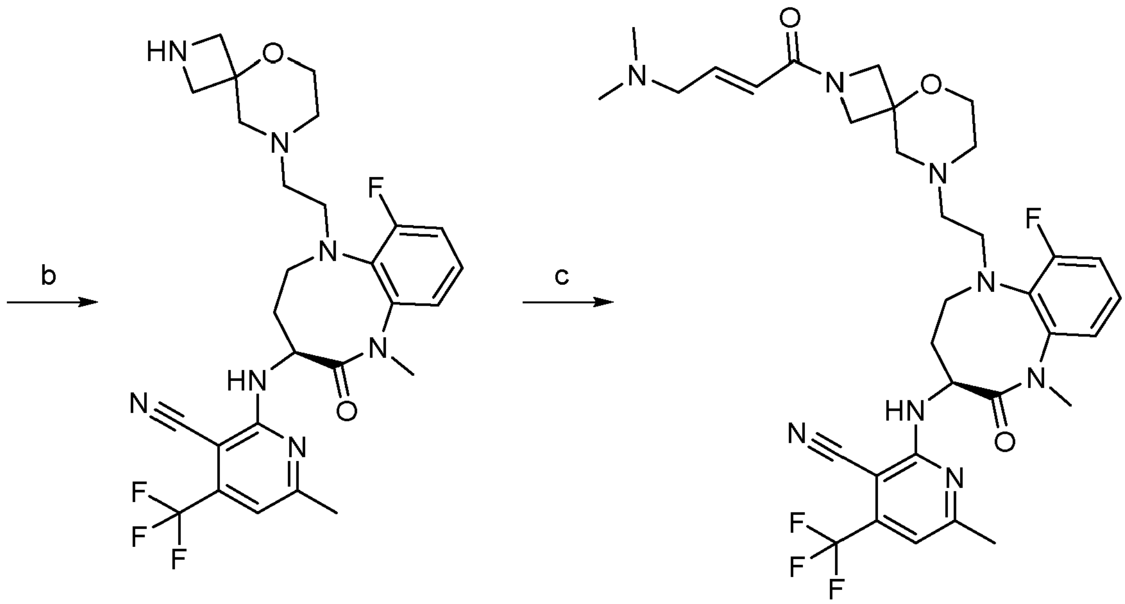


**Step a.** To a solution of **Intermediate 5** (230 mg, 0.51 mmol) and *tert-butyl* 5-oxa-2,8-diazaspiro[3.5]nonane-2-carboxylate (CAS Number 1251011-05-8; 140 mg, 0.62 mmol) in DCE (8 mL) was added $NaBH(OAc)_3$ (325 mg, 1.54 mmol). The mixture was stirred at rt for 12 h. Upon completion, the reaction mixture was quenched with water (0.5 mL) at 0 °C and evaporated. The residue was purified by column chromatography (EtOAc) to give (*S*)-*tert*-butyl 8-(2-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)-5-oxa-2,8-diazaspiro[3.5]nonane-2-carboxylate (200 mg, 56% yield) as a yellow solid.

m/z ES+ $[M+H]^+$ 662.4.

**Step b.** To a solution of (*S*)-*tert*-butyl 8-(2-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)-5-oxa-2,8-diazaspiro[3.5]nonane-2-carboxylate (200 mg, 0.30 mmol) in DCM (5 mL) was added TFA (1.54 g, 13.5 mmol). The mixture was stirred at rt for 1 h. Upon completion, The reaction mixture was evaporated to give (*S*)-2-((6-(2-(5-oxa-2,8-diazaspiro[3.5]nonan-8-yl)ethyl)-7-fluoro-1-methyl-2-oxo-1,2,3,4,5,6-hexahydrobenzo[b][1,4]diazocin-3-yl)amino)-6-methyl-4-(trifluoromethyl)nicotinonitrile as a TFA salt (200 mg, crude) as a brown gum.

m/z ES+ $[M+H]^+$ 562.3.

**Step c.** To a solution of (*S*)-2-((6-(2-(5-oxa-2,8-diazaspiro[3.5]nonan-8-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile (100 mg, 0.15 mmol) and (*E*)-4-(dimethylamino)but-2-enoic acid hydrochloride (29 mg, 0.18 mmol) in DCM (3 mL) was added HATU (67 mg, 0.18 mmol) and DIPEA (96 mg, 0.74 mmol). The mixture was stirred at rt for 12 h. Upon completion, the reaction mixture was filtered and evaporated. The residue was purified by Prep-HPLC to give the title compound (30 mg, 30% yield) as a white solid.

m/z ES+ $[M+H]^+$ 673.2; $^1H$ NMR (400 MHz, $CD_3OD$) δ ppm 7.40 - 7.32 (m, 1H), 7.31 - 7.26 (m, 1H), 7.19 (t, *J* = 9.6 Hz, 1H), 6.86 (s, 1H), 6.82 - 6.73 (m, 1H), 6.24 - 6.161 (m, 1H), 4.37 (d, *J* = 11.2 Hz, 1H), 4.18 - 4.12 (m, 1H), 4.10 - 4.03 (m, 1H), 3.88 - 3.77 (m, 2H), 3.65 (d, *J* = 4.0 Hz, 2H), 3.47 - 3.38 (m, 1H), 3.33 (s, 3H), 3.26 - 3.20 (m, 1H), 3.16 (d, *J* = 5.2 Hz, 3H), 3.12 - 3.05 (m, 1H), 2.66 - 2.48 (m, 2H), 2.43 (s, 1H), 2.40 (s, 3H), 2.40 - 2.28 (m, 4H), 2.27 - 2.21 (m, 6H), 1.81 (d, *J* = 13.6 Hz, 1H).

**Example 2**

**2-(((S)-6-(2-(2-((E)-4-(Dimethylamino)but-2-enoyl)-6-oxa-2,9-diazaspiro[4.5]decan-9-yl)ethyl)-7-fluoro-1-methyl-2-oxo-1,2,3,4,5,6-hexahydrobenzo[b][1,4]diazocin-3-yl)amino)-6-methyl-4-(trifluoromethyl)nicotinonitrile (E2)**

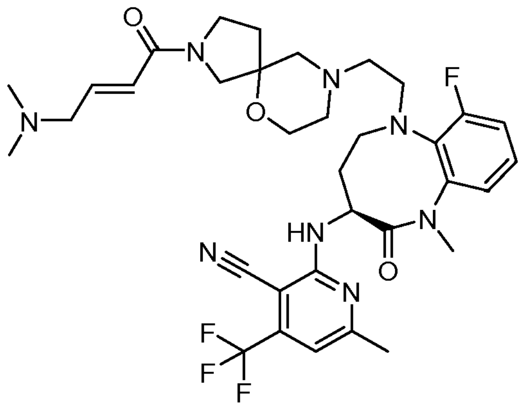

The title compound was prepared in a similar manner to **Example 1**, using *tert*-butyl 6-oxa-2,9-diazaspiro[4.5] decane-2-carboxylate (CAS Number 1366647-28-0) in step a.

m/z ES+ $[M+H]^+$ 687.4; $^1H$ NMR (400 MHz, $CDCl_3$) δ ppm 7.33 - 7.28 (m, 1H), 7.25 (s, 1H), 7.17 - 7.09 (m, 2H), 6.96 - 6.86 (m, 1H), 6.68 (d, *J* = 2.8 Hz, 1H), 6.30 - 6.24 (m, 1H), 6.23 - 6.17 (m, 1H), 4.49 - 4.41 (m, 1H), 3.80 (dd, *J* = 5.2, 13.6 Hz, 1H), 3.75 - 3.58 (m, 5H), 3.51 (dd, *J* = 4.8, 11.2 Hz, 1H), 3.39 - 3.33 (m, 1H), 3.31 - 3.28 (m, 3H), 3.19 - 3.11 (m, 3H), 3.10 - 3.06 (m, 2H), 2.52 - 2.39 (m, 2H), 2.37 (s, 3H), 2.29 (d, *J* = 6.8 Hz, 2H), 2.26 (s, 6H), 2.22 (s, 1H), 2.03 (td, *J* = 6.8, 12.8 Hz, 1H), 1.85 - 1.80 (m, 2H).

**Example 3**

**(S,E)-2-((6-(2-(2-(4-(Dimethylamino)but-2-enoyl)-5-oxa-2,8-diazaspiro[3.5]nonan-8-yl)ethyl)-1-methyl-2-oxo-1,2,3,4,5,6-hexahydrobenzo[b][1,4]diazocin-3-yl)amino)-6-methyl-4-(trifluoromethyl)nicotinonitrile (E3)**

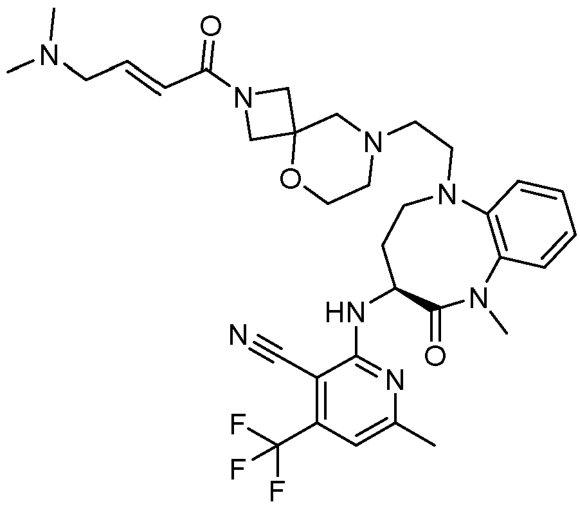

The title compound was prepared in a similar manner to **Example 1**, using (*S*)-2-((1-methyl-2-oxo-6-(2-oxoethyl)-1,2,3,4,5,6-hexahydrobenzo[b][1,4]diazocin-3-yl)amino)-6-methyl-4-(trifluoromethyl)nicotinonitrile (prepared in a similar manner to **Intermediate 5** starting with *tert*-butyl *N*-(2-aminophenyl)carbamate) in step a.

m/z ES+ $[M+H]^+$ 655.1; $^1H$ NMR (400 MHz, $CD_3OD$) δ ppm 7.44 - 7.33 (m, 3H), 7.23 - 7.13 (m, 1H), 6.88 (s, 1H), 6.85 - 6.75 (m, 1H), 6.28 - 6.17 (m, 1H), 4.55 (m, 1H), 4.21 - 4.10 (m, 1H), 4.10 - 4.06 (m, 1H), 3.93 - 3.79 (m, 2H), 3.68 (m, 2H), 3.53 (m, 1H), 3.45 - 3.36 (m, 1H), 3.34 - 3.32 (m, 2H), 3.27 (d, *J* = 6.8 Hz, 3H), 3.19 (t, *J* = 5.6 Hz, 2H), 3.14 - 3.07 (m, 1H), 2.66 - 2.57 (m, 1H), 2.54 - 2.43 (m, 4H), 2.41 (s, 3H), 2.29 (s, 6H), 2.14 - 1.96 (m, 2H).

**Example 4**

**(S,E)-2-((6-(2-(6-(4-(Dimethylamino)but-2-enoyl)-2,6-diazaspiro[3.3]heptan-2-yl)ethyl)-1-methyl-2-oxo-1,2,3,4,5,6-hexahydrobenzo[b][1,4]diazocin-3-yl)amino)-6-methyl-4-(trifluoromethyl)nicotinonitrile (E4)**

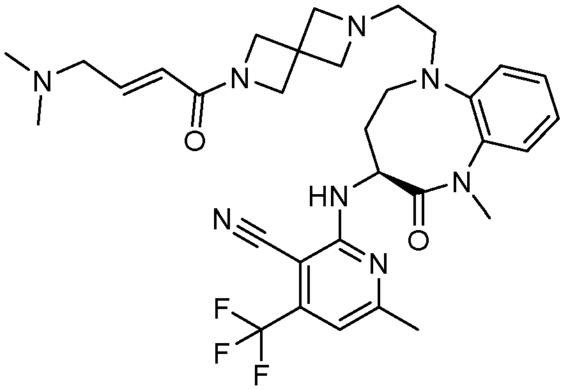

The title compound was prepared in a similar manner to **Example 3,** using tert-butyl 2,6-diazaspiro[3.3] heptane-2-carboxylate (CAS Number 1041026-70-3) in step a.

m/z ES+ $[M+H]^+$ 625.4; $^1H$ NMR (400 MHz, $CD_3OD$) δ ppm 7.42 - 7.27 (m, 3H), 7.21 - 7.13 (m, 1H), 6.85 (s, 1H), 6.74 (td, *J* = 6.4, 15.2 Hz, 1H), 6.13 (d, *J* = 15.2 Hz, 1H), 4.51 (dd, *J* = 3.2, 11.2 Hz, 1H), 4.33 (s, 2H), 4.07 (s, 2H), 3.54 - 3.34 (m, 5H), 3.26 (s, 3H), 3.23 - 3.15 (m, 1H), 3.14 - 3.09 (m, 3H), 3.07 (m, 1H), 2.64 - 2.53 (m, 1H), 2.51 - 2.43 (m, 1H), 2.39 (s, 3H), 2.24 (s, 6H), 2.13 - 2.00 (m, 1H), 2.00 - 1.87 (m, 1H).

**Example 5**

**(S,E)-2-((6-(2-(4-(4-(Dimethylamino)but-2-enoyl)piperazin-1-yl)ethyl)-1-methyl-2-oxo-1,2,3,4,5,6-hexahydrobenzo[b][1,4]diazocin-3-yl)amino)-6-methyl-4-(trifluoromethyl)nicotinonitrile (E5)**

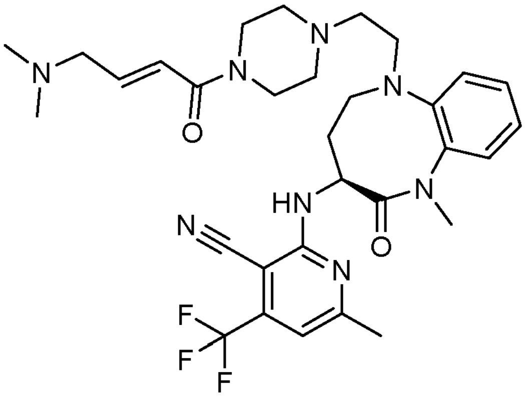

The title compound was prepared in a similar manner to **Example 3**, using 1-(tert-butoxycarbonyl)piperazine (CAS Number 57260-71-6) in step a.

m/z m/z ES+ $[M+H]^+$ 613.3; $^1H$ NMR (400 MHz, $CDCl_3$) δ ppm 7.36 - 7.30 (m, 1H), 7.29 - 7.20 (m, 2H), 7.14 - 7.08 (m, 1H), 6.87 - 6.80 (m, 1H), 6.68 (s, 1H), 6.41 (d, *J* = 15.2 Hz, 1H), 6.17 (d, *J* = 6.8 Hz, 1H), 4.67 - 4.61 (m, 1H), 3.66 (s, 2H), 3.61 - 3.48 (m, 3H), 3.44 - 3.33 (m, 1H), 3.29 (s, 3H), 3.28 - 3.22 (m, 1H), 3.15 - 3.05 (m, 3H), 2.54 - 2.42 (m, 6H), 2.37 (s, 3H), 2.28 (s, 6H), 2.06 - 1.96 (m, 2H).

**Example 6**

**(*S*)-2-((6-(2-(2-Acryloyl-5-methyl-2,5,8-triazaspiro[3.5]nonan-8-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile (E6)**

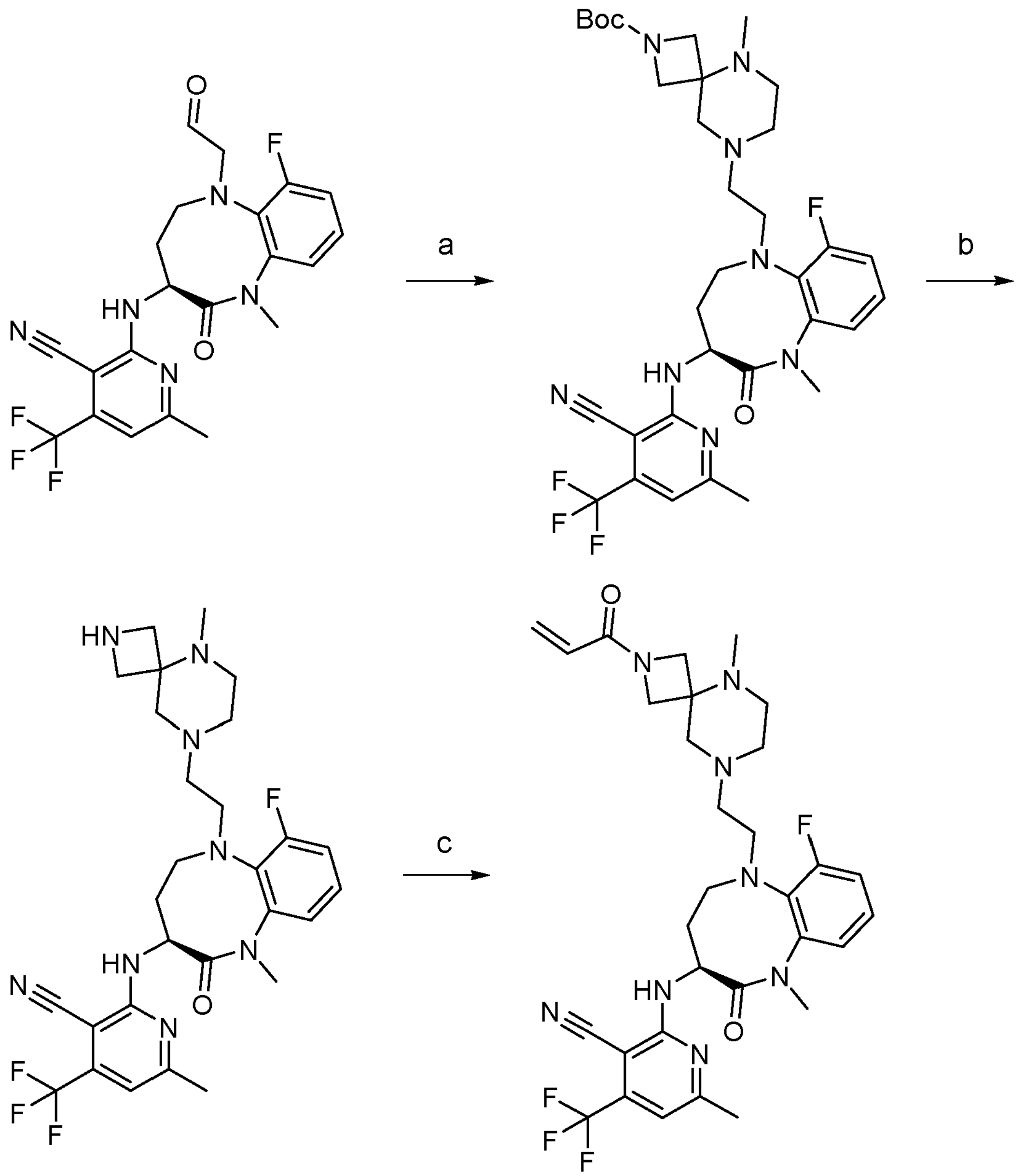


**Step** a. To a solution of **Intermediate 5** (250 mg, 0.56 mmol) and **Intermediate 6b** (147 mg, 0.611 mmol) in DCE (5 mL) was added $NaBH(OAc)_3$ (294 mg, 1.39 mmol). The mixture was stirred at rt for 1 h. Upon completion, the reaction mixture was diluted with DCM (5 mL) and washed with water (2 x 5 mL), brine (5 mL), dried over $Na_2SO_4$ and evaporated. The residue was purified by column chromatography (DCM/MeOH = 10/1) to afford *tert-butyl* (S)-8-(2-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)-5-methyl-2,5,8-triazaspiro[3.5]nonane-2-carboxylate (80 mg, crude) as a white solid.

m/z ES+ $[M+H]^+$675.4.

**Step b.** To a solution of *tert*-butyl (*S*)-8-(2-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)-5-methyl-2,5,8-triazaspiro[3.5]nonane-2-carboxylate (30 mg, 0.044 mmol) in DCM (2 mL) was added TFA (0.4 mL). The mixture was stirred at rt for 30 min. Upon completion, the reaction mixture was evaporated. The residue was diluted with DCM (8 mL) and washed with sat. aq. $NaHCO_3$ (3 x 5 mL). The combined organic layers were washed with brine (5 mL), dried over $Na_2SO_4$ and evaporated to give (*S*)-2-((7-fluoro-1-methyl-6-(2-(5-methyl-2,5,8-triazaspiro[3.5]nonan-8-yl)ethyl)-2-oxo-1,2,3,4,5,6-hexahydrobenzo

[*b*][1,4]diazocin-3-yl)amino)-6-methyl-4-(trifluoromethyl)nicotinonitrile (30.0 mg, crude) as a brown oil.

**Step c.** To a solution of (*S*)-2-((7-fluoro-1-methyl-6-(2-(5-methyl-2,5,8-triazaspiro[3.5]nonan-8-yl)ethyl)-2-oxo-1,2,3,4,5,6-hexahydrobenzo[b][1,4]diazocin-3-yl)amino)-6-methyl-4-(trifluoromethyl)nicotinonitrile (30 mg, 0.052 mmol) in DCM (1.5 mL) was added TEA (10.5 mg, 0.10 mmol) and acryloyl chloride (5 mg, 0.052 mmol). The mixture was stirred at rt for 30 min. Upon completion, the reaction mixture was diluted with DCM (5 mL), washed with water (5 mL) and brine (5 mL), dried over $Na_2SO_4$ and evaporated. The residue was purified by Prep-HPLC to give the title compound (10 mg, 29% yield) as a yellow solid.

m/z ES+ $[M+H]^+$629.3; $^1H$ NMR (400 MHz, DMSO-$d_6$) δ ppm 7.33 - 7.29 (m, 1H), 7.16 - 7.11 (m, 2H), 6.69 (s, 1H), 6.39 - 6.34 (m, 1H), 6.29 - 6.23 (m, 1H), 6.19 (d, *J* = 7.2 Hz, 1H), 5.74 - 5.70 (m, 1H), 4.47 - 4.35 (m, 3H), 4.19 (d, *J* = 10.4 Hz, 1H), 3.73 (d, *J* = 9.6 Hz, 1H), 3.39 - 3.32 (m, 4H), 3.18 - 3.10 (m, 2H), 2.79 - 2.62 (m, 7H), 2.49 (s, 3H), 2.27 - 2.20 (m, 1H), 1.80 (s, 3H), 2.24 (s, 1H), 1.82 - 1.78 (m, 2H).

**Example 7**

**(*S*)-2-((6-(2-(6-Acryloyl-2,6-diazaspiro[3.3]heptan-2-yl)ethyl)-8-chloro-1-methyl-2-oxo-1,2,3,4,5,6-hexahydrobenzo[*b*][1,4]diazocin-3-yl)amino)-6-methyl-4-(trifluoromethyl)nicotinonitrile (E7)**

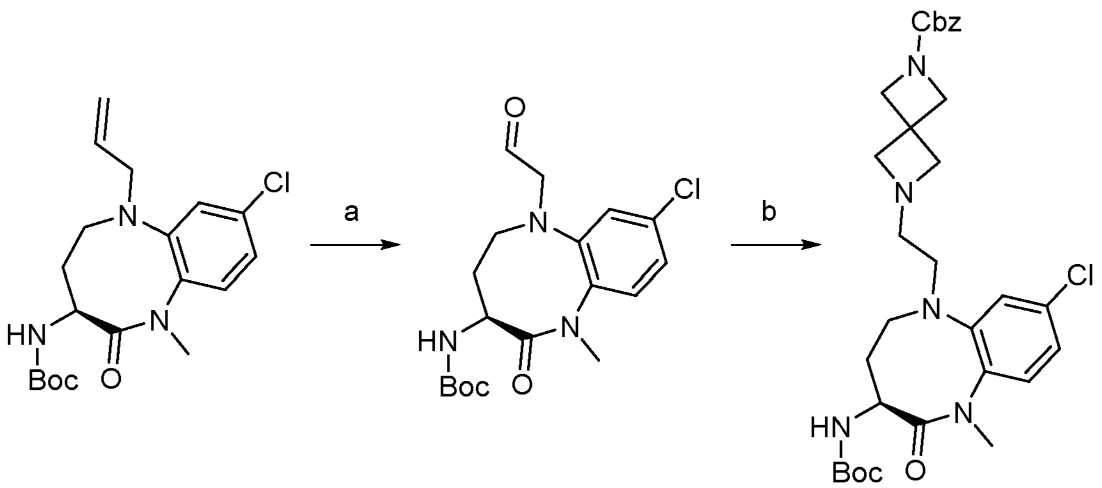

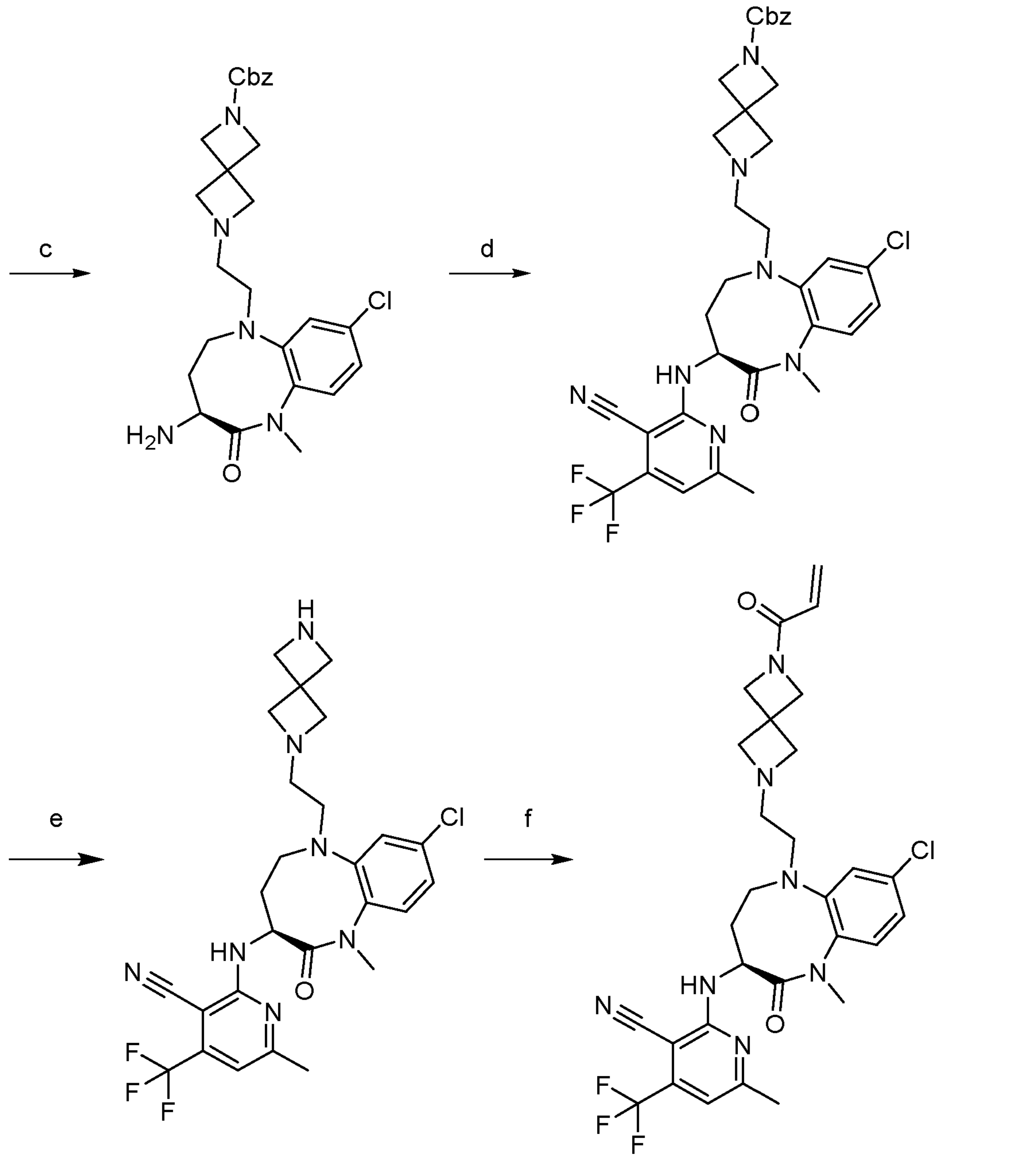


**Step** a. To a solution of tert-butyl (*S*)-(6-allyl-8-chloro-1-methyl-2-oxo-1,2,3,4,5,6-hexahydrobenzo[*b*][1,4]diazocin-3-yl)carbamate (prepared in a similar manner to **Intermediate 4** starting with **Intermediate 2c**; 0.7 g, 1.84 mmol) in THF (10 mL) and water (10 mL) was added $NaIO_4$ (1.18 g, 5.53 mmol). The mixture was treated with $OsO_4$ (47 mg, 0.18 mmol) and stirred at 0 °C for 3 h. Upon completion, the reaction mixture was quenched with sat. aq. $Na_2S_2O_3$ (15 mL) at rt and extracted with EtOAc (3 x 30 mL). The combined organic layers were washed with sat. aq. $Na_2S_2O_3$ (3 x 30 mL), dried over $Na_2SO_4$ and evaporated to give *tert*-butyl (*S*)-(8-chloro-1-methyl-2-oxo-6-(2-oxoethyl)-1,2,3,4,5,6-hexahydrobenzo [*b*][1,4]diazocin-3-yl)carbamate (0.8 g, crude) as a brown solid.

m/z ES+ $[M\text{-}tBu]^+$ 326.2.

**Step b.** To a solution of *tert-butyl* (*S*)-(8-chloro-1-methyl-2-oxo-6-(2-oxoethyl)-1,2,3,4,5,6-hexahydrobenzo[*b*] [1,4]diazocin-3-yl)carbamate (800 mg, 2.10 mmol) and benzyl 2,6-diazaspiro[3.3]heptane-2-carboxylate (535 mg, 2.30 mmol) in DCM (10 mL) was added acetic acid (13 mg, 0.21 mmol), $NaBH(OAc)_3$ (1.33 g, 6.29 mmol) and 4 Å molecular sieves (800 mg). The mixture was stirred at rt for 16 h. Upon completion, the reaction mixture was filtered and evaporated. The residue was purified by column chromatography (EtOAc/MeOH = 10/1) to give benzyl (*S*)-6-(2-(4-((*tert*-butoxycarbonyl)amino)-9-chloro-6-methyl-5-oxo-3,4,5,6-tetrahydrobenzo[*b*][1,4]diazocin-1(2*H*)-yl)ethyl)-2,6-diazaspiro[3.3]heptane-2-carboxylate (470 mg, 36% yield) as a brown solid.

m/z ES+ $[M+H]^+$ 598.5.

**Step c.** A solution of benzyl (*S*)-6-(2-(4-((*tert*-butoxycarbonyl)amino)-9-chloro-6-methyl-5-oxo-3,4,5,6-tetrahydrobenzo[*b*][1,4]diazocin-1(2*H*)-yl)ethyl)-2,6-diazaspiro[3.3]heptane-2-carboxylate (470 mg, 0.79 mmol) in DCM (5 mL) and TFA (0.5 mL) was stirred at rt for 16 h. Upon completion, the reaction mixture was evaporated. The residue was diluted with EtOAc (10 mL), treated with water (2 mL) and adjusted to pH 8-9 with sat. aq. $NaHCO_3$ (8 mL) and evaporated. The residue was diluted with EtOAc (10 mL), filtered and evaporated to give benzyl (*S*)-6-(2-(4-amino-9-chloro-6-methyl-5-oxo-3,4,5,6-tetrahydrobenzo[b][1,4]diazocin-1(2*H*)-yl)ethyl)-2,6-diazaspiro[3.3]heptane-2-carboxylate (400 mg, crude) as a yellow oil.

m/z ES+ $[M+H]^+$498.3.

**Step d.** To a solution of benzyl (S)-6-(2-(4-amino-9-chloro-6-methyl-5-oxo-3,4,5,6-tetrahydrobenzo[*b*][1,4]diazocin-1(2*H*)-yl)ethyl)-2,6-diazaspiro[3.3]heptane-2-carboxylate (400 mg, 0.80 mmol), 2-chloro-6-methyl-4-(trifluoromethyl)pyridine-3-carbonitrile (**Intermediate 1**, 213 mg, 0.96 mmol) in NMP (5 mL) was added DIPEA (311 mg, 2.41 mmol). The mixture was stirred at 100 °C for 1 h. Upon completion, the reaction mixture was diluted with water (20 mL) and extracted with EtOAc (3 x 20 mL). The combined organic layers were washed with brine (3 x 20 mL), dried over $Na_2SO_4$ and evaporated. The residue was purified by column chromatography (EtOAc/MeOH = 20/1) to give benzyl (S)-6-(2-(9-chloro-4-((3-cyano-6-methyl-4-(trifluoromethyl)pyridin-2-yl)amino)-6-methyl-5-oxo-3,4,5,6-tetrahydrobenzo[*b*][1,4]diazocin-1(2*H*)-yl)ethyl)-2,6-diazaspiro[3.3]heptane-2-carboxylate (130 mg, 21% yield) as a red oil.

m/z ES+ $[M+H]^+$682.2.

**Step e.** A solution of benzyl (S)-6-(2-(9-chloro-4-((3-cyano-6-methyl-4-(trifluoromethyl)pyridin-2-yl)amino)-6-methyl-5-oxo-3,4,5,6-tetrahydrobenzo[*b*][1,4]diazocin-1(2*H*)-yl)ethyl)-2,6-diazaspiro[3.3]heptane-2-carboxylate (120 mg, 0.18 mmol) in TFA (3 mL) was stirred at 40 °C for 16 h. Upon completion, the reaction mixture was evaporated. The residue was diluted with EtOAc (50 mL) and water (3 mL). The mixture was adjusted to pH 8-9 with sat. aq. $NaHCO_3$ (10 mL) and evaporated. The residue was diluted with EtOAc (10 mL), filtered and evaporated to give (S)-2-((6-(2-(2,6-diazaspiro[3.3]heptan-2-yl)ethyl)-8-chloro-1-methyl-2-oxo-1,2,3,4,5,6-hexahydrobenzo[b][1,4]diazocin-3-yl)amino)-6-methyl-4-(trifluoromethyl)nicotinonitrile (100 mg, crude) as a red oil.

m/z ES+ $[M+H]^+$ 548.4.

**Step f**. To a solution of (*S*)-2-((6-(2-(2,6-diazaspiro[3.3]heptan-2-yl)ethyl)-8-chloro-1-methyl-2-oxo-1,2,3,4,5,6-hexahydrobenzo[b][1,4]diazocin-3-yl)amino)-6-methyl-4-(trifluoromethyl)nicotinonitrile (90 mg, 0.16 mmol) and acrylic acid (15.4 mg, 0.21 mmol) in DCM (5 mL) was added HATU (74.9 mg, 0.20 mmol) and DIPEA (64 mg, 0.49 mmol). The mixture was stirred at rt for 1 h. Upon completion, the reaction mixture was diluted with water (10 mL) and extracted with EtOAc (2 x 10 mL). The combined organic layers were washed with brine (2 x 10 mL), dried over $Na_2SO_4$ and evaporated. The residue was purified by Prep-HPLC to give the title compound (17 mg, 15% yield) as a yellow solid.

m/z ES+ $[M+H]^+$ 602.3; $^1H$ NMR (400 MHz, $CDCl_3$) δ ppm 7.12 - 7.06 (m, 2H), 6.95 (dd, *J* = 2.4, 8.4 Hz, 1H), 6.71 (s, 1H), 6.38 - 6.29 (m, 1H), 6.21 - 6.12 (m, 1H), 6.09 (d, *J = 7.2* Hz, 1H), 5.68 (dd, *J* = 1.6, 10.4 Hz, 1H), 4.80 - 4.71 (m, 1H), 4.29 (s, 2H), 4.12 (s, 2H), 3.68 - 3.57 (m, 1H), 3.41 - 3.30 (m, 4H), 3.29 - 3.23 (m, 4H), 3.16 - 3.07 (m, 2H), 2.62 - 2.51 (m, 2H), 2.38 (s, 3H), 2.24 - 2.14 (m, 1H), 1.88 - 1.78 (m, 1H).

The examples in **Table 1** were prepared using methods similar to those described in the synthesis of **Example 6** and **Example 7**, using the listed amines in step a.

**Table 1**

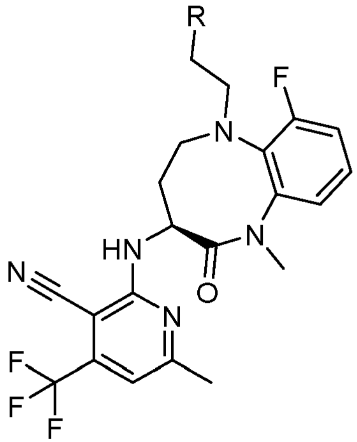

| Example Number | R | Name | Amine | $^1$H NMR (400 MHz) δ ppm | MI |
|---|---|---|---|---|---|
| **8** | 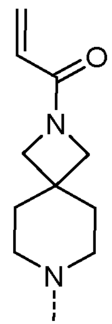 | (*S*)-2-((6-(2-(2-Acryloyl-2,7-diazaspiro[3.5]nonan-7-yl) ethyl)-7-fluoro-1-methyl-2-oxo-1,2,3,4,5,6-hexahydro-benzo[*b*][1,4]diazocin-3-yl) amino)-6-methyl-4-(trifluoromethyl)nicotinonitrile | *tert*-butyl 2,7-diazaspiro [3.5]nonane-2-carboxy-late (CAS Number 236406-55-6) | ($CDCl_3$) 7.31 - 7.27 (m, 1H), 7.16 - 7.09 (m, 2H), 6.69 (s, 1H), 6.37 - 6.31 (m, 1H), 6.22 - 6.14 (m, 2H), 5.67 (dd, *J* = 1.6, 10.4 Hz, 1H), 4.48 - 4.42 (m, 1H), 3.88 (s, 2H), 3.76 (s, 2H), 3.38 - 3.22 (m, 6H), 3.21 - 3.06 (m, 2H), 2.54 - 2.34 (m, 8H), 2.27 (dt, *J* = 5.2, 12.0 Hz, 2H), 1.82 - 1.77 (m, 4H) | 614.3 |
| **9** | 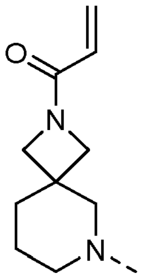 | (*S*)-2-((6-(2-(2-Acryloyl-2,6-diazaspiro[3.5]nonan-6-yl) ethyl)-7-fluoro-1-methyl-2-oxo-1,2,3,4,5,6-hexahydro-benzo[*b*][1,4]diazocin-3-yl) amino)-6-methyl-4-(trifluoromethyl)nicotinonitrile | *tert*-butyl 2,6-diazaspiro [3.5]nonane-2-carboxy-late (CAS Number 1086394-57-1) | ($DMSO-d_6$) 7.38 - 7.21 (m, 4H), 6.99 (s, 1H), 6.34 - 6.24 (m, 1H), 6.08 (dd, *J* = 2.4, 17.2 Hz, 1H), 5.65 (td, *J* = 2.4, 10.4 Hz, 1H), 4.28 - 4.21 (m, 1H), 3.77 (d, *J* = 4.0 Hz, 2H), 3.49 (s, 3H), 3.40 (d, *J* = 1.6 Hz, 1H), 3.31 (s, 1H), 3.19 (d, *J* = 1.6 Hz, 3H), 3.12 - 3.03 (m, 2H), 2.99 (d, *J* = 1.2 Hz, 1H), 2.38 (s, 3H), 2.30 - 2.12 (m, 5H), 1.77 (d, *J* = 13.6 Hz, 1H), 1.59 - 1.36 (m, 4H) | 614.3 |

(continued)

| Example Number | R | Name | Amine | $^1$H NMR (400 MHz) δ ppm | MI |
|---|---|---|---|---|---|
| **10** | 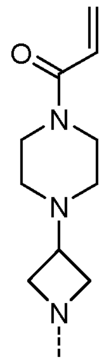 | (*S*)-2-((6-(2-(3-(4-Acryloylpiperazin-1-yl)azetidin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile | *tert*-butyl 4-(3-azetidinyl)-1-piperazinecarboxylate (CAS Number 219725-67-4) | (DMSO-$d_6$) 7.37 - 7.29 (m, 2H), 7.28 - 7.21 (m, 2H), 6.99 (s, 1H), 6.77 (dd, *J* = 10.4, 16.8 Hz, 1H), 6.08 (dd, *J* = 2.4, 16.8 Hz, 1H), 5.69 - 5.63 (m, 1H), 4.30 - 4.23 (m, 1H), 3.50 (dd, *J* = 4.8, 8.8 Hz, 4H), 3.40 - 3.35 (m, 1H), 3.29 - 3.22 (m, 2H), 3.20 (s, 3H), 3.02 - 2.88 (m, 3H), 2.82 - 2.76 (m, 1H), 2.74 - 2.67 (m, 2H), 2.38 (s, 3H), 2.34 - 2.30 (m, 2H), 2.16 (s, 5H), 1.81 - 1.75 (m, 1H) | 629.3 |
| **11** | 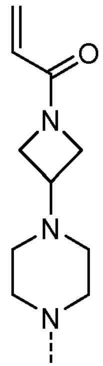 | (*S*)-2-((6-(2-(4-(1-Acryloylazetidin-3-yl)piperazin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile | *tert*-butyl 3-(1-piperazinyl)-1-azetidinecarboxylate (CAS Number 178311-48-3) | ($CDCl_3$) 7.32 (br d, *J* = 6.0 Hz, 1H), 7.18 - 7.11 (m, 2H), 6.70 (s, 1H), 6.37 - 6.30 (m, 1H), 6.23 - 6.12 (m, 2H), 5.71 - 5.65 (m, 1H), 4.50 - 4.39 (m, 1H), 4.27 - 4.20 (m, 1H), 4.15 - 4.03 (m, 2H), 3.91 (br s, 1H), 3.65 - 3.11 (m, 9H), 3.10 - 2.39 (m, 9H), 2.38 (s, 3H), 2.30 - 2.20 (m, 1H), 1.86 - 1.79 (m, 1H) | 629.3 |
| **12** | 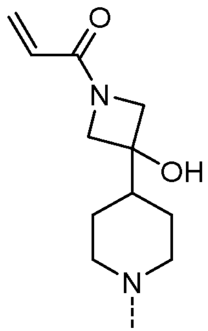 | (*S*)-2-((6-(2-(4-(1-Acryloyl-3-hydroxyazetidin-3-yl)piperidin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile | **Intermediate 6c** | ($CDCl_3$) 11.28 - 10.85 (m, 1H), 7.30 - 7.22 (m, 1H), 7.13 - 7.00 (m, 2H), 6.62 (s, 1H), 6.31 - 6.04 (m, 3H), 5.62 (br d, *J* = 11.6 Hz, 1H), 4.34 (br d, *J* = 10.4 Hz, 1H), 4.20 - 3.89 (m, 4H), 3.75 - 3.32 (m, 5H), 3.31 - 3.19 (m, 3H), 3.14 - 2.99 (m, 3H), 2.30 (s, 3H), 2.28 - 2.16 (m, 1H), 2.12 - 1.97 (m, 2H), 1.95 - 1.69 (m, 4H), 1.33 - 1.21 (m, 1H), 0.86 - 0.69 (m, 1H) | 644.4 |

(continued)

| Example Number | R | Name | Amine | $^1$H NMR (400 MHz) δ ppm | MI |
|---|---|---|---|---|---|
| **13** | 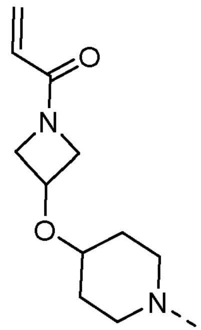 | (*S*)-2-((6-(2-(4-((1-Acryloylazetidin-3-yl)oxy)piperidin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile | **Intermediate 6d** | ($CDCl_3$) 7.38 - 7.31 (m, 1H), 7.20 - 7.13 (m, 2H), 6.73 - 6.69 (m, 1H), 6.40 - 6.30 (m, 1H), 6.25 - 6.12 (m, 2H), 5.75 - 5.66 (m, 1H), 4.50 - 4.38 (m, 3H), 4.35 - 4.26 (m, 1H), 4.11 (d, *J* = 2.4 Hz, 1H), 3.96 (d, *J* = 8.4 Hz, 1H), 3.84 - 3.64 (m, 3H), 3.57 - 3.33 (m, 6H), 3.23 - 2.87 (m, 5H), 2.62 - 2.43 (m, 2H), 2.38 (s, 3H), 2.29 - 2.16 (m, 1H), 2.00 - 1.89 (m, 2H), 1.84 (d, *J* = 12.0 Hz, 1H) | 644.3 |
| **14** | 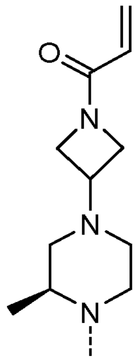 | 2-(((*S*)-6-(2-((*S*)-4-(1-Acryloylazetidin-3-yl)-2-methylpiperazin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile | **Intermediate 6e** | ($CDCl_3$) 7.31 - 7.26 (m, 1H), 7.18 - 7.09 (m, 2H), 6.70 (s, 1H), 6.38 - 6.30 (m, 1H), 6.25 - 6.14 (m, 2H), 5.68 (dd, *J* = 1.6, 10.4 Hz, 1H), 4.53 - 4.41 (m, 1H), 4.21 (t, *J* = 8.0 Hz, 1H), 4.11 - 4.03 (m, 2H), 3.93 (dd, *J* = 5.2, 10.4 Hz, 1H), 3.32 (s, 4H), 3.22 - 3.10 (m, 4H), 2.85 (dd, *J* = 2.8, 8.4 Hz, 1H), 2.75 - 2.52 (m, 4H), 2.38 (s, 7H), 2.22 - 2.10 (m, 2H), 1.04 - 0.99 (m, 3H) | 643.3 |
| **15** | 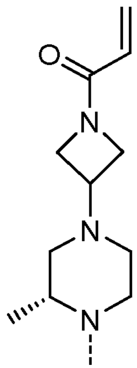 | 2-(((*S*)-6-(2-((*R*)-4-(1-Acryloylazetidin-3-yl)-2-methylpiperazin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile | **Intermediate 6f** | ($CDCl_3$) 7.40 - 7.31 (m, 1H), 7.21 - 7.13 (m, 2H), 6.71 (s, 1H), 6.39 - 6.30 (m, 1H), 6.23 - 6.11 (m, 2H), 5.70 (dd, *J* = 1.6, 10.4 Hz, 1H), 4.49 - 4.39 (m, 1H), 4.30 - 4.18 (m, 1H), 4.16 - 4.00 (m, 2H), 3.93 - 3.57 (m, 4H), 3.44 - 3.31 (m, 6H), 3.24 - 3.03 (m, 3H), 2.97 - 2.73 (m, 5H), 2.38 (s, 3H), 2.31 - 2.13 (m, 1H), 1.92 - 1.79 (m, 1H), 1.54 (d, *J* = 6.8 Hz, 3H) | 643.3 |

(continued)

| Example Number | R | Name | Amine | $^1$H NMR (400 MHz) δ ppm | MI |
|---|---|---|---|---|---|
| **16** | 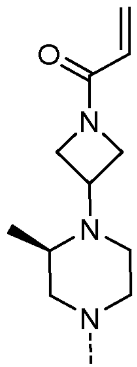 | 2-(((*S*)-6-(2-((*R*)-4-(1-Acryloylazetidin-3-yl)-3-methylpiperazin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile | **Intermediate 6g** | ($CDCl_3$) 7.35 - 7.27 (m, 1H), 7.18 - 7.08 (m, 2H), 6.69 (s, 1H), 6.37 - 6.28 (m, 1H), 6.23 - 6.12 (m, 2H), 5.68 (d, *J* = 10.4 Hz, 1H), 4.50 - 4.40 (m, 1H), 4.32 - 3.94 (m, 4H), 3.69 - 3.47 (m, 1H), 3.44 - 3.26 (m, 5H), 3.20 - 3.06 (m, 1H), 3.01 - 2.09 (m, 13H), 1.80 (d, *J* = 13.2 Hz, 2H), 1.03 (s, 3H). | 643.4 |
| **17** | 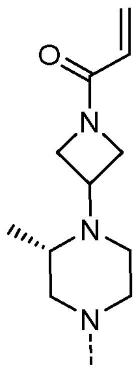 | 2-(((*S*)-6-(2-((*S*)-4-(1-Acryloylazetidin-3-yl)-3-methylpiperazin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile | **Intermediate 6h** | ($CDCl_3$) 7.35 - 7.27 (m, 1H), 7.18 - 7.07 (m, 2H), 6.70 (s, 1H), 6.41 - 6.28 (m, 1H), 6.25 - 6.13 (m, 2H), 5.68 (d, *J* = 10.4 Hz, 1H), 4.53 - 4.40 (m, 1H), 4.21 - 3.94 (m, 4H), 3.81 - 3.45 (m, 2H), 3.45 - 3.23 (m, 6H), 3.21 - 3.05 (m, 2H), 2.85 - 2.71 (m, 2H), 2.63 - 2.49 (m, 2H), 2.38 (s, 4H), 2.34 - 2.19 (m, 2H), 1.83 - 1.79 (m, 1H), 1.58 - 1.51 (m, 1H), 1.03 (d, *J* = 5.6 Hz, 3H) | 643.3 |
| **18** | 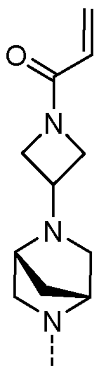 | 2-(((*S*)-6-(2-((1*R*,4*R*)-5-(1-Acryloylazetidin-3-yl)-2,5-diazabicyclo[2.2.1]heptan-2-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile | **Intermediate 6i** | ($CDCl_3$) 7.32 - 7.22 (m, 1H), 7.17 - 7.06 (m, 2H), 6.68 (s, 1H), 6.35 - 6.27 (m, 1H), 6.23 - 6.11 (m, 2H), 5.66 (d, *J* = 11.2 Hz, 1H), 4.50 - 4.41 (m, 1H), 4.29 - 4.16 (m, 1H), 4.15 - 4.03 (m, 1H), 4.03 - 3.93 (m, 1H), 3.90 - 3.80 (m, 1H), 3.64 - 3.32 (m, 3H), 3.31 (d, *J* = 0.8 Hz, 3H), 3.29 - 3.07 (m, 5H), 2.93 - 2.73 (m, 1H), 2.71 - 2.50 (m, 4H), 2.37 (s, 3H), 2.33 - 2.16 (m, 2H), 1.79 (d, *J* = 13.2 Hz, 2H) | 641.3 |

(continued)

| Example Number | R | Name | Amine | $^1$H NMR (400 MHz) δ ppm | MI |
|---|---|---|---|---|---|
| **19** | 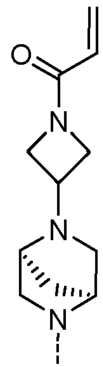 | 2-(((*S*)-6-(2-((1*S*,4*S*)-5-(1-Acryloylazetidin-3-yl)-2,5-diazabicyclo[2.2.1]heptan-2-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile | **Intermediate 6j** | ($CDCl_3$) 7.34 - 7.32 (m, 1H), 7.20 - 7.11 (m, 2H), 6.70 (s, 1H), 6.39 - 6.26 (m, 1H), 6.25 - 6.11 (m, 2H), 5.70 (d, *J* = 10.0 Hz, 1H), 4.54 - 4.36 (m, 3H), 4.34 (d, *J* = 3.6 Hz, 3H), 4.10 - 3.99 (m, 1H), 3.96 - 3.69 (m, 3H), 3.68 - 3.45 (m, 3H), 3.36 - 3.36 (m, 1H), 3.45 - 3.28 (m, 4H), 3.28 - 3.07 (m, 3H), 2.38 (s, 3H), 2.33 - 2.20 (m, 2H), 1.89 - 1.75 (m, 2H) | 641.3 |

The examples in **Table 2** were prepared using methods similar to those described in the synthesis of **Example 6**, using (S)-2-((1-methyl-2-oxo-6-(2-oxoethyl)-1,2,3,4,5,6-hexahydrobenzo[b][1,4]diazocin-3-yl)amino)-6-methyl-4-(trifluoromethyl)nicotinonitrile (prepared in a similar manner to Intermediate 5 starting with tert-butyl N-(2-aminophenyl) carbamate) and the listed amines in step a.

**Table 2**

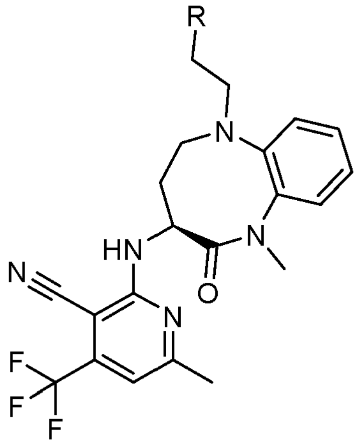

| Example Number | R | Name | Amine | $^1$H NMR (400 MHz) δ ppm | MI |
|---|---|---|---|---|---|
| **20** | 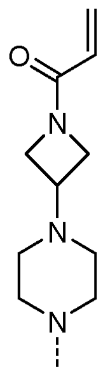 | *(S)*-2-((6-(2-(4-(1-Acryloylazetidin-3-yl)piperazin-1-yl)ethyl)-1-methyl-2-oxo-1,2,3,4,5,6-hexahydrobenzo[b][1,4]diazocin-3-yl)amino)-6-methyl-4-(trifluoromethyl)nicotinonitrile | tert-butyl 3-(1-piperazinyl)-1-azetidinecarboxylate (CAS Number 178311-48-3) | ($CD_3OD$) 7.42 - 7.34 (m, 3H), 7.18 (ddd, *J* = 2.0, 6.0, 8.0 Hz, 1H), 6.87 (s, 1H), 6.39 - 6.29 (m, 1H), 6.28 - 6.20 (m, 1H), 5.77 - 5.70 (m, 1H), 4.55 (dd, *J* = 3.6, 11.2 Hz, 1H), 4.33 (t, *J* = 8.0 Hz, 1H), 4.16 - 4.05 (m, 2H), 3.88 (dd, *J* = 5.2, 10.4 Hz, 1H), 3.52 (td, *J* = 4.8, 14.4 Hz, 1H), 3.35 (s, 2H), 3.27 (s, 3H), 3.25 - 3.19 (m, 1H), 3.15 - 3.07 (m, 1H), 2.79 - 2.29 (m, 13H), 2.15 - 1.96 (m, 2H). | 611.4 |
| **21** | 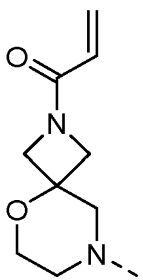 | *(S)*-2-((6-(2-(2-Acryloyl-5-oxa-2,8-diazaspiro[3.5]nonan-8-yl)ethyl)-1-methyl-2-oxo-1,2,3,4,5,6-hexahydrobenzo[b][1,4]diazocin-3-yl)amino)-6-methyl-4-(trifluoromethyl)nicotinonitrile | *tert*-butyl 5-oxa-2,8-diazaspiro[3.5]nonane-2-carboxylate (CAS Number 1251011-05-8) | ($CD_3OD$) 7.41 - 7.33 (m, 3H), 7.21 - 7.14 (m, 1H), 6.87 (s, 1H), 6.41 - 6.30 (m, 1H), 6.28 - 6.21 (m, 1H), 5.75 (d, *J* = 8.8 Hz, 1H), 4.59 - 4.52 (m, 1H), 4.20 - 4.10 (m, 1H), 4.07 (s, 1H), 3.93 - 3.81 (m, 2H), 3.68 (m, 2H), 3.60 - 3.50 (m, 1H), 3.46 - 3.36 (m, 1H), 3.35 (s, 1H), 3.27 (d, *J* = 4.0 Hz, 3H), 3.12 - 3.06 (m, 1H), 2.71 - 2.55 (m, 2H), 2.53 - 2.43 (m, 4H), 2.41 (s, 3H), 2.10 - 1.99 (m, 2H) | 598.4 |

(continued)

| Example Number | R | Name | Amine | $^1$H NMR (400 MHz) δ ppm | MI |
|---|---|---|---|---|---|
| **22** | 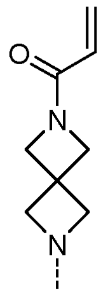 | *(S)*-2-((6-(2-(6-Acryloyl-2,6-diazaspiro[3.3]heptan-2-yl)ethyl)-1-methyl-2-oxo-1,2,3,4,5,6-hexahydrobenzo[b][1,4]diazocin-3-yl)amino)-6-methyl-4-(trifluoromethyl)nicotinonitrile | tert-butyl 2,6-diazaspiro[3.3]heptane-2-carboxylate (CAS Number 1041026-70-3) | ($CD_3OD$) 7.41 - 7.29 (m, 3H), 7.21 - 7.13 (m, 1H), 6.85 (s, 1H), 6.33 - 6.24 (m, 1H), 6.22 - 6.15 (m, 1H), 5.70 (dd, *J* = 2.4, 10.0 Hz, 1H), 4.51 (dd, *J* = 3.2, 11.2 Hz, 1H), 4.34 (s, 2H), 4.08 (s, 2H), 3.53 - 3.35 (m, 5H), 3.26 (s, 3H), 3.24 - 3.14 (m, 1H), 3.14 - 3.01 (m, 2H), 2.63 - 2.54 (m, 1H), 2.53 - 2.43 (m, 1H), 2.39 (s, 3H), 2.14 - 2.00 (m, 1H), 1.99 - 1.90 (m, 1H) | 568.4 |

**Example 23**

**(*S*)-2-((6-(2-(2-Acryloyl-2,5,8-triazaspiro[3.5]nonan-8-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile (E23)**

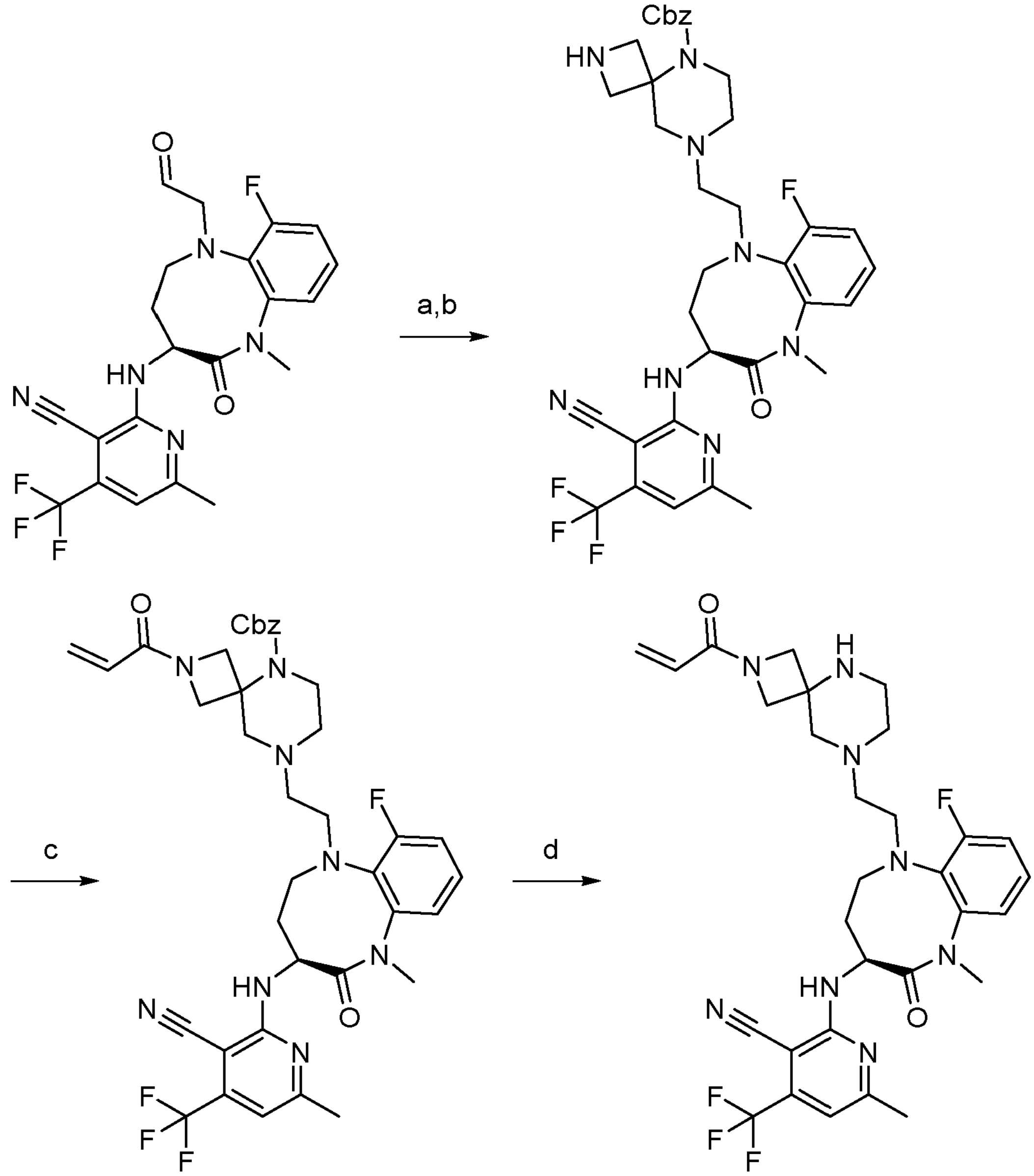


**Step a.** A mixture of **Intermediate 6a** (FA salt, 230 mg, 0.565 mmol), **Intermediate 5** (304 mg, 0.677 mmol), $NaBH_3CN$ (89 mg, 1.41 mmol), acetic acid (34 mg, 0.56 mmol) and 4 Å molecular sieves (200 mg) in MeOH (5 mL) was stirred at rt for 1 h. Upon completion, the reaction mixture was filtered and evaporated. The residue was purified by Prep-TLC (PE/EtOAc = 1/1) to give 5-benzyl *2-(tert-butyl)* (S)-8-(2-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)-2,5,8-triazaspiro[3.5]nonane-2,5-dicarboxylate (120 mg, 26% yield) as a yellow solid.

m/z ES+ $[M+H]^+$ 795.1.

**Step b.** To a solution of 5-benzyl 2-(*tert*-butyl) (*S*)-8-(2-(4-((3-cyano-6-methyl-4-(trifluoromethyl)pyridin-2-yl) amino)-10-fluoro-6-methyl-5-oxo-3,4,5,6-tetrahydrobenzo[*b*][1,4]diazocin-1(2H)-yl)ethyl)-2,5,8-triazaspiro[3.5]nonane-2,5-dicarboxylate (100 mg, 0.13 mmol) in DCM (3 mL) was added TFA (1.43 g, 12.6 mmol). The mixture was stirred at rt for 30 min. Upon completion, the reaction mixture was evaporated to give benzyl (*S*)-8-(2-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)-2,5,8-triazaspiro[3.5]nonane-5-carboxylate as a TFA salt (75 mg, crude) as a yellow solid.

m/z ES+ $[M+H]^+$ 695.1.

**Step c.** To a solution of benzyl (*S*)-8-(2-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)-2,5,8-triazaspiro[3.5]nonane-5-carboxylate (75 mg, 0.11 mmol) in DCM (3 mL) was added TEA (33 mg, 0.32 mmol) and acryloyl chloride (15 mg, 0.16 mmol). The mixture was stirred at 0 °C for 30 min. Upon completion, the reaction mixture was quenched with sat. aq. $NH_4Cl$ (20 mL) at rt, and then diluted with water (30 mL) and extracted with EtOAc (3 x 30 mL). The combined organic layers were washed with brine (3 x 20 mL), dried over $Na_2SO_4$ and evaporated. The residue was purified by Prep-TLC (PE/EtOAc = 2/1) to give benzyl (S)-2-acryloyl-8-(2-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)-2,5,8-triazaspiro[3.5]nonane-5-carboxylate (50 mg, 61% yield) as a yellow solid.

m/z ES+ $[M+H]^+$ 749.1; $^1$H NMR (400 MHz, $CDCl_3$) δ ppm 7.43 - 7.31 (m, 5H), 7.31 - 7.25 (m, 2H), 7.17 - 7.05 (m, 2H), 6.69 (s, 1H), 6.40 - 6.32 (m, 1H), 6.27 - 6.12 (m, 2H), 5.70 (t, *J* = 10.8 Hz, 1H), 5.12 (s, 2H), 4.51 - 4.33 (m, 2H), 4.22 - 4.00 (m, 2H), 3.98 - 3.88 (m, 1H), 3.53 - 3.32 (m, 2H), 3.29 (d, *J* = 2.0 Hz, 3H), 3.22 - 3.07 (m, 3H), 2.65 - 2.55 (m, 1H), 2.53 - 2.41 (m, 1H), 2.37 (s, 3H), 2.33 - 2.16 (m, 4H), 1.78 (d, *J* = 12.4 Hz, 2H).

**Step d.** A solution of benzyl (S)-2-acryloyl-8-(2-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)-2,5,8-triazaspiro[3.5]nonane-5-carboxylate (50 mg, 0.067 mmol) in TFA (2 mL) was stirred at 45 °C for 12 h. Upon completion, the reaction mixture was evaporated. The residue was purified by Prep-HPLC to give the title compound (11.5 mg, 26% yield) as a grey solid.

m/z ES+ $[M+H]^+$ 615.3; $^1$H NMR (400 MHz, $CDCl_3$) δ ppm 7.27 (s, 1H), 7.17 - 7.07 (m, 2H), 6.68 (s, 1H), 6.39 - 6.31 (m, 1H), 6.28 - 6.17 (m, 2H), 5.69 (dd, *J* = 9.6, 5.6 Hz, 1H), 4.45 (t, *J* = 8.8 Hz, 1H), 4.11 - 4.01 (m, 1H), 3.87 (d, *J* = 6.0 Hz, 2H), 3.72 (d, *J* = 10.0 Hz, 1H), 3.36 (d, *J* = 14.0 Hz, 1H), 3.30 (s, 3H), 3.21 - 3.06 (m, 3H) , 2.84 (s, 2H), 2.60 - 2.41 (m, 3H), 2.37 (s, 3H), 2.34 - 2.14 (m, 4H),1.80 (s, 1H).

## Example 24

**(*S*,*E*)-2-((6-(2-(2-(4-(Dimethylamino)but-2-enoyl)-6-oxo-5-oxa-2,7-diazaspiro[3.4]octan-7-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile (E24)**

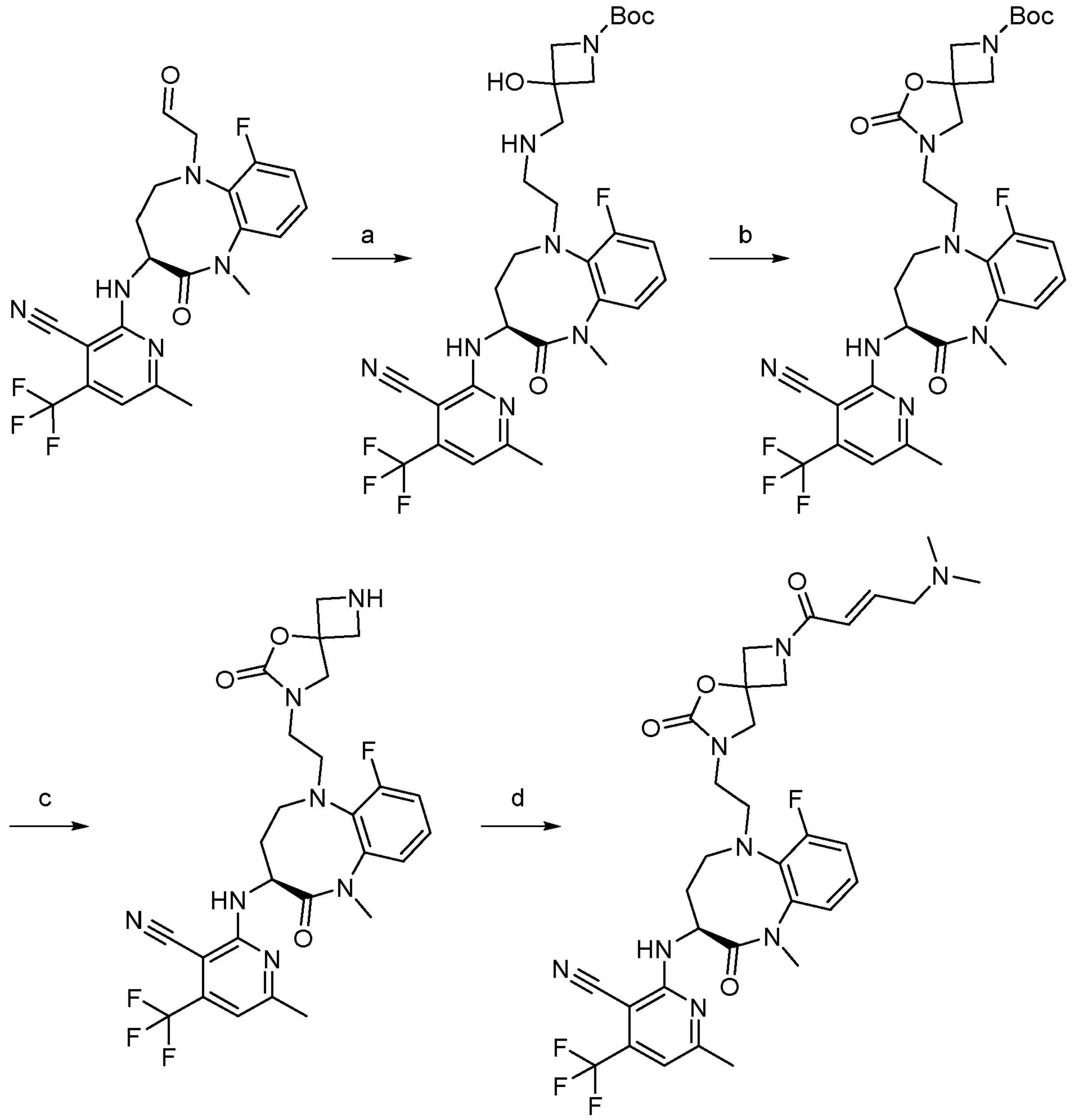


**Step** a. To a solution of **Intermediate** 5 (1.20 g, 2.67 mmol) and *tert-butyl* 3-(aminomethyl)-3-hydroxyazetidine-1-carboxylate (CAS Number 1008526-71-3; 594 mg, 2.94 mmol) in DCM (6 mL) was added $NaBH_3CN$ (419 mg, 6.68 mmol) and 4 Å molecular sieves (200 mg, 2.67 mmol). The mixture was stirred at rt for 16 h. Upon completion, the reaction mixture was filtered and evaporated. The residue was purified by column chromatography (EtOAc/EtOH = 1/20) and reverse phase flash chromatography (water (0.1% FA)/MeCN) to give *tert-butyl* (*S*)-3-(((2-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)amino)methyl)-3-hydroxyazetidine-1-carboxylate (200 mg, 10% yield) as a light yellow oil.

m/z ES+ $[M+H]^+$636.2; $^1H$ NMR (400 MHz, $CDCl_3$) δ ppm 7.39 - 7.30 (m, 1H), 7.22 - 7.09 (m, 2H), 6.75 - 6.67 (m, 1H), 6.24 - 6.17 (m, 1H), 4.54 - 4.40 (m, 1H), 4.05 - 3.88 (m, 4H), 3.85 - 3.78 (m, 1H), 3.57 - 3.51 (m, 2H), 3.46 - 3.38 (m, 1H), 3.37 (s, 3H), 3.34 - 3.30 (m, 2H), 3.28 - 3.19 (m, 2H), 3.19 - 3.08 (m, 2H), 3.05 - 2.96 (m, 1H), 2.41 - 2.37 (m, 3H), 1.89 - 1.73 (m, 1H), 1.46 - 1.42 (m, 9H).

**Step b.** To a solution of *tert*-butyl (*S*)-3-(((2-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)amino)methyl)-3-hydroxyazetidine-1-carboxylate (180 mg, 0.28 mmol) in water (9 mL) was added $K_2CO_3$ (51 mg, 0.37 mmol) and $KHSO_4$ (50 mg, 0.37 mmol), followed by a solution of bis(trichloromethyl) carbonate (168 mg, 0.57 mmol) in toluene (9 mL) dropwise at 0 °C. The mixture was stirred at rt for 2 h. Upon completion, the reaction mixture was quenched with sat. aq. $NaHCO_3$ (10 mL) at 0 °C

and extracted with EtOAc (3 x 10 mL). The combined organic layers were washed with brine (10 mL), dried over $Na_2SO_4$ and evaporated. The residue was purified by column chromatography (PE/EtOAc = 1/1) to give *tert*-butyl (*S*)-7-(2-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)-6-oxo-5-oxa-2,7-diazaspiro[3.4]octane-2-carboxylate (70 mg, 28% yield) as a colorless oil.

m/z ES+ $[M+H]^+$662.2.

**Step c.** To a solution of *tert*-butyl (*S*)-7-(2-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)-6-oxo-5-oxa-2,7-diazaspiro[3.4]octane-2-carboxylate (70 mg, 0.11 mmol) in DCM (1 mL) was added TFA (0.1 mL). The mixture was stirred at rt for 1 h. Upon completion, the reaction mixture was adjusted to pH 8-9 with sat. aq. $NaHCO_3$ (10 mL), and then extracted with EtOAc (3 x 5 mL). The combined organic layers were dried over $Na_2SO_4$ and evaporated to give (S)-2-((7-fluoro-1-methyl-2-oxo-6-(2-(6-oxo-5-oxa-2,7-diazaspiro[3.4]octan-7-yl)ethyl)-1,2,3,4,5,6-hexahydrobenzo[*b*][1,4]diazocin-3-yl)amino)-6-methyl-4-(trifluoromethyl)nicotinonitrile (70 mg, crude) as a pale yellow oil.

m/z ES+ $[M+H]^+$562.1.

**Step d.** To a solution of (S)-2-((7-fluoro-1-methyl-2-oxo-6-(2-(6-oxo-5-oxa-2,7-diazaspiro[3.4]octan-7-yl) ethyl)-1,2,3,4,5,6-hexahydrobenzo[*b*][1,4]diazocin-3-yl)amino)-6-methyl-4-(trifluoromethyl)nicotinonitrile (70 mg, 0.13 mmol), (*E*)-4-(dimethylamino)but-2-enoic acid (19 mg, 0.15 mmol) in DCM (0.5 mL) was added HATU (57 mg, 0.15 mmol) and DIPEA (48 mg, 0.37 mmol). The mixture was stirred at rt for 1 h. Upon completion, the reaction mixture was diluted with water (10 mL) and extracted with EtOAc (3 x 5 mL). The combined organic layers were washed with water (4 x 5 mL), dried over $Na_2SO_4$ and evaporated. The residue was purified by Prep-HPLC to give the title compound (20 mg, 23% yield) as a yellow solid.

m/z ES+ $[M+H]^+$673.3; $^1$H NMR (400 MHz, $CD_3OD$) δ ppm 7.45 - 7.38 (m, 1H), 7.36 - 7.30 (m, 1H), 7.28 - 7.20 (m, 1H), 6.89 (s, 1H), 6.75 (td, *J* = 7.2, 15.2 Hz, 1H), 6.54 (d, *J* = 15.2 Hz, 1H), 4.66 - 4.59 (m, 2H), 4.38 (br d, *J* = 11.2 Hz, 1H), 4.32 (br d, *J* = 8.8 Hz, 2H), 3.97 (d, *J* = 7.2 Hz, 2H), 3.93 - 3.83 (m, 2H), 3.50 - 3.43 (m, 1H), 3.30 (s, 3H), 3.29 - 3.06 (m, 5H), 2.91 (s, 6H), 2.43 (s, 3H), 2.33 - 2.27 (m, 1H), 1.88 - 1.81 (m, 1H).

### Example 25

**(*S*)-2-((6-(2-(2-Acryloyl-5-(prop-2-yn-1-yl)-2,5,8-triazaspiro[3.5]nonan-8-yl)ethyl)-7-fluoro-1-methyl-2-oxo-1,2,3,4,5,6-hexahydrobenzo[b][1,4]diazocin-3-yl)amino)-6-methyl-4-(trifluoromethyl)nicotinonitrile (E25)**

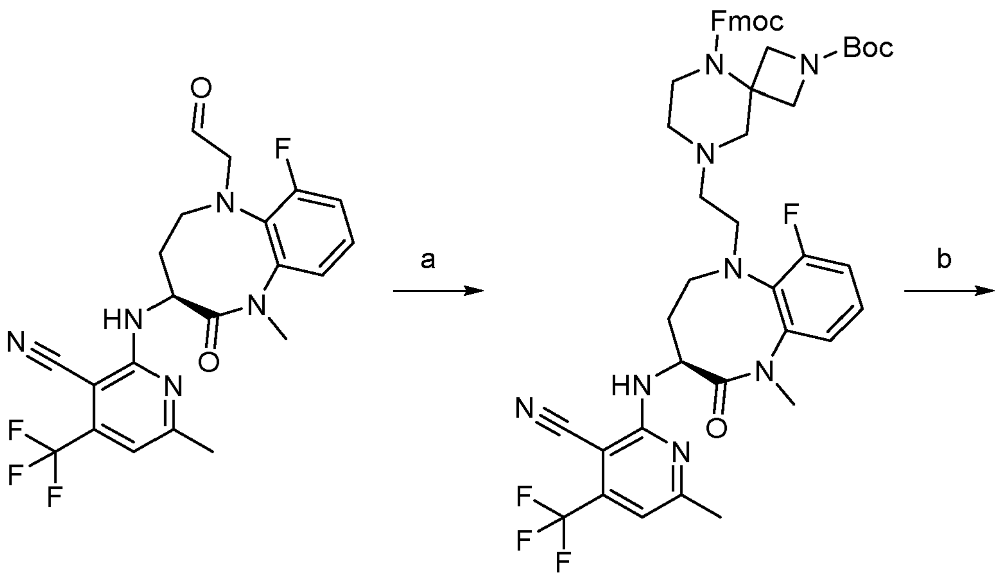

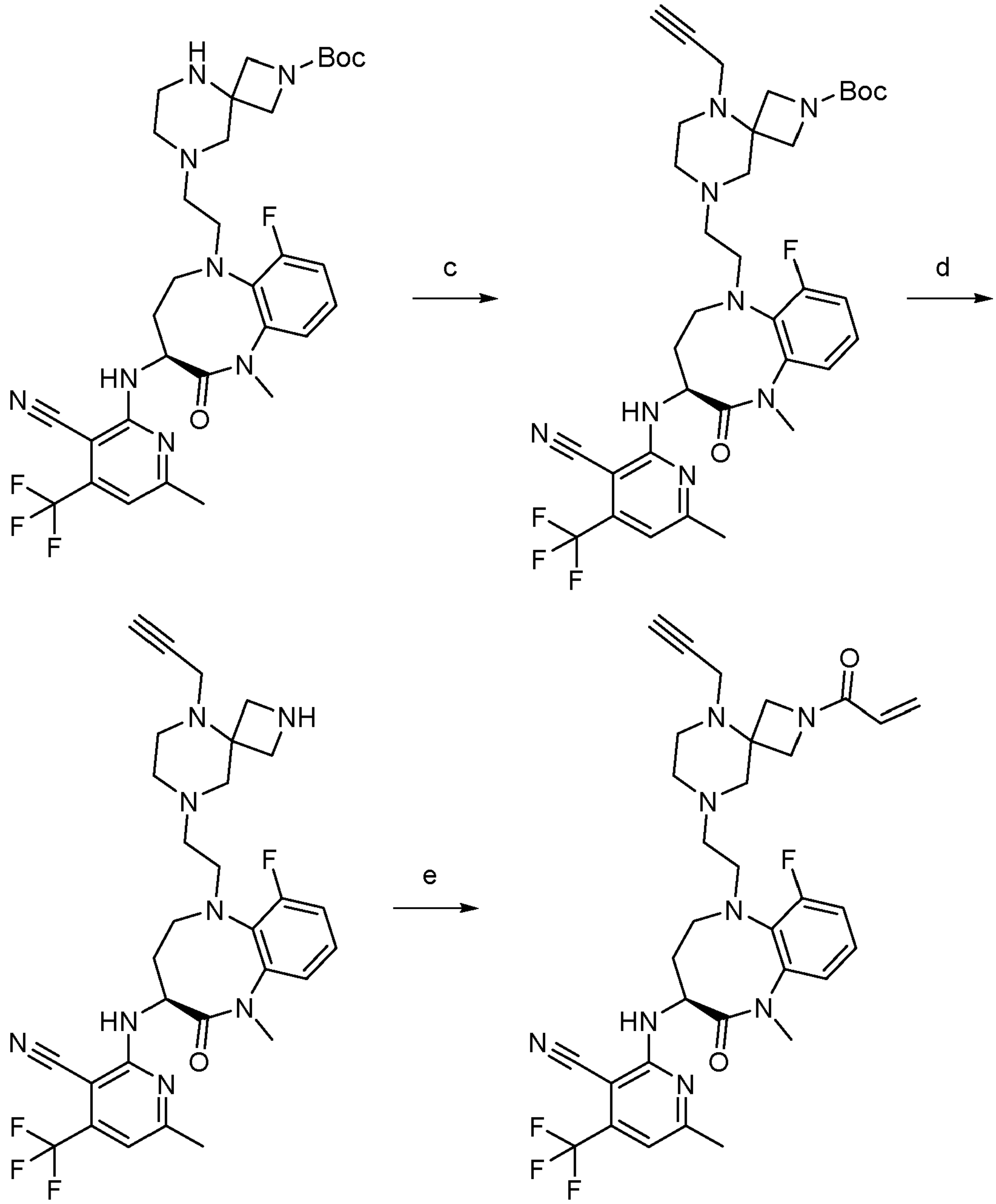


**Step a.** To a solution of **Intermediate 5** (640 mg, 1.42 mmol) and **Intermediate 6k** (800 mg, 1.42 mmol, 80% purity) in MeOH (10 mL) was added $NaBH_3CN$ (224 mg, 3.56 mmol), 4 Å molecular sieves (600 mg) and acetic acid (428 mg, 7.12 mmol). The mixture was stirred at rt for 12 h. Upon completion, the reaction mixture was filtered and evaporated. The resulting residue was purified by reverse phase column chromatography (water (0.1% FA)/MeCN) to give 5-((9*H*-fluoren-9-yl)methyl) *2-(tert-butyl)* (*S*)-8-(2-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)-2,5,8-triazaspiro[3.5]nonane-2,5-dicarboxylate (580 mg, 46% yield) as a yellow solid.

m/z ES+ $[M+H]^+$ 883.1; $^1H$ NMR (400 MHz, $CDCl_3$) δ ppm 7.82 - 7.72 (m, 2H), 7.62 - 7.52 (m, 2H), 7.46 - 7.30 (m, 5H), 7.19 - 7.07 (m, 2H), 6.69 (s, 1H), 6.20 (d, *J* = 7.0 Hz, 1H), 4.68 (s, 2H), 4.52 - 4.38 (m, 1H), 4.25 - 4.15 (m, 1H), 3.93 - 3.59 (m, 2H), 3.41 - 3.25 (m, 5H), 3.19 - 2.96 (m, 4H), 2.38 (s, 3H), 2.33 - 2.19 (m, 2H), 2.00 - 1.77 (m, 3H), 1.63 (s, 5H), 1.42 (s, 9H)

**Step b.** To a solution of 5-((9*H*-fluoren-9-yl)methyl) 2-(*tert*-butyl) (*S*)-8-(2-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)-2,5,8-triazaspiro[3.5]nonane-2,5-dicarboxylate (280 mg, 0.32 mmol) in DCM (5 mL) was added piperidine (1.08 g, 12.7 mmol) and the mixture was stirred at rt for 30 min. Upon completion, the reaction mixture was evaporated. The resulting residue was purified by reverse phase column chromatography (water (0.1% FA)/MeCN) to give *tert-butyl* (*S*)-8-(2-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)-2,5,8-triazaspiro[3.5]nonane-2-carboxylate (160 mg, 76% yield) as a yellow solid.

m/z ES+ $[M+H]^+$ 661.2

**Step c.** To a solution of *tert*-butyl (*S*)-8-(2-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)-2,5,8-triazaspiro[3.5]nonane-2-carboxylate (90 mg, 0.14 mmol) and 3-bromoprop-1-yne (40 mg, 0.27 mmol, 80% w/w in toluene) in MeCN (3 mL) was added $K_2CO_3$ (47 mg, 0.34 mmol) and potassium iodide (2.3 mg, 0.014 mmol). The mixture was stirred at rt for 6 h. Upon completion, the reaction mixture was quenched with sat. aq. $NH_4Cl$ (20 mL), diluted with water (30 mL) and extracted with EtOAc (3 x 50 mL). The combined organic layers were washed with brine (3 x 20 mL), dried over $Na_2SO_4$ and evaporated. The residue was purified by Prep-TLC (PE/EtOAc = 1/1) to give *tert-butyl* (*S*)-8-(2-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)-5-(prop-2-yn-1-yl)-2,5,8-triazaspiro[3.5]nonane-2-carboxylate (50 mg, 52% yield) as a yellow solid.

m/z ES+ $[M+H]^+$ 699.2

**Step d.** To a solution of *tert*-butyl (*S*)-8-(2-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)-5-(prop-2-yn-1-yl)-2,5,8-triazaspiro[3.5]nonane-2-carboxylate (50 mg, 0.072 mmol) in DCM (2 mL) was added TFA (816 mg, 7.2 mmol) and the mixture was stirred at rt for 30 min. Upon completion, the reaction mixture was quenched with sat. aq. $NaHCO_3$ (20 mL), diluted with water (30 mL) and extracted with DCM (3 x 50 mL). The combined organic layers were washed with brine (3 x 20 mL), dried over $Na_2SO_4$ and evaporated to give (*S*)-2-((7-fluoro-1-methyl-2-oxo-6-(2-(5-(prop-2-yn-1-yl)-2,5,8-triazaspiro[3.5]nonan-8-yl)ethyl)-1,2,3,4,5,6-hexahydrobenzo[*b*][1,4]diazocin-3-yl)amino)-6-methyl-4-(trifluoromethyl)nicotinonitrile (40 mg, 93% yield) as a yellow solid, which was used for next step directly.

m/z ES+ $[M+H]^+$ 599.3

**Step e.** To a solution of (*S*)-2-((7-fluoro-1-methyl-2-oxo-6-(2-(5-(prop-2-yn-1-yl)-2,5,8-triazaspiro[3.5]nonan-8-yl)ethyl)-1,2,3,4,5,6-hexahydrobenzo[*b*][1,4]diazocin-3-yl)amino)-6-methyl-4-(trifluoromethyl)nicotinonitrile (40 mg, 0.067 mmol) in DCM (3 mL) was added DIPEA (26 mg, 0.20 mmol) and acryloyl chloride (9.1 mg, 0.10 mmol). The mixture was stirred at 0 °C for 30 min. Upon completion, the reaction mixture was quenched with sat. aq. $NH_4Cl$ (20 mL), diluted with water (30 mL) and extracted with DCM (3 x 30 mL). The combined organic layers were washed with brine (3 x 20 mL), dried over $Na_2SO_4$ and evaporated. The residue was purified by Prep-HPLC to give the title compound (14 mg, 32% yield) as a white solid.

m/z ES+ $[M+H]^+$ 653.4; $^1$H NMR (400 MHz, $CDCl_3$) $\delta$ ppm 7.27 (m, 2H), 7.17 - 7.08 (m, 2H), 6.69 (s, 1H), 6.41 - 6.33 (m, 1H), 6.31 - 6.21 (m, 1H), 6.18 (d, *J* = 7.0 Hz, 1H), 5.78 - 5.64 (m, 1H), 4.50 - 4.32 (m, 2H), 4.17 (d, *J* = 10.2 Hz, 1H), 4.00 - 3.56 (m, 2H), 3.45 (s, 2H), 3.38 - 3.31 (m, 4H), 3.24 - 3.08 (m, 2H), 2.72 (s, 3H), 2.56 - 2.52 (m, 3H), 2.37 (s, 3H), 2.37 - 2.13 (m, 4H), 1.79 (d, *J* = 12.4 Hz, 1H).

### Example 26

**(*S*,*E*)-2-((6-(2-(2-(4-(Dimethylamino)but-2-enoyl)-5-(prop-2-yn-1-yl)-2,5,8-triazaspiro[3.5]nonan-8-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile (E26)**

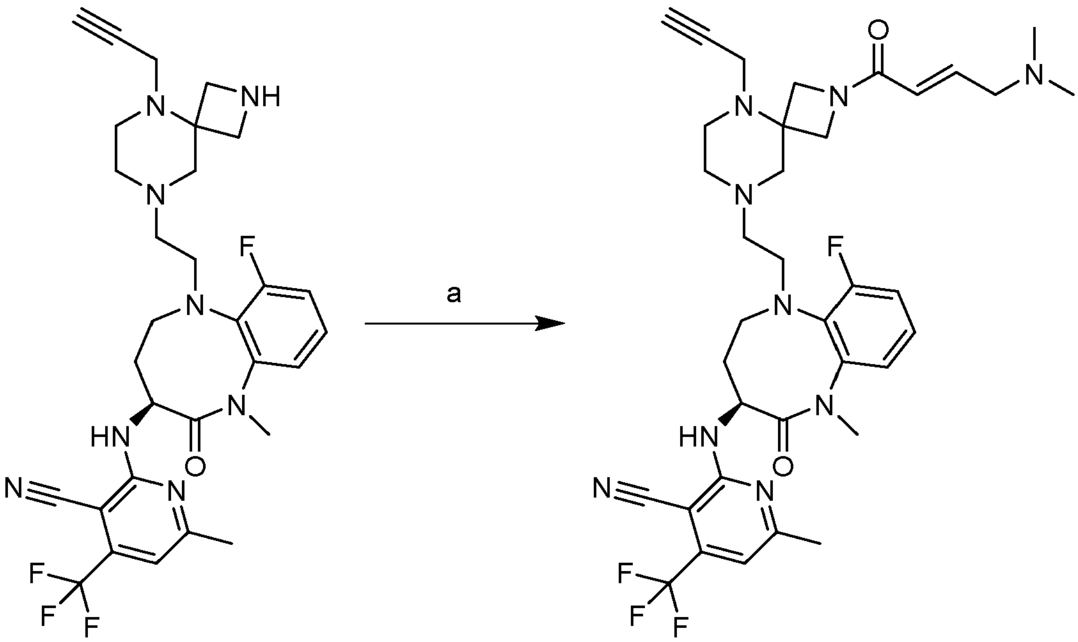


**Step a.** To a solution of (*S*)-2-((7-fluoro-1-methyl-2-oxo-6-(2-(5-(prop-2-yn-1-yl)-2,5,8-triazaspiro[3.5]nonan-8-yl)ethyl)-1,2,3,4,5,6-hexahydrobenzo[*b*][1,4]diazocin-3-yl)amino)-6-methyl-4-(trifluoromethyl)nicotinonitrile **(Example 25,** steps a-d; 30 mg, 0.050 mmol) and (*E*)-4-(dimethylamino)but-2-enoic acid hydrochloride (10 mg, 0.059 mmol) in DCM (2 mL) was added HATU (23 mg, 0.060 mmol) and DIPEA (19 mg, 0.15 mmol). The mixture was stirred at rt for 30 min. Upon completion, the reaction mixture was quenched with sat. aq. $NH_4Cl$ (20 mL), diluted with water (30 mL) and extracted with DCM (3 x 50 mL). The combined organic layers were washed with brine (3 x 20 mL), dried over $Na_2SO_4$ and evaporated. The residue was purified by Prep-HPLC to give the title compound (15 mg, 42% yield) as a yellow solid.

m/z ES+ $[M+H]^+$ 710.4; $^1H$ NMR (400 MHz, $CDCl_3$) δ ppm 7.32 - 7.27 (m, 2H), 7.18 - 7.08 (m, 2H), 6.93 - 6.84 (m, 1H), 6.68 (s, 1H), 6.41 - 6.29 (m, 1H), 6.25 (d, *J* = 7.2 Hz, 1H), 4.50 - 4.37 (m, 2H), 4.16 (d, *J* = 11.2 Hz, 1H), 3.98 - 3.87 (m, 1H), 3.65 (d, *J* = 11.2 Hz, 1H), 3.58 - 3.38 (m, 4H), 3.38 - 3.33 (m, 1H), 3.30 (d, *J* = 9.2 Hz, 3H), 3.21 - 3.06 (m, 3H), 2.80 (s, 1H), 2.64 (d, *J* = 11.2 Hz, 2H), 2.58 - 2.51 (m, 5H), 2.50 - 2.47 (m, 1H), 2.46 - 2.39 (m, 2H), 2.37 (s, 3H), 2.34 (d, *J* = 6.4 Hz, 1H), 2.31 - 2.27 (m, 2H), 2.24 - 2.19 (m, 1H), 1.80 (d, *J* = 11.6 Hz, 1H).

**Example 27**

**(*S*,*E*)-Prop-2-yn-1-yl 8-(2-(4-((3-cyano-6-methyl-4-(trifluoromethyl)pyridin-2-yl)amino)-10-fluoro-6-methyl-5-oxo-3,4,5,6-tetrahydrobenzo[b][1,4]diazocin-1(2H)-yl)ethyl)-2-(4-(dimethylamino)but-2-enoyl)-2,5,8-triazaspiro [3.5]nonane-5-carboxylate (E27)**

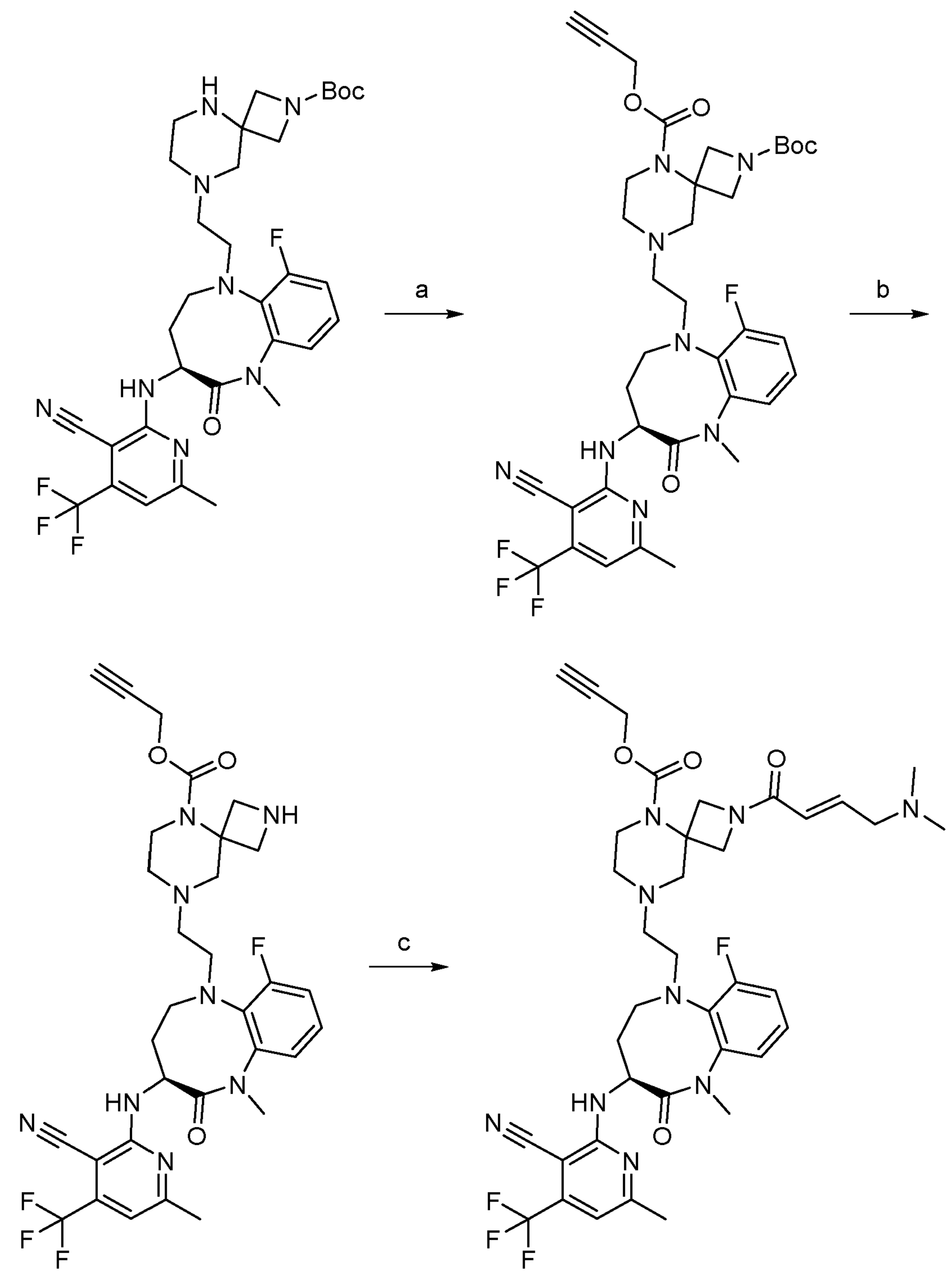


**Step a.** To a solution of *tert*-butyl (*S*)-8-(2-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)-2,5,8-triazaspiro[3.5]nonane-2-carboxylate **(Example 25,** steps a-b; 70 mg, 0.11 mmol) in DCM (2 mL) was added DIPEA (41 mg, 0.32 mmol) and propargyl chloroformate (19 mg, 0.16 mmol) at 0 °C. The mixture was stirred at rt for 1 h. Upon completion, the reaction mixture was quenched with sat. aq. $NH_4Cl$ (20 mL), diluted with water (30 mL) and extracted with DCM (3 x 30 mL). The combined organic layers were washed with brine (3 x 20 mL), dried over $Na_2SO_4$ and evaporated. The residue was purified by Prep-TLC (PE/EtOAc = 3/1) to give *2-(tert-butyl)* 5-(prop-2-yn-1-yl) (*S*)-8-(2-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)-2,5,8-triazaspiro[3.5]nonane-2,5-dicarboxylate (72 mg, 91% yield) as a yellow solid.

m/z ES+ $[M+H]^+$ 743.1; $^1H$ NMR (400 MHz, $CDCl_3$) δ ppm 7.32 - 7.27 (m, 1H), 7.18 - 7.05 (m, 2H), 6.69 (s, 1H), 6.18 (d, *J* =7.2 Hz, 1H), 4.73 - 4.64 (m, 2H), 4.45 (td, J = 9.2, 2.0 Hz, 1H), 4.16 - 3.98 (m, 2H), 3.78 - 3.65 (m, 2H), 3.50 (s, 1H),

3.35 (dd, *J* = 15.6, 4.8 Hz, 2H), 3.30 (s, 3H), 3.20 - 3.05 (m, 3H), 2.67- 2.51 (m, 2H), 2.39 - 2.52 (m, 3H), 2.37 (s, 3H), 2.34 - 2.17 (m, 3H), 1.79 (d, *J* = 13.2 Hz, 1H),1.45 (s, 9H).

**Step b.** To a solution of *2-(tert-butyl)* 5-(prop-2-yn-1-yl) (S)-8-(2-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)-2,5,8-triazaspiro[3.5]nonane-2,5-dicarboxylate (72 mg, 0.097 mmol) in DCM (3 mL) was added TFA (1.11 g, 9.69 mmol) and the mixture was stirred at rt for 30 min. Upon completion, the reaction mixture was quenched with sat. aq. $NaHCO_3$ (20 mL), diluted with water (30 mL) and extracted with DCM (3 x 30 mL). The combined organic layers were washed with sat. aq. $NaHCO_3$ (3 x 20 mL), dried over $Na_2SO_4$ and evaporated to give prop-2-yn-1-yl (*S*)-8-(2-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)-2,5,8-triazaspiro[3.5]nonane-5-carboxylate (55 mg, crude) as a yellow solid, which was used in the next step directly.

m/z ES+ $[M+H]^+$ 643.2.

**Step c.** To a solution of prop-2-yn-1-yl (*S*)-8-(2-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)-2,5,8-triazaspiro[3.5]nonane-5-carboxylate (55 mg, 0.86 mmol) and (E)-4-(dimethylamino)but-2-enoic acid hydrochloride (21 mg, 0.13 mmol) in DCM (3 mL) was added HATU (39 mg, 0.10 mmol) and DIPEA (33 mg, 0.26 mmol), the mixture was stirred at rt for 30 min. Upon completion, the reaction mixture was quenched with sat. aq. $NH_4Cl$ (100 mL), diluted with water (30 mL) and extracted with DCM (3 x 30 mL). The combined organic layers were washed with brine (3 x 20 mL), dried over $Na_2SO_4$ and evaporated. The residue was purified by Prep-HPLC to give the title compound (33 mg, 51% yield) as a yellow solid.

m/z ES+ $[M+H]^+$ 754.4; $^1H$ NMR (400 MHz, $CDCl_3$) $\delta$ ppm 7.32 - 7.27 (m, 2H), 7.19 - 7.07 (m, 2H), 6.94 - 6.83 (m, 1H), 6.69 (s, 1H), 6.35 - 6.20 (m, 2H), 4.70 (d, *J* = 2.0 Hz, 2H), 4.53 - 4.35 (m, 2H), 4.28 - 4.10 (m, 2H), 4.01 - 3.90 (m, 1H), 3.42 (s, 2H), 3.36 (s, 1H), 3.30 (d, *J* = 8.0 Hz, 3H), 3.16 (d, *J* = 6.8 Hz, 2H), 3.13 - 3.07 (m, 1H), 2.67 - 2.52 (m, 3H), 2.50 (d, *J* = 12.0 Hz, 6H), 2.42 (d, *J* = 6.0 Hz, 2H), 2.37 (s, 3H), 2.36 - 2.17 (m, 4H),1.80 - 1.78 (m, 1H).

### Example 28

**(*rel*-3*S*,4*R*)-N-(2-((*S*)-4-((3-Cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)-1-((*E*)-4-(dimethylamino)but-2-enoyl)-4-methoxy-N-methylpyrrolidine-3-carboxamide (E28)**

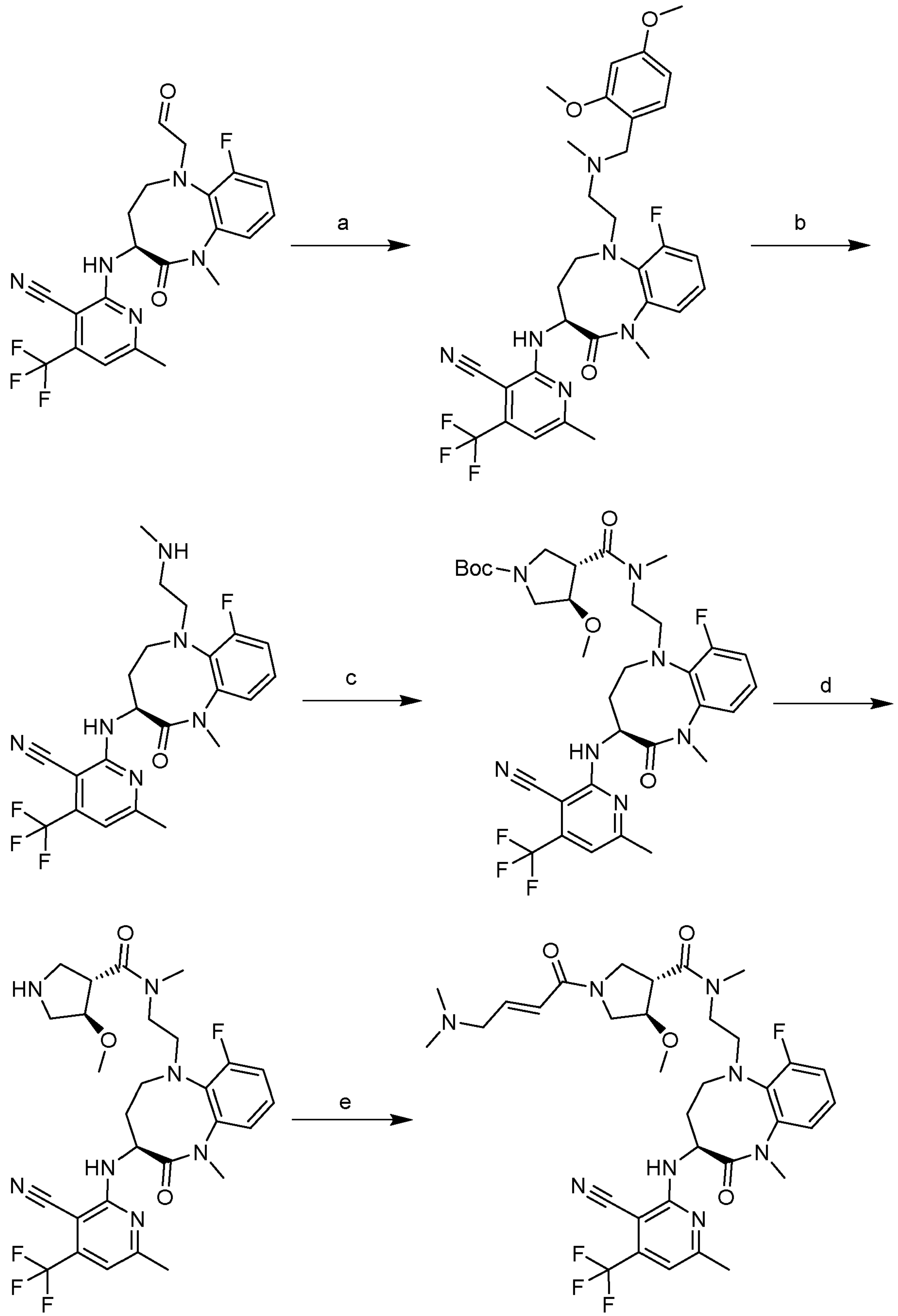


**Step a.** To a solution of **Intermediate 5** (200 mg, 0.44 mmol), *N*-methyl-3,4-dimethoxybenzylamine (CAS Number 63-64-9; 81 mg, 0.44 mmol) in DCM (3 mL) was added acetic acid (134 mg, 2.23 mmol), $NaBH_3CN$ (28 mg, 0.44

mmol) and 4 Å molecular sieves (200 mg). The mixture was stirred at rt for 3 h. Upon completion, the reaction mixture was filtered and evaporated. The residue was purified by reverse phase flash chromatography (water (0.1% FA)/MeCN) to give (*S*)-2-((6-(2-((2,4-dimethoxybenzyl)(methyl)amino)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile (140 mg, 50% yield) as a brown oil.

m/z ES+ $[M+H]^+$ 615.3.

**Step b.** To a solution of (*S*)-2-((6-(2-((2,4-dimethoxybenzyl)(methyl)amino)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile (80 mg, 0.13 mmol) in THF (2 mL) was added a solution of 2-chloroethyl chloroformate (18.6 mg, 0.13 mmol) in THF (0.4 mL) at 0 °C. The mixture was stirred for 15 min. Then TEA (40 mg, 0.39 mmol) was added dropwise and the mixture was stirred at 65 °C for 16 h. Upon completion, the reaction mixture was diluted with water (10 mL) and extracted with EtOAc (3 x 5 mL). The combined organic layers were washed with water (4 x 5 mL), dried over $Na_2SO_4$ and evaporated. The resulting residue was purified by Prep-TLC (PE/EtOAc = 1/1) to give (*S*)-2-((7-fluoro-1-methyl-6-(2-(methylamino)ethyl)-2-oxo-1,2,3,4,5,6-hexahydrobenzo[*b*][1,4]diazocin-3-yl)amino)-6-methyl-4-(trifluoromethyl)nicotinonitrile (25 mg, 41% yield) as a pale yellow oil.

m/z ES+ $[M+H]^+$ 465.0.

**Step c.** To a solution of (*S*)-2-((7-fluoro-1-methyl-6-(2-(methylamino)ethyl)-2-oxo-1,2,3,4,5,6-hexahydrobenzo[*b*][1,4]diazocin-3-yl)amino)-6-methyl-4-(trifluoromethyl)nicotinonitrile (20 mg, 0.043 mmol) and **Intermediate 7a** (11.6 mg, 0.047 mmol) in DCM (2 mL) was added DIPEA (16.7 mg, 0.13 mmol) and HATU (19.7 mg, 0.052 mmol). The mixture was stirred at rt for 1 h. Upon completion, the reaction mixture was diluted with water (5 mL) and extracted with EtOAc (3 x 3 mL). The combined organic layers were washed with water (3 x 3 mL), dried over $Na_2SO_4$ and evaporated. The residue was purified by Prep-TLC (PE/EtOAc = 1/2) to give *tert*-butyl (*rel*-3*S*,4*R*)-3-((2-((S)-4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)(methyl)carbamoyl)-4-methoxypyrrolidine-1-carboxylate (25 mg, 74% yield) as an oil.

m/z ES+ $[M+H]^+$ 692.3.

**Step d.** To a solution of *tert*-butyl (*rel*-3*S*,4*R*)-3-((2-((S)-4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)(methyl)carbamoyl)-4-methoxypyrrolidine-1-carboxylate (25 mg, 0.036 mmol) in DCM (0.5 mL) was added TFA (0.1 mL). The mixture was stirred at rt for 1 h. Upon completion, the reaction mixture was adjusted to pH 8-9 by the addition of sat. aq. $NaHCO_3$ (2 mL) at rt, diluted with water (3 mL) and extracted with EtOAc (3 x 3 mL). The combined organic layers were dried over $Na_2SO_4$ and evaporated to give (*rel*-3*S*,4*R*)-N-(2-((*S*)-4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)-4-methoxy-N-methylpyrrolidine-3-carboxamide (20 mg, crude) as a yellow oil, which was used in the next step without further purification.

m/z ES+ $[M+H]^+$ 592.3.

**Step** e. To a solution of (*rel*-3*S*,4*R*)-N-(2-((*S*)-4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)-4-methoxy-N-methylpyrrolidine-3-carboxamide (20 mg, 0.034 mmol) and (*E*)-4-(dimethylamino)but-2-enoic acid hydrochloride (6.2 mg, 0.037 mmol) in DCM (0.5 mL) was added DIPEA (13.1 mg, 0.10 mmol) and HATU (15.4 mg, 0.041 mmol). The mixture was stirred at rt for 1 h. Upon completion, the reaction mixture was diluted with water (5 mL) and extracted with EtOAc (3 x 3 mL). The combined organic layers were washed with water (3 x 3 mL), dried over $Na_2SO_4$, filtered and evaporated to give a residue. The residue was purified by Prep-HPLC to give the title compound (6 mg, 24% yield) as yellow gum.

m/z ES+ $[M+H]^+$ 703.3; $^1H$ NMR (400 MHz, $CDCl_3$) δ ppm 7.29 - 7.20 (m, 1H), 7.20 - 7.07 (m, 2H), 6.95 - 6.82 (m, 1H), 6.71 - 6.66 (m, 1H), 6.64 - 6.43 (m, 1H), 6.28 - 6.16 (m, 1H), 4.50 - 4.38 (m, 1H), 4.08 - 3.79 (m, 3H), 3.79 - 3.50 (m, 3H), 3.50 - 3.41 (m, 2H), 3.41 - 3.38 (m, 1H), 3.37 - 3.34 (m, 2H), 3.33 - 3.31 (m, 2H), 3.31 - 3.26 (m, 2H), 3.25 - 3.19 (m, 1H), 3.18 - 3.09 (m, 2H), 3.08 - 3.04 (m, 2H), 2.94 - 2.89 (m, 1H), 2.65 (s, 1H), 2.60 - 2.55 (m, 2H), 2.55 - 2.52 (m, 3H), 2.38 - 2.35 (m, 3H), 2.33 - 2.19 (m, 3H), 1.83 - 1.74 (m, 1H).

### Example 29

**(*S*,*E*)-*N*-(2-(4-((3-Cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)-1-(4-(dimethylamino)but-2-enoyl)-*N*-methylazetidine-3-carboxamide (E29)**

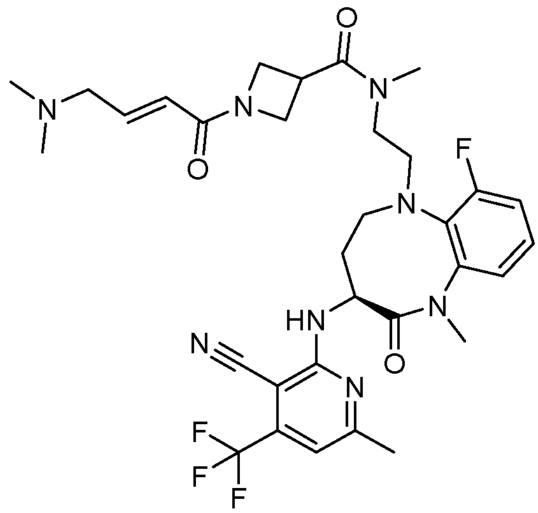

The title compound was prepared in a similar manner to **Example 28,** using 1-*tert*-butoxycarbonylazetidine-3-carboxylic acid (CAS Number 142253-55-2) in step c.

m/z ES+ $[M+H]^+$ 659.2; $^1H$ NMR (400 MHz, DMSO-$d_6$) δ ppm 7.43 - 7.33 (m, 2H), 7.33 - 7.22 (m, 2H), 6.99 (s, 1H), 6.60 - 6.53 (m, 1H), 6.14 - 6.06 (m, 1H), 4.37 - 4.27 (m, 1H), 4.26 - 4.18 (m, 2H), 4.08 - 3.99 (m, 1H), 3.92 - 3.87 (m, 1H), 3.71 - 3.62 (m, 1H), 3.38 (s, 2H), 3.28 (s, 1H), 3.22 - 3.19 (m, 3H), 3.13 - 3.03 (m, 3H), 3.01 (d, *J* = 4.8 Hz, 2H), 2.84 - 2.76 (m, 3H), 2.39 (s, 3H), 2.23 - 2.21 (m, 1H), 2.18 (s, 6H), 1.83 - 1.72 (m, 1H).

**Example 30**

**(*R*)-*N*-(2-((*S*)-4-((3-Cyano-6-methyl-4-(trifluoromethyl)pyridin-2-yl)amino)-6-methyl-5-oxo-3,4,5,6-tetrahydrobenzo[*b*][1,4]diazocin-1(2*H*)-yl)ethyl)-1-((*E*)-4-(dimethylamino)but-2-enoyl)-*N*-methylpyrrolidine-3-carboxamide (E30)**

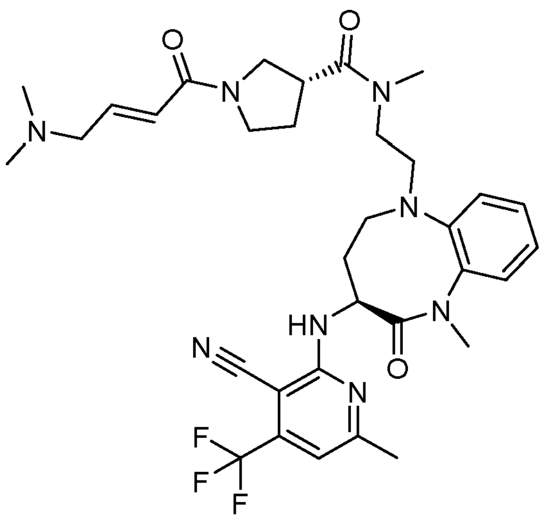

The title compound was prepared in a similar manner to **Example 28,** using (S)-2-((1-methyl-2-oxo-6-(2-oxoethyl)-1,2,3,4,5,6-hexahydrobenzo[b][1,4]diazocin-3-yl)amino)-6-methyl-4-(trifluoromethyl)nicotinonitrile (prepared in a similar manner to **Intermediate 5** starting with *tert-butyl* N-(2-aminophenyl)carbamate) in step a and (3R)-1-tert-butoxycarbonylpyrrolidine-3-carboxylic acid (CAS Number 72925-16-7) in step c.

m/z ES+ $[M+H]^+$ 655.2; $^1H$ NMR (400 MHz, $CDCl_3$) δ ppm 7.37 - 7.31 (m, 1H), 7.26 (d, *J* = 8.0 Hz, 1H), 7.20 - 7.19 (m, 1H), 7.12 - 7.09 (m, 1H), 6.91 - 6.85 (m, 1H), 6.65 (d, *J* = 3.2 Hz, 1H), 6.25 (d, *J* = 15.2 Hz, 1H), 6.19 - 6.15 (m, 1H), 4.62 - 4.48 (m, 1H), 3.90 - 3.84 (m, 1H), 3.81 - 3.68 (m, 2H), 3.60 - 3.40 (m, 5H), 3.26 (d, *J* = 0.8 Hz, 3H), 3.24 - 3.18 (m, 2H), 3.11 (s, 1H), 3.07 - 3.04 (m, 4H), 2.92 (d, *J* = 2.8 Hz, 1H), 2.33 (s, 3H), 2.27 - 2.20 (m, 6H), 2.17 - 2.11 (m, 1H), 2.04 - 1.89 (m, 3H).

**Example 31**

***(S,E)-N*-(2-(4-((3-Cyano-6-methyl-4-(trifluoromethyl)pyridin-2-yl)amino)-6-methyl-5-oxo-3,4,5,6-tetrahydrobenzo[b][1,4]diazocin-1(2H)-yl)ethyl)-1-(4-(dimethylamino)but-2-enoyl)-*N*-methylazetidine-3-carboxamide (E31)**

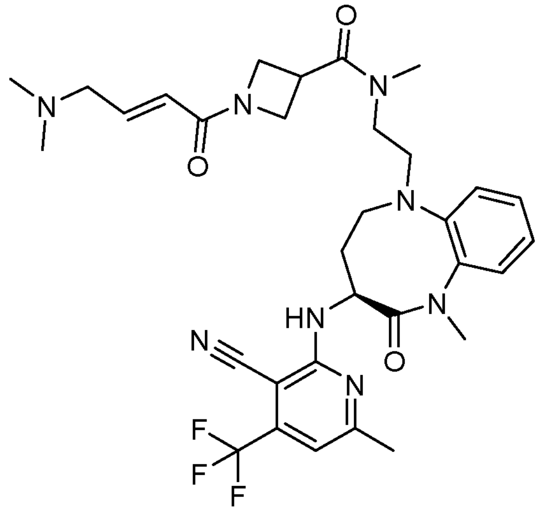

The title compound was prepared in a similar manner to **Example 30,** using 1-*tert*-butoxycarbonylazetidine-3-carboxylic acid (CAS Number 142253-55-2) in step c.

m/z ES+ $[M+H]^+$ 641.1; $^1H$ NMR (400 MHz, $CD_3OD$) δ ppm 7.48 - 7.31 (m, 3H), 7.28 - 7.10 (m, 1H), 6.87 - 6.83 (m, 1H), 6.80 - 6.75 (m, 1H), 6.21 - 6.11 (m, 1H), 4.58 - 4.46 (m, 1H), 4.45 - 4.31 (m, 2H), 4.26 - 4.11 (m, 1H), 4.10 - 3.97 (m, 1H), 3.82 - 3.71 (m, 1H), 3.70 - 3.54 (m, 1H), 3.53 - 3.40 (m, 1H), 3.40 - 3.30 (m, 3H), 3.26 - 3.22 (m, 3H), 3.17 - 3.06 (m, 3H), 2.97 - 2.89 (m, 3H), 2.42 - 2.37 (m, 3H), 2.25 (d, *J* = 2.8 Hz, 6H), 2.14 - 1.91 (m, 2H).

**Examples 32A and 32B**

**2-(((*S*)-6-((*S*/*R*)-3-(4-((*E*)-4-(Dimethylamino)but-2-enoyl)piperazin-1-yl)-2-hydroxypropyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile (E32A)** and

**2-(((*S*)-6-((*R*/*S*)-3-(4-((E)-4-(Dimethylamino)but-2-enoyl)piperazin-1-yl)-2-hydroxypropyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile (E32B)**

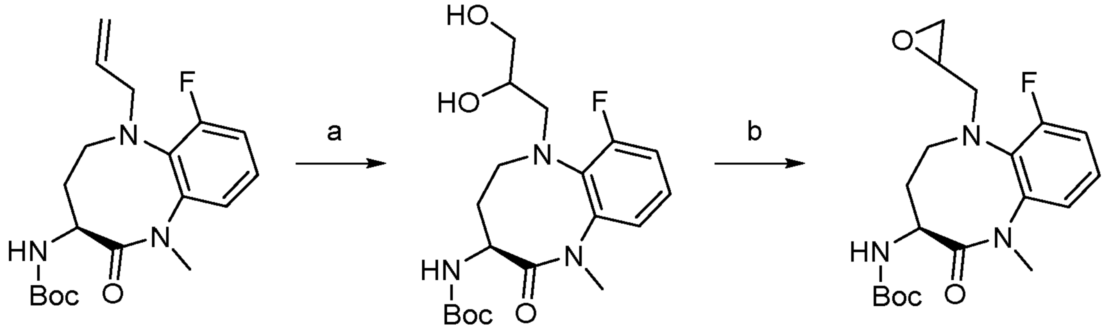

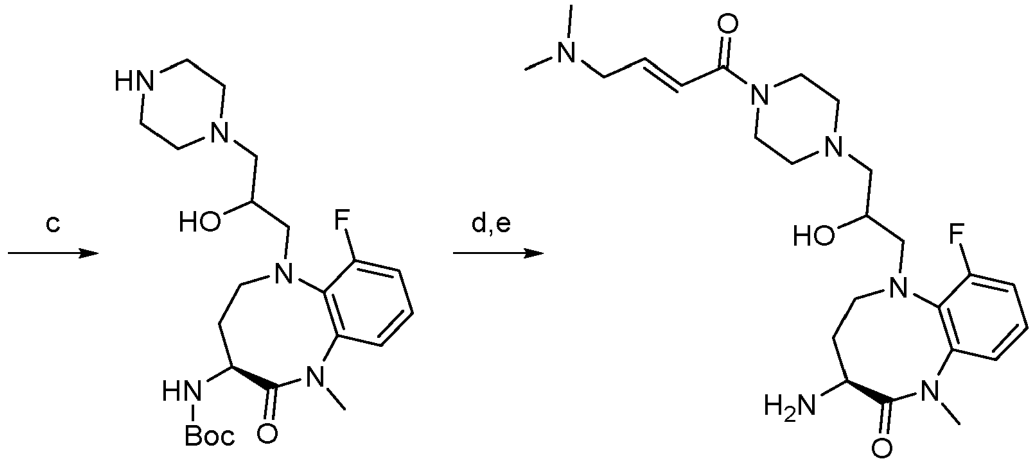


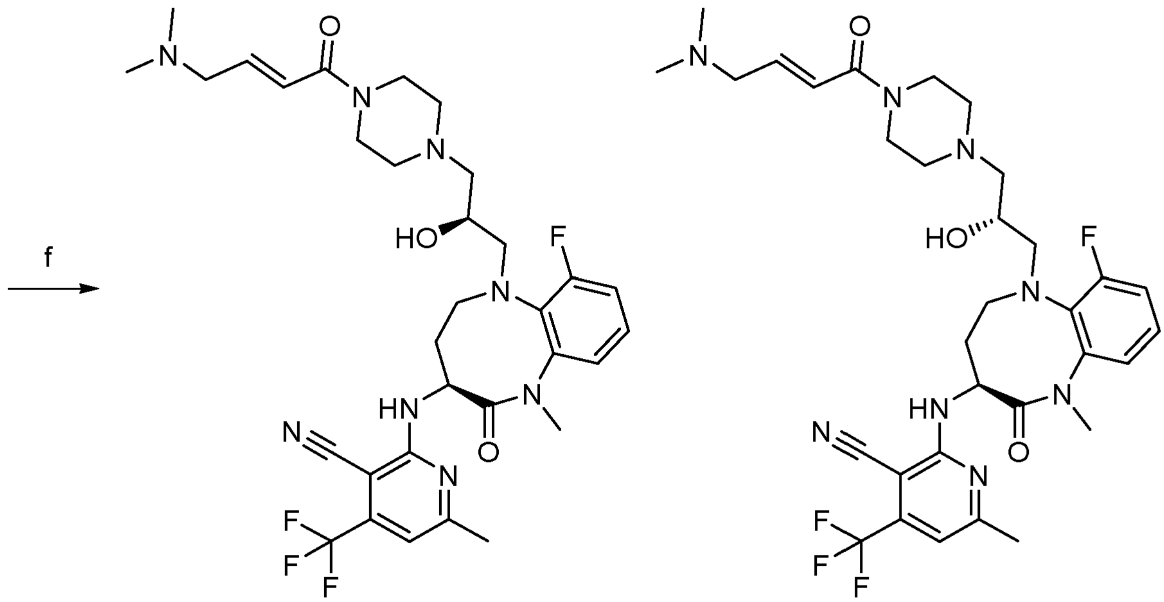


**Step a.** To a solution of **Intermediate 3** (720 mg, 1.98 mmol) in water (3 mL), MeCN (3 mL) and *t*-BuOH (3 mL) was added NMO (278 mg, 2.38 mmol), followed by $OsO_4$ (50 mg, 0.20 mmol). The mixture was stirred at rt for 1 h. Upon completion, the reaction mixture was quenched with sat. aq. $Na_2SO_3$ (5 mL) and stirred for 30 min. The mixture was diluted with water (10 mL) and extracted with EtOAc (3 x 10 mL). The combined organic layers were washed with brine (10 mL), dried over $Na_2SO_4$ and evaporated. The residue was purified by column chromatography (EtOAc) to give *tert-butyl* ((3*S*)-6-(2,3-dihydroxypropyl)-7-fluoro-1-methyl-2-oxo-1,2,3,4,5,6-hexahydrobenzo[b][1,4]diazocin-3-yl)carbamate (670 mg, 85% yield) as a brown oil.

m/z ES+ $[M+H]^+$ 398.2.

**Step b.** To a solution of *tert*-butyl ((3*S*)-6-(2,3-dihydroxypropyl)-7-fluoro-1-methyl-2-oxo-1,2,3,4,5,6-hexahydrobenzo[*b*][1,4]diazocin-3-yl)carbamate (650 mg, 1.64 mmol) in DCM (8 mL) was added 1,1,1-trimethoxyethane (785 mg, 6.54 mmol) and trimethylsilyl chloride (1.07 g, 9.81 mmol). The mixture was stirred at rt for 2 h and evaporated. The residue was dissolved in MeOH (7 mL) and $K_2CO_3$ (422 mg, 3.06 mmol) was added. The mixture was stirred at rt for 1 h. Upon completion, the reaction mixture was diluted with water (20 mL) and extracted with DCM (3 x 20 mL). The combined organic layers were washed with brine (20 mL), dried over $Na_2SO_4$ and evaporated. The residue was purified by column chromatography (DCM/ MeOH = 5/1) to give *tert*-butyl ((3S)-7-fluoro-1-methyl-6-(oxiran-2-ylmethyl)-2-oxo-1,2,3,4,5,6-hexahydrobenzo[b][1,4]diazocin-3-yl)carbamate (530 mg, 91% yield) as a brown oil.

m/z ES+ $[M+Na]^+$ 402.3.

**Step** c. To a solution of *tert-butyl* ((3*S*)-7-fluoro-1-methyl-6-(oxiran-2-ylmethyl)-2-oxo-1,2,3,4,5,6-hexahydrobenzo[*b*][1,4]diazocin-3-yl)carbamate (510 mg, 1.34 mmol) in EtOH (8 mL) was added $Na_2CO_3$ (284 mg, 2.69 mmol) and piperazine (578 mg, 6.72 mmol). The mixture was stirred at 60 °C for 2 h. Upon completion, the reaction was evaporated. The residue was diluted with water (10 mL) and extracted with DCM (3 x 10 mL). The combined organic layers were washed with brine (10 mL), dried over $Na_2SO_4$ and evaporated to give *tert-butyl* ((3S)-7-fluoro-6-(2-hydroxy-3-(piperazin-1-yl)propyl)-1-methyl-2-oxo-1,2,3,4,5,6-hexahydrobenzo[b][1,4]diazocin-3-yl)carbamate (630 mg, crude) as a

brown oil.

m/z ES+ $[M+H]^+$ 466.3.

**Step d.** To a solution of *tert*-butyl ((3*S*)-7-fluoro-6-(2-hydroxy-3-(piperazin-1-yl)propyl)-1-methyl-2-oxo-1,2,3,4,5,6-hexahydrobenzo[*b*][1,4]diazocin-3-yl)carbamate (620 mg, 1.33 mmol) and (*E*)-4-(dimethylamino) but-2-enoic acid hydrochloride (441 mg, 2.66 mmol) in DCM (10 mL) was added HATU (759 mg, 2.00 mmol) and DIPEA (516 mg, 4.00 mmol). The mixture was stirred at rt for 1 h. Upon completion, the reaction mixture was diluted with water (20 mL) and extracted with DCM (3 x 20 mL). The combined organic layers were washed with brine (20 mL), dried over $Na_2SO_4$, and evaporated. The residue was purified by column chromatography (DCM/MeOH = 5/1) to give *tert*-butyl ((3*S*)-6-(3-(4-((*E*)-4-(dimethylamino)but-2-enoyl)piperazin-1-yl)-2-hydroxypropyl)-7-fluoro-1-methyl-2-oxo-1,2,3,4,5,6-hexahydrobenzo[*b*][1,4]diazocin-3-yl)carbamate (530 mg, crude) as a brown oil.

$^1H$ NMR (400 MHz, DMSO-$d_6$) δ ppm 7.25 - 7.23 (m, 1H), 7.11 - 7.06 (m, 1H), 6.86 - 6.81 (m, 1H), 6.44 (d, *J* = 8.8 Hz, 1H), 5.25 (t, *J* = 8.0 Hz, 1H), 4.04 - 4.01 (m, 1H), 3.72 - 3.55 (m, 5H), 3.18 - 3.11 (m, 4H), 3.16 - 3.05 (m, 5H), 2.55 - 2.14 (m, 14H), 1.76 - 1.72 (m, 1H), 1.39 (s, 9H).

**Step e.** To a solution of *tert-butyl* ((3*S*)-6-(3-(4-((*E*)-4-(dimethylamino)but-2-enoyl)piperazin-1-yl)-2-hydroxypropyl)-7-fluoro-1-methyl-2-oxo-1,2,3,4,5,6-hexahydrobenzo[*b*][1,4]diazocin-3-yl)carbamate (530 mg, 0.92 mmol) in DCM (5 mL) was added TFA (1 mL). The mixture was stirred at rt for 1 h. Upon completion, the reaction mixture was evaporated. The residue was diluted with sat. aq. $NaHCO_3$ (5 mL) and extracted with DCM (3 x 10 mL). The combined organic layers were washed with brine (10 mL), dried over $Na_2SO_4$ and evaporated to give (3*S*)-3-amino-6-(3-(4-((*E*)-4-(dimethylamino)but-2-enoyl)piperazin-1-yl)-2-*hydroxypropyl)-7-fluoro-1-methyl-3,4,5,6-tetrahydrobenzo[b][1,4]diazocin-2(1H)-one* (395 mg, 90% yield) as a brown oil.

**Step f.** To a solution of (3*S*)-3-amino-6-(3-(4-((*E*)-4-(dimethylamino)but-2-enoyl)piperazin-1-yl)-2-hydroxypropyl)-7-fluoro-1-methyl-3,4,5,6-tetrahydrobenzo[*b*][1,4]diazocin-2(1*H*)-one (395 mg, 0.83 mmol) and **Intermediate 1** (274 mg, 1.24 mmol) in NMP (8 mL) was added DIPEA (214 mg, 1.66 mmol). The mixture was stirred at 90 °C for 2 h. Upon completion, the reaction mixture was diluted with water (20 mL) and extracted with EtOAc (3 x 20 mL). The combined organic layers were washed with brine (10 mL), dried over $Na_2SO_4$ and evaporated. The residue was purified by column chromatography (DCM/MeOH = 5/1) and re-purified by Prep-HPLC. The racemate was separated by cSFC (column: Phenomenex-Cellulose-2 (250×30mm, 10μm); mobile phase: A: $CO_2$; phase B: 0.1% $NH_4OH$ / EtOH; B%: 50%) to give two diastereoisomers, with unknown absolute configuration.

**Example 32A** (40 mg, 44% yield) as a white solid.

m/z ES+ $[M+H]^+$ 661.1; $^1H$ NMR (400 MHz, $CD_3OD$) δ ppm 7.40 - 7.33 (m, 1H), 7.31 - 7.26 (m, 1H), 7.24 - 7.16 (m, 1H), 6.86 (s, 1H), 6.76 - 6.62 (m, 2H), 4.44 - 4.27 (m, 1H), 3.77 - 3.68 (m, 1H), 3.67 - 3.57 (m, 4H), 3.47 (dd, *J* = 4.4, 14.4 Hz, 1H), 3.32 (s, 4H), 3.30 (m, 1H), 3.22 (dd, *J* = 3.6, 13.6 Hz, 1H), 3.17 - 3.13 (m, 1H), 3.13 - 3.08 (m, 1H), 2.98 - 2.88 (m, 1H), 2.56 (m, 3H), 2.52 - 2.43 (m, 2H), 2.43 - 2.37 (m, 10H), 1.79 (d, *J* = 13.2 Hz, 1H). **Example 32B** (15 mg, 16% yield) as a white solid.

m/z ES+ $[M+H]^+$ 661.1; $^1H$ NMR (400 MHz, $CD_3OD$) δ ppm 7.41 - 7.33 (m, 1H), 7.32 - 7.25 (m, 1H), 7.24 - 7.16 (m, 1H), 6.86 (s, 1H), 6.79 - 6.68 (m, 1H), 6.65 - 6.57 (m, 1H), 4.35 (d, *J* = 10.4 Hz, 1H), 3.77 - 3.67 (m, 1H), 3.67 - 3.57 (m, 4H), 3.42 (dd, *J* = 4.4, 14.4 Hz, 1H), 3.31 (s, 3H), 3.27 - 3.20 (m, 1H), 3.19 - 3.13 (m, 3H), 3.12 - 3.06 (m, 1H), 2.92 - 2.82 (m, 1H), 2.62 - 2.48 (m, 5H), 2.41 (s, 3H), 2.30 (m, 1H), 2.27 (s, 6H), 1.80 (d, *J* = 13.6 Hz, 1H).

**Examples 33A and 33B**

**2-(((*S*)-6-((*S*/*R*)-3-(4-Acryloylpiperazin-1-yl)-2-hydroxypropyl)-8-fluoro-1-methyl-2-oxo-1,2,3,4,5,6-hexahydrobenzo[*b*][1,4]diazocin-3-yl)amino)-6-methyl-4-(trifluoromethyl)nicotinonitrile (E33A)** and

**2-(((*S*)-6-((*R*/*S*)-3-(4-Acryloylpiperazin-1-yl)-2-hydroxypropyl)-8-fluoro-1-methyl-2-oxo-1,2,3,4,5,6-hexahydrobenzo[*b*][1,4]diazocin-3-yl)amino)-6-methyl-4-(trifluoromethyl)nicotinonitrile (E33B)**

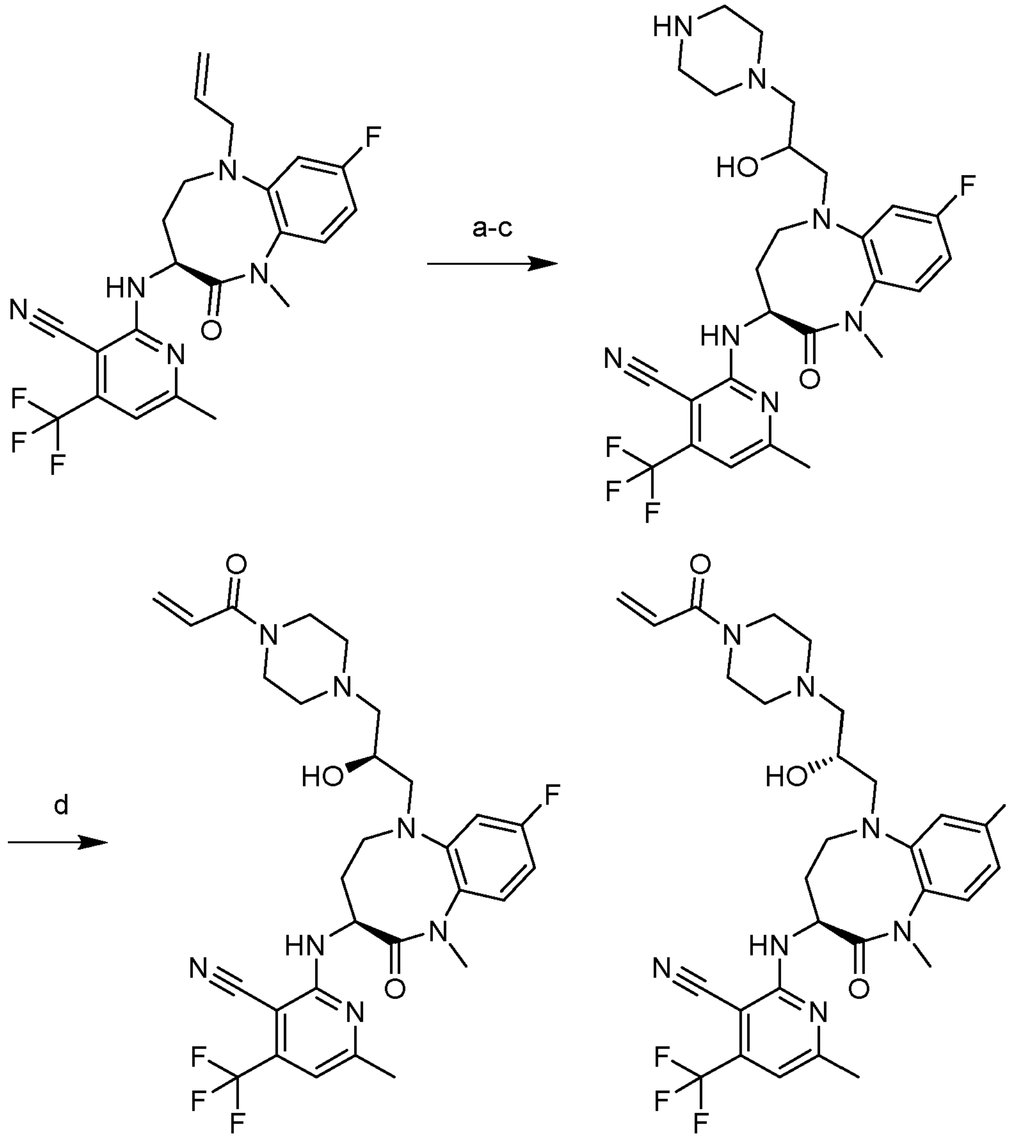


**Step a-c.** These steps were conducted in a similar manner to **Examples 32A/B,** steps a-c, using (S)-2-((6-allyl-8-fluoro-1-methyl-2-oxo-1,2,3,4,5,6-hexahydrobenzo[b][1,4]diazocin-3-yl)amino)-6-methyl-4-(trifluoromethyl)nicotinonitrile (prepared in a similar manner to **Intermediate 4** starting with **Intermediate 2b)** in step a.

m/z ES+ $[M+H]^+$ 550.4.

**Step d.** A mixture of 2-(((3S)-8-fluoro-6-(2-hydroxy-3-(piperazin-1-yl)propyl)-1-methyl-2-oxo-1,2,3,4,5,6-hexahydrobenzo[b][1,4]diazocin-3-yl)amino)-6-methyl-4-(trifluoromethyl)nicotinonitrile (200 mg, 0.31 mmol), acrylic acid (21 mg, 0.31 mmol), HATU (127 mg, 0.34 mmol) and DIPEA (118 mg, 0.92 mmol) in DCM (5 mL) was stirred at rt for 1 h under a $N_2$ atmosphere. Upon completion, the reaction mixture was diluted with DCM (50 mL) and washed with brine (10 mL), dried over $Na_2SO_4$ and evaporated. The residue was purified by Prep-HPLC to give two diastereoisomers, with unknown absolute configuration.

**Example 33A** (34 mg, 18% yield) as a white solid.

m/z ES+ $[M+H]^+$ 604.3; $^1$H NMR (400 MHz, $CDCl_3$) δ ppm 7.17 (dd, *J* = 6.0, 8.8 Hz, 1H), 6.96 - 6.91 (m, 1H), 6.82 - 6.74 (m, 1H), 6.71 (s, 1H), 6.60 - 6.54 (m, 1H), 6.33 - 6.29 (m, 1H), 6.15 (d, *J* = 7.2 Hz, 1H), 5.74 - 5.71 (m, 1H), 4.77-4.72 (m, 1H), 3.91 - 3.81 (m, 1H), 3.77 - 3.56 (m, 5H), 3.37 - 3.32 (m, 1H), 3.30 (s, 3H), 3.29 - 3.21 (m, 1H), 3.16 - 3.09 (m, 1H), 2.68 - 2.64 (m, 2H), 2.54 - 2.40 (m, 4H), 2.39 (s, 3H), 2.19 - 2.08 (m, 1H), 2.00 - 1.87 (m, 1H).

**Example 33B** (30 mg, 16% yield) as a white solid.

m/z ES+ $[M+H]^+$ 604.3; $^1$H NMR (400 MHz, $CDCl_3$) δ ppm 7.17 (dd, *J* = 6.0, 8.8 Hz, 1H), 6.90 (dd, *J* = 2.8, 11.2 Hz, 1H), 6.81 - 6.74 (m, 1H), 6.72 (s, 1H), 6.61 - 6.54 (m, 1H), 6.33 - 6.29 (m, 1H), 6.17 (d, *J* = 7.2 Hz, 1H), 5.74 - 5.71 (m, 1H), 4.77 - 4.71 (m, 1H), 3.97 - 3.89 (m, 1H), 3.87 - 3.57 (m, 5H), 3.38 - 3.34 (m, 1H), 3.29 (s, 3H), 3.24 - 3.11 (m, 2H), 2.65 (s, 2H), 2.59 - 2.45 (m, 3H), 2.43 - 2.37 (m, 4H), 2.28 - 2.17 (m, 1H), 2.01 - 1.89 (m, 1H).

**Examples 34A and 34B**

**2-(((*S*)-6-((*S*/*R*)-3-(4-((*E*)-4-(Dimethylamino)but-2-enoyl)piperazin-1-yl)-2-hydroxypropyl)-8-fluoro-1-methyl-2-oxo-1,2,3,4,5,6-hexahydrobenzo[*b*][1,4]diazocin-3-yl)amino)-6-methyl-4-(trifluoromethyl)nicotinonitrile (E34A)** and

**2-(((*S*)-6-((*R*/*S*)-3-(4-((*E*)-4-(Dimethylamino)but-2-enoyl)piperazin-1-yl)-2-hydroxypropyl)-8-fluoro-1-methyl-2-oxo-1,2,3,4,5,6-hexahydrobenzo[*b*][1,4]diazocin-3-yl)amino)-6-methyl-4-(trifluoromethyl)nicotinonitrile (E34B)**

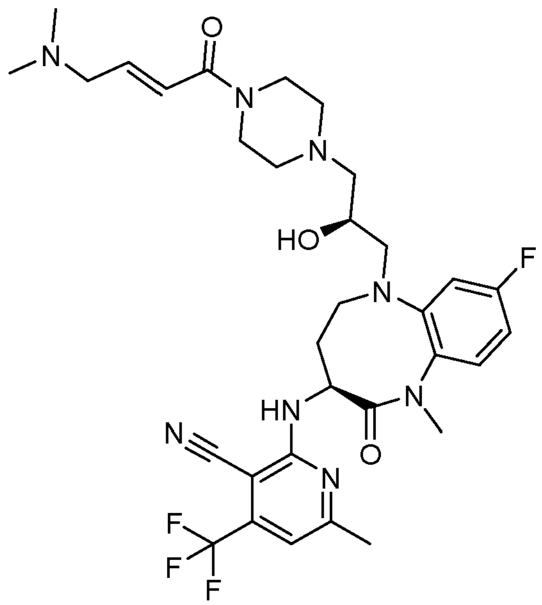

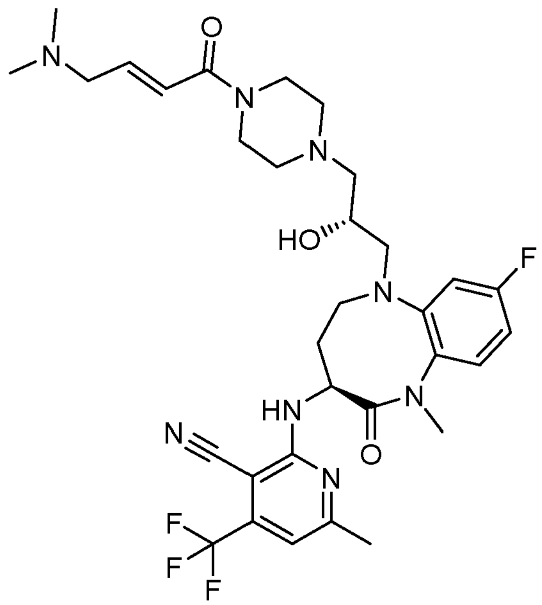

The title compounds were prepared in a similar manner to **Examples 33A/B,** using (*E*)-4-(dimethylamino)but-2-enoic acid in step d.

**Example 34A**

m/z ES+ $[M+H]^+$ 661.3; $^1H$ NMR (400 MHz, $CDCl_3$) δ ppm 7.18 - 7.13 (m, 1H), 6.93 - 6.89 (m, 1H), 6.88 - 6.83 (m, 1H), 6.77 - 6.72 (m, 1H), 6.70 (s, 1H), 6.43 - 6.39 (m, 1H), 6.15 - 6.12 (m, 1H), 4.77 - 4.72 (m, 1H), 3.84 - 3.75 (m, 1H), 3.70 - 3.51 (m, 5H), 3.37 - 3.32 (m, 1H), 3.29 (s, 3H), 3.26 - 3.20 (m, 1H), 3.10 - 3.07 (m, 3H), 2.63 - 2.61 (m, 2H), 2.48 - 2.39 (m, 4H), 2.37 (s, 3H), 2.26 (s, 6H), 2.26 - 2.20 (m, 1H), 1.99 - 1.88 (m, 1H).

**Example 34B**

m/z ES+ $[M+H]^+$ 661.3; $^1H$ NMR (400 MHz, $CDCl_3$) δ ppm 7.18 - 7.14 (m, 1H), 6.93 - 6.82 (m, 2H), 6.80 - 6.73 (m, 1H), 6.71 (s, 1H), 6.46 - 6.39 (m, 1H), 6.17 (d, *J* = 7.2 Hz, 1H), 4.77 - 4.72 (m, 1H), 3.96 - 3.88 (m, 1H), 3.79 (s, 1H), 3.74 - 3.58 (m, 4H), 3.37-3.33 (m, 1H), 3.29 (s, 3H), 3.24 - 3.06 (m, 4H), 2.63 (s, 2H), 2.57 - 2.43 (m, 3H), 2.43 - 2.36 (m, 4H), 2.28 (s, 6H), 2.26 - 2.20 (m, 1H), 1.99 - 1.88 (m, 1H).

**Example 35**

**2-(((3*S*)-6-(3-(4-Acryloylpiperazin-1-yl)-2-hydroxypropyl)-1-methyl-2-oxo-1,2,3,4,5,6-hexahydrobenzo[*b*][1,4]diazocin-3-yl)amino)-6-methyl-4-(trifluoromethyl)nicotinonitrile (E35)**

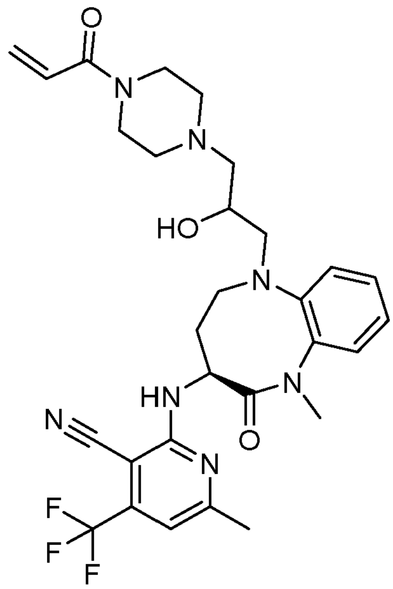

The title compound was prepared in a similar manner to **Examples 33A/B,** using (*S*)-2-((6-allyl-1-methyl-2-oxo-1,2,3,4,5,6-hexahydrobenzo[b][1,4]diazocin-3-yl)amino)-6-methyl-4-(trifluoromethyl)nicotinonitrile (prepared in a similar manner to **Intermediate 4** starting with *tert*-butyl *N*-(2-aminophenyl)carbamate) in step a.

m/z ES+ $[M+H]^+$ 586.3; $^1H$ NMR (400 MHz, $CDCl_3$) δ ppm 7.33 - 7.23 (m, 2H), 7.21 - 7.18 (m, 1H), 7.17 - 7.11 (m, 1H), 6.61 (s, 1H), 6.48 (dd, *J* = 10.4, 16.8 Hz, 1H), 6.21 (dd, *J* = 1.6, 16.8 Hz, 1H), 6.14 (d, *J* = 7.2 Hz, 1H), 5.63 (dd, *J* = 2.0, 10.4 Hz, 1H), 4.53 - 4.43 (m, 1H), 3.74 - 3.72 (m, 1H), 3.69 - 3.60 (m, 1H), 3.59 - 3.38 (m, 4H), 3.42 - 3.22 (m, 4H), 3.19 - 2.98 (m, 2H), 2.90 (dd, *J* = 6.8, 13.2 Hz, 1H), 2.76 - 2.60 (m, 2H), 2.49 - 2.36 (m, 3H), 2.37-2.25 (m, 4H), 2.14 - 2.03 (m, 1H), 1.90 - 1.78 (m, 1H).

**Example 36**

**2-(((3*S*)-6-(3-(6-Acryloyl-2,6-diazaspiro[3.3]heptan-2-yl)-2-hydroxypropyl)-1-methyl-2-oxo-1,2,3,4,5,6-hexahydrobenzo[*b*][1,4]diazocin-3-yl)amino)-6-methyl-4-(trifluoromethyl)nicotinonitrile (E36)**

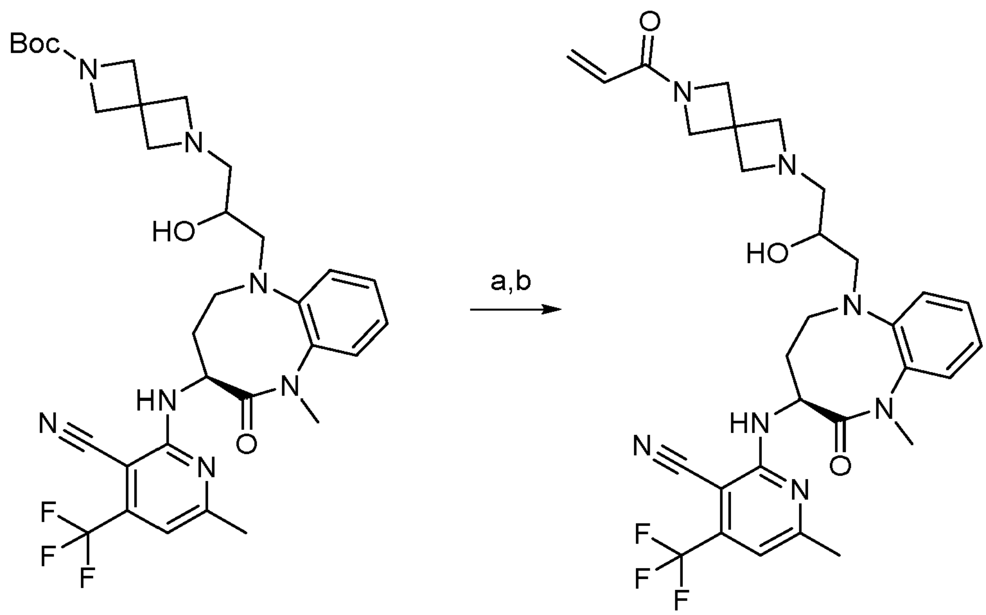


**Step** a. A mixture of *tert*-butyl 6-(3-((*S*)-4-((3-cyano-6-methyl-4-(trifluoromethyl)pyridin-2-yl)amino)-6-methyl-5-oxo-3,4,5,6-tetrahydrobenzo[*b*][1,4]diazocin-1(2*H*)-yl)-2-hydroxypropyl)-2,6-diazaspiro[3.3]heptane-2-carboxylate

(prepared in a similar manner to **Examples 32A/B,** steps a-c, using *tert*-butyl 2,6-diazaspiro[3.3]heptane-2-carboxylate (CAS Number 1041026-70-3) in step c; 30 mg, 0.047 mmol) and TFA (460 mg, 4.05 mmol), in DCM (1.5 mL) was stirred at rt for 30 min under a $N_2$ atmosphere. Upon completion, the reaction mixture was evaporated. The residue was diluted with water (5 mL), adjusted to pH 7 with sat. aq. $NaHCO_3$ and extracted with EtOAc (3 x 20 mL). The combined organic layers were washed with brine (20 mL), dried over $Na_2SO_4$ and evaporated to give 2-(((3*S*)-6-(2-hydroxy-3-(2,6-diazaspiro[3.3]heptan-2-yl)propyl)-1-methyl-2-oxo-1,2,3,4,5,6-hexahydrobenzo[*b*][1,4]diazocin-3-yl)amino)-6-methyl-4-(trifluoromethyl)nicotinonitrile (25 mg, crude) as a yellow oil.

m/z ES+ $[M+H]^+$ 544.2.

**Step b.** A mixture of 2-(((3*S*)-6-(2-hydroxy-3-(2,6-diazaspiro[3.3]heptan-2-yl)propyl)-1-methyl-2-oxo-1,2,3,4,5,6-hexahydrobenzo[*b*][1,4]diazocin-3-yl)amino)-6-methyl-4-(trifluoromethyl)nicotinonitrile (25 mg, 0.046 mmol), acrylic acid (3.6 mg, 0.050 mmol), HATU (17 mg, 0.046 mmol) and DIPEA (18 mg, 0.14 mmol) in DCM (1 mL) was stirred at rt for 1 h under a $N_2$ atmosphere. Upon completion, the reaction mixture was quenched with water (0.5 mL) and evaporated. The residue was purified by Prep-HPLC to give the title compound (2.5 mg, 8% yield) as a brown solid.

m/z ES+ $[M+H]^+$ 598.4; $^1$H NMR (400 MHz, $CDCl_3$) δ ppm 7.45 - 7.34 (m, 2H), 7.34 - 7.29 (m, 1H), 7.27 ( s, 1H), 6.70 (s, 1H), 6.39 - 6.29 (m, 1H), 6.28 - 6.11 (m, 2H), 5.73 - 5.64 (m, 1H), 4.62 - 4.46 (m, 1H), 4.30 (s, 2H), 4.14 (s, 2H), 3.61-3.60 (m, 1H), 3.52 - 3.37 (m, 5H), 3.34 - 3.31 (m, 3H), 3.28 - 3.05 (m, 3H), 3.02 - 2.87 (m, 1H), 2.56 - 2.41 (m, 2H), 2.39 (s, 3H), 2.23 - 2.11 (m, 1H), 1.98 - 1.88 (m, 1H).

### Example 37

**(*S*)-2-((6-(3-(4-Acryloylpiperazin-1-yl)propyl)-7-fluoro-1-methyl-2-oxo-1,2,3,4,5,6-hexahydrobenzo[b][1,4]diazocin-3-yl)amino)-6-methyl-4-(trifluoromethyl)nicotinonitrile (E37)**

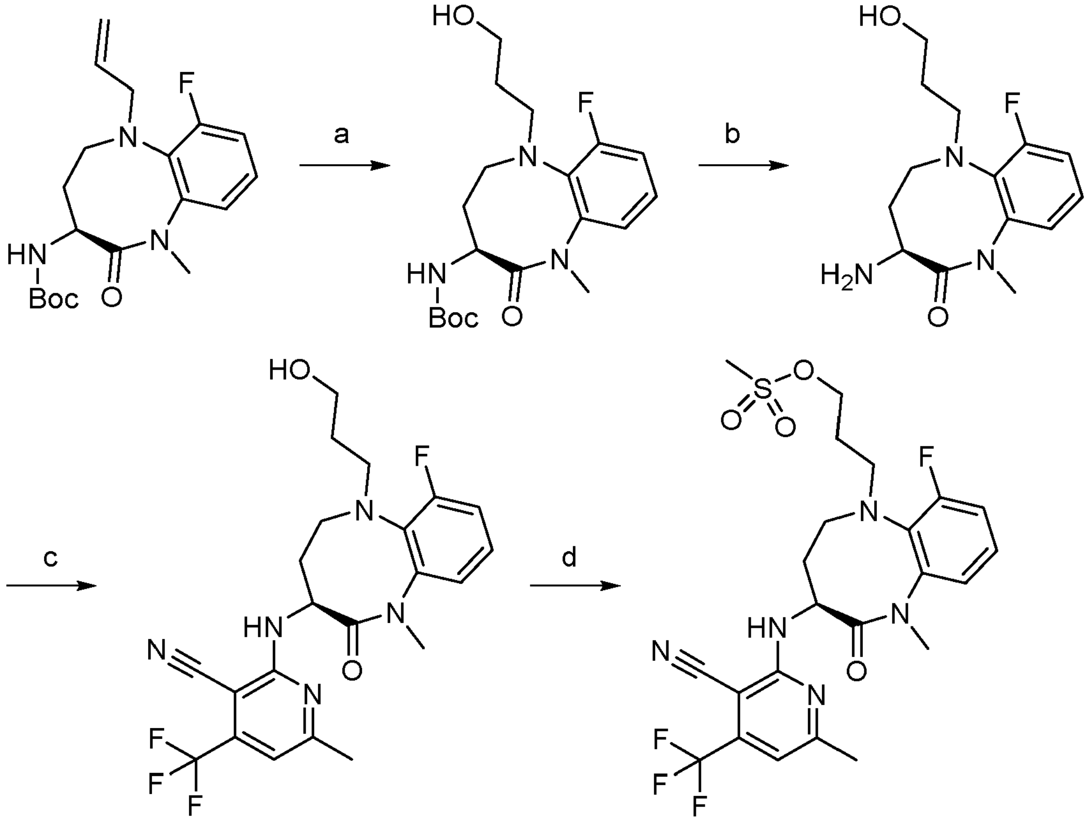

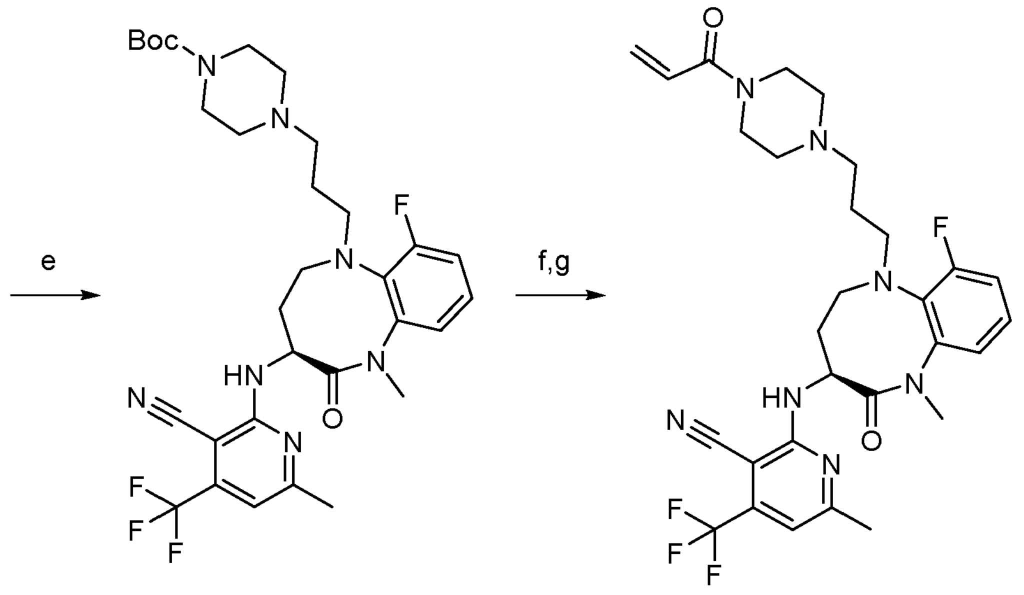


**Step** a. To a solution of **Intermediate 3** (1.0 g, 2.75 mmol) in THF (10 mL) was added borane dimethyl sulfide complex (10 M, 8.25 mmol). The mixture was stirred at rt for 2 h. The reaction mixture was diluted with MeOH (5 mL) and evaporated. The residue was dissolved in THF (10 mL) and treated with NaOH (330 mg, 8.25 mmol) and $H_2O_2$ (2.81 g, 24.8 mmol, 30% w/w). The resulting mixture was stirred at rt for 1 h. Upon completion, the reaction mixture was diluted with water (30 mL), extracted with EtOAc (30 mL). The organic layer was washed with water (2 x 30 mL), 1 M aq. $Na_2S_2O_3$ (30 mL), brine (30 mL), dried over $Na_2SO_4$ and evaporated. The residue was purified by column chromatography (PE/EtOAc = 1/1) to give *tert-butyl* (*S*)-(7-fluoro-6-(3-hydroxypropyl)-1-methyl-2-oxo-1,2,3,4,5,6-hexahydrobenzo[b][1,4]diazocin-3-yl)carbamate (0.5 g, 48% yield) as an oil.

m/z ES+ $[M+H]^+$ 382.3.

**Step b**. A solution of *tert*-butyl (*S*)-(7-fluoro-6-(3-hydroxypropyl)-1-methyl-2-oxo-1,2,3,4,5,6-hexahydrobenzo[*b*][1,4]diazocin-3-yl)carbamate (0.4 g, 1.05 mmol) in HCl (4 M in dioxane, 5 mL) was stirred at rt for 30 min. The reaction mixture was evaporated to give (*S*)-3-amino-7-fluoro-6-(3-hydroxypropyl)-1-methyl-3,4,5,6-tetrahydrobenzo[*b*][1,4]diazocin-2(1*H*)-one as a HCl salt (0.33 g, 99% yield) as a white solid, which was used in the next step directly.

m/z ES+ $[M+H]^+$ 282.1.

**Step c.** To a solution of (*S*)-3-amino-7-fluoro-6-(3-hydroxypropyl)-1-methyl-3,4,5,6-tetrahydrobenzo[*b*][1,4]diazocin-2(1H)-one (HCl salt, 0.33 g, 1.04 mmol) and DIPEA (671 mg, 5.19 mmol) in NMP (3 mL) was added **Intermediate 1** (344 mg, 1.56 mmol). The reaction mixture was stirred at 100 °C for 1 h. Upon completion, the reaction mixture was diluted with water (30 mL), extracted with EtOAc (30 mL), washed with brine (30 mL), dried over $Na_2SO_4$ and evaporated. The residue was purified by column chromatography (PE/EtOAc = 1/1) to give (*S*)-2-((7-fluoro-6-(3-hydroxypropyl)-1-methyl-2-oxo-1,2,3,4,5,6-hexahydrobenzo[*b*][1,4]diazocin-3-yl)amino)-6-methyl-4-(trifluoromethyl)nicotinonitrile (0.23 g, 48% yield) as a brown oil.

m/z ES+ $[M+H]^+$ 466.1; $^1$H NMR (400 MHz, $CDCl_3$) $\delta$ ppm 7.31 - 7.26 (m, 1H), 7.17 - 7.07 (m, 2H), 6.68 (s, 1H), 6.20 (d, *J* = 7.2 Hz, 1H), 4.50 - 4.41 (m, 1H), 3.76 - 3.67 (m, 2H), 3.43 - 3.34 (m, 2H), 3.34 - 3.29 (m, 3H), 3.21 - 3.15 (m, 2H), 2.38 - 2.36 (m, 3H), 2.34 - 2.19 (m, 1H), 2.04 - 1.98 (m, 1H), 1.69 - 1.53 (m, 2H).

**Step d.** To a solution of (*S*)-2-((7-fluoro-6-(3-hydroxypropyl)-1-methyl-2-oxo-1,2,3,4,5,6-hexahydrobenzo[*b*][1,4]diazocin-3-yl)amino)-6-methyl-4-(trifluoromethyl)nicotinonitrile (230 mg, 0.49 mmol) and TEA (100 mg, 0.99 mmol) in DCM (5 mL) was added methanesulfonyl chloride (85 mg, 0.74 mmol) at 0 °C. The reaction mixture was stirred at 0 °C for 1 h. Upon completion, the reaction mixture was diluted with water (30 mL) and extracted with DCM (30 mL). The organic layer was washed with brine (30 mL), dried over $Na_2SO_4$ and evaporated to give (*S*)-3-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)propyl methanesulfonate (0.25 g, 93% yield) as a brown oil.

m/z ES+ $[M+H]^+$ 544.3.

**Step e.** A mixture of (*S*)-3-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)propyl methanesulfonate (0.25 g, 0.46 mmol), *tert-butyl* piperazine-1-carboxylate (428 mg, 2.30 mmol) and $K_2CO_3$ (127 mg, 0.92 mmol) in DMF (5 mL) was stirred at 60 °C for 1 h. Upon completion, the reaction mixture was diluted with water (40 mL), extracted with EtOAc (40 mL). The organic layer was washed with brine (40 mL), dried over $Na_2SO_4$ and evaporated. The residue was purified by column chromatography (PE/EtOAc = 1/1) to give *tert*-butyl (*S*)-4-(3-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)propyl)piperazine-1-carboxylate (70 mg, 24% yield) as a yellow oil.

m/z ES+ $[M+H]^+$ 634.3; $^1H$ NMR (400 MHz, $CDCl_3$) δ ppm 7.29 - 7.22 (m, 1H), 7.14 - 7.06 (m, 2H), 6.67 (s, 1H), 6.24 - 6.14 (m, 1H), 4.49 - 4.38 (m, 1H), 3.50 - 3.40 (m, 4H), 3.34 - 3.25 (m, 4H), 3.20 - 2.94 (m, 4H), 2.54 - 2.38 (m, 5H), 2.36 (s, 3H), 2.33 - 2.18 (m, 2H), 1.66 - 1.50 (m, 2H), 1.45 (s, 9H).

**Step f.** A solution of *tert*-butyl (*S*)-4-(3-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)propyl)piperazine-1-carboxylate (0.06 g, 0.095 mmol) in HCl (4 M in EtOAc, 3 mL) was stirred at rt for 30 min. Upon completion, the reaction mixture was evaporated to give (*S*)-2-((7-fluoro-1-methyl-2-oxo-6-(3-(piperazin-1-yl)propyl)-1,2,3,4,5,6-hexahydrobenzo[*b*][1,4]diazocin-3-yl)amino)-6-methyl-4-(trifluoromethyl)nicotinonitrile as an HCl salt (0.05 g, 93% yield) as a white solid.

m/z ES+ $[M+H]^+$ 534.3.

**Step g.** To a solution of (*S*)-2-((7-fluoro-1-methyl-2-oxo-6-(3-(piperazin-1-yl)propyl)-1,2,3,4,5,6-hexahydrobenzo[*b*][1,4]diazocin-3-yl)amino)-6-methyl-4-(trifluoromethyl)nicotinonitrile (HCl salt, 50 mg, 0.088 mmol) and TEA (44.4 mg, 0.44 mmol) in DCM (3 mL) was added acryloyl chloride (11.9 mg, 0.13 mmol). The reaction mixture was stirred at rt for 30 min. Upon completion, the reaction mixture was diluted with water (30 mL) and extracted with DCM (30 mL). The organic layer was washed with brine (30 mL), dried over $Na_2SO_4$ and evaporated. The residue was purified by prep-HPLC to give the title compound (33 mg, 59% yield) as a white solid.

m/z ES+ $[M+H]^+$ 588.4; $^1H$ NMR (400 MHz, $CDCl_3$) δ ppm 7.32 - 7.27 (m, 1H), 7.17 - 7.08 (m, 2H), 6.69 (s, 1H), 6.60 - 6.50 (dd, *J* = 10.4, 16.8 Hz, 1H), 6.30 (dd, *J* = 1.6, 16.8 Hz, 1H), 6.18 (d, *J* = 7.2 Hz, 1H), 5.72 (d, *J* = 10.4 Hz, 1H), 4.50 - 4.40 (m, 1H), 3.92 - 3.46 (m, 4H), 3.38 - 3.28 (m, 4H), 3.27 - 2.94 (m, 4H), 2.79 - 2.41 (m, 5H), 2.38 (s, 3H), 2.36 - 2.20 (m, 2H), 1.84 - 1.71 (m, 2H).

**Example 38**

**(*S*)-2-((6-(3-(4-Acryloylpiperazin-1-yl)propyl)-8-fluoro-1-methyl-2-oxo-1,2,3,4,5,6-hexahydrobenzo[*b*][1,4]diazocin-3-yl)amino)-6-methyl-4-(trifluoromethyl)nicotinonitrile (E38)**

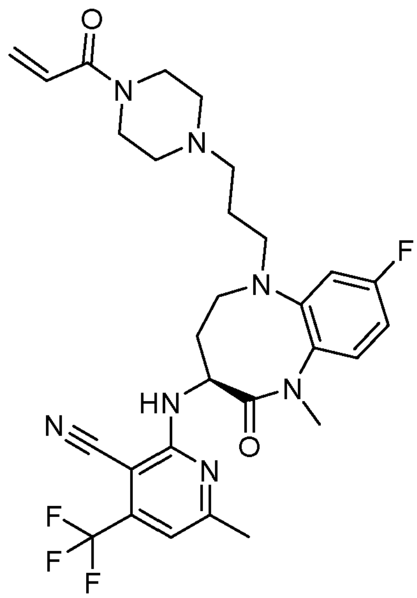

The title compound was prepared in a similar manner to **Example 37** using (*S*)-2-((6-allyl-8-fluoro-1-methyl-2-oxo-1,2,3,4,5,6-hexahydrobenzo[b][1,4]diazocin-3-yl)amino)-6-methyl-4-(trifluoromethyl)nicotinonitrile (prepared in a similar manner to **Intermediate 3** starting with **Intermediate 2b)** in step a.

m/z ES+ $[M+H]^+$ 588.3; $^1H$ NMR (400 MHz, $CD_3OD$) δ ppm 7.32 - 7.27 (m, 1H), 7.03 - 6.99 (m, 2H), 6.88 (s, 1H), 6.79 - 6.71 (m, 2H), 6.19 - 6.10 (m, 1H), , 5.72 (d, *J* = 10.0 Hz, 1H), 4.75 - 4.71 (m, 1H), 3.71 - 3.55 (m, 5H), 3.41 - 3.36 (m, 1H), 3.33 - 3.28 (m, 1H), 3.27 (s, 3H), 3.26 - 3.22 (m, 1H), 2.43 - 2.39 (m, 9H), 2.19 - 2.11 (m, 1H), 1.93. - 1.88 (m, 1H), 1.78. - 1.72 (m, 1H).

**Example 39**

**(*S*,*E*)-2-((6-(3-(4-(4-(Dimethylamino)but-2-enoyl)piperazin-1-yl)propyl)-8-fluoro-1-methyl-2-oxo-1,2,3,4,5,6-hexahydrobenzo[*b*][1,4]diazocin-3-yl)amino)-6-methyl-4-(trifluoromethyl)nicotinonitrile (E39)**

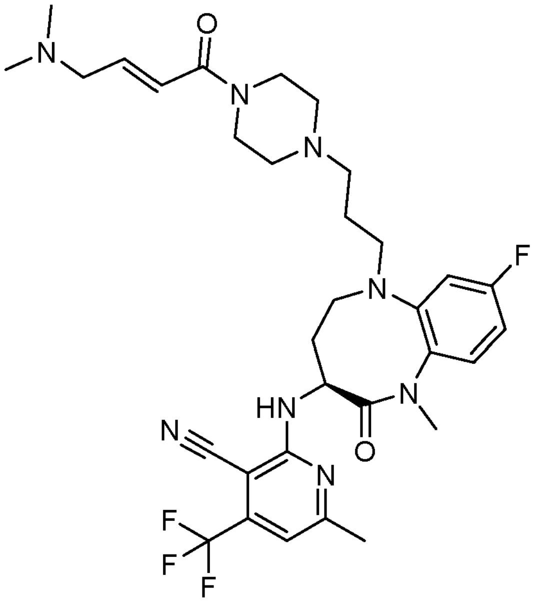

The title compound was prepared in a similar manner to **Example 38,** using (E)-4-(dimethylamino)but-2-enoic acid in the final step, according to **Example 1,** step c.

m/z ES+ $[M+H]^+$ 645.3; $^1H$ NMR (400 MHz, $CD_3OD$) δ ppm 7.34 - 7.27 (m, 1H), 7.05 (d, *J* = 11.6 Hz, 1H), 6.88 (s, 1H), 6.81 - 6.23 (m, 3H), 4.68 - 4.75 (m, 1H), 3.76 - 3.14 (m, 5H), 3.51 - 3.47 (m, 2H), 3.27 - 3.12 (m, 4H), 2.75 - 2.33 (m, 16H), 2.22 - 2.09 (m, 1H), 1.99 - 1.88 (m, 1H), 1.72 - 1.68 (m, 2H), 1.36. - 1.21 (m, 1H).

**Example 40**

**(*S*,*E*)-2-((6-(3-((1-(4-(Dimethylamino)but-2-enoyl)azetidin-3-yl)oxy)propyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile (E40)**

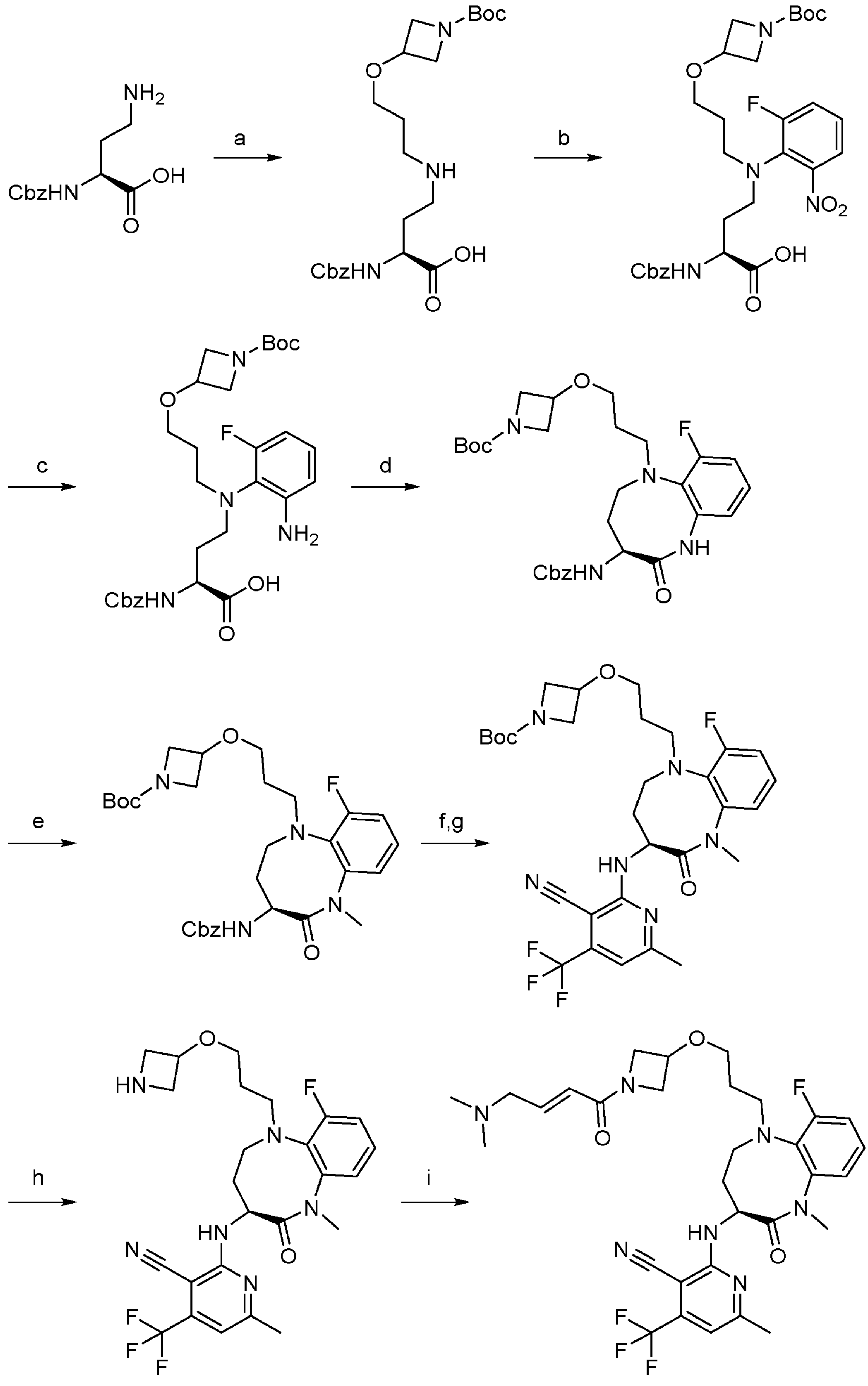
a
b
c
d
e
f,g
h
i

**Step a.** To a mixture of (*S*)-4-amino-2-(((benzyloxy)carbonyl)amino)butanoic acid (CAS Number 62234-40-6; 33.0 g, 131 mmol), 4 Å molecular sieves (8 g) and $NaBH_3CN$ (9.05 g, 144 mmol) in MeOH (50 mL) was added acetic acid (39.3 g, 654 mmol). A solution of **Intermediate 8a** (30 g, 131 mmol) in MeOH (300 mL) was added slowly and the mixture was stirred at rt for 30 min. The mixture was stirred at rt for 12 h. Upon completion, the reaction mixture was filtered and evaporated. The residue was purified by reverse phase flash chromatography (water (0.1% FA)/MeCN) to give (*S*)-2-(((benzyloxy)carbonyl)amino)-4-((3-((1-(*tert*-butoxycarbonyl)azetidin-3-yl)oxy)propyl)amino)butanoic acid (23.0 g, 38% yield) as a yellow solid.

m/z ES+ $[M+H]^+$ 466.3.

**Step b.** To a solution of (*S*)-2-(((benzyloxy)carbonyl)amino)-4-((3-((1-(*tert*-butoxycarbonyl)azetidin-3-yl)oxy)propyl)amino)butanoic acid (30.0 g, 64.4 mmol) and 1,2-difluoro-3-nitro-benzene (15.4 g, 96.7 mmol) in DMF (150 mL) was added $Cs_2CO_3$ (42.0 g, 129 mmol) and the mixture was stirred at 100 °C for 30 min. Upon completion, the reaction mixture was quenched with 1 M aq. HCl (30 mL) at rt, diluted with water (150 mL) and extracted with EtOAc (3 x 200 mL). The combined organic layers were washed with brine (3 x 150 mL) and evaporated. The residue was purified by column chromatography (PE/EtOAc = 1/1) to give (S)-2-(((benzyloxy)carbonyl)amino)-4-((3-((1-(tert-butoxycarbonyl)azetidin-3-yl)oxy)propyl)(2-fluoro-6-nitrophenyl)amino)butanoic acid (19.0 g, 49% yield) as a yellow solid.

m/z ES+ $[M+H]^+$ 605.3.

**Step c.** To a solution of (*S*)-2-(((benzyloxy)carbonyl)amino)-4-((3-((1-(*tert*-butoxycarbonyl)azetidin-3-yl)oxy)propyl)(2-fluoro-6-nitrophenyl)amino)butanoic acid (17.0 g, 28.1 mmol) in EtOAc (100 mL) was added 3% Pt-V/C (9.14 g, 1.41 mmol) and the mixture was stirred at rt for 1 h under $H_2$ (15 psi). Upon completion, the reaction mixture was filtered and evaporated to give (*S*)-4-((2-amino-6-fluorophenyl)(3-((1-(tert-butoxycarbonyl)azetidin-3-yl)oxy)propyl)amino)-2-(((benzyloxy)carbonyl)amino)butanoic acid (14.0 g, 87% yield) as a yellow oil.

m/z ES+ $[M+H]^+$ 575.3.

**Step d.** To a solution of (*S*)-4-((2-amino-6-fluorophenyl)(3-((1-(*tert*-butoxycarbonyl)azetidin-3-yl)oxy)propyl)amino)-2-(((benzyloxy)carbonyl)amino)butanoic acid (32 g, 55.7 mmol) in DCM (3.2 L) was added HATU (25.4 g, 66.8 mmol) and DIPEA (14.4 g, 112 mmol) and the mixture was stirred at rt for 1 h. Upon completion, the reaction mixture was washed with water (3 x 1 L), dried over $Na_2SO_4$ and evaporated. The residue was purified by column chromatography (PE/EtOAc = 1/1) to give *tert*-butyl (*S*)-3-(3-(4-(((benzyloxy)carbonyl)amino)-10-fluoro-5-oxo-3,4,5,6-tetrahydrobenzo[*b*][1,4]diazocin-1(2*H*)-yl)propoxy)azetidine-1-carboxylate (30.0 g, crude) as a yellow solid.

m/z ES+ $[M+H]^+$ 557.3.

**Step e.** To a solution of *tert*-butyl (*S*)-3-(3-(4-(((benzyloxy)carbonyl)amino)-10-fluoro-5-oxo-3,4,5,6-tetrahydrobenzo[*b*][1,4]diazocin-1(2*H*)-yl)propoxy)azetidine-1-carboxylate (30.0 g, 53.9 mmol) in DMF (300 mL) was added $K_2CO_3$ (22.4 g, 162 mmol) and methyl iodide (38.3 g, 270 mmol). The mixture was stirred at rt for 2 h. Upon completion, the reaction mixture was quenched with sat. aq. $NH_4Cl$ (200 mL) at rt, diluted with water (200 mL) and extracted with EtOAc (3 x 150 mL). The combined organic layers were washed with brine (2 x 150 mL), dried over $Na_2SO_4$ and evaporated. The residue was purified by column chromatography (PE/EtOAc = 3/1 to 1/1) to give *tert-butyl* (*S*)-3-(3-(4-(((benzyloxy)carbonyl)amino)-10-fluoro-6-methyl-5-oxo-3,4,5,6-tetrahydrobenzo[*b*][1,4]diazocin-1(2*H*)-yl)propoxy)azetidine-1-carboxylate (3.1 g, 76% yield) as a yellow solid.

m/z ES+ $[M+H]^+$ 571.3.

**Step f.** To a solution of *tert*-butyl (*S*)-3-(3-(4-(((benzyloxy)carbonyl)amino)-10-fluoro-6-methyl-5-oxo-3,4,5,6-tetrahydrobenzo[*b*][1,4]diazocin-1(2*H*)-yl)propoxy)azetidine-1-carboxylate (25.0 g, 43.8 mmol) in 2-propanol (250 mL) was added 10% Pd/C (5.0 g) and the mixture was stirred at 45 °C for 12 h under a $H_2$ atmosphere. Upon completion, the reaction mixture was filtered and evaporated to give *tert*-butyl (*S*)-3-(3-(4-amino-10-fluoro-6-methyl-5-oxo-3,4,5,6-tetrahydrobenzo[*b*][1,4]diazocin-1(2*H*)-yl)propoxy)azetidine-1-carboxylate (19 g, 95% yield) as a yellow oil.

m/z ES+ $[M+H]^+$ 437.1

**Step g.** A mixture of *tert-butyl* (S)-3-(3-(4-amino-10-fluoro-6-methyl-5-oxo-3,4,5,6-tetrahydrobenzo[*b*][1,4]diazocin-1(2H)-yl)propoxy)azetidine-1-carboxylate (19.0 g, 43.5 mmol), **Intermediate 1** (14.4 g, 65.3 mmol) and DIPEA (16.9 g, 131 mmol) in NMP (190 mL) was degassed and purged 3 times with $N_2$, and then the mixture was stirred at 100 °C for 1 h under a $N_2$ atmosphere. Upon completion, the reaction mixture was diluted with water (400 mL) and extracted with EtOAc (3 x 200 mL). The combined organic layers were washed with brine (200 mL), dried over $Na_2SO_4$ and evaporated. The residue was purified by column chromatography (PE/EtOAc = 10/1 to 1/1) to give *tert*-butyl (*S*)-3-(3-(4-((3-cyano-6-methyl-4-(trifluoromethyl)pyridin-2-yl)amino)-10-fluoro-6-methyl-5-oxo-3,4,5,6-tetrahydrobenzo[b][1,4]diazocin-1(2H)-yl)propoxy)azetidine-1-carboxylate (15.0 g, 56% yield) as a yellow oil.

m/z ES+ $[M+H]^+$ 621.3.

**Step h.** To a solution of *tert*-butyl (*S*)-3-(3-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)propoxy)azetidine-1-carboxylate (15.0 g, 24.2 mmol) in DCM (200 mL) was added TFA (27.6 g, 242 mmol) and the mixture was stirred at rt for 30 min. Upon completion, the reaction mixture was quenched with sat. aq. $NaHCO_3$ (200 mL), diluted with water (30 mL) and extracted with DCM (3 x 150 mL). The combined organic layers were washed with brine, dried over $Na_2SO_4$ and evaporated to give

(*S*)-2-((6-(3-(azetidin-3-yloxy)propyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile (11.0 g, 87% yield) as a yellow solid.

m/z ES+ $[M+H]^+$ 521.3.

**Step** i. A mixture of (S)-2-((6-(3-(azetidin-3-yloxy)propyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile (11 g, 21.1 mmol), (*E*)-4-(dimethylamino)but-2-enoic acid (4.2 g, 25.4 mmol), HATU (9.6 g, 25.4 mmol) and DIPEA (8.19 g, 63.4 mmol) in DCM (50 mL) was degassed and purged 3 times with $N_2$, and t the mixture was stirred at rt for 1 h under a $N_2$ atmosphere. Upon completion, the reaction mixture was diluted with DCM (150 mL), washed with brine (3 x 50 mL), dried over $Na_2SO_4$ and evaporated. The residue was purified by column chromatography (DCM/MeOH = 10/1) to give the title compound (8.00 g, 59% yield) as an off-white solid.

m/z ES+ $[M+H]^+$ 632.4; $^1$H NMR (400 MHz, $CDCl_3$) δ ppm 7.34 - 7.29 (m, 1H), 7.18 - 7.11 (m, 2H), 6.86 - 6.76 (m, 1H), 6.70 (s, 1H), 6.41 (d, *J* = 15.2 Hz, 1H), 6.26 (dd, *J* = 1.6, 7.2 Hz, 1H), 4.53 - 4.41 (m, 2H), 4.36 - 4.29 (m, 1H), 4.26 - 4.22 (m, 1H), 4.15 - 4.09 (m, 1H), 3.94 - 3.85 (m, 1H), 3.78 - 3.76 (m, 2H), 3.49 - 3.38 (m, 2H), 3.37 - 3.28 (m, 4H), 3.20 - 3.06 (m, 3H), 2.81 (s, 6H), 2.39 (s, 3H), 2.30 - 2.18 (m, 1H), 1.81 (d, *J* = 13.2 Hz, 1H), 1.66 - 1.52 (m, 2H).

**Example 41**

**(S, E)-2-((6-(3-((1-(4-(Dimethylamino)but-2-enoyl)azetidin-3-yl)oxy)propyl)-1-methyl-2-oxo-1,2,3,4,5,6-hexahydrobenzo[b][1,4]diazocin-3-yl)amino)-6-methyl-4-(trifluoromethyl)nicotinonitrile (E41)**

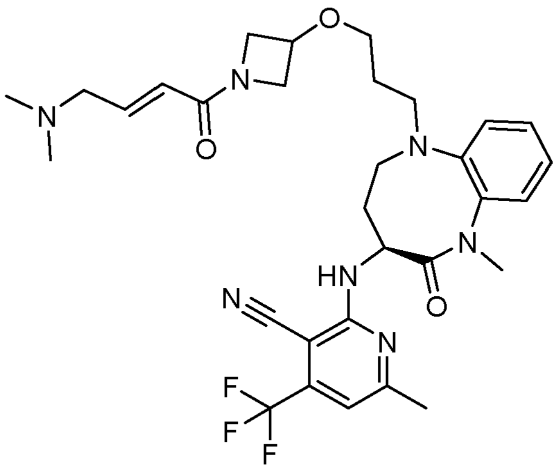

The title compound was prepared in a similar manner to **Example 40,** using 1-fluoro-2-nitrobenzene in step b.

m/z ES+ $[M+H]^+$ 614.3; $^1$H NMR (400 MHz, $CDCl_3$) δ ppm 7.38 - 7.32 (m, 1H), 7.30 - 7.27 (m, 1H), 7.24 (d, *J* = 7.6 Hz, 1H), 7.16 (s, 1H), 6.91 - 6.82 (m, 1H), 6.68 (s, 1H), 6.24 - 6.18 (m, 1H), 6.05-5.99 (m, 1H), 4.64 - 4.56 (m, 1H), 4.39 - 4.31 (m, 1H), 4.30 - 4.17 (m, 2H), 4.07 - 4.02 (m, 1H), 3.90 (d, *J* = 10.4 Hz, 1H), 3.48 - 3.38 (m, 3H), 3.30 - 3.27 (m, 3H), 3.27 - 3.12 (m, 2H), 3.10 - 3.01 (m, 3H), 2.37 (s, 3H), 2.26 (s, 6H), 2.10 - 1.99 (m, 1H), 1.98 - 1.87 (m, 1H), 1.73 - 1.69 (m, 2H).

**Example 42**

**(*S*,*E*)-2-((6-(3-((1-(4-(Dimethylamino)but-2-enoyl)azetidin-3-yl)oxy)propyl)-8-fluoro-1-methyl-2-oxo-1,2,3,4,5,6-hexahydrobenzo[b][1,4]diazocin-3-yl)amino)-6-methyl-4-(trifluoromethyl)nicotinonitrile (E42)**

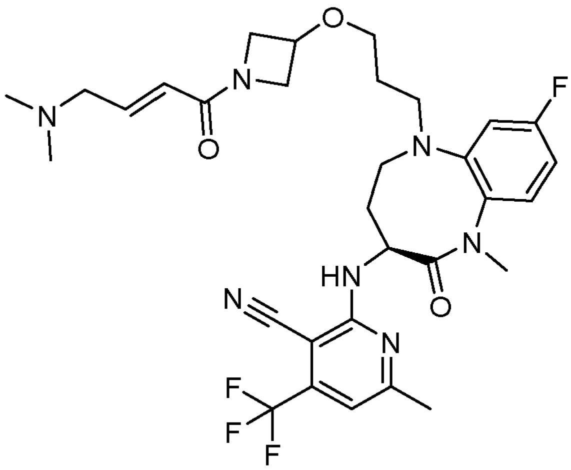

The title compound was prepared in a similar manner to **Example 40,** using 2,4-difluoro-1-nitrobenzene in step b.

m/z ES+ $[M+H]^+$ 632.5; $^1H$ NMR (400 MHz, $CDCl_3$) δ ppm 7.17 - 7.08 (m, 1H), 6.91 - 6.78 (m, 2H), 6.73 - 6.62 (m, 2H), 6.15 (t, *J* = 6.4 Hz, 1H), 6.09 (d, *J* = 15.6 Hz, 1H), 4.86 - 4.72 (m, 1H), 4.42 - 4.33 (m, 1H), 4.33 - 4.19 (m, 2H), 4.08 (dd, *J* = 3.2, 9.2 Hz, 1H), 3.97 - 3.88 (m, 1H), 3.68 - 3.55 (m, 1H), 3.49 - 3.40 (m, 2H), 3.40 - 3.27 (m, 2H), 3.27 (s, 3H), 3.20 - 3.05 (m, 3H), 2.37 (s, 3H), 2.33 (s, 6H), 2.24 - 2.10 (m, 1H), 1.90 - 1.76 (m, 3H).

**Example 43**

**(*S*,*E*)-2-((6-(3-((1-(4-(Dimethylamino)but-2-enoyl)azetidin-3-yl)oxy)propyl)-8,10-difluoro-1-methyl-2-oxo-1,2,3,4,5,6-hexahydrobenzo[*b*][1,4]diazocin-3-yl)amino)-6-methyl-4-(trifluoromethyl)nicotinonitrile (E43)**

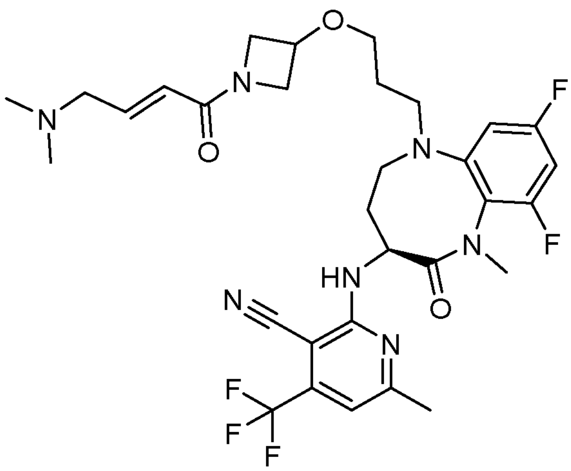

The title compound was prepared in a similar manner to **Example 40,** using 1,3,5-trifluoro-2-nitrobenzene in step b.

m/z ES+ $[M+H]^+$ 650.3; $^1H$ NMR (400 MHz, $CDCl_3$) δ ppm 6.88 - 6.81 (m, 1H), 6.73 (s, 1H), 6.58 - 6.48 (m, 1H), 6.46 - 6.30 (m, 2H), 5.92 - 5.88 (m, 1H), 4.99 - 4.87 (m, 1H), 4.50 - 4.44 (m, 1H), 4.37 - 4.23 (m, 2H), 4.19 - 4.12 (m, 1H), 4.02 - 3.91 (m, 1H), 3.85 - 3.71 (m, 1H), 3.53 - 3.29 (m, 6H), 3.21 (s, 3H), 3.20 - 3.11 (m, 1H), 2.54 (s, 6H), 2.39 (s, 3H), 2.32 - 2.24 (m, 1H), 1.94 - 1.86 (m, 2H), 1.81 - 1.73 (m, 1H).

**Example 44**

**(*S*,*E*)-2-((7-Chloro-6-(3-((1-(4-(dimethylamino)but-2-enoyl)azetidin-3-yl)oxy)propyl)-1-methyl-2-oxo-1,2,3,4,5,6-hexahydrobenzo[*b*][1,4]diazocin-3-yl)amino)-6-methyl-4-(trifluoromethyl)nicotinonitrile (E44)**

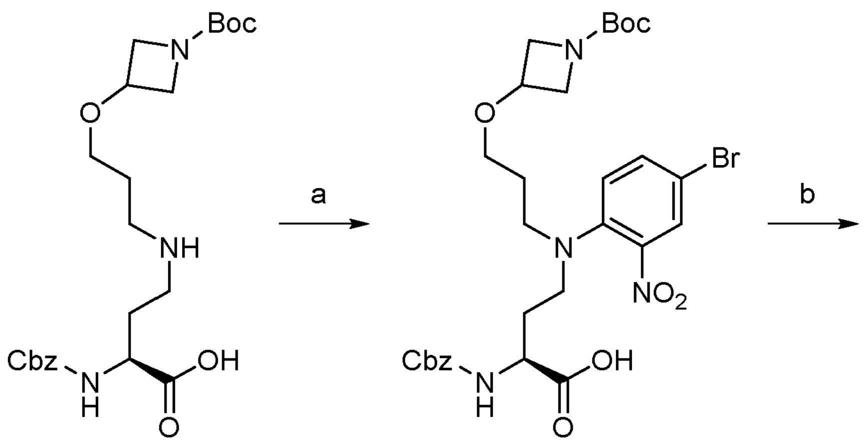
a
b

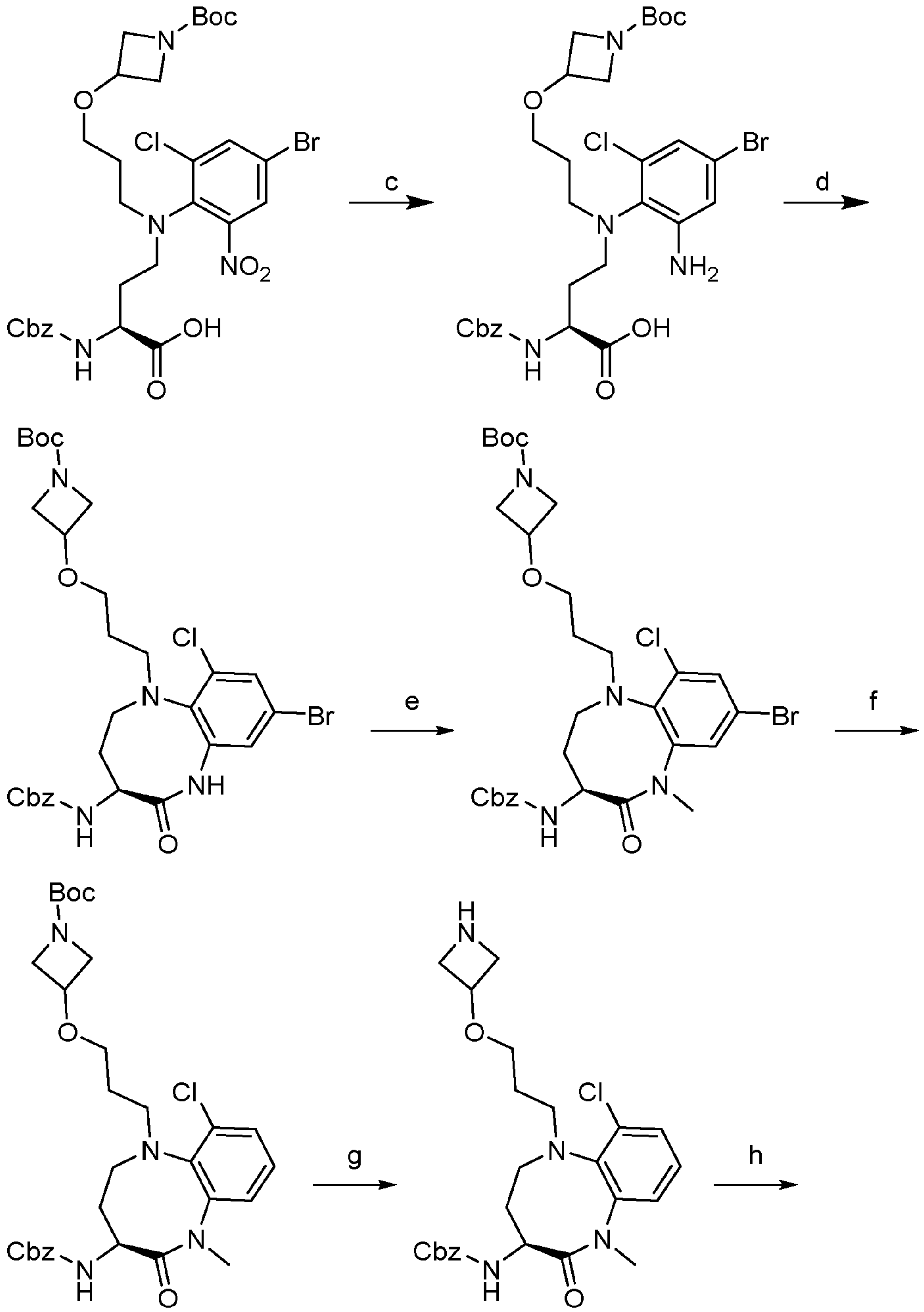
c
d
e
f
g
h

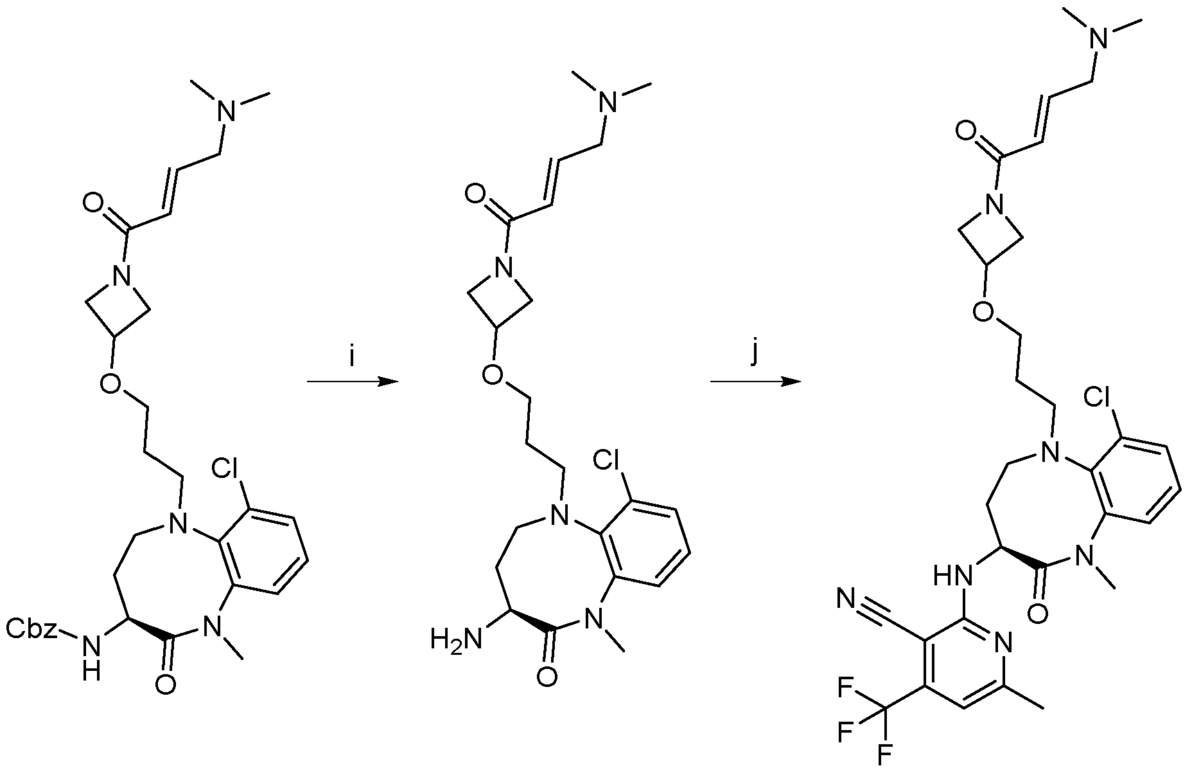


**Step** a. To a solution of (*S*)-2-(((benzyloxy)carbonyl)amino)-4-((3-((1-(*tert*-butoxycarbonyl)azetidin-3-yl)oxy) propyl)amino)butanoic acid (**Example 40,** step a; 5 g, 10.7 mmol), 4-bromo-1-fluoro-2-nitro-benzene (3.54 g, 16.1 mmol, 1.98 mL) in DMF (25 mL) was added $Cs_2CO_3$ (7.00 g, 21.5 mmol) and the mixture was stirred at 100 °C for 1 h. Upon completion, the reaction mixture was quenched with sat. aq. $NH_4Cl$ (20 mL) at rt, diluted with water (30 mL) and extracted with EtOAc (3 x 100 mL). The combined organic layers were washed with brine (3 x 50 mL), dried over $Na_2SO_4$ and evaporated. The residue was purified by reverse phase flash chromatography (water (0.1% FA)/MeCN) to give (S)-2-(((benzyloxy)carbonyl)amino)-4-((4-bromo-2-nitrophenyl)(3-((1-(tert-butoxycarbonyl)azetidin-3-yl)oxy)propyl) amino)butanoic acid (2.00 g, 28% yield) as a yellow solid.

m/z ES+ $[M+H]^+$ 667.3.

**Step b.** To a solution of (*S*)-2-(((benzyloxy)carbonyl)amino)-4-((4-bromo-2-nitrophenyl)(3-((1-(*tert*-butoxycarbonyl) azetidine-3-yl)oxy)propyl)amino)butanoic acid (2.00 g, 3.01 mmol) in acetic acid (4.2 mL) was added *N*-chlorosuccinimide (1.20 g, 9.02 mmol) and the mixture was stirred at 60 °C for 40 min. Upon completion, the reaction mixture was quenched with sat. aq. $NH_4Cl$ (20 mL) at rt, diluted with water (30 mL) and extracted with EtOAc (3 x 50 mL). The combined organic layers were washed with brine (3 x 20 mL), dried over $Na_2SO_4$ and evaporated. The residue was purified by reverse phase flash chromatography (water (0.1% FA)/MeCN) to give (*S*)-2-(((benzyloxy)carbonyl)amino)-4-((4-bromo-2-chloro-6-nitrophenyl)(3-((1-(*tert*-butoxycarbonyl)azetidin-3-yl)oxy)propyl)amino)butanoic acid (450 mg, 21% yield) as a yellow solid.

m/z ES+ $[M+H]^+$ 701.2.

**Step c.** To a solution of (*S*)-2-(((benzyloxy)carbonyl)amino)-4-((4-bromo-2-chloro-6-nitrophenyl)(3-((1-(*tert*-butoxycarbonyl)azetidin-3-yl)oxy)propyl)amino)butanoic acid (450 mg, 0.64 mmol) in EtOAc (3 mL) was added 5% Pt on carbon (12.5 mg) and the mixture was stirred at rt for 1 h under $H_2$ atmosphere (15 psi). Upon completion, the reaction mixture was filtered and evaporated to give (*S*)-4-((2-amino-4-bromo-6-chlorophenyl)(3-((1-(tert-butoxycarbonyl)azetidin-3-yl)oxy)propyl)amino)-2-(((benzyloxy)carbonyl)amino)butanoic acid (270 mg, 62% yield) as a yellow solid.

m/z ES+ $[M+H]^+$ 671.2.

**Step d.** To a solution of (*S*)-4-((2-amino-4-bromo-6-chlorophenyl)(3-((1-(*tert*-butoxycarbonyl)azetidin-3-yl)oxy) propyl)amino)-2-(((benzyloxy)carbonyl)amino)butanoic acid (270 mg, 0.40 mmol) in DCM (3 mL) was added HATU (184 mg, 0.48 mmol) and DIPEA (156 mg, 1.21 mmol, 0.21 mL) and the mixture was stirred at rt for 30 min. Upon completion, the reaction mixture was filtered and evaporated. The residue was purified by reverse phase flash chromatography (water (0.1% FA)/MeCN) to give *tert*-butyl (*S*)-3-(3-(4-(((benzyloxy)carbonyl)amino)-8-bromo-10-chloro-5-oxo-3,4,5,6-tetrahydrobenzo[b][1,4]diazocin-1(2*H*)-yl)propoxy)azetidine-1-carboxylate (180 mg, 68% yield) as a yellow solid.

m/z ES+ $[M+H]^+$ 651.3.

**Step e.** To a solution of *tert*-butyl (*S*)-3-(3-(4-(((benzyloxy)carbonyl)amino)-8-bromo-10-chloro-5-oxo-3,4,5,6-tetrahydrobenzo[*b*][1,4]diazocin-1(2*H*)-yl)propoxy)azetidine-1-carboxylate (180 mg, 0.28 mmol) in DMF (3 mL) was added $K_2CO_3$ (114 mg, 0.83 mmol) and methyl iodide (118 mg, 0.83 mmol, 0.05 mL). The mixture was stirred at rt for 1 h. Upon completion, the reaction mixture was quenched with sat. aq. $NH_4Cl$ (20 mL) at rt, and then diluted with water (30 mL) and extracted with EtOAc (3 x 30 mL). The combined organic layers were washed with brine (3 x 20 mL), dried over $Na_2SO_4$ and evaporated to give *tert*-butyl (*S*)-3-(3-(4-(((benzyloxy)carbonyl)amino)-8-bromo-10-chloro-6-methyl-5-oxo-3,4,5,6-tetrahydrobenzo[b][1,4]diazocin-1(2*H*)-yl)propoxy)azetidine-1-carboxylate (150 mg, 81% yield) as a yellow solid.

m/z ES+ $[M+H]^+$ 667.3.

**Step f.** To a solution of *tert*-butyl (*S*)-3-(3-(4-(((benzyloxy)carbonyl)amino)-8-bromo-10-chloro-6-methyl-5-oxo-3,4,5,6-tetrahydrobenzo[*b*][1,4]diazocin-1(2*H*)-yl)propoxy)azetidine-1-carboxylate (150 mg, 0.22 mmol) in 2-propanol (2 mL) was added $PtO_2$ (10.2 mg, 0.045 mmol) and the mixture was stirred at rt for 4 h under hydrogen atmosphere (15 psi). Upon completion, the reaction mixture was filtered and evaporated to give *tert*-butyl (*S*)-3-(3-(4-(((benzyloxy)carbonyl)amino)-10-chloro-6-methyl-5-oxo-3,4,5,6-tetrahydrobenzo[*b*][1,4]diazocin-1(2*H*)-yl)propoxy)azetidine-1-carboxylate (25 mg, 18% yield) as a yellow solid.

m/z ES+ $[M+H]^+$ 587.4.

**Step g.** To a solution of *tert-butyl* (*S*)-3-(3-(4-(((benzyloxy)carbonyl)amino)-10-chloro-6-methyl-5-oxo-3,4,5,6-tetrahydrobenzo[*b*][1,4]diazocin-1(2*H*)-yl)propoxy)azetidine-1-carboxylate (70 mg, 0.12 mmol) in DCM (3 mL) was added TFA (680 mg, 5.96 mmol, 0.44 mL) and the mixture was stirred at rt for 30 min. Upon completion, the reaction mixture was quenched with sat. aq. $NaHCO_3$ (100 mL) at rt, diluted with water (30 mL) and extracted with EtOAc (3 x 30 mL). The combined organic layers were washed with brine (3 x 20 mL), dried over $Na_2SO_4$ and evaporated to give benzyl (*S*)-(6-(3-(azetidin-3-yloxy)propyl)-7-chloro-1-methyl-2-oxo-1,2,3,4,5,6-hexahydrobenzo[*b*][1,4]diazocin-3-yl)carbamate (50 mg, 86% yield) as a yellow solid.

m/z ES+ $[M+H]^+$ 487.3.

**Step h.** To a solution of benzyl (*S*)-(6-(3-(azetidin-3-yloxy)propyl)-7-chloro-1-methyl-2-oxo-1,2,3,4,5,6-hexahydrobenzo[*b*][1,4]diazocin-3-yl)carbamate (40 mg, 0.082 mmol), (*E*)-4-(dimethylamino)but-2-enoic acid hydrochloride (16 mg, 0.099 mmol) in DCM (1 mL) was added HATU (37 mg, 0.099 mmol) and DIPEA (32 mg, 0.25 mmol, 43 μL). The mixture was stirred at rt for 12 h. Upon completion, the reaction mixture was filtered and evaporated. The residue was purified by reverse phase flash chromatography (water (0.1% FA)/MeCN) to give benzyl (*S*,*E*)-(7-chloro-6-(3-((1-(4-(dimethylamino)but-2-enoyl)azetidin-3-yl)oxy)propyl)-1-methyl-2-oxo-1,2,3,4,5,6-hexahydrobenzo[*b*][1,4]diazocin-3-yl)carbamate (35 mg, 71% yield) as a yellow solid.

m/z ES+ $[M+H]^+$ 598.4

**Step i.** A solution of benzyl (*S*,*E*)-(7-chloro-6-(3-((1-(4-(dimethylamino)but-2-enoyl)azetidin-3-yl)oxy)propyl)-1-methyl-2-oxo-1,2,3,4,5,6-hexahydrobenzo[*b*][1,4]diazocin-3-yl)carbamate (35 mg, 0.058 mmol) in TFA (1 mL) was stirred at rt for 12 h. Upon completion, the reaction mixture was filtered and evaporated. The residue was purified by reverse phase flash chromatography (water (0.1% FA)/MeCN) to give (*S*,*E*)-3-amino-7-chloro-6-(3-((1-(4-(dimethylamino)but-2-enoyl)azetidin-3-yl)oxy)propyl)-1-methyl-3,4,5,6-tetrahydrobenzo[b][1,4]diazocin-2(1*H*)-one (15 mg, 53% yield) as a yellow solid.

m/z ES+ $[M+H]^+$ 464.3.

**Step j.** To a solution of (*S*,*E*)-3-amino-7-chloro-6-(3-((1-(4-(dimethylamino)but-2-enoyl)azetidin-3-yl)oxy)propyl)-1-methyl-3,4,5,6-tetrahydrobenzo[*b*][1,4]diazocin-2(1*H*)-one (15 mg, 0.032 mmol) and **Intermediate 1** (11 mg, 0.048 mmol) in NMP (2 mL) was added DIPEA (12 mg, 0.097 mmol, 17 μL) and the mixture was stirred at 100 °C for 1 h. Upon completion, the reaction mixture was quenched with sat. aq. $NH_4Cl$ (20 mL) at rt, diluted with water (30 mL) and extracted with EtOAc (3 x 30 mL). The combined organic layers were washed with brine (3 x 20 mL), dried over $Na_2SO_4$ and evaporated. The residue was purified by Prep-HPLC to give the title compound (1.4 mg, 6% yield) as a brown solid.

m/z ES+ $[M+H]^+$ 648.2; $^1H$ NMR (400 MHz, $CDCl_3$) δ ppm 7.44 (d, *J* = 7.6 Hz, 1H), 7.26 - 7.17 (m, 2H), 6.85 (d, *J* = 15.2 Hz, 1H), 6.69 (s, 1H), 6.14 (d, *J* = 6.4 Hz, 1H), 6.01 (d, *J* = 15.6 Hz, 1H), 4.43 (t, *J* = 8.4 Hz, 1H), 4.33 (s, 1H), 4.29 - 4.16 (m, 2H), 4.08 - 3.94 (m, 1H), 3.92 - 3.77 (m, 1H), 3.44 (s, 2H), 3.35 (s, 4H), 3.28 - 3.15 (m, 1H), 3.06 (s, 4H), 2.38 (s, 3H), 2.25 (s, 6H), 1.74 (d, *J* = 13.6 Hz, 2H), 1.26 (s, 2H).

The examples in **Table 3** were prepared using methods similar to those described in the synthesis of **Example 40,** using the listed aldehydes in step a.

**Table 3**

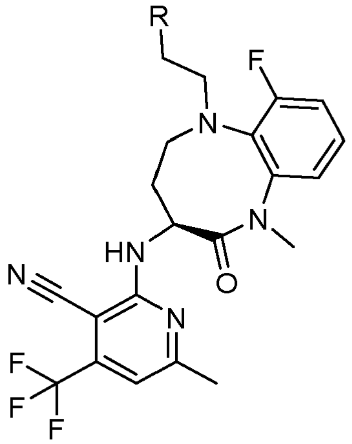

| Example Number | R | Name | Aldehyde | $^1$H NMR (400 MHz, $CDCl_3$) δ ppm | MI |
|---|---|---|---|---|---|
| **45** | 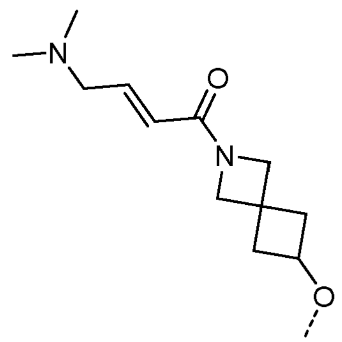 | (*S*,*E*)-2-((6-(2-((2-(4-(Dimethylamino)but-2-enoyl)-2-azaspiro [3.3]heptan-6-yl)oxy)ethyl)-7-fluoro-1-methyl-2-oxo-1,2,3,4,5,6-hexahydrobenzo[b][1,4]diazocin-3-yl)amino)-6-methyl-4-(trifluoromethyl)nicotinonitrile | **Intermediate 8b** | 7.28 - 7.25 (m, 1H), 7.18 - 7.08 (m, 2H), 6.89 - 6.78 (m, 1H), 6.69 (s, 1H), 6.43 - 6.33 (m, 1H), 6.26 - 6.20 (m, 1H), 4.53 - 4.44 (m, 1H), 4.33 - 4.24 (m, 2H), 4.03 (d, *J* = 14 Hz, 2H), 3.85 - 3.84 (m, 1H), 3.79 - 3.69 (m, 1H), 3.54 - 3.46 (m, 2H), 3.41 - 3.31 (m, 2H), 3.31 (s, 3H), 3.29 - 3.26 (m, 2H), 3.17 - 3.10 (m, 2H), 2.59 (s, 6H), 2.54 - 2.45 (m, 2H), 2.39 (s, 3H), 2.29 - 2.07 (m, 2H), 1.86 - 1.75 (m, 1H) | 658.2 |
| **46** | 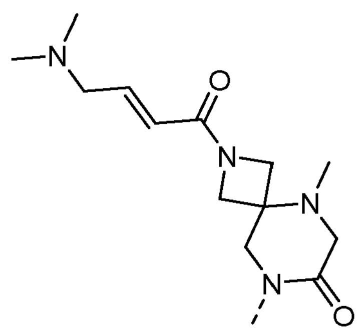 | (*S*,*E*)-2-((6-(2-(2-(4-(Dimethylamino)but-2-enoyl)-5-methyl-7-oxo-2,5,8-triazaspiro[3.5]nonan-8-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile | **Intermediate 8c** | 7.32 - 7.27 (m, 1H), 7.16 - 7.09 (m, 2H), 6.94 - 6.84 (m, 1H), 6.69 (s, 1H), 6.29 - 6.18 (m, 2H), 4.69 (s, 2H), 4.47 - 4.40 (m, 1H), 4.35 - 4.33 (m, 1H), 4.16 (d, *J* = 11 Hz, 1H), 3.94-3.90 (m, 1H), 3.74 - 3.72 (m, 1H), 3.55 (d, *J* = 5 Hz, 2H), 3.39 (d, *J* = 13 Hz, 3H), 3.34 (s, 3H), 3.31 (d, *J* = 7 Hz, 2H), 3.27 - 3.24 (m, 2H), 3.19 - 3.12 (m, 2H), 2.45 - 2.43 (m, 5H), 2.39 - 2.35 (m, 6H), 2.31-2.28 (m, 1H), 1.80 (d, *J* = 13 Hz, 1H) | 700.3 |

(continued)

| Example Number | R | Name | Aldehyde | $^1$H NMR (400 MHz, $CDCl_3$) δ ppm | MI |
|---|---|---|---|---|---|
| **47** | 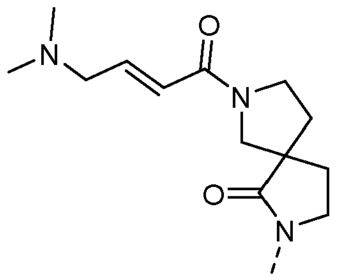 | 2-(((3*S*)-6-(2-(7-((E)-4-(Dimethylamino)but-2-enoyl)-1-oxo-2,7-diazaspiro[4.4]nonan-2-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile | **Intermediate 8d** | 7.33 - 7.27 (m, 1H), 7.14 - 7.08 (m, 1H), 7.17 - 7.08 (m, 2H), 6.97 - 6.85 (m, 1H), 6.69 (s, 1H), 6.43 - 6.28 (m, 1H), 6.23 - 6.13 (m, 1H), 4.51 - 4.40 (m, 1H), 3.93 - 3.75 (m, 2H), 3.67 - 3.60 (m, 1H), 3.59 - 3.48 (m, 2H), 3.48 - 3.39 (m, 2H), 3.36 - 3.33 (m, 4H), 3.31 - 3.24 (m, 2H), 3.21 - 3.17 (m, 2H), 3.16 - 3.13 (m, 1H), 3.12 - 3.04 (m, 1H), 2.40 (s, 2H), 2.37 (s, 3H), 2.36 - 2.34 (m, 3H), 2.29 - 2.14 (m, 2H), 2.10 - 1.99 (m, 2H), 1.95 1.82 (m, 1H), 1.78 (d, *J* = 13.6 Hz, 1H) | 685.4 |
| **48** | 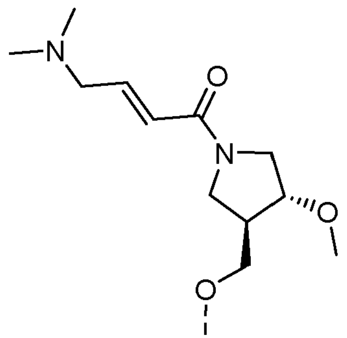 | 2-(((*S*)-6-(2-(((*rel*-3*R*,4*R*)-1-((*E*)-4-(Dimethylamino)but-2-enoyl)-4-methoxypyrrolidin-3-yl)methoxy)ethyl)-7-fluoro-1-methyl-2-oxo-1,2,3,4,5,6-hexahydrobenzo[b][1,4]diazocin-3-yl)amino)-6-methyl-4-(trifluoromethyl)nicotinonitrile | **Intermediate 8e** | 7.30 - 7.27 (m, 1H), 7.27 - 7.23 (m, 1H), 7.16 - 7.07 (m, 2H), 6.93 - 6.89 (m, 1H), 6.68 (s, 1H), 6.33 - 6.25 (m, 1H), 6.24 - 6.16 (m, 1H), 4.51 - 4.42 (m, 1H), 3.91 - 3.76 (m, 1H), 3.75 - 3.66 (m, 2H), 3.65 - 3.60 (m, 2H), 3.54 (dd, *J* = 2.8, 11.2 Hz, 1H), 3.42 - 3.37 (m, 3H), 3.36 - 3.35 (m, 1H), 3.34 - 3.32 (m, 2H), 3.31 - 3.28 (m, 3H), 3.23 - 3.17 (m, 2H), 3.13 (d, *J* = 3.2 Hz, 1H), 3.09 (d, *J* = 6.0 Hz, 2H), 2.57 - 2.41 (m, 2H), 2.37 (s, 3H), 2.26 (d, *J* = 1.6 Hz, 6H), 2.22 (d, *J* = 6.8 Hz, 1H) | 676.7 |
| **49** | | (*S*,*E*)-2-((6-(2-((1-(4-(Dimethylamino)but-2-enoyl)piperidin-4-yl)oxy)ethyl)-7-fluoro-1-methyl-2-oxo-1,2,3,4,5,6-hexahydrobenzo[*b*][1,4]diazocin- | *tert*-butyl 4-(2-oxo-ethoxy)-piperi-dine-1-car-boxylate | 7.31 - 7.24 (m, 1H), 7.17 - 7.09 (m, 2H), 6.86 - 6.75 (m, 2H), 6.70 (s, 1H), 6.22 (d, *J* = 7.2 Hz, 1H), 4.50 - 4.47 (m, 1H), 3.94 - 3.70 (m, 3H), 3.55 - 3.49 (m, 1H), 3.48 - 3.38 (m, 6H), 3.32 (s, 4H), 3.23 - 3.09 (m, 3H), 2.59 - 2.50 (m, 6H), 2.39 (s, | 646.3 |

(continued)

| Example Number | R | Name | Aldehyde | $^1H$ NMR (400 MHz, $CDCl_3$) δ ppm | MI |
|---|---|---|---|---|---|
| | 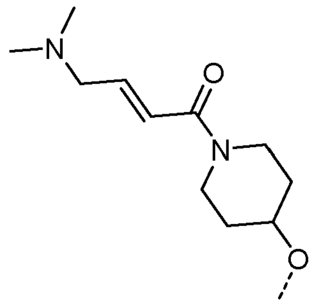 | 3-yl)amino)-6-methyl-4-(trifluoromethyl)nicotinonitrile | (CAS Number 919360-34-2) | 3H), 2.34 - 2.17 (m, 2H), 1.86 - 1.75 (m, 3H) | |

**Example 50**

**(*S*,*E*)-2-((6-(2-((1-(4-(Dimethylamino)but-2-enoyl)azetidin-3-yl)oxy)ethyl)-1-methyl-2-oxo-1,2,3,4,5,6-hexahydrobenzo[*b*][1,4]diazocin-3-yl)amino)-6-methyl-4-(trifluoromethyl)nicotinonitrile (E50)**

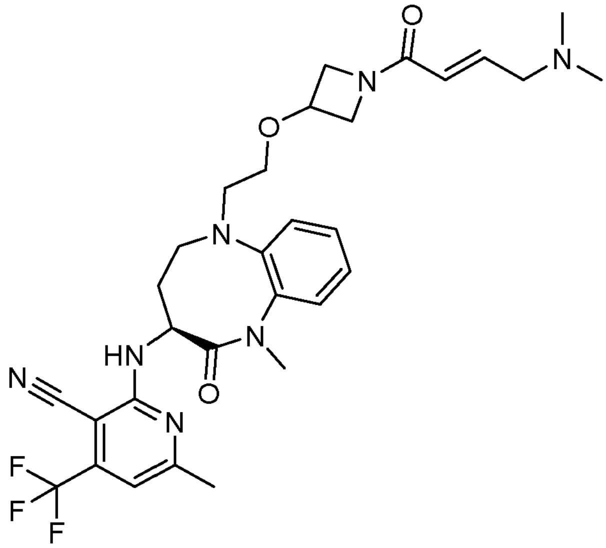

The title compound was prepared in a similar manner to **Example 41,** using **Intermediate 8f** in step a.

m/z ES+ $[M+H]^+$ 600.4; $^1H$ NMR (400 MHz, $CD_3OD$) δ ppm 7.39 - 7.31 (m, 3H), 7.19 - 7.13 (m, 1H), 6.87 (s, 1H), 6.80 - 6.73 (m, 1H), 6.18-6.13 (m, 1H), 4.61 - 4.59 (m, 1H), 4.45 - 4.39 (m, 1H), 4.36 - 4.30 (m, 1H), 4.21 - 4.15 (m, 1H), 4.10 - 4.03 (m, 1H), 3.79 (dd, *J* = 4.0, 11.2 Hz, 1H), 3.61 - 3.52 (m, 1H), 3.52 - 3.46 (m, 2H), 3.43 - 3.37 (m, 2H), 3.26 (s, 3H), 3.23 - 3.16 (m, 1H), 3.14 (d, *J* = 6.4 Hz, 2H), 2.40 (s, 3H), 2.26 (s, 6H), 2.10 - 2.01 (m, 2H).

**Example 51**

**(*S*)-2-((6-(2-((1-Acryloylazetidin-3-yl)oxy)ethyl)-1-methyl-2-oxo-1,2,3,4,5,6-hexahydrobenzo[*b*][1,4]diazocin-3-yl)amino)-6-methyl-4-(trifluoromethyl)nicotinonitrile (E51)**

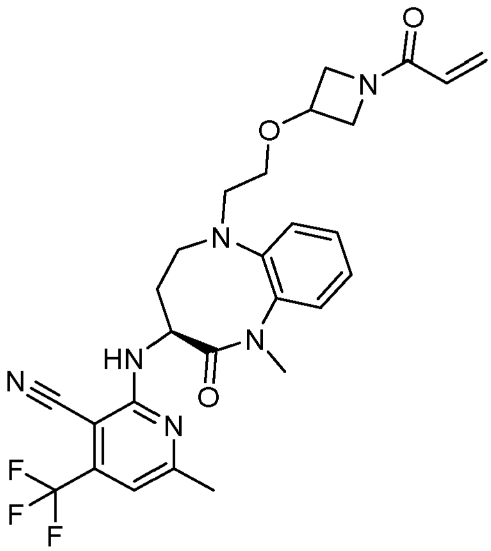

The title compound was prepared in a similar manner to **Example 50,** using acryloyl chloride in the final step, as

follows:

To a solution of (*S*)-2-((6-(2-(azetidin-3-yloxy)ethyl)-1-methyl-2-oxo-1,2,3,4,5,6-hexahydrobenzo[*b*][1,4]diazocin-3-yl)amino)-6-methyl-4-(trifluoromethyl)nicotinonitrile (20 mg, 0.04 mmol) in DCM (0.5 mL) was added DIPEA (11 mg, 0.08 mmol) followed by the dropwise addition of acryloyl chloride (6 mg, 0.06 mmol) at 0 °C. The mixture was stirred at 0 °C for 30 min. Upon completion, the reaction was evaporated. The residue was purified by Prep-HPLC to give the title compound (12 mg, 52% yield) as a white solid.

m/z ES+ $[M+H]^+$ 543.2; $^1H$ NMR (400 MHz, $CDCl_3$) δ ppm 7.35 - 7.30 (m, 1H), 7.23-7.20 (m, 2H), 7.14 - 7.09 (m, 1H), 6.68 (s, 1H), 6.36 - 6.30 (m, 1H), 6.23 - 6.14 (m, 2H), 5.69 (d, *J* = 10.4 Hz, 1H), 4.69 - 4.62 (m, 1H), 4.38 - 4.32 (m, 1H), 4.28 - 4.27 (m, 1H), 4.24 - 4.18 (m, 1H), 4.05 (d, *J* = 9.2 Hz, 1H), 3.90 - 3.87 (m, 1H), 3.61 - 3.52 (m, 1H), 3.51 - 3.43 (m, 2H), 3.42 - 3.31 (m, 2H), 3.29 (s, 3H), 3.20 - 3.12 (m, 1H), 2.37 (s, 3H), 2.03-1.98 (m, 2H).

**Example 52**

**(*S*)-2-((6-((2-Acryloyl-2-azaspiro[3.3]heptan-6-yl)methyl)-1-methyl-2-oxo-1,2,3,4,5,6-hexahydrobenzo[*b*][1,4]diazocin-3-yl)amino)-6-methyl-4-(trifluoromethyl)nicotinonitrile (E52)**

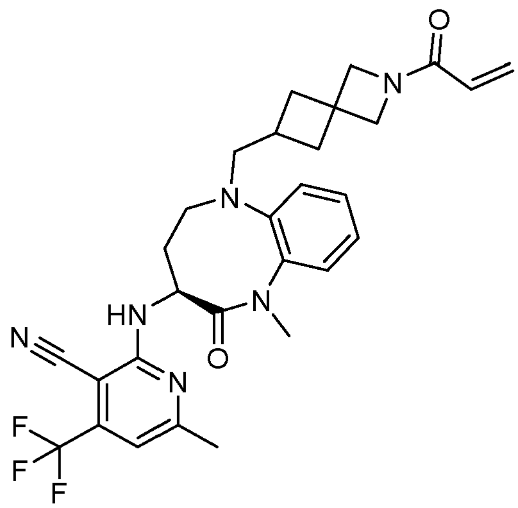

The title compound was prepared in a similar manner to **Example 51,** using *tert*-butyl 6-formyl-2-azaspiro[3.3]heptane-2-carboxylate (CAS Number 1440960-67-7) in step a.

m/z ES+ $[M+H]^+$ 553.2; $^1H$ NMR (400 MHz, DMSO-$d_6$) δ ppm 7.33 - 7.27 (m, 2H), 7.26 - 7.20 (m, 2H), 7.12 - 7.05 (m, 1H), 6.98 (s, 1H), 6.29 - 6.18 (m, 1H), 6.08 - 6.00 (m, 1H), 5.61 (d, *J* = 10.0 Hz, 1H), 4.45 - 4.33 (m, 1 H), 4.17 (s, 1H), 4.08 - 4.03 (m, 1H), 3.88 (s, 1H), 3.77 (d, *J* = 3.6 Hz, 1H), 3.41 (d, *J* = 9.6 Hz, 1H), 3.25 - 3.16 (m, 1H), 3.15 (s, 3H), 3.10 - 2.94 (m, 2H), 2.36 (s, 3H), 2.24 - 2.08 (m, 3H), 2.02 - 1.93 (m, 1H), 1.92 - 1.84 (m, 2H), 1.82 - 1.75 (m, 1H)

**Example 53**

**(*S*)-2-((6-(3-((1-Acryloylazetidin-3-yl)oxy)propyl)-1-methyl-2-oxo-1,2,3,4,5,6-hexahydrobenzo[*b*][1,4]diazocin-3-yl)amino)-6-methyl-4-(trifluoromethyl)nicotinonitrile (E53)**

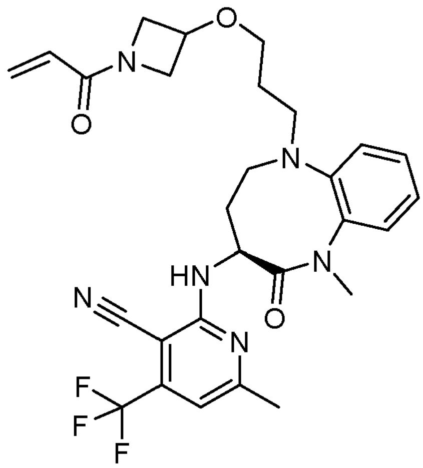

The title compound was prepared in a similar manner to **Example 51,** using **Intermediate 8a** in step a.

m/z ES+ $[M+H]^+$ 557.2; $^1$H NMR (400 MHz, $CDCl_3$) δ ppm 7.36 (d, *J* = 7.6 Hz, 1H), 7.32 - 7.02 (m, 1H), 7.25 (s, 1H), 7.21 - 7.14 (m, 1H), 6.70 (s, 1H), 6.40 - 6.31 (m, 1H), 6.28 - 6.15 (m, 2H), 5.74 - 5.66 (m, 1H), 4.65 - 4.58 (m, 1H), 4.36 (m, 1H), 4.40 - 4.34 (m, 2H), 4.09 - 4.05 (m, 1H), 3.95 - 3.91 (m, 1H), 3.73 - 3.41 (m, 3H), 3.30 (s, 3H), 3.27 - 3.00 (m, 3H), 2.39 (s, 3H), 2.1 - 2.0 (m, 1H), 1.98 - 1.97 (m, 1H), 1.76 - 1.67 (m, 2H).

**Example 54**

**2-(((*S*)-7-Fluoro-1-methyl-6-(3-((1-((*E*)-3-((*S*)-1-methylpyrrolidin-2-yl)acryloyl)azetidin-3-yl)oxy)propyl)-2-oxo-1,2,3,4,5,6-hexahydrobenzo[b][1,4]diazocin-3-yl)amino)-6-methyl-4-(trifluoromethyl)nicotinonitrile (E54)**

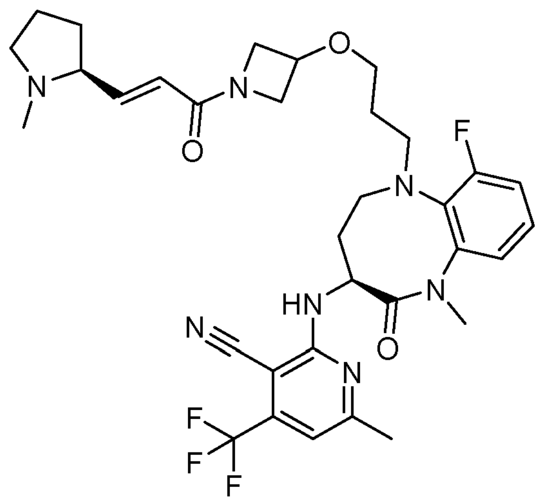

The title compound was prepared in a similar manner to **Example 40,** using **Intermediate 9a** in step i.

m/z ES+ $[M+H]^+$ 658.2; $^1$H NMR (400 MHz, $CDCl_3$) δ ppm 7.33 - 7.27 (m, 1H), 7.18 - 7.07 (m, 2H), 6.82 - 6.72 (m, 1H), 6.68 (s, 1H), 6.27 - 6.13 (m, 2H), 4.50 - 4.34 (m, 2H), 4.32 - 4.16 (m, 2H), 4.15 - 4.02 (m, 1H), 3.96 - 3.82 (m, 1H), 3.50 - 3.30 (m, 4H), 3.29 (s, 3H), 3.23 - 3.04 (m, 3H), 2.50 - 2.43 (m, 3H), 2.37 (s, 3H), 2.31 - 2.10 (m, 4H), 2.09 - 1.98 (m, 2H), 1.97 - 1.85 (m, 2H), 1.66 - 1.53 (m, 2H).

**Example 55**

**2-(((*S*)-7-Fluoro-1-methyl-6-(3-((1-((*E*)-3-((*R*)-1-methylpyrrolidin-2-yl)acryloyl)azetidin-3-yl)oxy)propyl)-2-oxo-1,2,3,4,5,6-hexahydrobenzo[*b*][1,4]diazocin-3-yl)amino)-6-methyl-4-(trifluoromethyl)nicotinonitrile (E55)**

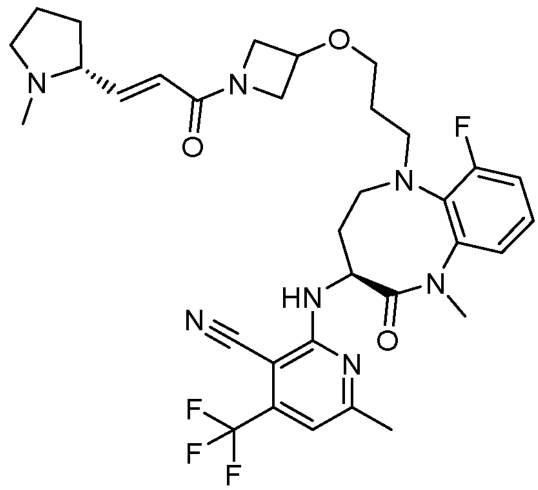

The title compound was prepared in a similar manner to **Example 40,** using **Intermediate 9b** in step i.

m/z ES+ $[M+H]^+$ 658.4; $^1H$ NMR (400 MHz, $CDCl_3$) $\delta$ ppm 7.32 - 7.27 (m, 1H), 7.17 - 7.07 (m, 2H), 6.81 - 6.71 (m, 1H), 6.68 (s, 1H), 6.22 (t, $J$ = 7.6 Hz, 1H), 6.13 (dd, $J$ = 4.4, 15.6 Hz, 1H), 4.49 - 4.42 (m, 1H), 4.42 - 4.35 (m, 1H), 4.31 - 4.24 (m, 1H), 4.24 - 4.17 (m, 1H), 4.12 - 4.02 (m, 1H), 3.94 - 3.84 (m, 1H), 3.47 - 3.31 (m, 3H), 3.29 (s, 3H), 3.17 - 3.02 (m, 4H), 2.43 - 2.34 (m, 6H), 2.32 - 2.19 (m, 2H), 2.16 - 2.06 (m, 2H), 2.03 - 1.93 (m, 2H), 1.91 - 1.81 (m, 2H), 1.65 - 1.52 (m, 2H).

**Example 56**

**(*S*,*E*)-2-((7-Fluoro-6-(3-((1-(4-((2-methoxyethyl)(methyl)amino)but-2-enoyl)azetidin-3-yl)oxy)propyl)-1-methyl-2-oxo-1,2,3,4,5,6-hexahydrobenzo[*b*][1,4]diazocin-3-yl)amino)-6-methyl-4-(trifluoromethyl)nicotinonitrile (E56)**

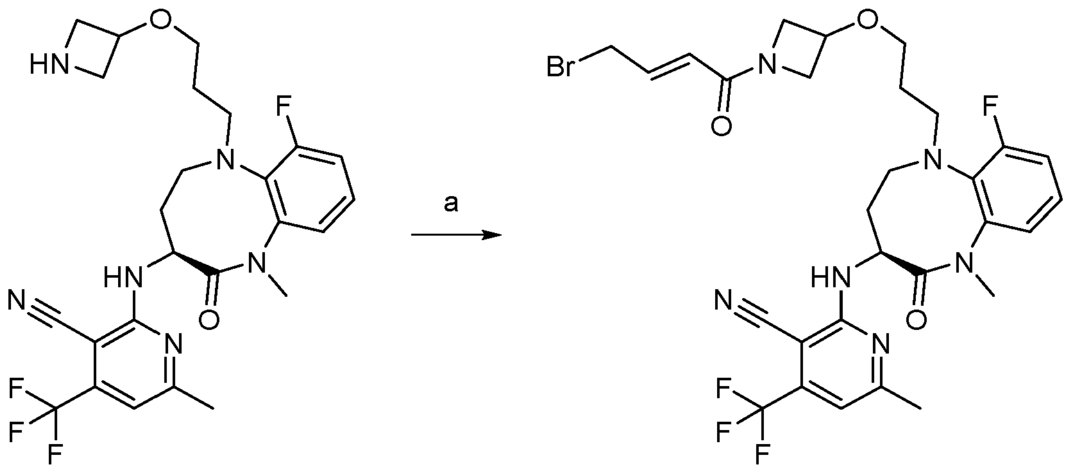

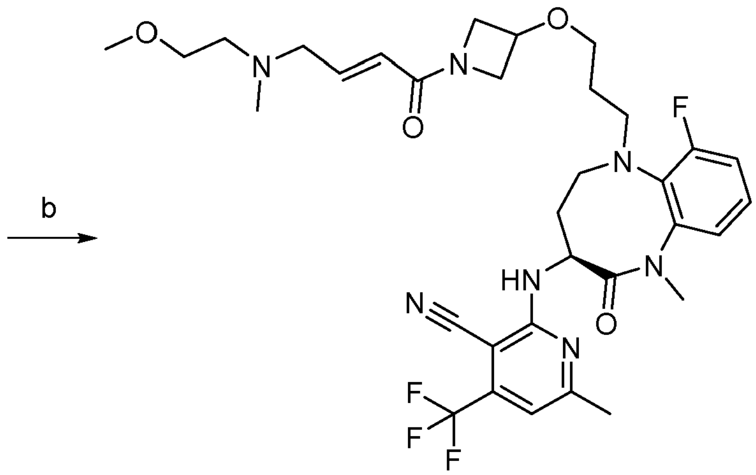


**Step** a. To a solution of (S)-2-((6-(3-(azetidin-3-yloxy)propyl)-7-fluoro-1-methyl-2-oxo-1,2,3,4,5,6-hexahydrobenzo[b][1,4]diazocin-3-yl)amino)-6-methyl-4-(trifluoromethyl)nicotinonitrile **(Example 40,** steps a-h; 100 mg, 0.19 mmol) in DCM (1 mL) was added TEA (58 mg, 0.58 mmol). The mixture was cooled to 0 °C and a solution of **Intermediate 10** (46 mg, 0.25 mmol) in DCM (0.5 mL) was added at 0 °C. The mixture was stirred at 0 °C for 30 min. Upon completion, the reaction mixture was used directly in the following step.

m/z ES+ $[M+H]^+$ 669.1.

**Step b.** To a solution of crude (*S*,*E*)-2-((6-(3-((1-(4-bromobut-2-enoyl)azetidin-3-yl)oxy)propyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile in DCM (1.5 mL) was added 2-methoxy-*N*-methylethanamine (27 mg, 0.30 mmol). The mixture was stirred at rt for 2 h. Upon completion, the reaction mixture was diluted with water (5 mL), extracted with DCM (3 x 6 mL). The combined organic layers were washed with brine (2 x 10 mL), dried over $Na_2SO_4$ and evaporated. The residue was purified by Prep-HPLC to give the title compound (10 mg, 8% yield) as a brown gum.

m/z ES+ $[M+H]^+$ 676.4; $^1H$ NMR (400 MHz, $CDCl_3$) δ ppm 7.31 - 7.25 (s, 1H), 7.16 - 7.09 (m, 2H), 6.90-6.83 (m, 1H), 6.69 (s, 1H), 6.19 (d, *J* = 7.2 Hz, 1H), 6.09-6.04 (m, 1H), 4.50 - 4.42 (m, 1H), 4.38 - 4.31 (m, 1H), 4.30 - 4.24 (m, 1H), 4.22 - 4.19 (m, 1H), 4.07 - 4.00 (m, 1H), 3.91 - 3.83 (m, 1H), 3.50 - 3.48 (m, 2H), 3.41 - 3.40 (m, 2H), 3.37 - 3.32 (m, 4H), 3.30 (s, 3H), 3.24 (s, 2H), 3.16 - 3.06 (m, 3H), 2.62 - 2.60 (m, 2H), 2.38 (s, 3H), 2.32 (s, 3H), 2.26 - 2.23 (m, 1H), 1.80 - 1.79 (m, 1H), 1.75 - 1.70 (m, 2H).

**Example 57**

**(*S*,*E*)-2-((7-Fluoro-1-methyl-6-(3-((1-(4-(methyl(prop-2-yn-1-yl)amino)but-2-enoyl)azetidin-3-yl)oxy)propyl)-2-oxo-1,2,3,4,5,6-hexahydrobenzo[*b*][1,4]diazocin-3-yl)amino)-6-methyl-4-(trifluoromethyl)nicotinonitrile (E57)**

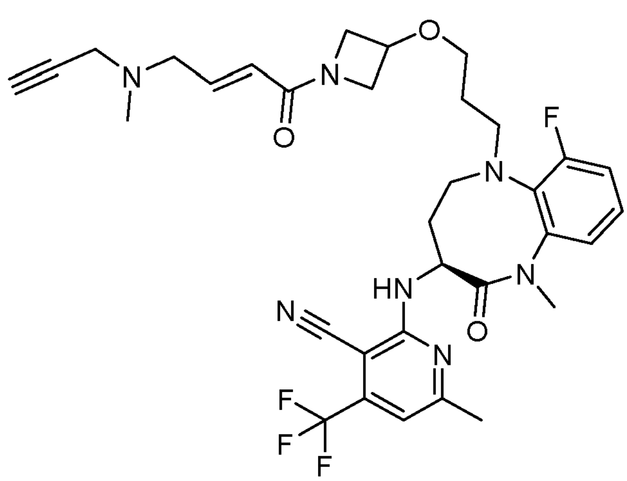

The title compound was prepared in a similar manner to **Example 56,** using *N*-methylprop-2-yn-1-amine in step

b.

m/z ES+ $[M+H]^+$ 656.3; $^1H$ NMR (400 MHz, $CDCl_3$) δ ppm 7.32 - 7.27 (m, 1H), 7.16 - 7.09 (m, 2H), 6.86 - 6.79 (m, 1H), 6.69 (s, 1H), 6.18 (d, *J* = 7.2 Hz, 1H), 6.08 - 6.04 (m, 1H), 4.46 - 4.45 (m, 1H), 4.39 - 4.30 (m, 1H), 4.30 - 4.24 (m, 1H), 4.23 - 4.17 (m, 1H), 4.10 - 4.02 (m, 1H), 3.91 - 3.84 (m, 1H), 3.41 (t, *J* = 6.4 Hz, 2H), 3.35 (s, 2H), 3.30 (s, 4H), 3.24 - 3.20 (m, 2H), 3.17 - 3.07 (m, 3H), 2.37 (s, 3H), 2.33 (d, *J* = 2.4 Hz, 3H), 2.31 - 2.20 (m, 2H), 1.80-1.78 (m, 1H), 1.60 (t, *J* = 6.4 Hz, 2H).

**Examples 58A and 58B**

**2-(((*S*)-6-(((*S*/*R*)-4-(1-Acryloylazetidin-3-yl)-1-methyl-6-oxopiperazin-2-yl)methyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile (E58A)** and

**2-(((*S*)-6-(((*R*/*S*)-4-(1-Acryloylazetidin-3-yl)-1-methyl-6-oxopiperazin-2-yl)methyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile (E58B)**

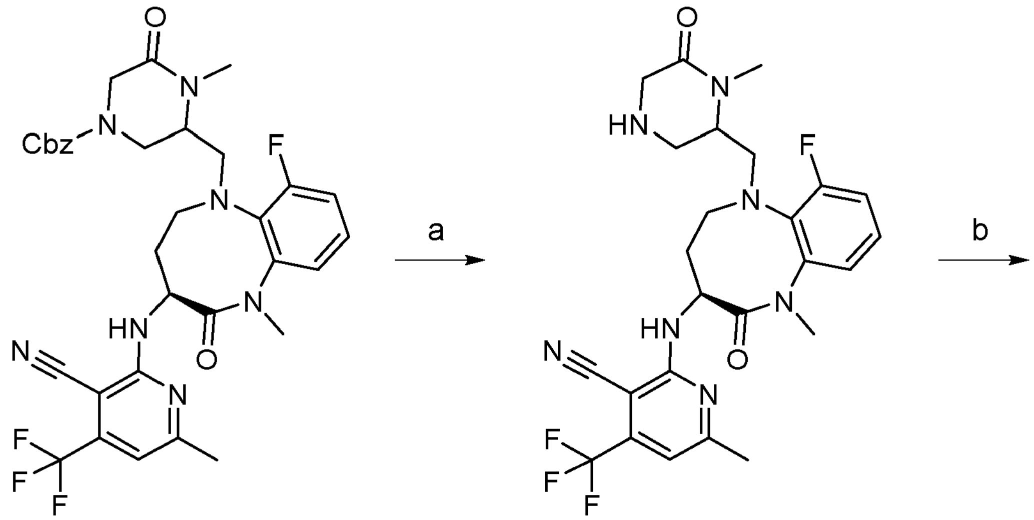

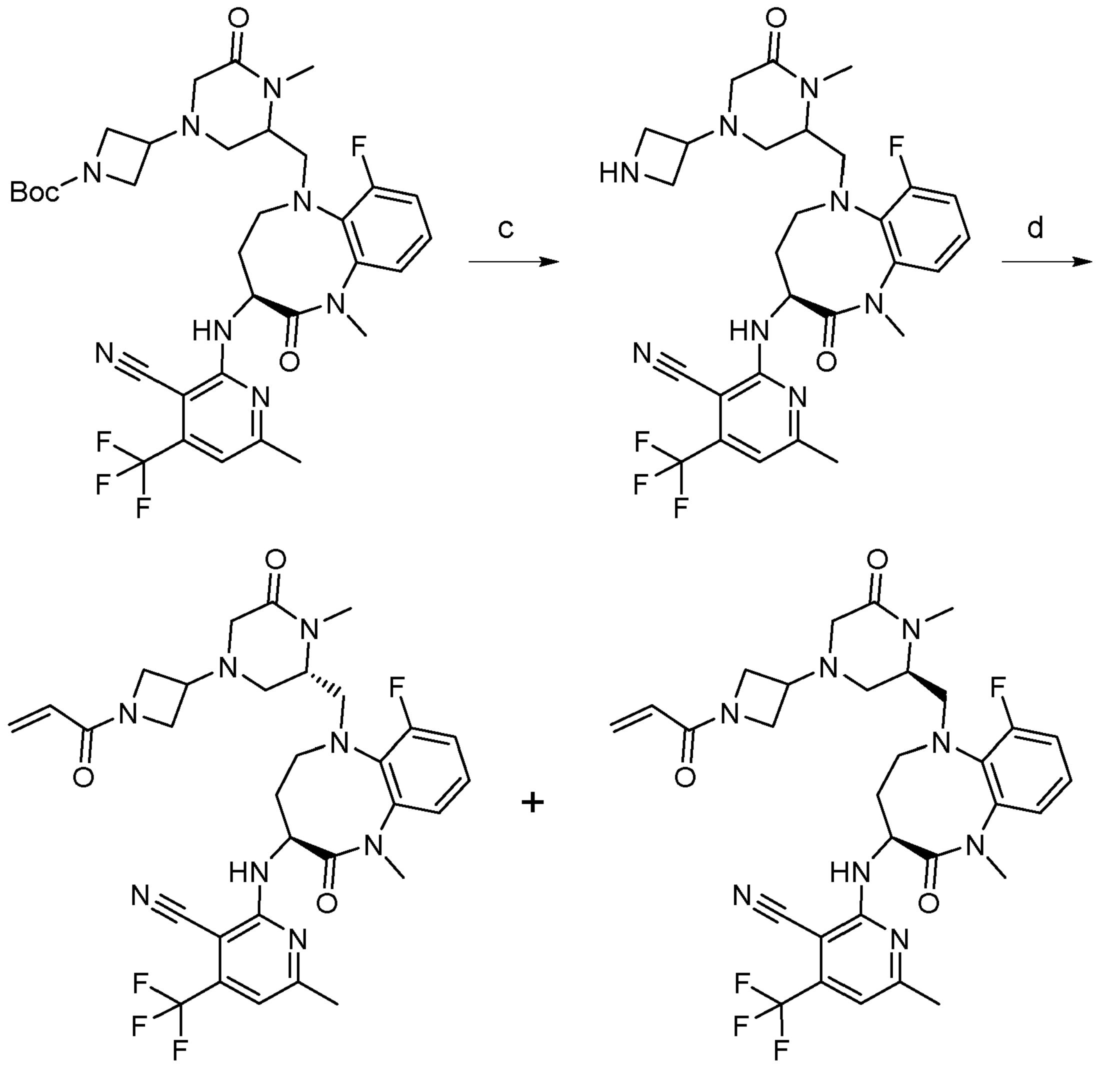


**Step** a. A solution of benzyl 3-(((*S*)-4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)methyl)-4-methyl-5-oxopiperazine-1-carboxylate (prepared in a similar manner to **Example 40,** steps a-g, using **Intermediate 8g** in step a; 180 mg, 0.27 mmol) in TFA (4.62 g, 40.5 mmol) was stirred at 40 °C for 16 h. Upon completion, the reaction mixture was evaporated. The residue was diluted with EtOAc (10mL) and MeOH (10 mL) and treated with 1 M aq. $K_2CO_3$ solution (10 mL). The mixture was extracted with EtOAc (30 mL) and washed with water (30 mL).

The organic layer was washed with brine (30 mL), dried over $Na_2SO_4$ and evaporated to give 2-(((3S)-7-fluoro-1-methyl-6-((1-methyl-6-oxopiperazin-2-yl)methyl)-2-oxo-1,2,3,4,5,6-hexahydrobenzo[b][1,4]diazocin-3-yl)amino)-6-methyl-4-(trifluoromethyl)nicotinonitrile (0.13 g, 90% yield) as a yellow solid.

m/z ES+ $[M+H]^+$ 534.4.

**Step b.** A mixture of 2-(((3S)-7-fluoro-1-methyl-6-((1-methyl-6-oxopiperazin-2-yl)methyl)-2-oxo-1,2,3,4,5,6-hexahydrobenzo[b][1,4]diazocin-3-yl)amino)-6-methyl-4-(trifluoromethyl)nicotinonitrile (120 mg, 0.22 mmol), *tert*-butyl 3-oxoazetidine-1-carboxylate (77 mg, 0.45 mmol), 4 Å molecular sieves (10 mg) and acetic acid (1.3 mg, 0.022 mmol) in DCE (2 mL) was stirred at rt for 30 min. The reaction mixture was cooled to 0 °C and treated with $NaBH_3CN$ (28 mg, 0.45 mmol). The reaction mixture was stirred at rt for 30 min. Upon completion, the reaction mixture was filtered and evaporated. The residue was diluted with water (30 mL) and extracted with EtOAc (30 mL). The organic layer was dried over $Na_2SO_4$ and evaporated. The residue was purified by column chromatography (EtOAc) to give *tert*-butyl 3-(3-(((*S*)-4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)methyl)-4-methyl-5-oxopiperazin-1-yl)azetidine-1-carboxylate (0.1 g, 65% yield) as a yellow oil.

m/z ES+ $[M+H]^+$ 689.6.

**Step** c. To a solution of *tert*-butyl 3-(3-(((*S*)-4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)methyl)-4-methyl-5-oxopiperazin-1-yl)azetidine-1-carboxylate (100 mg, 0.14 mmol) in DCM (3 mL) was added TFA (1.54 g, 13.5 mmol). The reaction mixture was

stirred at rt for 15 min. Upon completion, the reaction mixture was evaporated. The residue was diluted with MeOH (3 mL) and EtOAc (30 mL) and washed with 1 M aq. $K_2CO_3$ (30 mL). The organic layer was washed with brine (30 mL), dried over $Na_2SO_4$ and evaporated to give 2-(((3S)-6-((4-(azetidin-3-yl)-1-methyl-6-oxopiperazin-2-yl)methyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile (0.05 g, 59% yield) as a yellow oil, which was used in the next step without purification.

m/z ES+ $[M+H]^+$ 589.5.

**Step d.** To a solution of 2-(((3S)-6-((4-(azetidin-3-yl)-1-methyl-6-oxopiperazin-2-yl)methyl)-7-fluoro-1-methyl-2-oxo-1,2,3,4,5,6-hexahydrobenzo[b][1,4]diazocin-3-yl)amino)-6-methyl-4-(trifluoromethyl)nicotinonitrile (50 mg, 0.085 mmol) and TEA (17 mg, 0.17 mmol) in DCM (2 mL) was added acryloyl chloride (11 mg, 0.13 mmol). The reaction mixture was stirred at rt for 10 min. Upon completion, the reaction mixture was diluted with DCM (20 mL) and water (20 mL). The organic layer was separated, washed with brine (20 mL), dried over $Na_2SO_4$ and evaporated. The residue was purified by Prep-TLC (EtOAc) and further purified by Prep-HPLC to give two diastereoisomers, the absolute configuration was unknown.

**Example 58A** (3.5 mg, 6% yield) as a white solid.

m/z ES+ $[M+H]^+$ 643.3; $^1H$ NMR (400 MHz, $CDCl_3$) δ ppm 7.39 - 7.29 (m, 1H), 7.23 - 7.10 (m, 2H), 6.71 (s, 1H), 6.34 (d, *J* = 17.2 Hz, 1H), 6.26 - 6.09 (m, 2H), 5.74 - 5.65 (m, 1H), 4.49 - 4.39 (m, 1H), 4.30 - 4.16 (m, 1H), 4.14 -3.65 (m, 3H), 3.41 - 3.36 (m, 3H), 3.36 - 3.31 (m, 2H), 3.27 - 3.20 (m, 2H), 3.19 - 3.12 (m, 2H), 2.99 - 2.92 (m, 3H), 2.90 - 2.69 (m, 3H), 2.39 (s, 3H), 2.35 - 2.17 (m, 2H), 1.83 (d, *J* = 13.6 Hz, 1H).

**Example 58B** (6.2 mg, 11% yield) as a yellow gum.

m/z ES+ $[M+H]^+$ 643.3; $^1H$ NMR (400 MHz, $CDCl_3$) δ ppm 7.35 - 7.23 (m, 1H), 7.15 - 7.04 (m, 2H), 6.63 (d, *J* = 6.0 Hz, 1H), 6.33 - 6.07 (m, 3H), 5.72 - 5.58 (m, 1H), 4.45 - 4.31 (m, 1H), 4.24 - 4.07 (m, 1H), 4.06 - 3.92 (m, 1H), 3.91 - 3.56 (m, 2H), 3.44 - 3.24 (m, 3H), 3.24 (s, 3H), 3.18 - 3.06 (m, 3H), 2.95 - 2.84 (m, 2H), 2.82 - 2.77 (m, 3H), 2.72 - 2.57 (m, 1H), 2.30 (d, *J* = 2.8 Hz, 3H), 2.28 - 2.11 (m, 2H), 1.91 - 1.80 (m, 1H).

**Example 59**

**2-(((*S*)-6-(((*S*)-4-(1-((*E*)-4-(Dimethylamino)but-2-enoyl)azetidin-3-yl)morpholin-2-yl)methyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile (E59)**

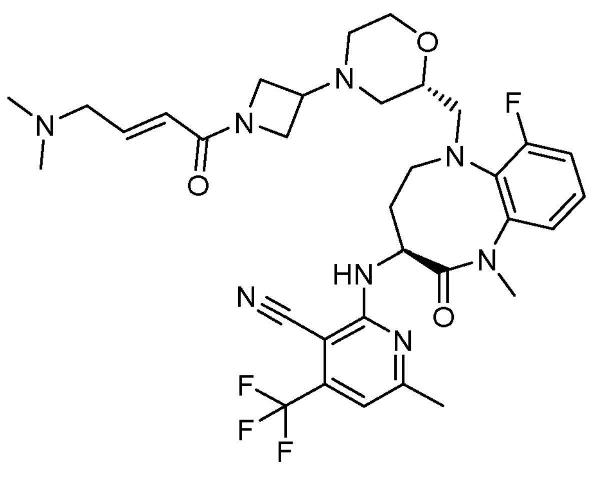

The title compound was prepared in a similar manner to **Example 58A/58B,** starting from **Intermediate 8h.**

m/z ES+ $[M+H]^+$ 673.2; $^1H$ NMR (400 MHz, $CDCl_3$) δ ppm 7.27 (s, 1H), 7.17 - 7.05 (m, 2H), 6.87 - 6.82 (m, 1H), 6.68 (s, 1H), 6.28 - 6.20 (m, 1H), 6.19 - 6.07 (m, 1H), 4.45 - 4.42 (m, 1H), 4.32 - 4.04 (m, 3H), 3.99 - 3.80 (m, 2H), 3.58 - 3.55 (m, 1H), 3.49 - 3.33 (m, 2H), 3.30 (d, *J* = 2.8 Hz, 3H), 3.18 - 3.16 (m, 4H), 3.14 - 3.01 (m, 2H), 2.81 - 2.52 (m, 2H), 2.52 - 2.45 (m, 1H), 2.37 - 2.32 (m, 8H), 2.17 - 2.01 (m, 1H), 1.86 (t, *J* = 10.4 Hz, 1H), 2.00 - 1.82 (m, 1H),1.75 (d, *J* = 12.8 Hz, 1H).

**Example 60**

**2-(((*S*)-6-(((*R*)-4-(1-((*E*)-4-(Dimethylamino)but-2-enoyl)azetidin-3-yl)morpholin-2-yl)methyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile (E60)**

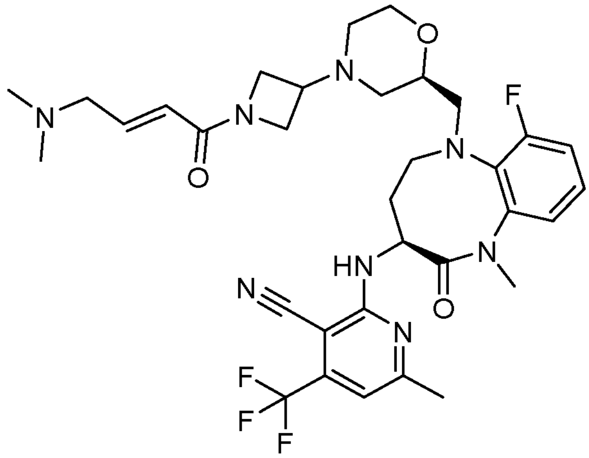

The title compound was prepared in a similar manner to **Example 59,** starting from

**Intermediate 8i.**

m/z ES+ $[M+H]^+$ 673.3; $^1$H NMR (400 MHz, $CDCl_3$) δ ppm 7.36 - 7.29 (m, 1H), 7.19 - 7.09 (m, 2H), 6.93 - 6.81 (m, 1H), 6.73 - 6.68 (m, 1H), 6.29 - 6.12 (m, 2H), 4.51 - 4.41 (m, 1H), 4.37 - 4.19 (m, 2H), 4.13 - 3.87 (m, 3H), 3.76 - 3.42 (m, 3H), 3.41 - 3.32 (m, 2H), 3.29 (s, 4H), 3.23 - 3.10 (m, 2H), 2.98 - 2.75 (m, 2H), 2.67 (d, *J* = 10.8 Hz, 1H), 2.59 (d, *J* = 11.2 Hz, 1H), 2.46 - 2.33 (m, 9H), 2.32 - 2.14 (m, 2H), 2.10 - 1.99 (m, 1H).

**Example 61**

**2-(((*S*)-6-(2-((*R*)-1-Acryloyltetrahydro-1'*H*-spiro[azetidine-3,6'-pyrazino[2,1-*c*][1,4]oxazin]-8'(7'*H*)-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile (E61)**

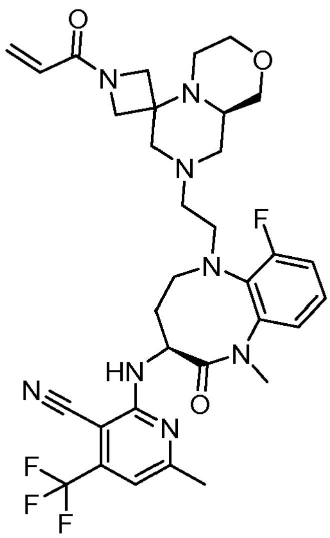

The title compound was prepared in a similar manner to **Example** 6, using **Intermediate 6l** in step a.
m/z ES+ $[M+H]^+$ 671.3; $^1$H NMR (400 MHz, $CDCl_3$) δ ppm 7.26 - 7.22 (m, 1H), 7.10 -7.00 (m, 2H), 6.62 (s, 1H),

6.35 - 6.25 (m, 1H), 6.23 - 6.13 (m, 1H), 6.09 (d, *J* = 7.2 Hz, 1H), 5.70 - 5.60 (m, 1H), 4.44 - 4.03 (m, 3H), 3.89 - 3.81 (m, 1H), 3.78 - 3.47 (m, 4H), 3.35 - 3.21 (m, 4H), 3.19 - 3.01 (m, 4H), 2.85 - 2.78 (m, 2H), 2.60 - 2.39 (m, 3H), 2.30 (s, 4H), 2.20 - 2.16 (m, 3H), 1.75 - 1.70 (m, 2H).

**Example 62**

**2-(((*S*)-6-(2-((*S*)-1-Acryloyltetrahydro-1'*H*-spiro[azetidine-3,6'-pyrazino[2,1-*c*][1,4]oxazin]-8'(7'*H*)-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile (E62)**

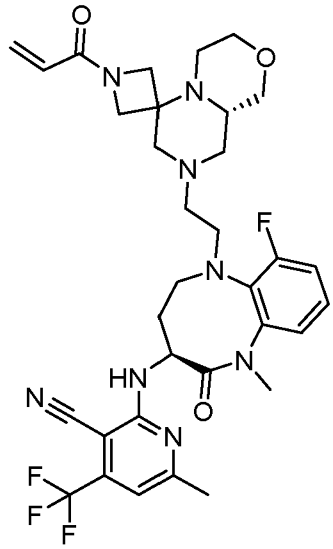

The title compound was prepared in a similar manner to **Example 6,** using **Intermediate 6m** in step a.

m/z ES+ $[M+H]^+$ 671.3; $^1H$ NMR (400 MHz, $CDCl_3$) δ ppm 7.33 - 7.27 (m, 1H), 7.18 - 7.08 (m, 2H), 6.69 (s, 1H), 6.41 - 6.20 (m, 2H), 6.20 - 6.12 (m, 1H), 5.77 - 5.64 (m, 1H), 4.50 - 4.41 (m, 1H), 4.39 - 4.11 (m, 2H), 3.97 - 3.75 (m, 2H), 3.72 - 3.58 (m, 3H), 3.38 - 3.32 (m, 1H), 3.32 - 3.27 (m, 3H), 3.25 - 3.11 (m, 4H), 3.05 - 2.83 (m, 2H), 2.66 - 2.60 (m, 1H), 2.59 - 2.52 (m, 2H), 2.50 - 2.40 (m, 2H), 2.37 (s, 3H), 2.35 - 2.25 (m, 2H), 2.23 - 2.15 (m, 1H),1.80 - 1.75 (m, 1H).

**Example 63**

**2-(((*S*)-6-(2-((*R*)-2-Acryloyl-6-methyl-2,5,8-triazaspiro[3.5]nonan-8-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile (E63)**

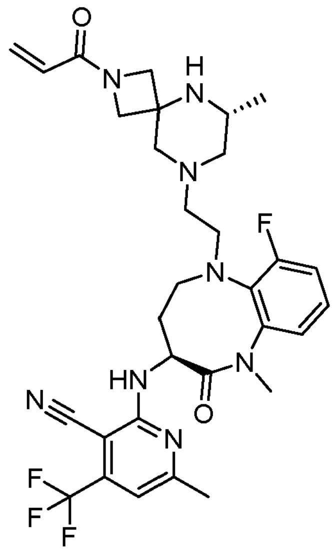

The title compound was prepared in a similar manner to Example 23, using Intermediate 6n in step a.

m/z ES+ $[M+H]^+$ 629.3; $^1$H NMR (400 MHz, $CDCl_3$) δ ppm 7.36 - 7.28 (m, 1H), 7.18 - 7.11 (m, 2H), 6.70 (s, 1H), 6.39 - 6.13 (m, 3H), 5.79 - 5.60 (m, 1H), 4.58 - 3.77 (m, 6H), 3.38 - 3.29 (m, 4H), 3.27 - 3.12 (m, 4H), 3.04 - 2.85 (m, 2H), 2.60 - 2.49 (m, 2H), 2.38 (s, 3H), 2.30 - 2.18 (m, 2H), 1.83 - 1.76 (m, 1H), 1.44 - 1.16 (m, 4H).

**Example 64**

**2-(((*S*)-6-(2-((*R*)-2-Acryloyl-6-methyl-2,5,8-triazaspiro[3.5]nonan-8-yl)ethyl)-7-chloro-1-methyl-2-oxo-1,2,3,4,5,6-hexahydrobenzo[*b*][1,4]diazocin-3-yl)amino)-6-methyl-4-(trifluoromethyl)nicotinonitrile (E64)**

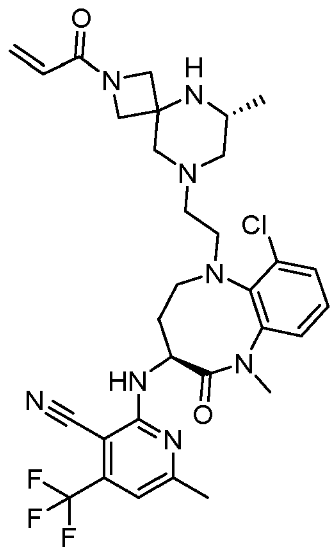

The title compound was prepared in a similar manner to **Example 6,** using **Intermediate 12** and **Intermediate 6o** in step a.

m/z ES+ $[M+H]^+$ 645.1; $^1$H NMR (400 MHz, $CDCl_3$) δ ppm 7.45 (d, *J* = 7.6 Hz, 1H), 7.28 (s, 1H), 7.26 - 7.21 (m, 1H), 6.70 (s, 1H), 6.39 - 6.30 (m, 1H), 6.26 - 6.14 (m, 2H), 5.75 - 5.67 (m, 1H), 4.47 - 4.38 (m, 1H), 4.17 (br s, 1H), 4.10 - 3.94 (m, 2H), 3.92 - 3.85 (m, 1H), 3.40 - 3.33 (m, 4H), 3.29 - 3.16 (m, 2H), 3.12 - 3.10 (m, 2H), 2.91 - 2.87 (m, 1H), 2.84 - 2.75 (m, 1H), 2.50 - 2.40 (m, 2H), 2.39 - 2.38 (m, 4H), 2.34 - 2.30 (m, 2H), 2.04 - 1.88 (m, 1H), 1.78 - 1.74 (m, 1H), 1.25 - 1.18 (m, 3H).

Examples **65A and 65A**

**2-(((*S*)-6-(2-((3*S*/*R*,4*S*/*R*)-3-((1-Acryloylazetidin-3-yl)oxy)-4-hydroxypyrrolidin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile (E65A)** and **2-(((*S*)-6-(2-((3*R*/*S*,4*R*/*S*)-3-((1-acryloylazetidin-3-yl)oxy)-4-hydroxypyrrolidin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile (E65B)**

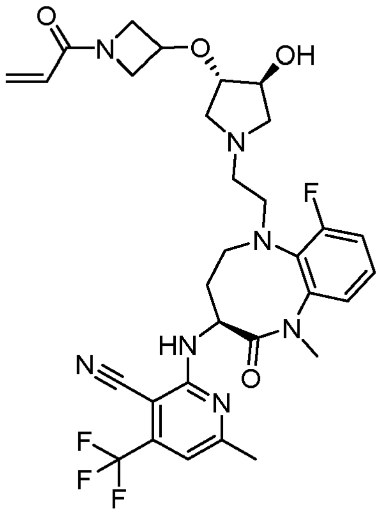

+

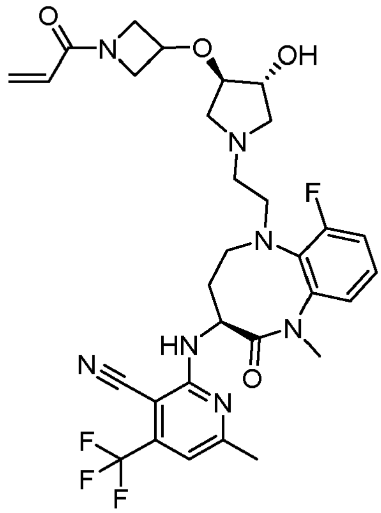

The title compounds were prepared in a similar manner to **Example 6,** using **Intermediate 7b** in step a. An additional chiral SFC step was conducted after step a. The separated diastereoisomers were arbitrarily assigned and were used in subsequent steps b-c.

**Example 65A**

m/z ES+ $[M+H]^+$ 646.3; $^1H$ NMR (400 MHz, $CDCl_3$) δ ppm 7.37 - 7.29 (m, 1H), 7.20 - 7.12 (m, 2H), 6.70 (s, 1H), 6.36 - 6.28 (m, 1H), 6.24 - 6.13 (m, 2H), 5.70 - 5.67 (m, 1H), 4.57 - 4.49 (m, 1H), 4.46 - 4.44 (m, 2H), 4.33 - 4.25 (m, 2H), 4.16 - 4.08 (m, 1H), 4.05 - 4.03 (m, 1H), 3.97 - 3.90 (m, 1H), 3.71 - 3.59 (m, 1H), 3.53 - 3.43 (m, 1H), 3.42 - 3.36 (m, 2H), 3.34 (s, 3H), 3.29 - 3.27 (m, 1H), 3.19 - 3.07 (m, 2H), 3.01 - 2.81 (m, 3H), 2.40 - 2.36 (m, 3H), 2.32 - 2.29 (m, 2H), 1.82 - 1.78 (m, 1H).

**Example 65B**

m/z ES+ $[M+H]^+$ 646.3; $^1H$ NMR (400 MHz, $CDCl_3$) δ ppm 7.33 - 7.27 (m, 1H), 7.17 - 7.08 (m, 2H), 6.68 (s, 1H), 6.37 - 6.28 (m, 1H), 6.23 - 6.11 (m, 2H), 5.72 - 5.63 (m, 1H), 4.50 - 4.34 (m, 3H), 4.31 - 4.21 (m, 1H), 4.12 - 4.01 (m, 2H), 3.99 - 3.88 (m, 1H), 3.80 - 3.78 (m, 1H), 3.75 - 3.39 (m, 1H), 3.36 - 3.30 (m, 1H), 3.28 (s, 3H), 3.17 - 3.15 (m, 2H), 3.13 - 3.04 (m, 2H), 2.75 - 2.71 (m, 1H), 2.65 - 2.57 (m, 1H), 2.45 - 2.42 (m, 2H), 2.37 (s, 3H), 2.33 - 2.21 (m, 1H), 2.14 - 2.10 (m, 1H), 1.78 - 1.75 (m, 1H).

**Example 66**

**(*S*)-2-((6-((1-(1-Acryloylazetidin-3-yl)-1*H*-1,2,4-triazol-3-yl)methyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile (E66)**

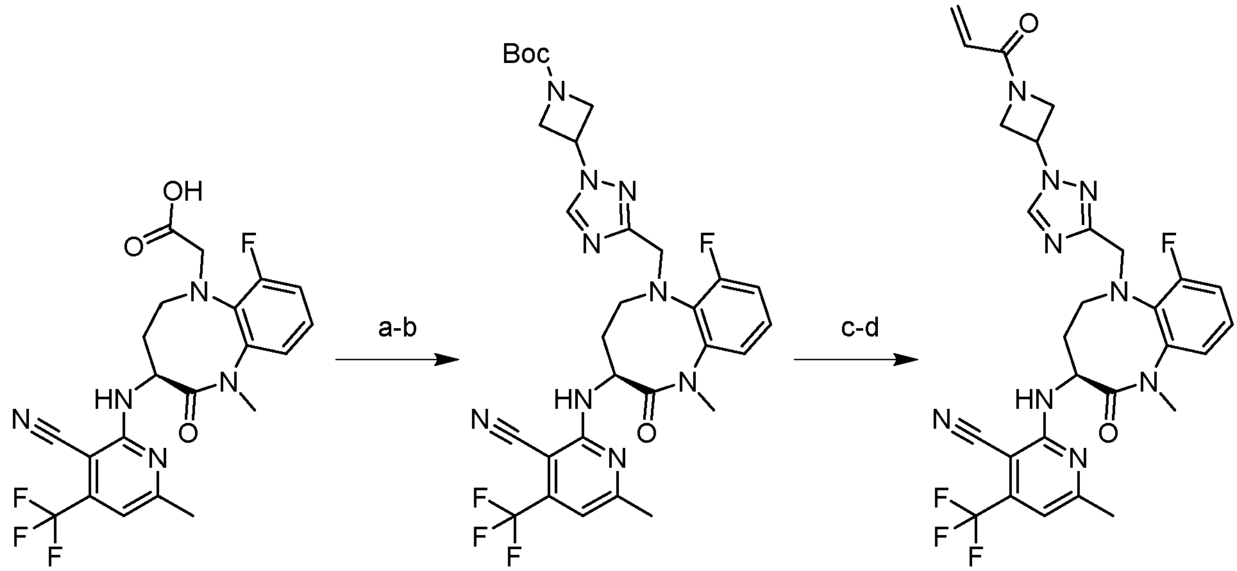


**Step a.** A mixture of **Intermediate 15** (300 mg, 0.65 mmol), **Intermediate 7c** (157 mg, 0.84 mmol), EDCI (161 mg, 0.84 mmol), HOBt (174 mg, 1.3 mmol) and DIPEA (208 mg, 1.6 mmol) in DCM (3 mL) was stirred at 25 °C for 1 h under a $N_2$ atmosphere. Upon completion, the mixture was evaporated and purified by reversed phase flash chromatography (water (0.1% FA)/MeCN) to give *tert*-butyl (*S*)-3-(2-(2-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)acetyl)hydrazineyl)azetidine-1-carboxylate (120 mg, 29% yield) as a colourless oil.

m/z ES+ $[M+H]^+$ 635.2.

**Step b.** To a solution of *tert*-butyl (*S*)-3-(2-(2-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)acetyl)hydrazineyl)azetidine-1-carboxylate (80 mg, 0.13 mmol) in acetic acid (1 mL) and *i*-PrOH (1 mL) was added formamidine acetate (263 mg, 2.5 mmol). The mixture was stirred at 70 °C for 12 h. Upon completion, the mixture was evaporated and purified by reversed phase flash chromatography (water (0.1% FA)/MeCN) to give *tert-butyl* (S)-3-(3-((4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)methyl)-1*H*-1,2,4-triazol-1-yl)azetidine-1-carboxylate (35 mg, 43% yield) as a yellow oil.

m/z ES+ $[M+H]^+$ 644.2.

**Steps c-d.** These 2 steps were conducted in a similar manner to **Example 6,** steps b-c.

m/z ES+ $[M+H]^+$ 598.2; $^1H$ NMR (400 MHz, $CDCl_3$) δ ppm 8.09 - 8.02 (m, 1H), 7.27 - 7.21 (m, 1H), 7.19 - 7.02 (m, 2H), 6.67 (s, 1H), 6.46 - 6.36 (m, 1H), 6.32 - 6.22 (m, 1H), 6.18 (d, *J* = 7.2 Hz, 1H), 5.77 (t, *J* = 7.6 Hz, 1H), 5.29 - 5.08 (m, 1H), 4.82 - 4.62 (m, 2H), 4.61 - 4.48 (m, 2H), 4.40 (d, *J* = 13.2 Hz, 2H), 4.29 - 4.11 (m, 1H), 3.69 - 3.51 (m, 1H), 3.32 - 3.10 (m, 4H), 2.37 (s, 3H), 2.22 - 2.03 (m, 1H), 1.87 - 1.78 (m, 1H).

**Example 67**

**(*S*)-2-((6-((3-(1-Acryloylazetidin-3-yl)-1,2,4-oxadiazol-5-yl)methyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile (E67)**

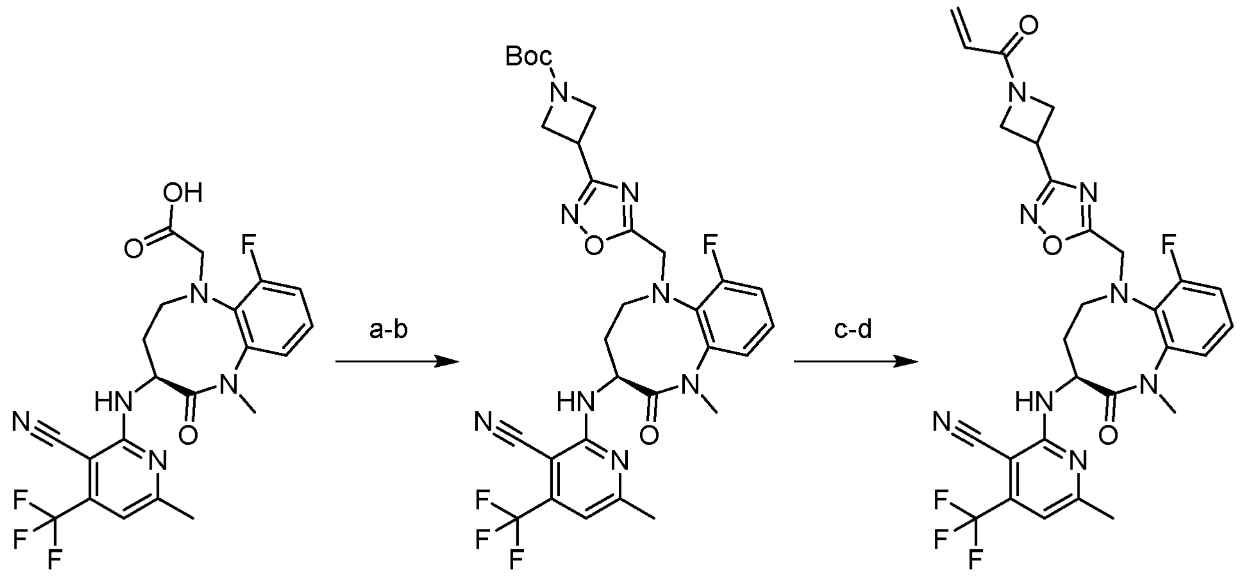


**Step** a. To a solution of Intermediate 15 (70 mg, 0.15 mmol) and **Intermediate 7d** (49 mg, 0.23 mmol) in DCM (1 mL) was added EDCI (35 mg, 0.18 mmol) and HOBt (26 mg, 0.20 mmol). The reaction mixture was stirred at 20 °C for 12 h. Upon completion, the reaction mixture was diluted with water (20 mL), extracted with EtOAc (20 mL), dried over $Na_2SO_4$ and evaporated. The residue was purified by Prep-TLC (EtOAc) to give *tert*-butyl (*S*,*E*)-3-(*N*-(2-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)acetyl)-*N*'-hydroxycarbamimidoyl)azetidine-1-carboxylate (50 mg, 50% yield) as yellow gum.

m/z ES+ $[M+H]^+$ 663.1.

**Step b.** To a solution of *tert*-butyl (*S*,*E*)-3-(*N*-(2-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)acetyl)-*N*'-hydroxycarbamimidoyl)azetidine-1-carboxylate (50 mg, 0.076 mmol) in 1,4-dioxane (2 mL) was added DBU (23 mg, 0.15 mmol). The reaction mixture was stirred at 50 °C for 12 h. Upon completion, the reaction mixture was diluted with water (20 mL) and extracted with EtOAc (20 mL). The organic layer was washed with brine (20 mL), dried over $Na_2SO_4$, evaporated and purified by column chromatography (PE/EtOAc=1/1) to give *tert*-butyl (*S*)-3-(5-((4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)methyl)-1,2,4-oxadiazol-3-yl)azetidine-1-carboxylate (40 mg, 81% yield) as yellow gum.

m/z ES+ $[M+H]^+$ 645.2.

**Steps c-d.** These 2 steps were conducted in a similar manner to **Example 6,** steps b-c.

m/z ES+ $[M+H]^+$ 599.1; $^1H$ NMR (400 MHz, $CDCl_3$) δ ppm 7.36 - 7.28 (m, 1H), 7.21 -7.14 (m, 1H), 7.14 - 7.09 (m, 1H), 6.69 (s, 1H), 6.41 - 6.33 (m, 1H), 6.27 - 6.15 (m, 2H), 5.76 - 5.68 (m, 1H), 4.62 - 4.56 (m, 1H), 4.55 - 4.47 (m, 4H), 4.46 - 4.41 (m, 1H), 4.25 - 4.16 (m, 1H), 4.05 - 3.96 (m, 1H), 3.62 - 3.51 (m, 1H), 3.35 - 3.23 (m, 4H), 2.37 (s, 3H), 2.32 - 2.15 (m, 1H), 1.95 - 1.85 (m, 1H).

**Example 68**

**(*S*)-2-((6-((5-(1-Acryloylazetidin-3-yl)-4*H*-1,2,4-triazol-3-yl)methyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile (E68)**

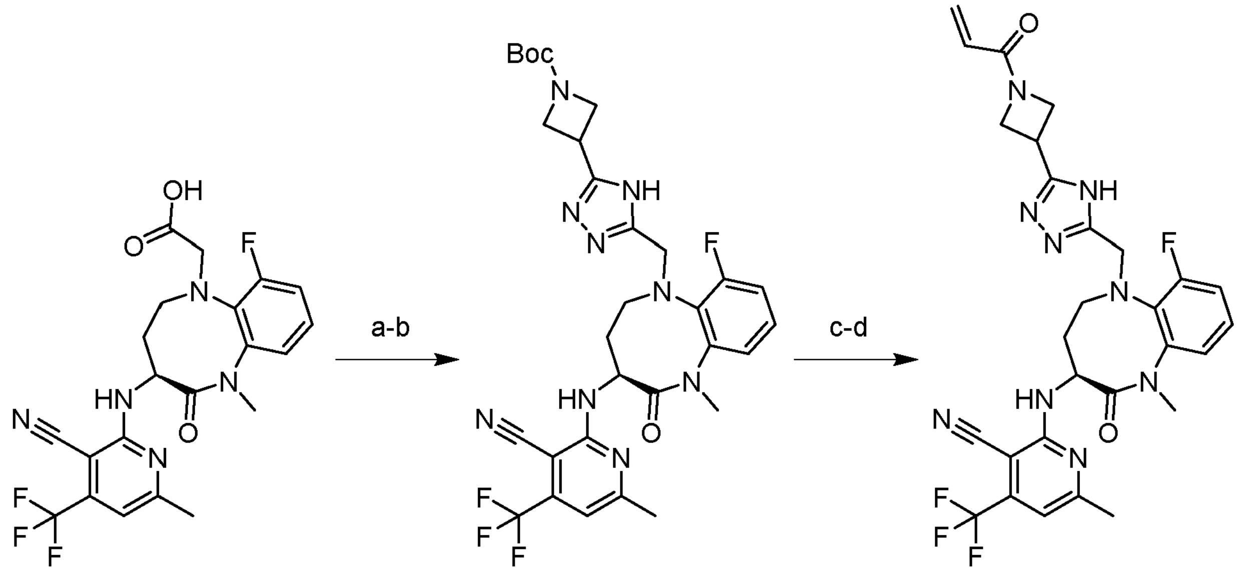


**Step a.** To a solution of **Intermediate 15** (100 mg, 0.21 mmol) and hydrazine monohydrate (54 mg, 1.07 mmol) in DCM (2 mL) was added EDCI (49 mg, 0.26 mmol) and HOBt (41 mg, 0.30 mmol). The mixture was stirred at 25 °C for 3 h. Upon completion, the mixture was diluted with water (20 mL), extracted with DCM (3 x 10 mL) and evaporated. The residue was purified by Prep-TLC (EtOAc/MeOH = 20/1) to give (S)-2-(4-((3-cyano-6-methyl-4-(trifluoromethyl)pyridin-2-yl) amino)-10-fluoro-6-methyl-5-oxo-3,4,5,6-tetrahydrobenzo[*b*][1,4]diazocin-1(2*H*)-yl)acetohydrazide (70 mg, 61% yield) as a yellow solid.

m/z ES+ $[M+H]^+$ 479.9; $^1$H NMR (400 MHz, $CDCl_3$) δ ppm 8.26 - 8.09 (m, 1H), 7.38 - 7.32 (m, 1H), 7.22 - 7.13 (m, 2H), 6.72 (s, 1H), 6.19 (d, J = 7.2 Hz, 1H), 4.58 - 4.51 (m, 1H), 3.82 - 3.67 (m, 2H), 3.37 (s, 3H), 3.35 - 3.17 (m, 4H), 2.46 - 2.33 (m, 4H), 1.90 - 1.78 (m, 1H).

**Step b.** A mixture of (*S*)-2-(4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)acetohydrazide (60 mg, 0.13 mmol), *tert*-butyl 3-cyanoazetidine-1-carboxylate (CAS: 142253-54-1; 137 mg, 0.75 mmol) and $Na_2CO_3$ (16 mg, 0.15 mmol) in n-BuOH (2 mL) was stirred at 100 °C for 48 h under a $N_2$ atmosphere. Upon completion, the mixture was diluted with water (10 mL) and extracted with EtOAc (3 x 10 mL). The combined organic layers were washed with brine (10 mL), dried over $Na_2SO_4$, evaporated and purified by column chromatography (PE/EtOAc = 1/3) to give *tert*-butyl (*S*)-3-(5-((4-((3-cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)methyl)-4*H*-1,2,4-triazol-3-yl)azetidine-1-carboxylate (70 mg, 70% yield) as a yellow solid.

m/z ES+ $[M+H]^+$ 644.3; $^1$H NMR (400 MHz, $CDCl_3$) δ ppm 7.43 - 7.35 (m, 1H), 7.25 - 7.18 (m, 2H), 6.73 (s, 1H), 6.17 (d, *J* = 7.2 Hz, 1H), 4.73 - 4.62 (m, 2H), 4.39 - 4.34 (m, 2H), 4.28 - 4.26 (m, 2H), 4.07 - 3.99 (m, 2H), 3.93 - 3.82 (m, 1H), 3.34 - 3.31 (m, 1H), 3.26 (s, 3H), 3.24 - 3.19 (m, 1H), 2.40 (s, 3H), 2.38 - 2.30 (m, 1H), 1.86 - 1.80 (m, 1H), 1.44 (s, 9H).

**Steps c-d.** These 2 steps were conducted in a similar manner to **Example 6,** steps b-c.

m/z ES+ $[M+H]^+$ 598.2; $^1$H NMR (400 MHz, $CDCl_3$) δ ppm 7.43 - 7.35 (m, 1H), 7.25 - 7.18 (m, 2H), 6.73 (s, 1H), 6.38 - 6.31 (m, 1H), 6.27 - 6.18 (m, 2H), 5.70 - 5.65 (m, 1H), 4.72 - 4.60 (m, 2H), 4.58 - 4.51 (m, 2H), 4.45 - 4.43 (m, 1H), 4.30 - 4.21 (m, 2H), 4.02 - 3.93 (m, 1H), 3.39 - 3.19 (m, 5H), 2.40 (s, 3H), 2.38 - 2.28 (m, 1H), 1.84 (d, *J* = 12.8 Hz, 1H).

## Example 69

### (*S*)-2-((6-(2-(2-Acryloyl-2,5,8-triazaspiro[3.5]nonan-8-yl)ethyl)-1,7-dimethyl-2-oxo-1,2,3,4,5,6-hexahydrobenzo [*b*][1,4]diazocin-3-yl)amino)-6-methyl-4-(trifluoromethyl)nicotinonitrile (E69)

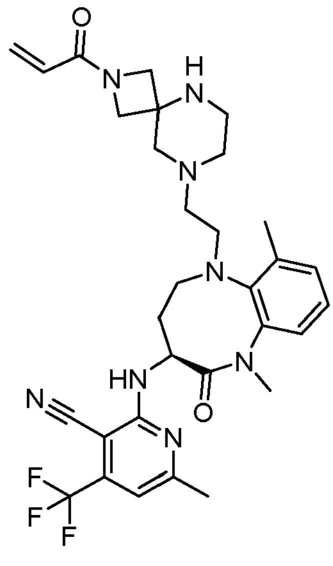

The title compound was prepared in a similar manner to **Example 23,** using **Intermediate 13** in step a.

m/z ES+ $[M+H]^+$ 611.2; $^1H$ NMR (400 MHz, $CDCl_3$) δ ppm 7.24 - 7.16 (m, 2H), 7.09 (d, *J* = 7.6 Hz, 1H), 6.68 (s, 1H), 6.39 - 6.31 (m, 1H), 6.25 - 6.14 (m, 2H), 5.69 (d, *J* = 10.4 Hz, 1H), 4.48 - 4.43 (m, 1H), 4.07 - 3.92 (m, 1H), 3.92 - 3.83 (m, 2H), 3.74 (d, *J* = 10.8 Hz, 1H), 3.43 - 3.38 (m, 1H), 3.35 (d, *J* = 2.4 Hz, 3H), 3.10 - 2.99 (m, 2H), 2.98 - 2.90 (m, 1H), 2.90 - 2.80 (m, 2H), 2.57 - 2.50 (m, 1H), 2.49 (d, *J* = 4.0 Hz, 2H), 2.39 (s, 4H), 2.37 (s, 4H), 2.36 - 2.34 (m, 1H), 2.33 - 2.31 (m, 1H), 1.72 (s, 1H), 1.70 - 1.69 (m, 1H).

**Example 70**

**(*S*)-2-((6-(2-(2-Acryloyl-2,5,8-triazaspiro[3.5]nonan-8-yl)ethyl)-1-ethyl-7-fluoro-2-oxo-1,2,3,4,5,6-hexahydrobenzo[*b*][1,4]diazocin-3-yl)amino)-6-methyl-4-(trifluoromethyl)nicotinonitrile (E70)**

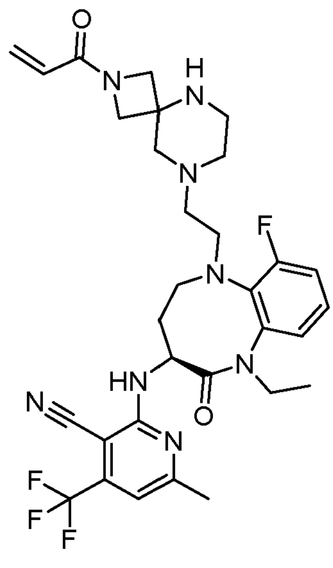

The title compound was prepared in a similar manner to **Example 23,** using **Intermediate 14** in step a.

m/z ES+ $[M+H]^+$ 629.2; $^1H$ NMR (400 MHz, $CDCl_3$) δ ppm 7.31 - 7.23 (m, 1H), 7.18 - 7.06 (m, 2H), 6.67 (s, 1H), 6.39 - 6.30 (m, 1H), 6.27 - 6.14 (m, 2H), 5.69 (d, *J* = 10.4 Hz, 1H), 4.43 - 4.34 (m, 1H), 4.11 - 3.85 (m, 4H), 3.80 - 3.75 (m, 1H), 3.71 - 3.57 (m, 1H), 3.51 - 3.26 (m, 2H), 3.14 - 2.95 (m, 2H), 2.95 - 2.80 (m, 2H), 2.65 - 2.48 (m, 3H), 2.46 - 2.39 (m, 2H), 2.37 (s, 3H), 2.32 - 2.09 (m, 3H), 1.81 - 1.78 (m, 1H), 1.19 - 1.06 (m, 3H).

**Example 71**

**(*S*)-2-((6-(2-(5-Acetyl-2-acryloyl-2,5,8-triazaspiro[3.5]nonan-8-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile (E71)**

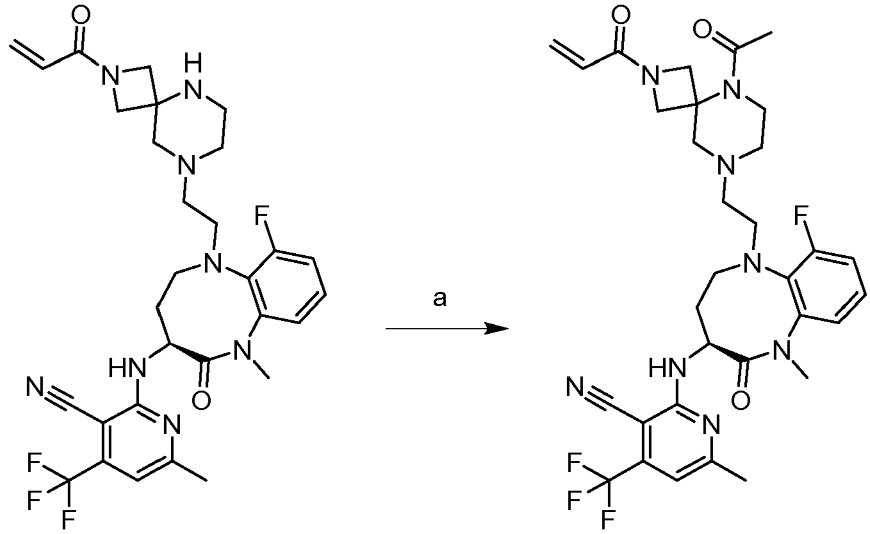


**Step a.** A mixture of Example 23 (0.1 g, 0.16 mmol), acetic acid (15 mg, 0.24 mmol), HATU (74 mg, 0.20 mmol) and DIPEA (42 mg, 0.33 mmol) in DCM (1 mL) was stirred at 15 °C for 1 h under a $N_2$ atmosphere. Upon completion, the mixture was evaporated. The residue was purified by Prep-HPLC to give the title compound (20 mg, 19% yield) as a white solid.

m/z ES+ $[M+H]^+$ 657.3; $^1H$ NMR (400 MHz, $CDCl_3$) δ ppm 7.31 - 7.27 (m, 1H), 7.21 - 7.07 (m, 2H), 6.70 (s, 1H), 6.42 - 6.31 (m, 1H), 6.29 - 6.14 (m, 2H), 5.71 (t, *J* = 10.4 Hz, 1H), 4.63 - 4.38 (m, 2H), 4.22 - 3.95 (m, 3H), 3.54 - 3.21 (m, 6H), 3.20 - 3.05 (m, 3H), 2.83 - 2.41 (m, 4H), 2.39 (s, 3H), 2.35 - 2.16 (m, 3H), 2.09 (s, 3H), 1.80 (d, *J* = 12.4 Hz, 1H).

**Examples 72A and 72B**

**2-(((*S*)-6-(2-((*R*/*S*)-8-Acryloyloctahydro-2*H*-pyrazino[1,2-a]pyrazin-2-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile (E72A)** and **2-(((*S*)-6-(2-((*S*/*R*)-8-acryloyloctahydro-2*H*-pyrazino[1,2-*a*]pyrazin-2-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile (E72B)**

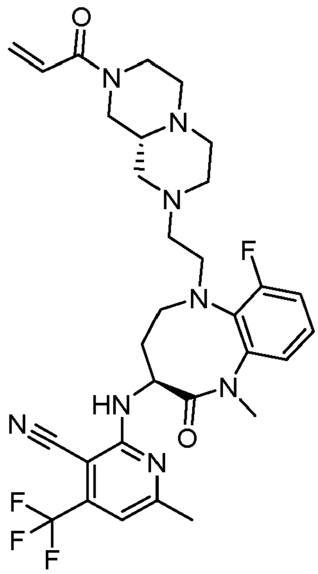

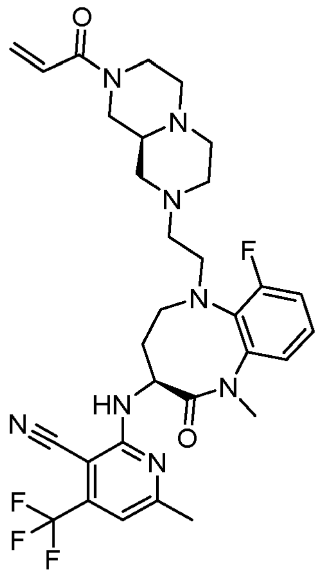

The title compounds were prepared in a similar manner to **Example 6,** using *tert*-butyl octahydro-2*H*-pyrazino [1,2-*a*]pyrazine-2-carboxylate (CAS: 1159825-34-9) in step a. An additional chiral SFC step was conducted after step a. The separated diastereoisomers were arbitrarily assigned and were used in subsequent steps b-c.

**Example 72A**

m/z ES+ $[M+H]^+$ 629.3; $^1H$ NMR (400 MHz, $CDCl_3$) $\delta$ ppm 7.28 - 7.26 (m, 1H), 7.16 - 7.07 (m, 2H), 6.68 (s, 1H), 6.56 - 6.52 (m, 1H), 6.31 - 6.26 (m, 1H), 6.20 - 6.17 (m, 1H), 5.71 - 5.69 (m, 1H), 4.62 - 4.59 (m, 1H), 4.48 - 4.43 (m, 1H), 3.94 - 3.69 (m, 1H), 3.39 - 3.30 (m, 4H), 3.23 - 3.09 (m, 3H), 2.85 - 2.80 (m, 1H), 2.77 - 2.72 (m, 4H), 2.49 - 2.15 (m, 10H), 2.18 - 2.06 (m, 1H), 1.93 - 1.83 (m, 1H), 1.83 - 1.76 (m, 1H).

**Example 72B**

m/z ES+ $[M+H]^+$ 629.3; $^1H$ NMR (400 MHz, $CDCl_3$) $\delta$ ppm 7.34 - 7.28 (m, 1H), 7.21 - 7.10 (m, 2H), 6.69 (s, 1H), 6.67 - 6.48 (m, 1H), 6.33 - 6.28 (m, 1H), 6.20 - 6.18 (m, 1H), 5.75 - 5.73 (m, 1H), 4.70 - 4.53 (m, 1H), 4.51 - 4.41 (m, 1H), 4.08 - 3.89 (m, 1H), 3.51-3.46 (m, 2H), 3.41 - 3.28(m, 5H), 3.19 - 3.08 (m, 2H), 2.95 - 2.80 (m, 4H), 2.76 - 2.50 (m, 4H), 2.38 - 2.36 (m, 4H), 2.34 - 2.18 (m, 2H), 1.82 - 1.79 (m, 2H).

## BIOLOGICAL DATA

### Pol$\theta$ Polymerase Domain $K_{inact}/K_I$ Assay

A PicoGreen assay was used to measure the $K_{inact}$ and $K_I$ values of covalent compounds that inhibit the activity of Pol$\theta$ *in vitro.*

Human Pol$\theta$ polymerase domain (aa1820-2590) was expressed in *E. Coli*, purified, aliquoted and stored at -80 °C until required. Pol$\theta$ substrate was generated from a 1.2:1 mix of DNA II Short to DNA II Long to give a final concentration of 20 mM substrate in annealing buffer (20 mM Tris pH 7.5, 50 mM NaCl). The substrate was heated in 50 mL aliquots to 95 °C in a heating block for 5 min before the heating block switched off, the reaction left to cool to rt and stored at -20 °C until required.

| Name | Sequence |
|---|---|
| DNA II short | 5'-GCGGCTGTCATAAG-3' (SEQ ID NO: 1) |
| DNA II long | 5'-GCTACATTGACAATGGCA-TCAAATCTCAGATTGCGTCTTATGACAGCCGCG-3' (SEQ ID NO: 2) |

Assay measurements were performed with 1X buffer comprising 25 mM Tris pH 7.5, 12.5 mM NaCl, 0.5 mM

NaCl, 5% (v/v) glycerol, 0.01 % v/v Triton x-100, 0.1 mg/ml BSA, 1 mM DTT. Test compounds were prepared by dilution in 100% DMSO to give a 12 μM intermediate stock of each (100x final top concentration). 100 nL of 23x 1:1.5-fold serial dilutions and a DMSO only control were dispensed using the Tecan dispenser into Greiner 384 well black low volume plates (product code 784076). DMSO concentration was maintained at 1% of the final assay volume by back filling with DMSO.

2x Working stock of substrate (200 nM of DNA substrate and 100 mM dNTPs) and enzyme (1.4 nM PolΘ) were made up in assay buffer. 5 μL / well of both enzyme and substrate 2x solutions were dispensed using a Tempest dispenser (Formulatrix) into assay plates that had been pre-dispensed with compound to give a final assay concentration of 100 nM DNA substrate, 50 mM dNTPs and 0.8 nM Polθ. To stop the reaction, 5 mL of a solution containing 25 mM Tris-HCl pH 7.5 and 20 mM EDTA was added at 6 timepoints (t=0, 2.5 , 5, 7.5, 10, 12.5, 15, 20, 25, 30, 35, 40, 45, 50, 55 and 60 min) using the Tempest's time delay function. The plates were covered during the time course to prevent evaporation. After completing the assay, 5 μL of detection reagent (25 mM Tris-HCl pH 7.5 and 2.5% (v/v) PicoGreen) was dispensed into the wells using a Tempest liquid handler (Formulatrix) and plates subsequently read on the CLARIOstar Plus (BMG Labtech) using the default optical settings for fluorescein and the auto gain / focus settings.

All data analysis was carried out using GraphPad Prism V.8 (GraphPad Software Inc, San Diego, CA) Time course data for each inhibitor concentration was fitted to equation 1 to determine $k_{obs}$ values.[1] Any time points where the control (DMSO alone) reaction was no longer linear were excluded from the analysis.

$$[P] = \frac{v_i}{k_{obs}}[1 - \exp(-k_{obs}\,t)]$$

(equation 1)

A secondary plot of $k_{obs}$ vs [I] is used to determine the $K_{inact}$ and $K_I$ parameters (for a 2-step covalent inhibitor) by fitting to equation 2.[1]

$$k_{obs} = \frac{k_{inact}\ [I]}{K_I\ +\ [I]}$$

(equation 2)

1) Evaluation of Enzyme Inhibitors in Drug Discovery (Ch9), R.A Copeland, Wiley, 2nd Edition 2013.

The compounds of **Examples 1 to 72** were tested in the above mentioned Polθ Polymerase Domain $K_{inact}/K_I$ Assay and the results are shown in the following table:

| Example Number | $K_{inact}/K_I$ ($\mu M^{-1}s^{-1}$) |
|---|---|
| 1 | 3.1 |
| 2 | 0.51 |
| 3 | 1.1 |
| 4 | 0.30 |
| 5 | 0.11 |
| 6 | 0.98 |
| 7 | 0.46 |
| 8 | 0.49 |
| 9 | 0.30 |
| 10 | 0.19 |
| 11 | 0.50 |
| 12 | 0.44 |
| 13 | 0.32 |

(continued)

| Example Number | $K_{inact}/K_I$ ($\mu M^{-1}s^{-1}$) |
|---|---|
| 14 | 0.78 |
| 15 | 0.28 |
| 16 | 0.54 |
| 17 | 0.57 |
| 18 | 0.23 |
| 19 | 0.16 |
| 20 | 0.50 |
| 21 | 1.2 |
| 22 | 0.50 |
| 23 | 0.94 |
| 24 | 0.63 |
| 25 | 1.6 |
| 26 | 2.1 |
| 27 | 0.94 |
| 28 | 0.56 |
| 29 | 1.2 |
| 30 | 0.69 |
| 31 | 0.92 |
| 32A | 0.77 |
| 32B | 1.0 |
| 33A | 0.15 |
| 33B | 0.12 |
| 34A | 0.19 |
| 34B | 0.68 |
| 35 | 0.34 |
| 36 | 0.36 |
| 37 | 0.34 |
| 38 | 0.48 |
| 39 | 1.0 |
| 40 | 3.0 |
| 41 | 1.4 |
| 42 | 0.82 |
| 43 | 0.90 |
| 44 | 1.3 |
| 45 | 5.7 |
| 46 | 0.56 |
| 47 | 0.38 |
| 48 | 0.68 |
| 49 | 0.11 |

(continued)

| Example Number | $K_{inact}/K_I$ ($\mu M^{-1}s^{-1}$) |
|---|---|
| 50 | 0.11 |
| 51 | 0.07 |
| 52 | 0.15 |
| 53 | 0.50 |
| 54 | 0.70 |
| 55 | 0.81 |
| 56 | 11 |
| 57 | 0.30 |
| 58A | 0.06 |
| 58B | 0.04 |
| 59 | 0.12 |
| 60 | 0.24 |
| 61 | 0.50 |
| 62 | 1.8 |
| 63 | 0.86 |
| 64 | 1.8 |
| 65A | 0.46 |
| 65B | 0.55 |
| 66 | 2.4 |
| 67 | 1.3 |
| 68 | 0.13 |
| 69 | 4.1 |
| 70 | 1.3 |
| 71 | 1.4 |
| 72A | 0.18 |
| 72B | 0.63 |

## Claims

**1.** A compound of formula (I):

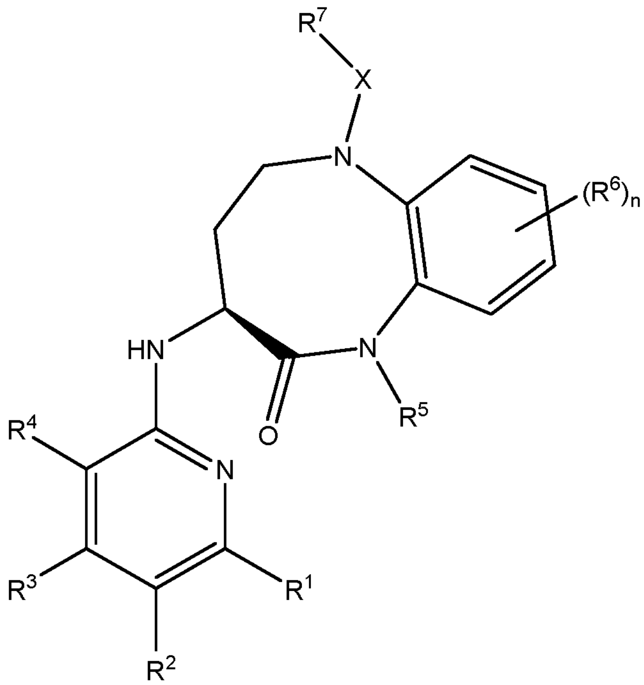

(I)

or a tautomeric or a stereochemically isomeric form, a pharmaceutically acceptable salt or a solvate thereof, wherein:

$R^1$, $R^2$, $R^3$ and $R^4$ independently represent hydrogen, $C_{1-6}$ alkyl, $C_{2-6}$ alkenyl, $C_{1-6}$ alkoxy, halogen, halo$C_{1-6}$ alkyl, halo$C_{1-6}$ alkoxy, $C_{3-8}$ cycloalkyl, cyano or $-NR^xR^y$;
$R^5$ represents hydrogen or $C_{1-6}$ alkyl;
n represents an integer selected from 0, 1, 2 or 3;
$R^6$ represents halogen or $C_{1-6}$ alkyl;
X represents a $C_1$-$C_4$ alkylene group optionally substituted by an O, OH or $CONR^8$ group;
$R^8$ represents hydrogen or $C_{1-6}$ alkyl;
$R^7$ represents a $-Het^A-CO-CH=CHR^9$ or $-Het^B-Y-Het^C-CO-CH=CHR^9$ group;
Y represents a bond or -O-;
$R^9$ represents hydrogen, $-CH_2-NR^xR^y$ or $-Het^D$;
$Het^A$, $Het^B$, $Het^C$ and $Het^D$ each represent a heterocyclyl or a heteroaryl group optionally substituted by one or more (e.g. 1, 2 or 3) substituents such as $C_{1-6}$ alkyl, $C_{2-6}$ alkenyl, $C_{2-6}$ alkynyl, $C_{1-6}$ alkoxy, $-COO-C_{2-6}$ alkynyl, $-CO-C_{1-6}$ alkyl, hydroxy and oxo; and
$R^x$ and $R^y$ independently represent hydrogen, $C_{1-6}$ alkyl, $C_{2-6}$ alkenyl, $C_{2-6}$ alkynyl, $C_{1-6}$ alkoxy, or $R^x$ and $R^y$ together with the nitrogen atom to which they are attached join to form a nitrogen containing heterocyclic ring which may be optionally substituted by one or more $C_{1-6}$ alkyl groups.

**2.** The compound as defined in claim 1, wherein:

(a) $R^1$ represents:

hydrogen;
$C_{1-6}$ alkyl (such as methyl or ethyl);
$C_{2-6}$ alkenyl (such as ethenyl);$C_{1-6}$ alkoxy (such as methoxy);
halogen (such as chlorine); or
$-NR^xR^y$ (such as $-N(Me)_2$ or -N(Me)(Et));

such as $R^1$ represents $C_{1-6}$ alkyl, in particular methyl; and/or
(b) $R^2$ represents:

hydrogen;

halogen (such as chlorine); or
$C_{1-6}$ alkyl (such as methyl);

such as hydrogen; and/or
(c) $R^3$ represents:

$C_{1-6}$ alkyl (such as methyl, ethyl or isopropyl);
$C_{2-6}$ alkenyl (such as $-C(=CH_2)(Me)$);
halogen (such as bromine);
halo$C_{1-6}$ alkyl (such as trifluoromethyl or $-C(H)(Me)-CF_3$); or
halo$C_{1-6}$ alkoxy (such as difluoromethoxy);

such as halo$C_{1-6}$ alkyl, in particular trifluoromethyl; and/or
(d) $R^4$ represents cyano.

**3.** The compound as defined in claim 1 or claim 2, wherein $R^5$ represents hydrogen, methyl or ethyl, such as hydrogen or methyl, in particular methyl.

**4.** The compound as defined in any one of claims 1 to 3, wherein n represents an integer selected from 0, 1 or 2, such as 0.

**5.** The compound as defined in any one of claims 1 to 4, wherein $R^6$ represents fluorine, chlorine or methyl, such as fluorine or chlorine, in particular fluorine, such as wherein n represents an integer which is 1 and $R^6$ represents fluorine; or n represents an integer which is 1 and $R^6$ represents chlorine; or n represents an integer which is 2 and both $R^6$ groups represent fluorine.

**6.** The compound as defined in any one of claims 1 to 5, wherein X represents a $C_1$-$C_4$ alkylene group (such as $-CH_2-$, $-(CH_2)_2-$, $-(CH_2)_3-$ or $-(CH_2)_4-$) optionally substituted by an O (such as $-(CH_2)_2-O-$, $-(CH_2)_2-O-CH_2-$ or $-(CH_2)_3-O-$), OH (such as $-CH_2-CH(OH)-CH_2-$) or $CONR^8$ (such as $-(CH_2)_2-N(Me)-CO-$) group, in particular X represents $-CH_2-$, $-(CH_2)_2-$, - $(CH_2)_2-O-$, $-(CH_2)_2-O-CH_2-$, $-(CH_2)_3-$, $-(CH_2)_3-O-$, $-(CH_2)_2-N(Me)-CO-$ or $-CH_2-CH(OH)-CH_2-$.

**7.** The compound as defined in any one of claims 1 to 6, wherein $R^8$ represents hydrogen or methyl, such as methyl.

**8.** The compound as defined in any one of claims 1 to 7, wherein $R^7$ is a:

(a) -$Het^A$-CO-CH=$CHR^9$ group and $Het^A$ represents 2-azaspiro[3.3]heptan-6-yl, azetidinyl, 2,6-diazaspiro[3.3]heptan-2-yl, 2,6-diazaspiro[3.5]nonan-6-yl, 2,7-diazaspiro[3.5]nonan-7-yl, 2,7-diazaspiro[4.4]nonan-2-yl, 5-oxa-2,7-diazaspiro[3.4]octan-7-yl, 5-oxa-2,8-diazaspiro[3.5]nonan-8-yl, 6-oxa-2,9-diazaspiro[4.5]decan-9-yl, piperazinyl, piperidinyl, pyrrolidinyl, 2,5,8-triazaspiro[3.5]nonan-8-yl, tetrahydro-1'*H*-spiro[azetidine-3,6'-pyrazino[2,1-*c*][1,4]oxazin]-8'(7'*H*)-yl, or octahydro-2*H*-pyrazino[1,2-a]pyrazin-2-yl, optionally substituted by one or more (e.g. 1, 2 or 3) substituents such as $C_{1-6}$ alkyl (e.g. methyl), oxo, $C_{2-6}$ alkynyl (e.g. propynyl), -COO-$C_{2-6}$ alkynyl (e.g. -COO-propynyl), -CO-$C_{1-6}$ alkyl (e.g. - CO-Me), or $C_{1-6}$ alkoxy (e.g. methoxy); or
(b) -$Het^B$-Y-$Het^C$-CO-CH=$CHR^9$ group, such as -azetidinyl-piperazinyl-CO-CH=$CHR^9$, - morpholinyl-azetidinyl-CO-CH=$CHR^9$, -piperazinyl-azetidinyl-CO-CH=$CHR^9$, -piperidinyl-azetidinyl-CO-CH=$CHR^9$, -piperidinyl-O-azetidinyl-CO-CH=$CHR^9$, -2,5-diazabicyclo[2.2.1]heptan-2-yl-azetidinyl-CO-CH=$CHR^9$, -pyrrolidinyl-O-azetidinyl-CO-CH=$CHR^9$, -triazolyl-azetidinyl-CO-CH=$CHR^9$, or -oxadiazolyl-azetidinyl-CO-CH=$CHR^9$, optionally substituted by one or more (e.g. 1, 2 or 3) substituents such as $C_{1-6}$ alkyl (e.g. methyl), hydroxy or oxo.

**9.** The compound as defined in any one of claims 1 to 8, wherein $R^9$ represents hydrogen; or $-CH_2-NR^xR^y$ (such as $-CH_2-N(Me)_2$, $-CH_2-N(Me)(-(CH_2)_2-OMe)$ or $-CH_2-N(Me)(-CH_2-C{\equiv}CH)$; or -$Het^D$ (such as a monocyclic heterocyclyl, in particular pyrrolidinyl, especially C-linked pyrrolidinyl (e.g. pyrrolidin-2-yl) optionally substituted by one or more (e.g. 1, 2 or 3) substituents such as $C_{1-6}$ alkyl (e.g. methyl)).

**10.** The compound as defined in any one of claims 1 to 9, wherein $R^x$ and $R^y$ independently represent hydrogen, $C_{1-6}$ alkyl (such as methyl), $C_{2-6}$ alkynyl (such as $-CH_2-C{\equiv}CH$) or $C_{1-6}$ alkoxy (such as $-(CH_2)_2-OMe$); such as both of $R^x$ and $R^y$ represent $C_{1-6}$ alkyl (such as methyl); or one of $R^x$ and $R^y$ represents $C_{1-6}$ alkyl (such as methyl) and the other represents $C_{2-6}$ alkynyl (such as $-CH_2-C{\equiv}CH$) or $C_{1-6}$ alkoxy (such as -OMe).

**11.** The compound as defined in any one of claims 1 to 10, which is a compound of formula $(I)^a$:

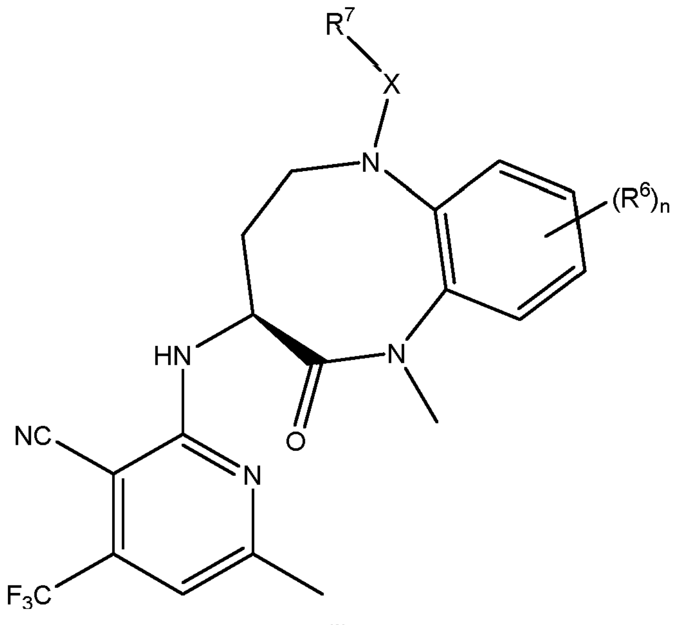

(I)[a]

or a tautomeric or a stereochemically isomeric form, a pharmaceutically acceptable salt or a solvate thereof, wherein $R^6$, n, X and $R^7$ are as defined in any one of claims 1 and 4 to 10.

**12.** The compound as defined in claim 1, wherein the compound is the free base of a compound of Examples 1-72:

Example 1: (*S*,*E*)-2-((6-(2-(2-(4-(Dimethylamino)but-2-enoyl)-5-oxa-2,8-diazaspiro[3.5]nonan-8-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 2: 2-(((S)-6-(2-(2-((E)-4-(Dimethylamino)but-2-enoyl)-6-oxa-2,9-diazaspiro[4.5]decan-9-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 3: (S,E)-2-((6-(2-(2-(4-(Dimethylamino)but-2-enoyl)-5-oxa-2,8-diazaspiro[3.5]nonan-8-yl)ethyl)-1-methyl-2-oxo-1,2,3,4,5,6-hexahydrobenzo[b][1,4]diazocin-3-yl)amino)-6-methyl-4-(trifluoromethyl)nicotinonitrile;
Example 4: (S,E)-2-((6-(2-(6-(4-(Dimethylamino)but-2-enoyl)-2,6-diazaspiro[3.3]heptan-2-yl)ethyl)-1-methyl-2-oxo-1,2,3,4,5,6-hexahydrobenzo[b][1,4]diazocin-3-yl)amino)-6-methyl-4-(trifluoromethyl)nicotinonitrile;
Example 5: (S,E)-2-((6-(2-(4-(4-(Dimethylamino)but-2-enoyl)piperazin-1-yl)ethyl)-1-methyl-2-oxo-1,2,3,4,5,6-hexahydrobenzo[b][1,4]diazocin-3-yl)amino)-6-methyl-4-(trifluoromethyl)nicotinonitrile;
Example 6: (*S*)-2-((6-(2-(2-Acryloyl-5-methyl-2,5,8-triazaspiro[3.5]nonan-8-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 7: (*S*)-2-((6-(2-(6-Acryloyl-2,6-diazaspiro[3.3]heptan-2-yl)ethyl)-8-chloro-1-methyl-2-oxo-1,2,3,4,5,6-hexahydrobenzo[b][1,4]diazocin-3-yl)amino)-6-methyl-4-(trifluoromethyl)nicotinonitrile;
Example 8: (*S*)-2-((6-(2-(2-Acryloyl-2,7-diazaspiro[3.5]nonan-7-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 9: (*S*)-2-((6-(2-(2-Acryloyl-2,6-diazaspiro[3.5]nonan-6-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 10: (*S*)-2-((6-(2-(3-(4-Acryloylpiperazin-1-yl)azetidin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 11: (*S*)-2-((6-(2-(4-(1-Acryloylazetidin-3-yl)piperazin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 12: (*S*)-2-((6-(2-(4-(1-Acryloyl-3-hydroxyazetidin-3-yl)piperidin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 13: (*S*)-2-((6-(2-(4-((1-Acryloylazetidin-3-yl)oxy)piperidin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 14: 2-(((*S*)-6-(2-((S)-4-(1-Acryloylazetidin-3-yl)-2-methylpiperazin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;

Example 15: 2-(((*S*)-6-(2-((*R*)-4-(1-Acryloylazetidin-3-yl)-2-methylpiperazin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 16: 2-(((*S*)-6-(2-((*R*)-4-(1-Acryloylazetidin-3-yl)-3-methylpiperazin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 17: 2-(((*S*)-6-(2-((*S*)-4-(1-Acryloylazetidin-3-yl)-3-methylpiperazin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 18: 2-(((*S*)-6-(2-((1*R*,4*R*)-5-(1-Acryloylazetidin-3-yl)-2,5-diazabicyclo[2.2.1]heptan-2-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 19: 2-(((*S*)-6-(2-((1*S*,4*S*)-5-(1-Acryloylazetidin-3-yl)-2,5-diazabicyclo[2.2.1]heptan-2-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 20: *(S)*-2-((6-(2-(4-(1-Acryloylazetidin-3-yl)piperazin-1-yl)ethyl)-1-methyl-2-oxo-1,2,3,4,5,6-hexahydrobenzo[b][1,4]diazocin-3-yl)amino)-6-methyl-4-(trifluoromethyl)nicotinonitrile;
Example 21: *(S)*-2-((6-(2-(2-Acryloyl-5-oxa-2,8-diazaspiro[3.5]nonan-8-yl)ethyl)-1-methyl-2-oxo-1,2,3,4,5,6-hexahydrobenzo[b][1,4]diazocin-3-yl)amino)-6-methyl-4-(trifluoromethyl)nicotinonitrile;
Example 22: *(S)*-2-((6-(2-(6-Acryloyl-2,6-diazaspiro[3.3]heptan-2-yl)ethyl)-1-methyl-2-oxo-1,2,3,4,5,6-hexahydrobenzo[b][1,4]diazocin-3-yl)amino)-6-methyl-4-(trifluoromethyl)nicotinonitrile;
Example 23: *(S)*-2-((6-(2-(2-Acryloyl-2,5,8-triazaspiro[3.5]nonan-8-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 24: (*S,E*)-2-((6-(2-(2-(4-(Dimethylamino)but-2-enoyl)-6-oxo-5-oxa-2,7-diazaspiro[3.4]octan-7-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 25: (*S*)-2-((6-(2-(2-Acryloyl-5-(prop-2-yn-1-yl)-2,5,8-triazaspiro[3.5]nonan-8-yl)ethyl)-7-fluoro-1-methyl-2-oxo-1,2,3,4,5,6-hexahydrobenzo[b][1,4]diazocin-3-yl)amino)-6-methyl-4-(trifluoromethyl)nicotinonitrile;
Example 26: (*S,E*)-2-((6-(2-(2-(4-(Dimethylamino)but-2-enoyl)-5-(prop-2-yn-1-yl)-2,5,8-triazaspiro[3.5]nonan-8-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 27: (*S,E*)-Prop-2-yn-1-yl 8-(2-(4-((3-cyano-6-methyl-4-(trifluoromethyl)pyridin-2-yl)amino)-10-fluoro-6-methyl-5-oxo-3,4,5,6-tetrahydrobenzo[b][1,4]diazocin-1(2H)-yl)ethyl)-2-(4-(dimethylamino)but-2-enoyl)-2,5,8-triazaspiro[3.5]nonane-5-carboxylate;
Example 28: (*rel*-3*S*,4*R*)-N-(2-((*S*)-4-((3-Cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)-1-((*E*)-4-(dimethylamino)but-2-enoyl)-4-methoxy-N-methylpyrrolidine-3-carboxamide;
Example 29: (*S,E*)-*N*-(2-(4-((3-Cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)-1-(4-(dimethylamino)but-2-enoyl)-*N*-methylazetidine-3-carboxamide;
Example 30: (*R*)-*N*-(2-((*S*)-4-((3-Cyano-6-methyl-4-(trifluoromethyl)pyridin-2-yl)amino)-6-methyl-5-oxo-3,4,5,6-tetrahydrobenzo[*b*][1,4]diazocin-1(2*H*)-yl)ethyl)-1-((*E*)-4-(dimethylamino)but-2-enoyl)-*N*-methylpyrrolidine-3-carboxamide;
Example 31: (*S*,*E*)-*N*-(2-(4-((3-Cyano-6-methyl-4-(trifluoromethyl)pyridin-2-yl)amino)-6-methyl-5-oxo-3,4,5,6-tetrahydrobenzo[*b*][1,4]diazocin-1(2*H*)-yl)ethyl)-1-(4-(dimethylamino)but-2-enoyl)-*N*-methylazetidine-3-carboxamide;
Example 32A: 2-(((*S*)-6-((*S*/*R*)-3-(4-((*E*)-4-(Dimethylamino)but-2-enoyl)piperazin-1-yl)-2-hydroxypropyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 32B: 2-(((*S*)-6-((*R*/*S*)-3-(4-((*E*)-4-(Dimethylamino)but-2-enoyl)piperazin-1-yl)-2-hydroxypropyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 33A: 2-(((*S*)-6-((*S*/*R*)-3-(4-Acryloylpiperazin-1-yl)-2-hydroxypropyl)-8-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 33B: 2-(((*S*)-6-((*R*/*S*)-3-(4-Acryloylpiperazin-1-yl)-2-hydroxypropyl)-8-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 34A: 2-(((*S*)-6-((*S*/*R*)-3-(4-((*E*)-4-(Dimethylamino)but-2-enoyl)piperazin-1-yl)-2-hydroxypropyl)-8-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 34B: 2-(((*S*)-6-((*R*/*S*)-3-(4-((*E*)-4-(Dimethylamino)but-2-enoyl)piperazin-1-yl)-2-hydroxypropyl)-8-fluoro-1-methyl-2-oxo-1,2,3,4,5,6-hexahydrobenzo[*b*][1,4]diazocin-3-yl)amino)-6-methyl-4-(trifluoromethyl)nicotinonitrile;

Example 35: 2-(((3*S*)-6-(3-(4-Acryloylpiperazin-1-yl)-2-hydroxypropyl)-1-methyl-2-oxo-1,2,3,4,5,6-hexahydrobenzo[*b*][1,4]diazocin-3-yl)amino)-6-methyl-4-(trifluoromethyl)nicotinonitrile;

Example 36: 2-(((3*S*)-6-(3-(6-Acryloyl-2,6-diazaspiro[3.3]heptan-2-yl)-2-hydroxypropyl)-1-methyl-2-oxo-1,2,3,4,5,6-hexahydrobenzo[*b*][1,4]diazocin-3-yl)amino)-6-methyl-4-(trifluoromethyl)nicotinonitrile;

Example 37: (*S*)-2-((6-(3-(4-Acryloylpiperazin-1-yl)propyl)-7-fluoro-1-methyl-2-oxo-1,2,3,4,5,6-hexahydrobenzo[b][1,4]diazocin-3-yl)amino)-6-methyl-4-(trifluoromethyl)nicotinonitrile;

Example 38: (*S*)-2-((6-(3-(4-Acryloylpiperazin-1-yl)propyl)-8-fluoro-1-methyl-2-oxo-1,2,3,4,5,6-hexahydrobenzo[*b*][1,4]diazocin-3-yl)amino)-6-methyl-4-(trifluoromethyl)nicotinonitrile;

Example 39: (*S,E*)-2-((6-(3-(4-(4-(Dimethylamino)but-2-enoyl)piperazin-1-yl)propyl)-8-fluoro-1-methyl-2-oxo-1,2,3,4,5,6-hexahydrobenzo[*b*][1,4]diazocin-3-yl)amino)-6-methyl-4-(trifluoromethyl)nicotinonitrile;

Example 40: (*S,E*)-2-((6-(3-((1-(4-(Dimethylamino)but-2-enoyl)azetidin-3-yl)oxy)propyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;

Example 41: (S, E)-2-((6-(3-((1-(4-(Dimethylamino)but-2-enoyl)azetidin-3-yl)oxy)propyl)-1-methyl-2-oxo-1,2,3,4,5,6-hexahydrobenzo[b][1,4]diazocin-3-yl)amino)-6-methyl-4-(trifluoromethyl)nicotinonitrile;

Example 42: (*S,E*)-2-((6-(3-((1-(4-(Dimethylamino)but-2-enoyl)azetidin-3-yl)oxy)propyl)-8-fluoro-1-methyl-2-oxo-1,2,3,4,5,6-hexahydrobenzo[b][1,4]diazocin-3-yl)amino)-6-methyl-4-(trifluoromethyl)nicotinonitrile;

Example 43: (*S,E*)-2-((6-(3-((1-(4-(Dimethylamino)but-2-enoyl)azetidin-3-yl)oxy)propyl)-8,10-difluoro-1-methyl-2-oxo-1,2,3,4,5,6-hexahydrobenzo[*b*][1,4]diazocin-3-yl)amino)-6-methyl-4-(trifluoromethyl)nicotinonitrile;

Example 44: (*S,E*)-2-((7-Chloro-6-(3-((1-(4-(dimethylamino)but-2-enoyl)azetidin-3-yl)oxy)propyl)-1-methyl-2-oxo-1,2,3,4,5,6-hexahydrobenzo[*b*][1,4]diazocin-3-yl)amino)-6-methyl-4-(trifluoromethyl)nicotinonitrile;

Example 45: (*S,E*)-2-((6-(2-((2-(4-(Dimethylamino)but-2-enoyl)-2-azaspiro[3.3]heptan-6-yl)oxy)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;

Example 46: (*S,E*)-2-((6-(2-(2-(4-(Dimethylamino)but-2-enoyl)-5-methyl-7-oxo-2,5,8-triazaspiro[3.5]nonan-8-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;

Example 47: 2-(((3*S*)-6-(2-(7-((E)-4-(Dimethylamino)but-2-enoyl)-1-oxo-2,7-diazaspiro[4.4]nonan-2-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;

Example 48: 2-(((*S*)-6-(2-(((*rel*-3*R*,4*R*)-1-((*E*)-4-(Dimethylamino)but-2-enoyl)-4-methoxypyrrolidin-3-yl)methoxy)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;

Example 49: (*S,E*)-2-((6-(2-((1-(4-(Dimethylamino)but-2-enoyl)piperidin-4-yl)oxy)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;

Example 50: (*S,E*)-2-((6-(2-((1-(4-(Dimethylamino)but-2-enoyl)azetidin-3-yl)oxy)ethyl)-1-methyl-2-oxo-1,2,3,4,5,6-hexahydrobenzo[*b*][1,4]diazocin-3-yl)amino)-6-methyl-4-(trifluoromethyl)nicotinonitrile;

Example 51: (*S*)-2-((6-(2-((1-Acryloylazetidin-3-yl)oxy)ethyl)-1-methyl-2-oxo-1,2,3,4,5,6-hexahydrobenzo[*b*][1,4]diazocin-3-yl)amino)-6-methyl-4-(trifluoromethyl)nicotinonitrile;

Example 52: (*S*)-2-((6-((2-Acryloyl-2-azaspiro[3.3]heptan-6-yl)methyl)-1-methyl-2-oxo-1,2,3,4,5,6-hexahydrobenzo[*b*][1,4]diazocin-3-yl)amino)-6-methyl-4-(trifluoromethyl)nicotinonitrile;

Example 53: (*S*)-2-((6-(3-((1-Acryloylazetidin-3-yl)oxy)propyl)-1-methyl-2-oxo-1,2,3,4,5,6-hexahydrobenzo[*b*][1,4]diazocin-3-yl)amino)-6-methyl-4-(trifluoromethyl)nicotinonitrile;

Example 54: 2-(((*S*)-7-Fluoro-1-methyl-6-(3-((1-((*E*)-3-((*S*)-1-methylpyrrolidin-2-yl)acryloyl)azetidin-3-yl)oxy)propyl)-2-oxo-1,2,3,4,5,6-hexahydrobenzo[b][1,4]diazocin-3-yl)amino)-6-methyl-4-(trifluoromethyl)nicotinonitrile;

Example 55: 2-(((*S*)-7-Fluoro-1-methyl-6-(3-((1-((*E*)-3-((*R*)-1-methylpyrrolidin-2-yl)acryloyl)azetidin-3-yl)oxy)propyl)-2-oxo-1,2,3,4,5,6-hexahydrobenzo[*b*][1,4]diazocin-3-yl)amino)-6-methyl-4-(trifluoromethyl)nicotinonitrile;

Example 56: (*S,E*)-2-((7-Fluoro-6-(3-((1-(4-((2-methoxyethyl)(methyl)amino)but-2-enoyl)azetidin-3-yl)oxy)propyl)-1-methyl-2-oxo-1,2,3,4,5,6-hexahydrobenzo[*b*][1,4]diazocin-3-yl)amino)-6-methyl-4-(trifluoromethyl)nicotinonitrile;

Example 57: (*S,E*)-2-((7-Fluoro-1-methyl-6-(3-((1-(4-(methyl(prop-2-yn-1-yl)amino)but-2-enoyl)azetidin-3-yl)oxy)propyl)-2-oxo-1,2,3,4,5,6-hexahydrobenzo[*b*][1,4]diazocin-3-yl)amino)-6-methyl-4-(trifluoromethyl)nicotinonitrile;

Example 58A: 2-(((*S*)-6-(((*S*/*R*)-4-(1-Acryloylazetidin-3-yl)-1-methyl-6-oxopiperazin-2-yl)methyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 58B: 2-(((*S*)-6-(((*R*/*S*)-4-(1-Acryloylazetidin-3-yl)-1-methyl-6-oxopiperazin-2-yl)methyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 59: 2-(((*S*)-6-(((*S*)-4-(1-((*E*)-4-(Dimethylamino)but-2-enoyl)azetidin-3-yl)morpholin-2-yl)methyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 60: 2-(((*S*)-6-(((*R*)-4-(1-((*E*)-4-(Dimethylamino)but-2-enoyl)azetidin-3-yl)morpholin-2-yl)methyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 61: 2-(((*S*)-6-(2-((*R*)-1-Acryloyltetrahydro-1'*H*-spiro[azetidine-3,6'-pyrazino[2,1-*c*][1,4]oxazin]-8'(7'*H*)-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 62: 2-(((*S*)-6-(2-((*S*)-1-Acryloyltetrahydro-1'*H*-spiro[azetidine-3,6'-pyrazino[2,1-*c*][1,4]oxazin]-8'(7'*H*)-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 63: 2-(((*S*)-6-(2-((*R*)-2-Acryloyl-6-methyl-2,5,8-triazaspiro[3.5]nonan-8-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 64: 2-(((*S*)-6-(2-((*R*)-2-Acryloyl-6-methyl-2,5,8-triazaspiro[3.5]nonan-8-yl)ethyl)-7-chloro-1-methyl-2-oxo-1,2,3,4,5,6-hexahydrobenzo[*b*][1,4]diazocin-3-yl)amino)-6-methyl-4-(trifluoromethyl)nicotinonitrile;
Example 65A: 2-(((*S*)-6-(2-((3*S*/*R*,4*S*/*R*)-3-((1-Acryloylazetidin-3-yl)oxy)-4-hydroxypyrrolidin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 65B: 2-(((*S*)-6-(2-((3*R*/*S*,4*R*/*S*)-3-((1-acryloylazetidin-3-yl)oxy)-4-hydroxypyrrolidin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 66: (*S*)-2-((6-((1-(1-Acryloylazetidin-3-yl)-1*H*-1,2,4-triazol-3-yl)methyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 67: (*S*)-2-((6-((3-(1-Acryloylazetidin-3-yl)-1,2,4-oxadiazol-5-yl)methyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 68: (*S*)-2-((6-((5-(1-Acryloylazetidin-3-yl)-4H-1,2,4-triazol-3-yl)methyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 69: (*S*)-2-((6-(2-(2-Acryloyl-2,5,8-triazaspiro[3.5]nonan-8-yl)ethyl)-1,7-dimethyl-2-oxo-1,2,3,4,5,6-hexahydrobenzo[*b*][1,4]diazocin-3-yl)amino)-6-methyl-4-(trifluoromethyl)nicotinonitrile;
Example 70: (*S*)-2-((6-(2-(2-Acryloyl-2,5,8-triazaspiro[3.5]nonan-8-yl)ethyl)-1-ethyl-7-fluoro-2-oxo-1,2,3,4,5,6-hexahydrobenzo[*b*][1,4]diazocin-3-yl)amino)-6-methyl-4-(trifluoromethyl)nicotinonitrile;
Example 71: (*S*)-2-((6-(2-(5-Acetyl-2-acryloyl-2,5,8-triazaspiro[3.5]nonan-8-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
Example 72A: 2-(((*S*)-6-(2-((*R*/*S*)-8-Acryloyloctahydro-2*H*-pyrazino[1,2-*a*]pyrazin-2-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile; and
Example 72B: 2-(((*S*)-6-(2-((*S*/*R*)-8-acryloyloctahydro-2*H*-pyrazino[1,2-*a*]pyrazin-2-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitrile;
or a pharmaceutically acceptable salt or solvate thereof.

**13.** A pharmaceutical composition comprising a compound of formula (I) as defined in any of claims 1 to 12, optionally in combination with one or more therapeutic agents.

**14.** A compound as defined in any of claims 1 to 12 for use in:

(a) therapy; or
(b) the prophylaxis or treatment of cancer.

**15.** A process for preparing a compound of formula (I) as defined in claim 1 which comprises:

(a) reacting a compound of formula (II):

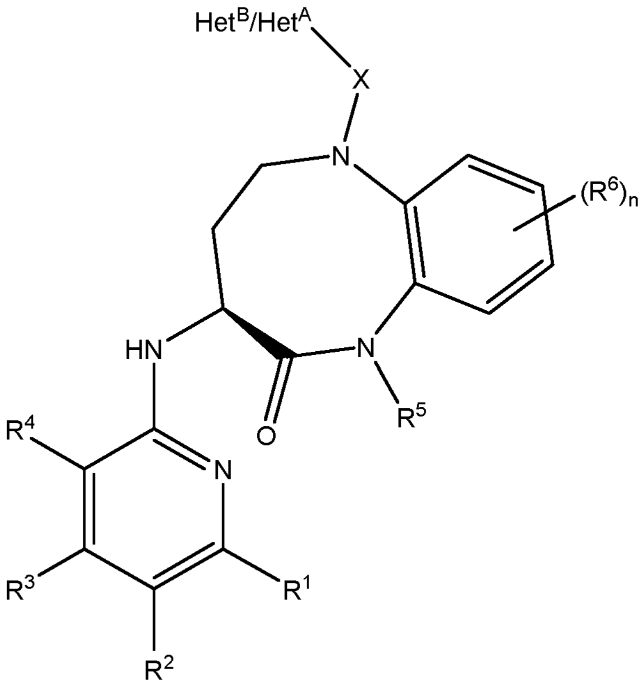

(II)

wherein $R^1$, $R^2$, $R^3$, $R^4$, $R^5$, $R^6$, n, X, $Het^A$ and $Het^B$ are as defined in claim 1, with a compound of formula -CO-CH=CHR$^9$ or -Y-Het$^C$-CO-CH=CHR$^9$, wherein Y, $Het^C$ and $R^9$ are as defined in claim 1;

(b) reacting a compound of formula (III):

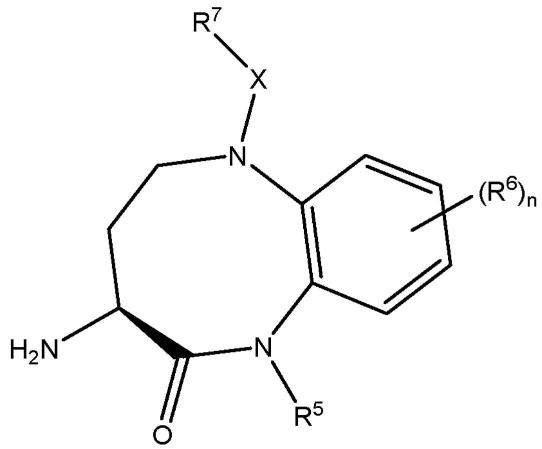

(III)

wherein $R^5$, $R^6$, n, X and $R^7$ are as defined in claim 1, with a compound of formula (IV):

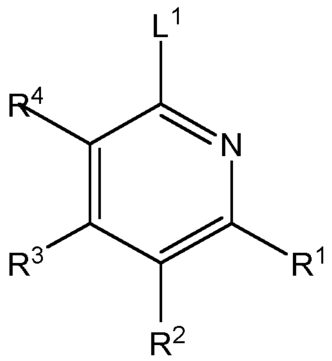

(IV)

wherein $R^1$, $R^2$, $R^3$ and $R^4$ are as defined in claim 1 and $L^1$ represents a suitable leaving group, such as a halogen atom (e.g. chlorine);
(c) deprotection of a protected derivative of a compound of formula (I);
(d) interconversion of a compound of formula (I) or protected derivative thereof to a further compound of formula (I) or protected derivative thereof; and
(e) optional formation of a pharmaceutically acceptable salt of a compound of formula (I).

**Patentansprüche**

**1.** Verbindung der Formel (I):

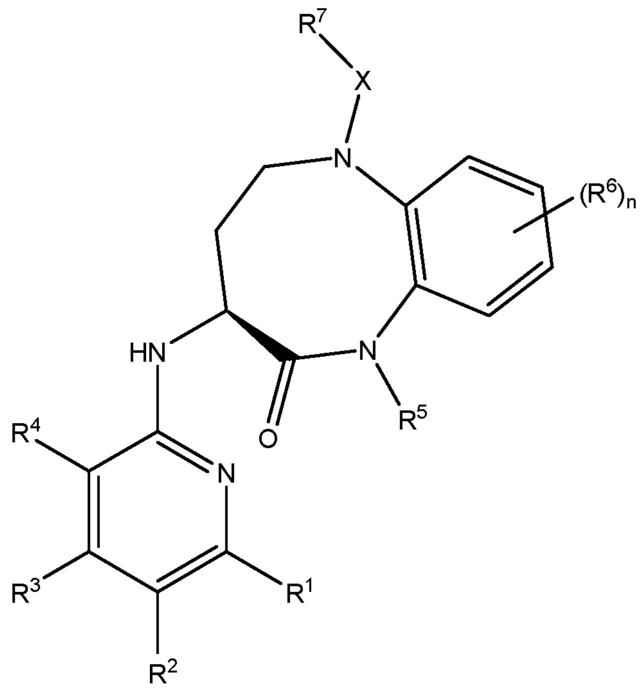

(I)

oder eine tautomere oder eine stereochemisch isomere Form, ein pharmazeutisch verträgliches Salz oder ein Solvat davon, wobei:

$R^1$, $R^2$, $R^3$ und $R^4$ unabhängig voneinander für Wasserstoff, $C_{1-6}$-Alkyl, $C_{2-6}$-Alkenyl, $C_{1-6}$-Alkoxy, Halogen, Halogen$C_{1-6}$-Alkyl, Halogen$C_{1-6}$-Alkoxy, $C_{3-8}$-Cycloalkyl, Cyano oder -$NR^xR^y$ stehen;
$R^5$ für Wasserstoff oder $C_{1-6}$-Alkyl steht;
n für eine ganze Zahl steht, ausgewählt aus 0, 1, 2 oder 3;

$R^6$ für Halogen oder $C_{1-6}$-Alkyl steht;
X für eine optional durch eine O-, OH- oder $CONR^8$-Gruppe substituierte $C_1$-$C_4$-Alkylengruppe steht;
$R^8$ für Wasserstoff oder $C_{1-6}$-Alkyl steht;
$R^7$ für eine -$Het^A$-CO-CH=$CHR^9$ oder -$Het^B$-Y-$Het^C$-CO-CH=$CHR^9$-Gruppe steht;
Y für eine Bindung oder -O- steht;
$R^9$ für Wasserstoff, -$CH_2$-$NR^xR^y$ oder -$Het^D$ steht;
$Het^A$, $Het^B$, $Het^C$ und $Het^D$ jeweils für eine Heterocyclyl- oder eine Heteroaryl-Gruppe stehen, die optional durch einen oder mehrere (z. B. 1, 2 oder 3) Substituenten substituiert ist, wie etwa $C_{1-6}$-Alkyl, $C_{2-6}$-Alkenyl, $C_{2-6}$-Alkynyl, $C_{1-6}$-Alkoxy, -COO-$C_{2-6}$-Alkynyl, -CO-$C_{1-6}$-Alkyl, Hydroxy und Oxo; und
$R^x$ und $R^y$ unabhängig voneinander für $C_{1-6}$-Alkyl, $C_{2-6}$-Alkenyl, $C_{2-6}$-Alkynyl, $C_{1-6}$-Alkoxy, oder $R^x$ und $R^y$ stehen, an die sie mit dem Stickstoffatom gebunden sind, um einen Stickstoff enthaltenden heterocyclischen Ring zu bilden, der optional durch eine oder mehrere $C_{1-6}$-Alkyl-Gruppen substituiert sein kann.

**2.** Verbindung nach Anspruch 1, wobei:

(a) $R^1$ für Folgendes steht:
Wasserstoff;

$C_{1-6}$-Alkyl (wie etwa Methyl oder Ethyl);
$C_{2-6}$-Alkenyl (wie etwa Ethenyl);$C_{1-6}$-Alkoxy (wie etwa Methoxy); Halogen (wie etwa Chlor); oder
-$NR^xR^y$ (wie etwa -$N(Me)_2$ oder -N(Me)(Et));
wie etwa $R^1$ für $C_{1-6}$-Alkyl, insbesondere Methyl, steht; und/oder

(b) $R^2$ für Folgendes steht:

Wasserstoff;
Halogen (wie etwa Chlor); oder
$C_{1-6}$-Alkyl (wie etwa Methyl);
wie etwa Wasserstoff; und/oder

(c) $R^3$ für Folgendes steht:

$C_{1-6}$-Alkyl (wie etwa Methyl, Ethyl oder Isopropyl);
$C_{2-6}$-Alkenyl (wie -C(=$CH_2$)(Me));
Halogen (wie etwa Brom);
Halogen$C_{1-6}$-Alkyl (wie etwa Trifluormethyl oder -C(H)(Me)-$CF_3$); oder
Halogen$C_{1-6}$-Alkoxy (wie etwa Difluormethoxy);
wie etwa Halogen$C_{1-6}$-Alkyl, insbesondere Trifluormethyl; und/oder

(d) $R^4$ für Cyano steht.

**3.** Verbindung nach Anspruch 1 oder Anspruch 2, wobei $R^5$ für Wasserstoff, Methyl oder Ethyl, wie etwa Wasserstoff oder Methyl, insbesondere Methyl, steht.

**4.** Verbindung nach einem der Ansprüche 1 bis 3, wobei n für eine ganze Zahl, ausgewählt aus 0, 1 oder 2, wie etwa 0, steht.

**5.** Verbindung nach einem der Ansprüche 1 bis 4, wobei $R^6$ für Fluor, Chlor oder Methyl steht, wie etwa Fluor oder Chlor, insbesondere Fluor, wie etwa wobei n für eine ganze Zahl steht, die 1 ist, und $R^6$ für Fluor steht; oder n für eine ganze Zahl steht, die 1 ist und $R^6$ für Chlor steht; oder n für eine ganze Zahl steht, die 2 ist, und beide $R^6$-Gruppen für Fluor stehen.

**6.** Eine Verbindung nach einem der Ansprüche 1 bis 5, wobei X für eine $C_1$-$C_4$-Alkylen-Gruppe (wie etwa -$CH_2$-, -$(CH_2)_2$-, -$(CH_2)_3$- oder -$(CH_2)_4$-) optional substituiert durch ein O (wie etwa - $(CH_2)_2$-O-, -$(CH_2)_2$-O-$CH_2$- oder -$(CH_2)_3$-O-), OH (wie etwa -$CH_2$-CH (OH) -$CH_2$-) oder $CONR^8$ (wie etwa - $(CH_2)_2$-N(Me) -CO-) -Gruppe, insbesondere X für -$CH_2$-, -$(CH_2)_2$-, -$(CH_2)_2$-O-, -$(CH_2)_2$-O-$CH_2$-, - $(CH_2)_3$-, - $(CH_2)_3$-O-, - $(CH_2)_2$-N(Me)-CO- oder -$CH_2$-CH(OH)-$CH_2$-steht.

**7.** Verbindung nach einem der Ansprüche 1 bis 6, wobei $R^8$ für Wasserstoff oder Methyl steht, wie etwa Methyl.

**8.** Verbindung nach einem der Ansprüche 1 bis 7, wobei $R^7$ eine Folgende ist:

(a) -$Het^A$-CO-CH=CHR $^9$-Gruppe und $Het^A$ steht für 2-Azaspiro[3.3]heptan-6-yl, Azetidinyl, 2,6-Diazaspiro[3.3]heptan-2-yl, 2,6-Diazaspiro[3.5]nonan-6-yl, 2,7-Diazaspiro[3.5]nonan-7-yl, 2,7-Diazaspiro[4.4]nonan-2-yl, 5-Oxa-2,7-Diazaspiro[3.4]octan-7-yl, 5-Oxa-2,8-diazaspiro[3.5]nonan-8-yl, 6-Oxa-2,9-diazaspiro[4.5]decan-9-yl, Piperazinyl, Piperidinyl, Pyrrolidinyl, 2,5,8-Triazaspiro[3.5]nonan-8-yl, Tetrahydro-1'*H*-spiro[azetidin-3,6'-pyrazino[2,1-*c*][1,4]oxazin]-8'(7'*H*)-yl oder Octahydro-2H-pyrazino[1,2-*a* ]pyrazin-2-yl,, optional substituiert durch einen oder mehrere (z. B. 1, 2 oder 3) Substituenten wie $C_{1-6}$-Alkyl (z. B. Methyl), Oxo, $C_{2-6}$-Alkinyl (z. B. Propinyl), -COO-$C_{2-6}$-Alkinyl (z. B. -COO-Propinyl), -CO-$C_{1-6}$-Alkyl (z. B. -CO-Me) oder $C_{1-6}$-Alkoxy (z. B. Methoxy); oder (b) -$Het^B$-Y-$Het^C$-CO-CH=CHR$^9$-Gruppe, wie etwa -Azetidinyl-Piperazinyl-CO-CH=CHR$^9$, -Morpholinyl-Azetidinyl-CO-CH=CHR$^9$, - Piperazinyl-Azetidinyl-CO-CH=CHR$^9$, -Piperidinyl-Azetidinyl-CO-CH=CHR$^9$, -Piperidinyl-O-Azetidinyl-CO-CH=CHR$^9$, -2,5-Diazabicyclo[2.2.1]Heptan-2-yl-Azetidinyl-CO-CH=CHR$^9$, - Pyrrolidinyl-O-Azetidinyl-CO-CH=CHR$^9$, -Triazolyl-Azetidinyl-CO-CH=CHR$^9$, oder -Oxadiazolyl-Azetidinyl-CO-CH=CHR$^9$, optional substituiert durch eine oder mehrere (z. B. 1, 2 oder 3) Substituenten, wie etwa $C_{1-6}$-Alkyl (z. B. Methyl), Hydroxy oder Oxo.

**9.** Verbindung nach einem der Ansprüche 1 bis 8, wobei $R^9$ für Wasserstoff; oder -$CH_2$-$NR^xR^y$ steht (wie etwa -$CH_2$-$N(Me)_2$, -$CH_2$-N(Me)(-$(CH_2)_2$-OMe) oder -$CH_2$-N(Me)(-$CH_2$-C≡CH); oder -$Het^D$ (wie etwa monocyclisches Heterocyclyl, insbesondere Pyrrolidinyl, speziell C-gebundenes Pyrrolidinyl (z. B. Pyrrolidin-2-yl) steht, optional substituiert durch einen oder mehrere (z. B. 1, 2 oder 3) Substituenten, wie etwa $C_{1-6}$-Alkyl (z. B. Methyl)).

**10.** Verbindung nach einem der Ansprüche 1 bis 9, wobei $R^x$ und $R^y$ unabhängig voneinander für Wasserstoff stehen, $C_{1-6}$-Alkyl (wie etwa Methyl), $C_{2-6}$-Alkynyl (wie etwa -$CH_2$-C≡CH) oder $C_{1-6}$-Alkoxy (wie etwa -$(CH_2)_2$-OMe); wie etwa beide von $R^x$ und $R^y$für $C_{1-6}$-Alkyl stehen (wie etwa Methyl); oder eines von $R^x$ und $R^y$ für $C_{1-6}$-Alkyl steht (wie etwa Methyl) und das andere für $C_{2-6}$-Alkynyl steht (wie etwa -$CH_2$-C≡CH) oder $C_{1-6}$-Alkoxy (wie etwa -OMe) .

**11.** Verbindung nach einem der Ansprüche 1 bis 10, die eine Verbindung der Formel $(I)^a$ ist:

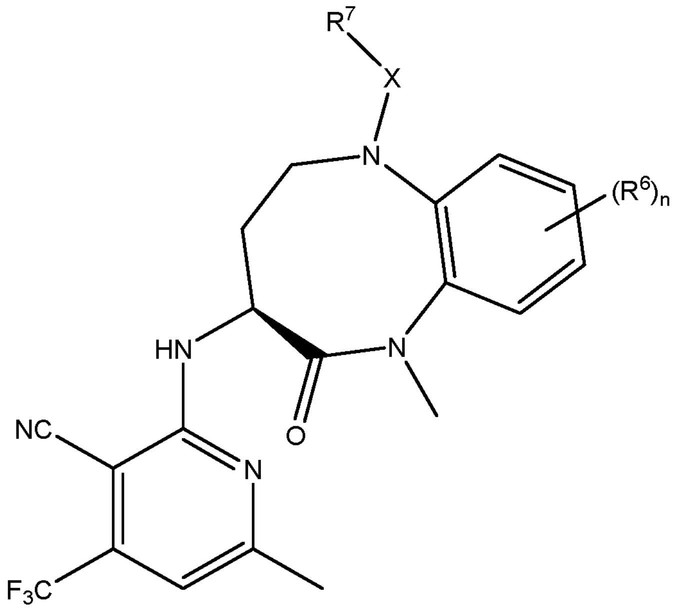

(I) a

oder eine tautomere oder eine stereochemisch isomere Form, ein pharmazeutisch verträgliches Salz oder ein Solvat davon, wobei $R^6$, n, X und $R^7$ nach einem der Ansprüche 1 und 4 bis 10 definiert sind.

**12.** Verbindung nach Anspruch 1, wobei die Verbindung die freie Base einer Verbindung der Beispiele 1-72 ist:

Beispiel 1: (*S*,*E*)-2-((6-(2-(2-(4-(Dimethylamino)but-2-enoyl)-5-oxa-2,8-diazaspiro[3.5]nonan-8-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 2: 2-(((*S*)-6-(2-(2-((*E*)-4-(Dimethylamino)but-2-enoyl)-6-oxa-2,9-diazaspiro[4.5]decan-9-yl)ethyl)-7-fluoro-1-methyl-2-oxo-1,2,3,4,5,6-hexahydrobenzo[b][1,4]diazocin-3-yl)amino)-6-methyl-4-(trifluoromethyl)nicotinonitril;

Beispiel 3: (S,E)-2-((6-(2-(2-(4-(Dimethylamino)but-2-enoyl)-5-oxa-2,8-diazaspiro[3.5]nonan-8-yl)ethyl)-1-methyl-2-oxo-1,2,3,4,5,6-hexahydrobenzo[b][1,4]diazocin-3-yl)amino)-6-methyl-4-(trifluoromethyl)nicotinonitril;

Beispiel 4: (S,E)-2-((6-(2-(6-(4-(Dimethylamino)but-2-enoyl)-2,6-diazaspiro[3.3]heptan-2-yl)ethyl)-1-methyl-2-oxo-1,2,3,4,5,6-hexahydrobenzo[b][1,4]diazocin-3-yl)amino)-6-methyl-4-(trifluoromethyl)nicotinonitril;

Beispiel 5: (S,E)-2-((6-(2-(4-(4-(Dimethylamino)but-2-enoyl)piperazin-1-yl)ethyl)-1-methyl-2-oxo-1,2,3,4,5,6-hexahydrobenzo[b][1,4]diazocin-3-yl)amino)-6-methyl-4-(trifluoromethyl)nicotinonitril;

Beispiel 6: (*S*)-2-((6-(2-(2-Acryloyl-5-methyl-2,5,8-triazaspiro[3.5]nonan-8-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 7: (*S*)-2-((6-(2-(6-Acryloyl-2,6-diazaspiro[3.3]heptan-2-yl)ethyl)-8-chloro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 8: (*S*)-2-((6-(2-(2-Acryloyl-2,7-diazaspiro[3.5]nonan-7-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 9: (*S*)-2-((6-(2-(2-Acryloyl-2,6-diazaspiro[3.5]nonan-6-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 10: (*S*)-2-((6-(2-(3-(4-Acryloylpiperazin-1-yl)azetidin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 11: (*S*)-2-((6-(2-(4-(1-Acryloylazetidin-3-yl)piperazin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 12: (*S*)-2-((6-(2-(4-(1-Acryloyl-3-hydroxyazetidin-3-yl)piperidin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 13: (*S*)-2-((6-(2-(4-((1-Acryloylazetidin-3-yl)oxy)piperidin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 14: 2-(((*S*)-6-(2-((*S*)-4-(1-Acryloylazetidin-3-yl)-2-methylpiperazin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 15: 2-(((*S*)-6-(2-((*R*)-4-(1-Acryloylazetidin-3-yl)-2-methylpiperazin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 16: 2-(((*S*)-6-(2-((*R*)-4-(1-Acryloylazetidin-3-yl)-3-methylpiperazin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 17: 2-(((*S*)-6-(2-((*S*)-4-(1-Acryloylazetidin-3-yl)-3-methylpiperazin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 18: 2-(((*S*)-6-(2-((1*R*,4*R*)-5-(1-Acryloylazetidin-3-yl)-2,5-diazabicyclo[2.2.1]heptan-2-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 19: 2-(((*S*)-6-(2-((1*S*,4*S*)-5-(1-Acryloylazetidin-3-yl)-2,5-diazabicyclo[2.2.1]heptan-2-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 20: *(S)*-2-((6-(2-(4-(1-Acryloylazetidin-3-yl)piperazin-1-yl)ethyl)-1-methyl-2-oxo-1,2,3,4,5,6-hexahydrobenzo[b][1,4]diazocin-3-yl)amino)-6-methyl-4-(trifluoromethyl)nicotinonitril;

Beispiel 21: *(S)*-2-((6-(2-(2-Acryloyl-5-oxa-2,8-diazaspiro[3.5]nonan-8-yl)ethyl)-1-methyl-2-oxo-1,2,3,4,5,6-hexahydrobenzo[b][1,4]diazocin-3-yl)amino)-6-methyl-4-(trifluoromethyl)nicotinonitril;

Beispiel 22: *(S)*-2-((6-(2-(6-Acryloyl-2,6-diazaspiro[3.3]heptan-2-yl)ethyl)-1-methyl-2-oxo-1,2,3,4,5,6-hexahydrobenzo[b][1,4]diazocin-3-yl)amino)-6-methyl-4-(trifluoromethyl)nicotinonitril;

Beispiel 23: *(S)*-2-((6-(2-(2-Acryloyl-2,5,8-triazaspiro[3.5]nonan-8-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 24: (*S*,*E*)-2-((6-(2-(2-(4-(Dimethylamino)but-2-enoyl)-6-oxo-5-oxa-2,7-diazaspiro[3.4]octan-7-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 25: (*S*)-2-((6-(2-(2-Acryloyl-5-(prop-2-yn-1-yl)-2,5,8-triazaspiro[3.5]nonan-8-yl)ethyl)-7-fluoro-1-methyl-2-oxo-1,2,3,4,5,6-hexahydrobenzo[b][1,4]diazocin-3-yl)amino)-6-methyl-4-(trifluoromethyl)nicotinonitril;

Beispiel 26: (*S*,*E*)-2-((6-(2-(2-(4-(Dimethylamino)but-2-enoyl)-5-(prop-2-yn-1-yl)-2,5,8-triazaspiro[3.5]nonan-8-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 27: (*S*,*E*)-Prop-2-yn-1-yl 8-(2-(4-((3-cyano-6-methyl-4-(trifluoromethyl)pyridin-2-yl)amino)-10-fluoro-6-methyl-5-oxo-3,4,5,6-tetrahydrobenzo[b][1,4]diazocin-1(2H)-yl)ethyl)-2-(4-(dimethylamino)but-2-enoyl)-2,5,8-triazaspiro[3.5]nonane-5-carboxylat;

Beispiel 28: (rel-3*S*,4*R*)-N-(2-((S)-4-((3-Cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2H)-yl)ethyl)-1-((*E*)-4-(dimethylamino)but-2-enoyl)-4-methoxy-N-methylpyrrolidine-3-carboxamid;

Beispiel 29: (*S*,*E*)-*N*-(2-(4-((3-Cyano-6-methyl-4-(trifluoromethyl)pyridin-2-yl)amino)-10-fluoro-6-methyl-5-oxo-3,4,5,6-tetrahydrobenzo[*b*][1,4]diazocin-1(2*H*)-yl)ethyl)-1-(4-(dimethylamino)but-2-enoyl)-*N*-methylazetidine-3-carboxamid;

Beispiel 30: (*R*)-*N*-(2-((S)-4-((3-Cyano-6-methyl-4-(trifluoromethyl)pyridin-2-yl)amino)-6-methyl-5-oxo-3,4,5,6-tetrahydrobenzo[*b*][1,4]diazocin-1(2*H*)-yl)ethyl)-1-((*E*)-4-(dimethylamino)but-2-enoyl)-*N*-methylpyrrolidine-3-carboxamid;

Beispiel 31: (*S*,*E*)-*N*-(2-(4-((3-Cyano-6-methyl-4-(trifluoromethyl)pyridin-2-yl)amino)-6-methyl-5-oxo-3,4,5,6-tetrahydrobenzo[*b*][1,4]diazocin-1(2*H*)-yl)ethyl)-1-(4-(dimethylamino)but-2-enoyl)-*N*-methylazetidine-3-carboxamid;

Beispiel 32A: 2-(((*S*)-6-((*S*/*R*)-3-(4-((*E*)-4-(Dimethylamino)but-2-enoyl)piperazin-1-yl)-2-hydroxypropyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 32B: 2-(((*S*)-6-((*R*/*S*)-3-(4-((*E*)-4-(Dimethylamino)but-2-enoyl)piperazin-1-yl)-2-hydroxypropyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 33A: 2-(((*S*)-6-((*S*/*R*)-3-(4-Acryloylpiperazin-1-yl)-2-hydroxypropyl)-8-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 33B: 2-(((*S*)-6-((*R*/*S*)-3-(4-Acryloylpiperazin-1-yl)-2-hydroxypropyl)-8-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 34A: 2-(((*S*)-6-((*S*/*R*)-3-(4-((*E*)-4-(Dimethylamino)but-2-enoyl)piperazin-1-yl)-2-hydroxypropyl)-8-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 34B: 2-(((*S*)-6-((*R*/*S*)-3-(4-((*E*)-4-(Dimethylamino)but-2-enoyl)piperazin-1-yl)-2-hydroxypropyl)-8-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 35: 2-(((3*S*)-6-(3-(4-Acryloylpiperazin-1-yl)-2-hydroxypropyl)-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 36: 2-(((3*S*)-6-(3-(6-Acryloyl-2,6-diazaspiro[3.3]heptan-2-yl)-2-hydroxypropyl)-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 37: (*S*)-2-((6-(3-(4-Acryloylpiperazin-1-yl)propyl)-7-fluoro-1-methyl-2-oxo-1,2,3,4,5,6-hexahydrobenzo[b][1,4]diazocin-3-yl)amino)-6-methyl-4-(trifluoromethyl)nicotinonitril;

Beispiel 38: (*S*)-2-((6-(3-(4-Acryloylpiperazin-1-yl)propyl)-8-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 39: (*S*,*E*)-2-((6-(3-(4-(4-(Dimethylamino)but-2-enoyl)piperazin-1-yl)propyl)-8-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 40: (*S*,*E*)-2-((6-(3-((1-(4-(Dimethylamino)but-2-enoyl)azetidin-3-yl)oxy)propyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 41: (S, E)-2-((6-(3-((1-(4-(Dimethylamino)but-2-enoyl)azetidin-3-yl)oxy)propyl)-1-methyl-2-oxo-1,2,3,4,5,6-hexahydrobenzo[b][1,4]diazocin-3-yl)amino)-6-methyl-4-(trifluoromethyl)nicotinonitril;

Beispiel 42: (*S*,*E*)-2-((6-(3-((1-(4-(Dimethylamino)but-2-enoyl)azetidin-3-yl)oxy)propyl)-8-fluoro-1-methyl-2-oxo-1,2,3,4,5,6-hexahydrobenzo[b][1,4]diazocin-3-yl)amino)-6-methyl-4-(trifluoromethyl)nicotinonitril;

Beispiel 43: (*S*,*E*)-2-((6-(3-((1-(4-(Dimethylamino)but-2-enoyl)azetidin-3-yl)oxy)propyl)-8,10-difluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 44: (*S*,*E*)-2-((7-Chloro-6-(3-((1-(4-(dimethylamino)but-2-enoyl)azetidin-3-yl)oxy)propyl)-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 45: (*S*,*E*)-2-((6-(2-((2-(4-(Dimethylamino)but-2-enoyl)-2-azaspiro[3.3]heptan-6-yl)oxy)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 46: (*S*,*E*)-2-((6-(2-(2-(4-(Dimethylamino)but-2-enoyl)-5-methyl-7-oxo-2,5,8-triazaspiro[3.5]nonan-8-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 47: 2-(((3*S*)-6-(2-(7-((E)-4-(Dimethylamino)but-2-enoyl)-1-oxo-2,7-diazaspiro[4.4]nonan-2-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 48: 2-(((*S*)-6-(2-(((rel-3*R*,4*R*)-1-((*E*)-4-(Dimethylamino)but-2-enoyl)-4-methoxypyrrolidin-3-yl)methoxy)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 49: (*S*,*E*)-2-((6-(2-((1-(4-(Dimethylamino)but-2-enoyl)piperidin-4-yl)oxy)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 50: (*S*,*E*)-2-((6-(2-((1-(4-(Dimethylamino)but-2-enoyl)azetidin-3-yl)oxy)ethyl)-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 51: (*S*)-2-((6-(2-((1-Acryloylazetidin-3-yl)oxy)ethyl)-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 52: (*S*)-2-((6-((2-Acryloyl-2-azaspiro[3.3]heptan-6-yl)methyl)-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 53: (*S*)-2-((6-(3-((1-Acryloylazetidin-3-yl)oxy)propyl)-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 54: 2-(((*S*)-7-Fluoro-1-methyl-6-(3-((1-((*E*)-3-((*S*)-1-methylpyrrolidin-2-yl)acryloyl)azetidin-3-yl)oxy)propyl)-2-oxo-1,2,3,4,5,6-hexahydrobenzo[b][1,4]diazocin-3-yl)amino)-6-methyl-4-(trifluoromethyl)nicotinonitril;

Beispiel 55: 2-(((*S*)-7-Fluoro-1-methyl-6-(3-((1-((*E*)-3-((*R*)-1-methylpyrrolidin-2-yl)acryloyl)azetidin-3-yl)oxy)propyl)-2-oxo-1,2,3,4,5,6-hexahydrobenzo[*b*][1,4]diazocin-3-yl)amino)-6-methyl-4-(trifluoromethyl)nicotinonitril;

Beispiel 56: (*S*,*E*)-2-((7-Fluoro-6-(3-((1-(4-((2-methoxyethyl)(methyl)amino)but-2-enoyl)azetidin-3-yl)oxy)propyl)-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 57: (*S*,*E*)-2-((7-Fluoro-1-methyl-6-(3-((1-(4-(methyl(prop-2-yn-1-yl)amino)but-2-enoyl)azetidin-3-yl)oxy)propyl)-2-oxo-1,2,3,4,5,6-hexahydrobenzo[*b*][1,4]diazocin-3-yl)amino)-6-methyl-4-(trifluoromethyl)nicotinonitril;

Beispiel 58A: 2-(((*S*)-6-(((*S*/*R*)-4-(1-Acryloylazetidin-3-yl)-1-methyl-6-oxopiperazin-2-yl)methyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 58B: 2-(((*S*)-6-(((*R*/*S*)-4-(1-Acryloylazetidin-3-yl)-1-methyl-6-oxopiperazin-2-yl)methyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 59: 2-(((*S*)-6-(((*S*)-4-(1-((*E*)-4-(Dimethylamino)but-2-enoyl)azetidin-3-yl)morpholin-2-yl)methyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 60: 2-(((*S*)-6-(((*R*)-4-(1-((*E*)-4-(Dimethylamino)but-2-enoyl)azetidin-3-yl)morpholin-2-yl)methyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 61: 2-(((*S*)-6-(2-((*R*)-1-Acryloyltetrahydro-1'*H*-spiro[azetidine-3,6'-pyrazino[2,1-*c*][1,4]oxazin]-8'(7'*H*)-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 62: 2-(((*S*)-6-(2-((*S*)-1-Acryloyltetrahydro-1'*H*-spiro[azetidine-3,6'-pyrazino[2,1-*c*][1,4]oxazin]-8'(7'*H*)-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 63: 2-(((*S*)-6-(2-((*R*)-2-Acryloyl-6-methyl-2,5,8-triazaspiro[3.5]nonan-8-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 64: 2-(((*S*)-6-(2-((*R*)-2-Acryloyl-6-methyl-2,5,8-triazaspiro[3.5]nonan-8-yl)ethyl)-7-chloro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 65A: 2-(((*S*)-6-(2-((3*S*/*R*,4*S*/*R*)-3-((1-Acryloylazetidin-3-yl)oxy)-4-hydroxypyrrolidin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 65B: 2-(((*S*)-6-(2-((3*R*/*S*,4*R*/*S*)-3-((1-acryloylazetidin-3-yl)oxy)-4-hydroxypyrrolidin-1-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 66: (*S*)-2-((6-((1-(1-Acryloylazetidin-3-yl)-1*H*-1,2,4-triazol-3-yl)methyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 67: (*S*)-2-((6-((3-(1-Acryloylazetidin-3-yl)-1,2,4-oxadiazol-5-yl)methyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;

Beispiel 68: (*S*)-2-((6-((5-(1-Acryloylazetidin-3-yl)-4*H*-1,2,4-triazol-3-yl)methyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;
Beispiel 69: (*S*)-2-((6-(2-(2-Acryloyl-2,5,8-triazaspiro[3.5]nonan-8-yl)ethyl)-1,7-dimethyl-2-oxo-1,2,3,4,5,6-hexahydrobenzo[*b*][1,4]diazocin-3-yl)amino)-6-methyl-4-(trifluoromethyl)nicotinonitril;
Beispiel 70: (*S*)-2-((6-(2-(2-Acryloyl-2,5,8-triazaspiro[3.5]nonan-8-yl)ethyl)-1-ethyl-7-fluoro-2-oxo-1,2,3,4,5,6-hexahydrobenzo[*b*][1,4]diazocin-3-yl)amino)-6-methyl-4-(trifluoromethyl)nicotinonitril;
Beispiel 71: (*S*)-2-((6-(2-(5-Acetyl-2-acryloyl-2,5,8-triazaspiro[3.5]nonan-8-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;
Beispiel 72A: 2-(((*S*)-6-(2-((*R*/*S*)-8-Acryloyloctahydro-2*H*-pyrazino[1,2-*a*]pyrazin-2-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril; und
Beispiel 72B: 2-(((*S*)-6-(2-((*S*/*R*)-8-acryloyloctahydro-2*H*-pyrazino[1,2-*a*]pyrazin-2-yl)ethyl)-7-fluoro-1-methyl-2-oxo-1,2,3,4,5,6-hexahydrobenzo[*b*][1,4]diazocin-3-yl)amino)-6-methyl-4-(trifluoromethyl)nicotinonitril;
oder ein pharmazeutisch verträgliches Salz oder Solvat davon.

**13.** Pharmazeutische Zusammensetzung, umfassend eine Verbindung der Formel (I) nach einem der Ansprüche 1 bis 12, optional in Kombination mit einem oder mehreren therapeutischen Mitteln.

**14.** Verbindung nach einem der Ansprüche 1 bis 12 zur Verwendung bei Folgendem:

(a) Therapie; oder
(b) der Prophylaxe oder Behandlung von Krebs.

**15.** Prozess zum Herstellen einer Verbindung der Formel (I) nach Anspruch 1, der Folgendes umfasst:

(a) Umsetzen einer Verbindung der Formel (II):

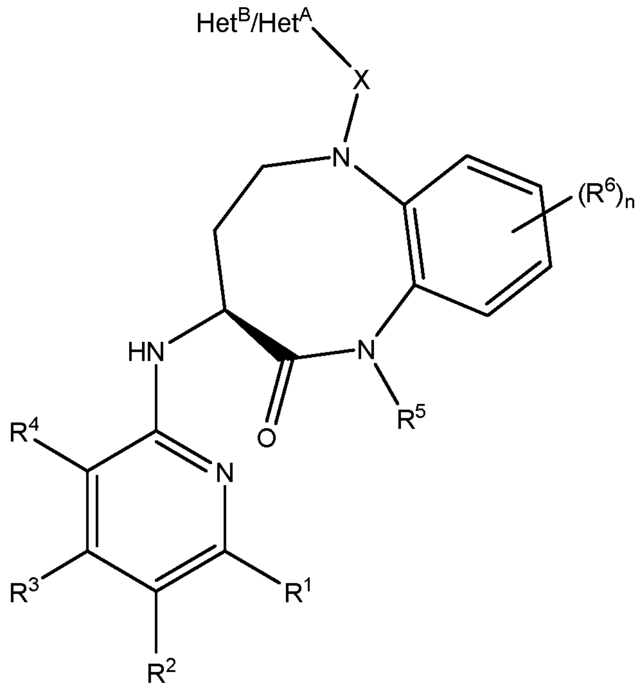

(II)

wobei $R^1$, $R^2$, $R^3$, $R^4$, $R^5$, $R^6$, n, X, $Het^A$ und $Het^B$ nach Anspruch 1 definiert sind, mit einer Verbindung der Formel $-CO-CH=CHR^9$ oder $-Y-Het^C-CO-CH=CHR^9$, wobei Y, $Het^C$ und $R^9$ nach Anspruch 1 definiert sind;
(b) Umsetzen einer Verbindung der Formel (III):

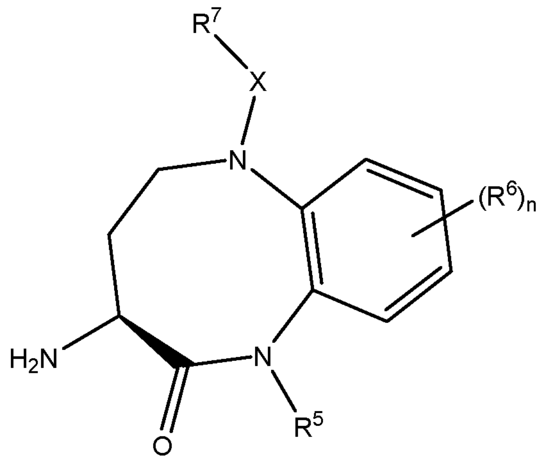

(III)

wobei $R^5$, $R^6$, n, X und $R^7$ nach Anspruch 1 definiert sind, mit einer Verbindung der Formel (IV):

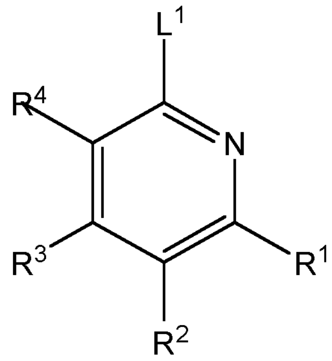

(IV)

wobei $R^1$, $R^2$, $R^3$ und $R^4$ nach Anspruch 1 definiert sind und $L^1$ für eine geeignete Abgangsgruppe, wie etwa ein Halogenatom (z. B. Chlor), steht;

(c) Entschützung eines geschützten Derivats einer Verbindung der Formel (I);
(d) Umwandlung einer Verbindung der Formel (I) oder eines geschützten Derivats davon in eine weitere Verbindung der Formel (I) oder ein geschütztes Derivat davon; und
(e) optionale Bildung eines pharmazeutisch verträglichen Salzes einer Verbindung der Formel (I).

**Revendications**

**1.** Composé de formule (I) :

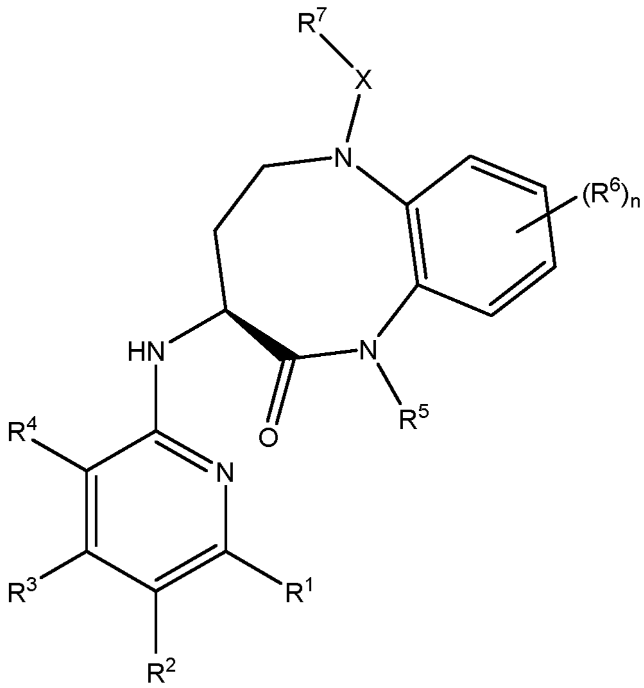

(I)

ou une forme tautomère ou stéréochimiquement isomère, un sel ou un solvate pharmaceutiquement acceptable de celui-ci, dans lequel :

$R^1$, $R^2$, $R^3$ et $R^4$ représentent indépendamment hydrogène, alkyle en $C_{1-6}$, alcényle en $C_{2-6}$, alcoxy en $C_{1-6}$, halogène, haloalkyle en $C_{1-6}$, haloalcoxy en $C_{1-6}$, cycloalkyle en $C_{3-8}$, cyano ou $-NR^xR^y$ ;
$R^5$ représente hydrogène ou alkyle en $C_{1-6}$ ;
n représente un entier choisi parmi 0, 1, 2 ou 3 ;
$R^6$ représente halogène ou alkyle en $C_{1-6}$ ;
X représente un groupe alkylène en $C_1$-$C_4$ éventuellement substitué par un groupe O, OH ou $CONR^8$ ;
$R^8$ représente hydrogène ou alkyle en $C_{1-6}$ ;
$R^7$ représente un groupe $-Het^A-CO-CH=CHR^9$ ou $-Het^B-Y-Het^C-CO-CH=CHR^9$ ;
Y représente une liaison ou -O- ;
$R^9$ représente hydrogène, $-CH_2-NR^xR^y$ ou $-Het^D$ ;
$Het^A$, $Het^B$, $Het^C$ et $Het^D$ représentent chacun un groupe hétérocyclyle ou hétéroaryle éventuellement substitué par un ou plusieurs substituants (par exemple 1, 2 ou 3) tels qu'alkyle en $C_{1-6}$, alcényle en $C_{2-6}$, alcynyle en $C_{2-6}$, alcoxy en $C_{1-6}$, -COO-alcynyle en $C_{2-6}$, -CO-alkyle en $C_{1-6}$, hydroxy et oxo ; et
$R^x$ et $R^y$ représentent indépendamment hydrogène, alkyle en $C_{1-6}$, alcényle en $C_{2-6}$, alcynyle en $C_{2-6}$, alcoxy en $C_{1-6}$, ou $R^x$ et $R^y$ avec l'atome d'azote auquel ils sont attachés se joignent pour former un cycle hétérocyclique contenant de l'azote qui peut être éventuellement substitué par un ou plusieurs groupes alkyle en $C_{1-6}$.

**2.** Composé selon la revendication 1, dans lequel :

(a) $R^1$ représente :

hydrogène ;
alkyle en $C_{1-6}$ (tel que méthyle ou éthyle) ;
alcényle en $C_{2-6}$ (tel qu' éthényle) ; alcoxy en $C_{1-6}$ (tel que méthoxy) ;
halogène (tel que chlore) ; ou
$-NR^xR^y$ (tel que $-N(Me)_2$ ou $-N(Me)(Et)$) ;
tel que $R^1$ représente alkyle en $C_{1-6}$, en particulier méthyle ; et/ou

(b) $R^2$ représente :

hydrogène ;
halogène (tel que chlore) ; ou
alkyle en $C_{1-6}$ (tel que méthyle) ;
tel qu'hydrogène ; et/ou

(c) $R^3$ représente :

alkyle en $C_{1-6}$ (tel que méthyle, éthyle ou isopropyle) ;
alcényle en $C_{2-6}$ (tel que $-C(=CH_2)(Me)$) ;
halogène (tel que brome) ;
haloalkyle en $C_{1-6}$ (tel que trifluorométhyle ou $-C(H)(Me)-CF_3$) ; ou
haloalcoxy en $C_{1-6}$ (tel que difluorométhoxy) ;
tel qu'haloalkyle en $C_{1-6}$, en particulier trifluorométhyle ; et/ou

(d) $R^4$ représente cyano.

**3.** Composé selon la revendication 1 ou la revendication 2, dans lequel $R^5$ représente hydrogène, méthyle ou éthyle, tel qu'hydrogène ou méthyle, en particulier méthyle.

**4.** Composé selon l'une quelconque des revendications 1 à 3, dans lequel n représente un entier choisi parmi 0, 1 ou 2, tel que 0.

**5.** Composé selon l'une quelconque des revendications 1 à 4, dans lequel $R^6$ représente fluor, chlore ou méthyle, tel que fluor ou chlore, en particulier fluor, tel que dans lequel n représente un entier qui est 1 et $R^6$ représente fluor ; ou n représente un entier qui est 1 et $R^6$ représente chlore ; ou n représente un entier qui est 2 et les deux groupes $R^6$ représentent fluor.

**6.** Composé selon l'une quelconque des revendications 1 à 5, dans lequel X représente un groupe alkylène en $C_1$-$C_4$ (tel que - $CH_2$-, $-(CH_2)_2$-, $-(CH_2)_3$- ou $-(CH_2)_4$-) éventuellement substitué par un groupe O (tel que $-(CH_2)_2$-O-, $-(CH_2)_2$-O-$CH_2$- ou $-(CH_2)_3$-O-), OH (tel que $-CH_2$-CH(OH)-$CH_2$-) ou $CONR^8$ (tel que $-(CH_2)_2$-N(Me)-CO-), en particulier X représente $-CH_2$-, $-(CH_2)_2$-, $-(CH_2)_2$-O-, $-(CH_2)_2$-O-$CH_2$-, $-(CH_2)_3$-, $-(CH_2)_3$-O-, $-(CH_2)_2$-N(Me)-CO- ou $-CH_2$-CH(OH)-$CH_2$-.

**7.** Composé selon l'une quelconque des revendications 1 à 6, dans lequel $R^8$ représente hydrogène ou méthyle, tel que méthyle.

**8.** Composé selon l'une quelconque des revendications 1 à 7, dans lequel $R^7$ est un :

(a) groupe -$Het^A$-CO-CH=$CHR^9$ et $Het^A$ représente 2-azaspiro[3.3]heptan-6-yle, azétidinyle, 2,6-diazaspiro[3.3]heptan-2-yle, 2,6-diazaspiro[3.5]nonan-6-yle, 2,7-diazaspiro[3.5]nonan-7-yle, 2,7-diazaspiro[4.4]nonan-2-yle, 5-oxa-2,7-diazaspiro[3.4]octan-7-yle, 5-oxa-2,8-diazaspiro[3.5]nonan-8-yle, 6-oxa-2,9-diazaspiro[4.5]décan-9-yle, pipérazinyle, pipéridinyle, pyrrolidinyle, 2,5,8-triazaspiro[3.5]nonan-8-yle, tétrahydro-1'*H*-spiro[azétidin-3,6'-pyrazino[2,1-*c*][1,4]oxazin]-8'(7'*H*)-yle, ou octahydro-2*H*-pyrazino[1,2-*a*]pyrazin-2-yle, éventuellement substitué par un ou plusieurs substituants (par exemple 1, 2 ou 3) tels qu'alkyle en $C_{1-6}$ (par exemple méthyle), oxo, alcynyle en $C_{2-6}$ (par exemple propynyle), -COO-alcynyle en $C_{2-6}$ (par exemple -COO-propynyle), -CO-alkyle en $C_{1-6}$ (par exemple -CO-Me), ou alcoxy en $C_{1-6}$ (par exemple méthoxy) ; ou
(b) groupe -$Het^B$-Y-$Het^C$-CO-CH=$CHR^9$, tel que -azétidinyl-pipérazinyl-CO-CH=$CHR^9$, -morpholinyl-azétidinyl-CO-CH=$CHR^9$, - pipérazinyl-azétidinyl-CO-CH=$CHR^9$, -pipéridinyl-azétidinyl-CO-CH=$CHR^9$, -pipéridinyl-O-azétidinyl-CO-CH=$CHR^9$, -2,5-diazabicyclo[2.2.1]heptan-2-yl-azétidinyl-CO-CH=$CHR^9$, - pyrrolidinyl-O-azétidinyl-CO-CH=$CHR^9$, -triazolyl-azétidinyl-CO-CH=$CHR^9$ ou -oxadiazolyl-azétidinyl-CO-CH=$CHR^9$, éventuellement substitué par un ou plusieurs substituants (par exemple 1, 2 ou 3) tels qu'alkyle en $C_{1-6}$ (par exemple méthyle), hydroxy ou oxo.

**9.** Composé selon l'une quelconque des revendications 1 à 8, dans lequel $R^9$ représente hydrogène ; ou $-CH_2-NR^xR^y$ (tel que - $CH_2$-$N(Me)_2$, $-CH_2$-N(Me)(-$(CH_2)_2$-OMe) ou $-CH_2$-N(Me)(-$CH_2$-C≡CH) ; ou -$Het^D$ (tel qu'un hétérocyclyle monocyclique, en particulier pyrrolidinyle, en particulier pyrrolidinyle lié en C (par exemple un pyrrolidin-2-yle) éventuellement substitué par un ou plusieurs (par exemple 1, 2 ou 3) substituants tels qu'alkyle en $C_{1-6}$ (par exemple méthyle)).

**10.** Composé selon l'une quelconque des revendications 1 à 9, dans lequel $R^x$ et $R^y$ représentent indépendamment hydrogène, alkyle en $C_{1-6}$ (tel que méthyle), alcynyle en $C_{2-6}$ (tel que $-CH_2-C=CH$) ou alcoxy en $C_{1-6}$ (tel que $-(CH_2)_2-OMe$) ; tel que $R^x$ et $R^y$ représentent tous deux alkyle en $C_{1-6}$ (tel que méthyle) ; ou l'un de $R^x$ et $R^y$ représente alkyle en $C_{1-6}$ (tel que méthyle) et l'autre représente alcynyle en $C_{2-6}$ (tel que $-CH_2-C\equiv CH$) ou alcoxy en $C_{1-6}$ (tel que -OMe).

**11.** Composé selon l'une quelconque des revendications 1 à 10, qui est un composé de formule $(I)^a$ :

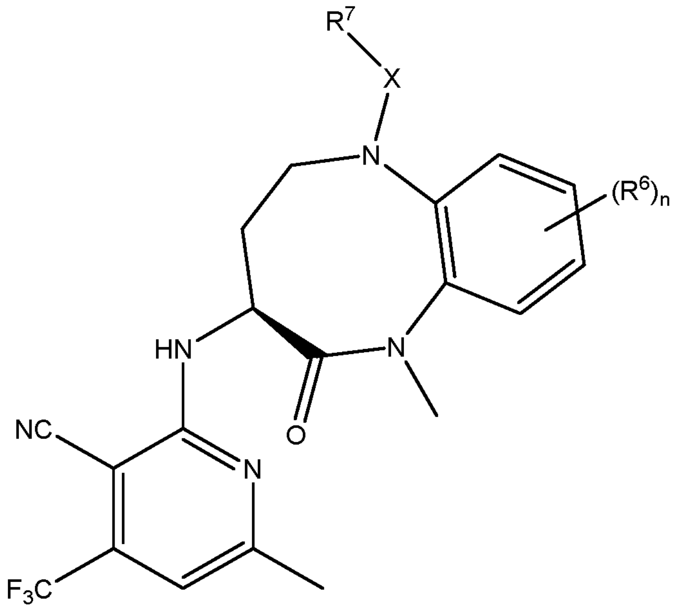

$(I)^a$

ou une forme tautomère ou stéréochimiquement isomère, un sel ou un solvate pharmaceutiquement acceptable de celui-ci, dans lequel $R^6$, n, X et $R^7$ sont tels que définis dans l'une quelconque des revendications 1 et 4 à 10.

**12.** Composé selon la revendication 1, dans lequel le composé est la base libre d'un composé des exemples 1 à 72 :

Exemple 1 : (*S,E*)-2-((6-(2-(2-(4-(diméthylamino)but-2-énoyl)-5-oxa-2,8-diazaspiro[3.5]nonan-8-yl)éthyl)-7-fluoro-1-méthyl-2-oxo-1,2,3,4,5,6-hexahydrobenzo[b][1,4]diazocin-3-yl)amino)-6-méthyl-4-(trifluorométhyl)nicotinonitrile ;
Exemple 2 : 2-(((S)-6-(2-(2-((E)-4-(diméthylamino)but-2-énoyl)-6-oxa-2,9-diazaspiro[4.5]décan-9-yl)éthyl)-7-fluoro-1-méthyl-2-oxo-1,2,3,4,5,6-hexahydrobenzo[b][1,4]diazocin-3-yl)amino)-6-méthyl-4-(trifluorométhyl)nicotinonitrile ;
Exemple 3 : (S,E)-2-((6-(2-(2-(4-(diméthylamino)but-2-énoyl)-5-oxa-2,8-diazaspiro[3.5]nonan-8-yl)éthyl)-1-méthyl-2-oxo-1,2,3,4,5,6-hexahydrobenzo[b][1,4]diazocin-3-yl)amino)-6-méthyl-4-(trifluorométhyl)nicotinonitrile ;
Exemple 4 : (S,E)-2-((6-(2-(6-(4-(diméthylamino)but-2-énoyl)-2,6-diazaspiro[3.3]heptan-2-yl)éthyl)-1-méthyl-2-oxo-1,2,3,4,5,6-hexahydrobenzo[b][1,4]diazocin-3-yl)amino)-6-méthyl-4-(trifluorométhyl)nicotinonitrile ;
Exemple 5 : (S,E)-2-((6-(2-(4-(4-(diméthylamino)but-2-énoyl)pipérazin-1-yl)éthyl)-1-méthyl-2-oxo-1,2,3,4,5,6-hexahydrobenzo[b][1,4]diazocin-3-yl)amino)-6-méthyl-4-(trifluorométhyl)nicotinonitrile ;
Exemple 6 : (*S*)-2-((6-(2-(2-acryloyl-5-méthyl-2,5,8-triazaspiro[3.5]nonan-8-yl)éthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 7 : (*S*)-2-((6-(2-(6-acryloyl-2,6-diazaspiro[3.3]heptan-2-yl)éthyl)-8-chloro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 8 : (*S*)-2-((6-(2-(2-acryloyl-2,7-diazaspiro[3.5]nonan-7-yl)éthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 9 : (*S*)-2-((6-(2-(2-acryloyl-2,6-diazaspiro[3.5]nonan-6-yl)éthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 10 : (*S*)-2-((6-(2-(3-(4-acryloylpipérazin-1-yl)azétidin-1-yl)éthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;

Exemple 11 : (*S*)-2-((6-(2-(4-(1-acryloylazétidin-3-yl)pipérazin-1-yl)éthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 12 : (*S*)-2-((6-(2-(4-(1-acryloyl-3-hydroxyazétidin-3-yl)pipéridin-1-yl)éthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 13 : (*S*)-2-((6-(2-(4-((1-acryloylazétidin-3-yl)oxy)pipéridin-1-yl)éthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 14 : 2-(((*S*)-6-(2-((*S*)-4-(1-acryloylazétidin-3-yl)-2-méthylpipérazin-1-yl)éthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 15 : 2-(((*S*)-6-(2-((*R*)-4-(1-acryloylazétidin-3-yl)-2-méthylpipérazin-1-yl)éthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 16 : 2-(((*S*)-6-(2-((*R*)-4-(1-acryloylazétidin-3-yl)-3-méthylpipérazin-1-yl)éthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 17 : 2-(((*S*)-6-(2-((*S*)-4-(1-acryloylazétidin-3-yl)-3-méthylpipérazin-1-yl)éthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 18 : 2-(((*S*)-6-(2-((1*R*,4*R*)-5-(1-acryloylazétidin-3-yl)-2,5-diazabicyclo[2.2.1]heptan-2-yl)éthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 19 : 2-(((*S*)-6-(2-((1*S*,4*S*)-5-(1-acryloylazétidin-3-yl)-2,5-diazabicyclo[2.2.1]heptan-2-yl)éthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 20 : (*S*)-2-((6-(2-(4-(1-acryloylazétidin-3-yl)pipérazin-1-yl)éthyl)-1-méthyl-2-oxo-1,2,3,4,5,6-hexahydrobenzo[b][1,4]diazocin-3-yl)amino)-6-méthyl-4-(trifluorométhyl)nicotinonitrile ;
Exemple 21 : (*S*)-2-((6-(2-(2-acryloyl-5-oxa-2,8-diazaspiro[3.5]nonan-8-yl)éthyl)-1-méthyl-2-oxo-1,2,3,4,5,6-hexahydrobenzo[b][1,4]diazocin-3-yl)amino)-6-méthyl-4-(trifluorométhyl)nicotinonitrile ;
Exemple 22 : (*S*)-2-((6-(2-(6-acryloyl-2,6-diazaspiro[3.3]heptan-2-yl)éthyl)-1-méthyl-2-oxo-1,2,3,4,5,6-hexahydrobenzo[b][1,4]diazocin-3-yl)amino)-6-méthyl-4-(trifluorométhyl)nicotinonitrile ;
Exemple 23 : (*S*)-2-((6-(2-(2-acryloyl-2,5,8-triazaspiro[3.5]nonan-8-yl)éthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 24 : (*S,E*)-2-((6-(2-(2-(4-(diméthylamino)but-2-énoyl)-6-oxo-5-oxa-2,7-diazaspiro[3.4]octan-7-yl)éthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 25 : (*S*)-2-((6-(2-(2-acryloyl-5-(prop-2-yn-1-yl)-2,5,8-triazaspiro[3.5]nonan-8-yl)éthyl)-7-fluoro-1-méthyl-2-oxo-1,2,3,4,5,6-hexahydrobenzo[b][1,4]diazocin-3-yl)amino)-6-méthyl-4-(trifluorométhyl)nicotinonitrile ;
Exemple 26 : (*S*,*E*)-2-((6-(2-(2-(4-(diméthylamino)but-2-énoyl)-5-(prop-2-yn-1-yl)-2,5,8-triazaspiro[3.5]nonan-8-yl)éthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 27 : (*S*,*E*)-prop-2-yn-1-yl 8-(2-(4-((3-cyano-6-méthyl-4-(trifluorométhyl)pyridin-2-yl)amino)-10-fluoro-6-méthyl-5-oxo-3,4,5,6-tétrahydrobenzo[b][1,4]diazocin-1(2H)-yl)éthyl)-2-(4-(diméthylamino)but-2-énoyl)-2,5,8-triazaspiro[3.5]nonane-5-carboxylate ;
Exemple 28 : (*rel*-3*S*,4*R*)-N-(2-((*S*)-4-((3-cyano-6-méthyl-4-(trifluorométhyl)pyridin-2-yl)amino)-10-fluoro-6-méthyl-5-oxo-3,4,5,6-tétrahydrobenzo[*b*][1,4]diazocin-1(2*H*)-yl)éthyl)-1-((*E*)-4-(diméthylamino)but-2-énoyl)-4-methoxy-N-méthylpyrrolidin-3-carboxamide ;
Exemple 29 : (*S,E)-N*-(2-(4-((3-cyano-6-méthyl-4-(trifluorométhyl)pyridin-2-yl)amino)-10-fluoro-6-méthyl-5-oxo-3,4,5,6-tétrahydrobenzo[*b*][1,4]diazocin-1(2*H*)-yl)éthyl)-1-(4-(diméthylamino)but-2-énoyl)-*N*-méthylazétidin-3-carboxamide ;
Exemple 30 : (*R*)-*N*-(2-((*S*)-4-((3-cyano-6-méthyl-4-(trifluorométhyl)pyridin-2-yl)amino)-6-méthyl-5-oxo-3,4,5,6-tétrahydrobenzo[*b*][1,4]diazocin-1(2*H*)-yl)éthyl)-1-((*E*)-4-(diméthylamino)but-2-énoyl)-*N*-méthylpyrrolidin-3-carboxamide ;
Exemple 31 : (*S,E*)-*N*-(2-(4-((3-cyano-6-méthyl-4-(trifluorométhyl)pyridin-2-yl)amino)-6-méthyl-5-oxo-3,4,5,6-tétrahydrobenzo[*b*][1,4]diazocin-1(2*H*)-yl)éthyl)-1-(4-(diméthylamino)but-2-énoyl)-*N*-méthylazétidin-3-carboxamide ;
Exemple 32A : 2-(((*S*)-6-((*S*/*R*)-3-(4-((*E*)-4-(diméthylamino)but-2-énoyl)pipérazin-1-yl)-2-hydroxypropyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 32B : 2-(((*S*)-6-((*R*/*S*)-3-(4-((*E*)-4-(diméthylamino)but-2-énoyl)pipérazin-1-yl)-2-hydroxypropyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 33A : 2-(((*S*)-6-((*S*/*R*)-3-(4-acryloylpipérazin-1-yl)-2-hydroxypropyl)-8-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 33B : 2-(((*S*)-6-((*R*/*S*)-3-(4-acryloylpipérazin-1-yl)-2-hydroxypropyl)-8-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 34A : 2-(((*S*)-6-((*S*/*R*)-3-(4-((E)-4-(diméthylamino)but-2-énoyl)pipérazin-1-yl)-2-hydroxypropyl)-8-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 34B : 2-(((*S*)-6-((*R*/*S*)-3-(4-((*E*)-4-(diméthylamino)but-2-énoyl)pipérazin-1-yl)-2-hydroxypropyl)-8-fluoro-1-méthyl-2-oxo-1,2,3,4,5,6-hexahydrobenzo[b][1,4]diazocin-3-yl)amino)-6-méthyl-4-(trifluorométhyl)nicotinonitrile ;
Exemple 35 : 2-(((3*S*)-6-(3-(4-acryloylpipérazin-1-yl)-2-hydroxypropyl)-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 36 : 2-(((3*S*)-6-(3-(6-Acryloyl-2,6-diazaspiro[3.3]heptan-2-yl)-2-hydroxypropyl)-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 37 : (*S*)-2-((6-(3-(4-acryloylpipérazin-1-yl)propyl)-7-fluoro-1-méthyl-2-oxo-1,2,3,4,5,6-hexahydrobenzo[b][1,4]diazocin-3-yl)amino)-6-méthyl-4-(trifluorométhyl)nicotinonitrile ;
Exemple 38 : (*S*)-2-((6-(3-(4-acryloylpipérazin-1-yl)propyl)-8-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 39 : (*S,E*)-2-((6-(3-(4-(4-(diméthylamino)but-2-énoyl)pipérazin-1-yl)propyl)-8-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 40 : (*S,E*)-2-((6-(3-((1-(4-(diméthylamino)but-2-énoyl)azétidin-3-yl)oxy)propyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 41 : (S, E)-2-((6-(3-(1-(4-(diméthylamino)but-2-énoyl)azétidin-3-yl)oxy)propyl)-1-méthyl-2-oxo-1,2,3,4,5,6-hexahydrobenzo[b][1,4]diazocin-3-yl)amino)-6-méthyl-4-(trifluorométhyl)nicotinonitrile ;
Exemple 42 : (*S,E*)-2-((6-(3-((1-(4-(diméthylamino)but-2-énoyl)azétidin-3-yl)oxy)propyl)-8-fluoro-1-méthyl-2-oxo-1,2,3,4,5,6-hexahydrobenzo[b][1,4]diazocin-3-yl)amino)-6-méthyl-4-(trifluorométhyl)nicotinonitrile ;
Exemple 43 : (*S,E*)-2-((6-(3-((1-(4-(diméthylamino)but-2-énoyl)azétidin-3-yl)oxy)propyl)-8,10-difluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 44 : (*S,E*)-2-((7-chloro-6-(3-((1-(4-(diméthylamino)but-2-énoyl)azétidin-3-yl)oxy)propyl)-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 45 : (*S,E*)-2-((6-(2-((2-(4-(diméthylamino)but-2-énoyl)-2-azaspiro[3.3]heptan-6-yl)oxy)éthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 46 : (*S,E*)-2-((6-(2-(2-(4-(diméthylamino)but-2-énoyl)-5-méthyl-7-oxo-2,5,8-triazaspiro[3,5]nonan-8-yl)éthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 47 : 2-(((3*S*)-6-(2-(7-((E)-4-(diméthylamino)but-2-énoyl)-1-oxo-2,7-diazaspiro[4.4]nonan-2-yl) éthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 48 : 2-(((*S*)-6-(2-(((rel-3*R*,4*R*)-1-((*E*)-4-(diméthylamino)but-2-énoyl)-4-méthoxypyrrolidin-3-yl)méthoxy)éthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 49 : (*S*,*E*)-2-((6-(2-((1-(4-(diméthylamino)but-2-énoyl)pipéridin-4-yl)oxy)éthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 50 : (*S,E*)-2-((6-(2-((1-(4-(diméthylamino)but-2-énoyl)azétidin-3-yl)oxy)éthyl)-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 51 : (*S*)-2-((6-(2-((1-acryloylazétidin-3-yl)oxy)éthyl)-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 52 : (*S*)-2-((6-((2-acryloyl-2-azaspiro[3.3]heptan-6-yl)méthyl)-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 53 : (*S*)-2-((6-(3-((1-acryloylazétidin-3-yl)oxy)propyl)-1-méthyl-2-oxo-1,2,3,4,5,6-hexahydrobenzo[*b*][1,4]diazocin-3-yl)amino)-6-méthyl-4-(trifluorométhyl)nicotinonitrile ;
Exemple 54 : 2-(((*S*)-7-fluoro-1-méthyl-6-(3-((1-((*E*)-3-((*S*)-1-méthylpyrrolidin-2-yl)acryloyl)azétidin-3-yl)oxy)propyl)-2-oxo-1,2,3,4,5,6-hexahydrobenzo[b][1,4]diazocin-3-yl)amino)-6-méthyl-4-(trifluorométhyl)nicotinonitrile ;
Exemple 55 : 2-(((*S*)-7-fluoro-1-méthyl-6-(3-((1-((*E*)-3-((*R*)-1-méthylpyrrolidin-2-yl)acryloyl)azétidin-3-yl)oxy)propyl)-2-oxo-1,2,3,4,5,6-hexahydrobenzo[*b*][1,4]diazocin-3-yl)amino)-6-méthyl-4-(trifluorométhyl)nicotinonitrile ;
Exemple 56 : (*S,E*)-2-((7-fluoro-6- (3-((1-(4-((2-méthoxyéthyl)(méthyl)amino)but-2-énoyl)azétidin-3-yl)oxy)propyl)-1-méthyl-2-oxo-1,2,3,4,5,6-hexahydrobenzo[*b*][1,4]diazocin-3-yl)amino)-6-méthyl-4-(trifluorométhyl)nicotinonitrile ;

Exemple 57 : (*S*,*E*)-2-((7-fluoro-1-méthyl-6-(3-((1-(4-(méthyl(prop-2-yn-1-yl)amino)but-2-énoyl)azétidin-3-yl)oxy)propyl)-2-oxo-1,2,3,4,5,6-hexahydrobenzo[*b*][1,4]diazocin-3-yl)amino)-6-méthyl-4-(trifluorométhyl)nicotinonitrile ;

Exemple 58A : 2-(((*S*)-6-(((*S*/*R*)-4-(1-acryloylazétidin-3-yl)-1-méthyl-6-oxopipérazin-2-yl)méthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;

Exemple 58B : 2-(((*S*)-6-(((*R*/*S*)-4-(1-acryloylazétidin-3-yl)-1-méthyl-6-oxopipérazin-2-yl)méthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;

Exemple 59 : 2-(((*S*)-6-(((*S*)-4-(1-((*E*)-4-(diméthylamino)but-2-énoyl)azétidin-3-yl)morpholin-2-yl)méthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;

Exemple 60 : 2-(((*S*)-6-(((*R*)-4-(1-((*E*)-4-(diméthylamino)but-2-énoyl)azétidin-3-yl)morpholin-2-yl)méthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;

Exemple 61 : 2-(((*S*)-6-(2-((*R*)-1-acryloyltétrahydro-1'*H*-spiro[azétidin-3,6'-pyrazino[2,1-*c*][1,4]oxazin]-8'(7'*H*)-yl)éthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;

Exemple 62 : 2-(((*S*)-6-(2-((*S*)-1-acryloyltétrahydro-1'*H*-spiro[azétidin-3,6'-pyrazino[2,1-*c*][1,4]oxazin]-8'(7'*H*)-yl)éthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;

Exemple 63 : 2-(((*S*)-6-(2-((*R*)-2-acryloyl-6-méthyl-2,5,8-triazaspiro[3,5]nonan-8-yl)éthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;

Exemple 64 : 2-(((*S*)-6-(2-((*R*)-2-acryloyl-6-méthyl-2,5,8-triazaspiro[3,5]nonan-8-yl)éthyl)-7-chloro-1-méthyl-2-oxo-1,2,3,4,5,6-hexahydrobenzo[*b*][1,4]diazocin-3-yl)amino)-6-méthyl-4-(trifluorométhyl)nicotinonitrile ;

Exemple 65A : 2-(((*S*)-6-(2-((3*S*/*R*, 4*S*/*R*)-3-((1-acryloylazétidin-3-yl)oxy)-4-hydroxypyrrolidin-1-yl)éthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;

Exemple 65B : 2-(((*S*)-6-(2-((3*R*/*S*,4*R*/*S*)-3-((1-acryloylazétidin-3-yl)oxy)-4-hydroxypyrrolidin-1-yl)éthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;

Exemple 66 : (*S*)-2-((6-((1-(1-acryloylazétidin-3-yl)-1*H*-1,2,4-triazol-3-yl)méthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;

Exemple 67 : (*S*)-2-((6-((3-(1-acryloylazétidin-3-yl)-1,2,4-oxadiazol-5-yl)méthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;

Exemple 68 : (*S*)-2-((6-((5-(1-acryloylazétidin-3-yl)-4*H*-1,2,4-triazol-3-yl)méthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;

Exemple 69 : (*S*)-2-((6-(2-(2-acryloyl-2,5,8-triazaspiro[3,5]nonan-8-yl)éthyl)-1,7-diméthyl-2-oxo-1,2,3,4,5,6-hexahydrobenzo[*b*][1,4]diazocin-3-yl)amino)-6-méthyl-4-(trifluorométhyl)nicotinonitrile ;

Exemple 70 : (*S*)-2-((6-(2-(2-acryloyl-2,5,8-triazaspiro[3,5]nonan-8-yl)éthyl)-1-éthyl-7-fluoro-2-oxo-1,2,3,4,5,6-hexahydrobenzo[b][1,4]diazocin-3-yl)amino)-6-méthyl-4-(trifluorométhyl)nicotinonitrile ;

Exemple 71 : (*S*)-2-((6-(2-(5-acétyl-2-acryloyl-2,5,8-triazaspiro[3,5]nonan-8-yl)éthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;

Exemple 72A : 2-(((*S*)-6-(2-((*R*/*S*)-8-acryloyloctahydro-2*H*-pyrazino[1,2-*a*]pyrazin-2-yl)éthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ; et

Exemple 72B : 2-(((*S*)-6-(2-((*S*/*R*)-8-acryloyloctahydro-2*H*-pyrazino[1,2-*a*]pyrazin-2-yl)éthyl)-7-fluoro-1-méthyl-2-oxo-1,2,3,4,5,6-hexahydrobenzo[*b*][1,4]diazocin-3-yl)amino)-6-méthyl-4-(trifluorométhyl)nicotinonitrile ;

ou un sel ou un solvate pharmaceutiquement acceptable de celui-ci,

**13.** Composition pharmaceutique comprenant un composé de formule (I) selon l'une quelconque des revendications 1 à 12, éventuellement en combinaison avec un ou plusieurs agents thérapeutiques.

**14.** Composé selon l'une quelconque des revendications 1 à 12 destiné à être utilisé :

(a) en thérapie ; ou

(b) dans la prophylaxie ou le traitement du cancer.

**15.** Processus de préparation d'un composé de formule (I) selon la revendication 1 qui comprend :

(a) la réaction d'un composé de formule (II) :

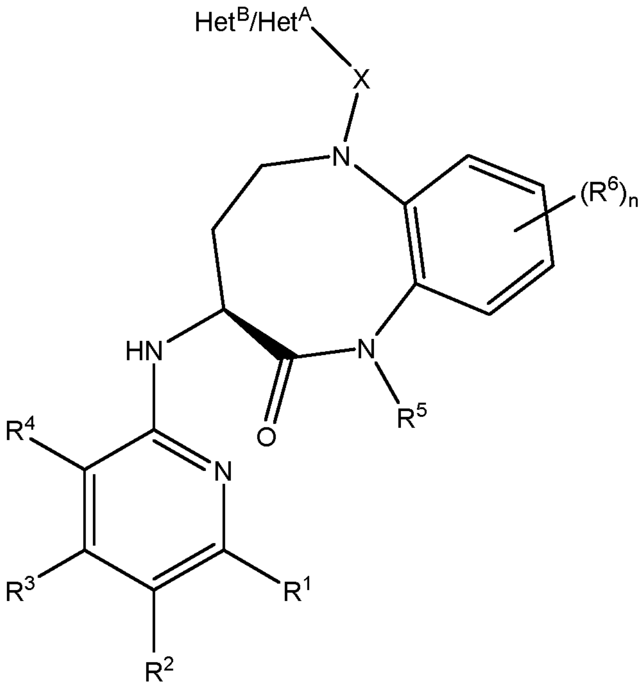

(II)

dans lequel $R^1$, $R^2$, $R^3$, $R^4$, $R^5$, $R^6$, n, X, $Het^A$ et $Het^B$ sont tels que définis selon la revendication 1, avec un composé de formule -CO-CH=CHR$^9$ ou -Y-Het$^C$-CO-CH=CHR$^9$, dans lequel Y, $Het^C$ et $R^9$ sont tels que définis selon la revendication 1 ;

(b) la réaction d'un composé de formule (III) :

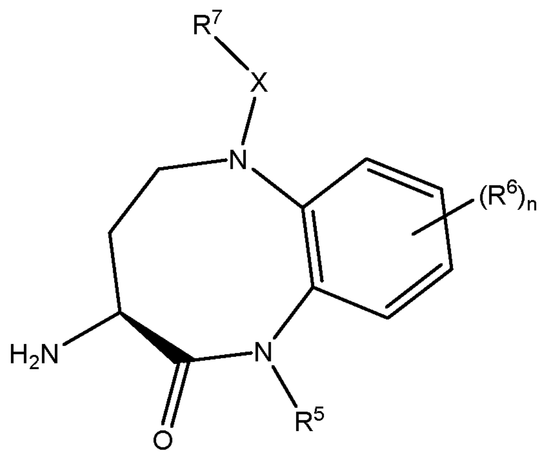

(III)

dans lequel $R^5$, $R^6$, n, X et $R^7$ sont tels que définis selon la revendication 1, avec un composé de formule (IV) :

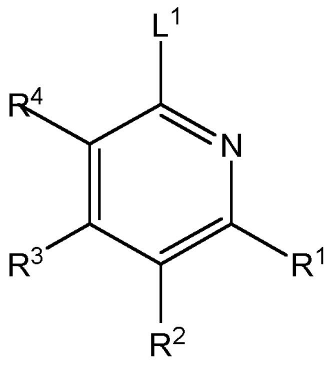

(IV)

dans lequel $R^1$, $R^2$, $R^3$ et $R^4$ sont tels que définis selon la revendication 1 et $L^1$ représente un groupe partant approprié, tel qu'un atome d'halogène (par exemple chlore) ;
(c) la déprotection d'un dérivé protégé d'un composé de formule (I) ;
(d) l'interconversion d'un composé de formule (I) ou d'un dérivé protégé de celui-ci en un autre composé de formule (I) ou en un dérivé protégé de celui-ci ; et
(e) la formation éventuelle d'un sel pharmaceutiquement acceptable d'un composé de formule (I).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2017062754 A

### Non-patent literature cited in the description


- **TRUONG et al.** *PNAS*, 2013, vol. 110 (19), 7720-7725
- **KENT et al.** *Nature Structural & Molecular Biology*, 2015, vol. 22 (3), 230-237
- **MATEOS-GOMEZ et al.** *Nature*, 2015, vol. 518 (7538), 254-257
- **WOOD** ; **DOUBLIÉ**. *DNA Repair*, 2016, vol. 44, 22-32
- **CECCALDI et al.** *Nature*, 2015, vol. 518 (7538), 258-262
- **HIGGINS et al.** *Oncotarget*, 2010, vol. 1, 175-184
- **LEMÉE et al.** *PNAS*, 2010, vol. 107 (30), 13390-13395
- **KAWAMURA et al.** *International Journal of Cancer*, 2004, vol. 109 (1), 9-16
- **BERGE** ; **BIGHLEY** ; **MONKHOUSE**. *J. Pharm. Sci.*, 1977, vol. 66, 1-19
- **EMAMI S et al.** *BioImpacts*, 2018, vol. 8 (4), 305-320
- **JERRY MARCH**. Advanced Organic Chemistry. Wiley Interscience
- **L. W. DEADY**. *Syn. Commun.*, 1977, vol. 7, 509-514
- **JERRY MARCH**. Advanced Organic Chemistry. John Wiley & Sons, 1992
- **JIRO TSUJI**. Palladium Reagents and Catalysts. Wiley
- Handbook of OrganoPalladium Chemistry for Organic Synthesis. Wiley, vol. 1
- **T. GREEN** ; **P. WUTS**. Protective Groups in Organic Synthesis. John Wiley and Sons, 2007
- Remington's Pharmaceutical Sciences. Mack Publishing Company
- **R. G. STRICKLY**. Solubilizing Excipients in oral and injectable formulations. *Pharmaceutical Research*, 2004, vol. 21 (2), 201-230
- Nanoparticle Technology for Drug Delivery. Informa Healthcare, 13 March 2006
- *J. Control. Release*, 2003, vol. 91 (1-2), 167-172
- **SINHA et al.** *Mol. Cancer Ther.*, 01 August 2006, vol. 5, 1909
- **CHIOU** ; **RIEGELMAN**. *J. Pharm. Sci.*, 1971, vol. 60, 1281-1300
- **CHOU TC** ; **TALALAY P**. Quantitative analysis of dose-effect relationships: the combined effects of multiple drugs or enzyme inhibitors. *Adv Enzyme Regulat*, 1984, vol. 22, 27-55
- *CHEMICAL ABSTRACTS*, 34582-32-6
- *CHEMICAL ABSTRACTS*, 741288-31-3
- *CHEMICAL ABSTRACTS*, 1187933-47-6
- *CHEMICAL ABSTRACTS*, 14316-06-4
- *CHEMICAL ABSTRACTS*, 1428775-89-6
- *CHEMICAL ABSTRACTS*, 31865-25-5
- *CHEMICAL ABSTRACTS*, 141699-55-0
- *CHEMICAL ABSTRACTS*, 142253-54-1
- *CHEMICAL ABSTRACTS*, 51673-84-8
- *CHEMICAL ABSTRACTS*, 2106-49-2
- *CHEMICAL ABSTRACTS*, 1251011-05-8
- *CHEMICAL ABSTRACTS*, 1366647-28-0
- *CHEMICAL ABSTRACTS*, 1041026-70-3
- *CHEMICAL ABSTRACTS*, 57260-71-6
- *CHEMICAL ABSTRACTS*, 236406-55-6
- *CHEMICAL ABSTRACTS*, 1086394-57-1
- *CHEMICAL ABSTRACTS*, 219725-67-4
- *CHEMICAL ABSTRACTS*, 178311-48-3
- *CHEMICAL ABSTRACTS*, 1008526-71-3
- *CHEMICAL ABSTRACTS*, 63-64-9
- *CHEMICAL ABSTRACTS*, 142253-55-2
- *CHEMICAL ABSTRACTS*, 72925-16-7
- *CHEMICAL ABSTRACTS*, 62234-40-6
- *CHEMICAL ABSTRACTS*, 919360-34-2
- *CHEMICAL ABSTRACTS*, 1440960-67-7
- *CHEMICAL ABSTRACTS*, 1159825-34-9
- **R.A COPELAND**. Evaluation of Enzyme Inhibitors in Drug Discovery. Wiley, 2013